# EUROPEAN PATENT SPECIFICATION

(11) **EP 2 942 356 B1**
(45) Date of publication and mention of the grant of the patent: **23.08.2017**
(21) Application number: 14167808.6
(22) Date of filing: 09.05.2014
(51) Int. Cl.: C07K 14/47, C07K 14/755, C12N 15/85

(54) **Mutation for type 1 diabetes mellitus and animal model**
Mutation für Diabetes mellitus Typ 1 und Tiermodell
Mutation pour le diabète sucré de type 1 et modèle animal

(30) Priority: 05.05.2014 EP 14167082
(43) Date of publication of application: 11.11.2015
(73) Proprietor: Medizinische Hochschule Hannover, 30625 Hannover (DE)
(72) Inventor: Lenzen, Sigurd, 30625 Hannover (DE); Jörns, Anne, 30655 Hannover (DE); Wedekind, Dirk, 31535 Neustadt a. Rübenberge (DE); Arndt, Tanja, 31303 Burgdorf (DE)
(74) Representative: Taruttis, Stefan Georg

(56) References cited:
- WO-A2-2005/054512
- WO-A2-2009/064901
- Arndt T. et al.: "Identifizierung der Mutation im Dock8 Gen in der LEW.1AR1-iddm Ratte, einem Tiermodell des humanen Typ 1 Diabetes mellitus", Diabetologie und Stoffwechsel, vol. 9, no. FV8 May 2014 (2014-05), XP002738020, Retrieved from the Internet: URL:https://www.thieme-connect.com/product s/ejournals/abstract/10.1055/s-0034-137486 5 [retrieved on 2015-03-31]
- ANNE JÖRNS ET AL: "Immune Cell Infiltration, Cytokine Expression, and beta-Cell Apoptosis During the Development of Type 1 Diabetes in the Spontaneously Diabetic LEW.1AR1/Ztm-iddm Rat", DIABETES, vol. 54, 1 January 2005 (2005-01-01), pages 2041-2052, XP055180550,
- YANG JING ET AL: "Activation of Rho GTPases by DOCK exchange factors is mediated by a nucleotide sensor.", SCIENCE, vol. 325, no. 5946, 11 September 2009 (2009-09-11), pages 1398-1402, XP002738019, ISSN: 1095-9203
- Y. HARADA ET AL: "DOCK8 is a Cdc42 activator critical for interstitial dendritic cell migration during immune responses", BLOOD, vol. 119, no. 19, 28 March 2012 (2012-03-28), pages 4451-4461, XP055180498, ISSN: 0006-4971, DOI: 10.1182/blood-2012-01-407098

## Description

The invention relates to a mutation that is causative for type 1 diabetes mellitus at least in the rat (rattus norvegicus) or mouse, the rat or mouse preferably having an MHC predisposing towards type 1 diabetes mellitus and/or a mutation in the von Willebrand factor Vwa2. Embodiments of the invention comprise a process for generating a rodent by genetic manipulation to introduce in the rodent at least heterozygously, preferably homozygously, the mutation causative for type 1 diabetes mellitus, in combination with an MHC predisposing for type 1 diabetes mellitus and/or with a mutation in the von Willebrand factor Vwa2. Further, the invention relates to a rodent that is genetically manipulated to contain at least heterozygously, preferably homozygously, the mutation causative for type 1 diabetes mellitus, in combination with an MHC predisposing for type 1 diabetes mellitus and/or with a mutation in the von Willebrand factor Vwa2, a process for analysing the activity of a test compound by administration of the test compound to a non-human mammal containing at least heterozygously, preferably homozygously, the mutation causative for type 1 diabetes mellitus, in combination with an MHC predisposing for type 1 diabetes mellitus and/or with a mutation in the von

Willebrand factor Vwa2, and relates to a process for analysis of the genetic predisposition of a human being or of a non-human mammal for the genetic predisposition towards type 1 diabetes mellitus by analysing for the presence of the mutation, preferably in combination with analysing for the presence of an MHC predisposing towards type 1 diabetes mellitus and/or for a mutation in the von Willebrand factor Vwa2.

### State of the art

Lenzen et al., Diabetologia 1189-1196 (2001), describe a spontaneous insulin-dependent diabetes mellitus rat strain designated LEW.1AR1/Ztm-*iddm*, having the MHC haplotype RT1.A^{a} B/D^{u} C^{u}. This rat develops diabetes at 58±2 d at 60% incidence without major sex preference, showing islets heavily infiltrated with B and T lymphocytes, macrophages and NK cells, destroying beta cells through apoptosis.

WO2005/054512 A2 describes the measurement of a type 1 diabetes mellitus specific gene product, as e.g. the expression of CDC42 among others was found elevated in type 1 diabetes mellitus patients.

Jörns et al., Diabetes 2041-2052 (2005), describe the immune cell infiltration comprising CD4+ T-cells, CD8+ T-cells and macrophages, and beta cell apoptosis during development of insulin-dependent diabetes mellitus in rat strain LEW.1AR1/Ztm-*iddm*.

Yang et al., Science 1398-1402 (2009) describe the activation of Rho GTPases by DOCK exchange factors and involvement of the DHR2 region of DOCK in Cdc42 binding.

### Objective of the invention

The objective of the invention is to provide an experimental rodent for use as an animal model for research purposes, especially for identification of compounds having activity against type 1 diabetes mellitus, as well as to provide a method for generating such an experimental rodent for research purposes. A further object is to provide an analytical process for analysing a human or a non-human mammal for its predisposition towards type 1 diabetes mellitus. A further objective is to provide a process for analysing the activity of a compound, especially against type 1 diabetes.

### Description of the invention

The invention achieves the objectives by the features of the claims and specifically provides a mutant rat Dock8 encoding gene and mutant rat Dock8 protein and a mutant mouse Dock8 protein which in rodents, especially in rat (e.g. rattus norvegicus) and in mouse (mus musculus), induces insulin-dependent diabetes mellitus (iddm), closely resembling human type 1 diabetes. For the mutation, an autosomal recessive mode of inheritance with an incomplete penetrance of the diabetes phenotype of about 60% was found in the LEW.1AR1-*iddm* rat. The mutation was found in animals of the rat strain LEW.1AR1-*iddm* that showed type 1 diabetes (T1D), whereas the wild-type strain LEW.1AR1 is diabetes resistant and did not show this mutation. The mutant strain is designated LEW.1AR1-*iddm*. Both the wild-type strain LEW.1AR1 and the mutant strain LEW.1AR1-*iddm* carry the MHC II RT1-B/D^{u} haplotype which is indispensable for T1D in the rat.

Following linkage analysis on N2 backcross populations of the LEW.1AR1-*iddm* with the Brown Norway rat (BN) and respectively with the Paris rat (PAR), respectively, analyses of diabetic BN and PAR rats of N2 using SNP (single nucleotide polymorphisms) indicated candidate gene regions. Sequencing of candidate gene regions revealed a mutation in exon 44 of the dedicator of cytokinesis 8 gene (*Dock8*), resulting in an amino acid exchange from glutamine to glutamate at amino acid position 1864 (Q1864E) of Dock8 protein (SEQ ID NO: 1). The mutation is from the wild-type C to G at position 181980 of the Dock8 encoding sequence *Dock8* (SEQ ID NO: 2), corresponding to position 228 622 763 on RNO1 (rattus norvegicus gene 1, RGSC genome assembly version 3.4). In SEQ ID NO: 2, which is based on RGSC genome assembly version 5.0, DHR2 is encoded by nucleotides No. 170118 to 192368, and exon 44 is at nucleotides No. 181971 to 182207.

The mutation resides in the β4 region of the DHR2 (DOCK homology region 2) of Dock8. The mutation is assumed to change the binding of the DHR2 region to Cdc42, resulting in the T1D phenotype.

The DHR2 region is at amino acids No. 1620-2070, preferably No. 1632-2070, exon 44 corresponds to amino acids No. 1861 - 1939, and the β4 region is at amino acids No. 1860 - 1867, preferably No. 1861 - 1867 of the rat Dock8 protein SEQ ID NO: 1 (mutated) and of SEQ ID NO: 10 (wild-type), respectively.

Accordingly, the invention in one embodiment relates to rodents, preferably mouse and rat, which contain a mutation in the DHR2 encoding portion of the Dock8 encoding gene (*Dock8*), especially in the β4 region encoding gene region of DHR2, the mutation changing, e.g. increasing or decreasing, the interactions with Cdc42. The mutation causes the Q1864E mutation in the Dock8 (SEQ ID NO: 1) encoding region in the rat and the corresponding Q1865E mutation in the Dock8 (SEQ ID NO: 12) encoding region in the mouse.

In rat, the mutation Q1864E of Dock8 protein causes the infiltration of islets by T-cells with a predominance of CD8+ T-cells over CD4+ T-cells, whereas in the wild-type rat strain without this mutation, well-preserved beta cells were observed.

Further, the invention relates to a process for generating rodents, preferably mouse and rat, by genetic manipulation to contain a mutation in the DHR2 encoding portion of the Dock8 encoding gene, in exon 44, in the β4 region encoding gene of DHR2, the mutation changing, e.g. increasing or decreasing, the interactions with Cdc42. The rodentis a rat or a mouse and the mutation causes the Q1864E mutation in Dock8 in the rat and respectively the Q1865E mutation in Dock8 in the mouse, respectively. In rodents other than rat or mouse, the mutation preferably exchanges the amino acid Q for amino acid E in the site corresponding to position No. 1864 in rat Dock8 or corresponding to position No. 1865 in mouse Dock8, respectively. The amino acid corresponding to position 1864 in rat and 1865 in mouse can be found by sequence alignment, as Dock8 encoding genes have been found to be conserved, and preferably, the position is in the amino acids encoded by exon 44 and/or in the β4 region.

In a further embodiment, the invention relates to rodents, preferably rat and mouse, having a gene encoding a mutation in the DHR2 encoding portion of the Dock8 encoding gene, in exon 44, in the β4 region encoding gene of DHR2, the mutation changing, e.g. increasing or decreasing, the interactions with Cdc42., The mutation causes the Q1864E mutation in Dock8 in the rat and the Q1865E mutation in Dock8 in the mouse, respectively, preferably homozygously. The rodents can be generated by genetic manipulation introducing the mutation into the Dock8 encoding gene, e.g. by the process for generating rodents by genetic manipulation or can be obtained by selection of mutant animals, e.g. of spontaneous or induced mutant rodents. These rodents carry an MHC haplotype that is permissive for T1D, the *H²G⁷* haplotype in the mouse or *RT1-B*/*D^{u}* haplotype in the rat, optionally in combination with a mutation in the von Willebrandt factor Vwa2 encoding gene as described herein.

As the rodents having such a mutation in the Dock8 encoding gene, preferably homozygously, are predisposed for T1D, the invention also relates to a process for the analysis of a compound with respect to its activity against T1D in the rodent, e.g. a screening method. Generally, in the method, a compound to be tested is administered to the rodent having such a mutation in combination with an MHC susceptible to T1D, the *H²G⁷* haplotype in the mouse or *RT1-B*/*D^{u}* haplotype in the rat, or in combination with a mutation in *Vwa2*, e.g. in exon 11 of the Vwa2 encoding gene, preferably causing the mutation R681 W in rat Vwa2 or the mutation from R to W in a position corresponding to amino acid position No. 681 in rat Vwa2 protein (SEQ ID NO: 3), corresponding to amino acid position No. 159 in the amino acid sequence section encoded by exon 11 of the *Vwa2* gene. The reaction of rodent to the compound administered is detected, e.g. by analysis of blood levels of glucose prior to and following administration of the compound.

The amino acid sequences of Dock8 are highly conserved between rat and mouse (98% amino acid identity), and between Dock8 in rat and human DOCK8 (92% amino acid identity).

Further, the invention relates to a nucleic acid sequence, optionally isolated, the nucleic acid sequence encoding non-human Dock8 or human DOCK8 encoding a mutation in the Dock8 and DOCK8, respectively, encoding gene, the mutation e.g. encoding a mutation of glutamine at an amino acid position corresponding to amino acid position 1864 (Q1864) in rat Dock 8 and human DOCK8, respectively, or at amino acid position 1865 (Q1865) in mouse Dock8, preferably to glutamate (E) resulting in the mutation Q1864E in rat Dock8 or human DOCK8 and Q1865E in mouse Dock8.

Further, the invention relates to an analytical process of determining in a sample obtained from a human or in a sample obtained from a non-human mammal mutations in the DHR2 encoding section, in exon 44, in the β4 encoding section of the Dock8 encoding gene, the mutation encoding a mutation of glutamine at an amino acid position corresponding to amino acid position 1864 (Q1864) in rat or to amino acid position 1865 (Q1865) in mouse Dock8 (Dock8 mutant Q1865E in SEQ ID NO: 12, Dock8 wild-type in SEQ ID NO: 11) or to amino acid position 1864 in human DOCK8 (wild-type in SEQ ID NO: 8), preferably to glutamate (E) resulting in the mutation Q1864E in rat and respectively human, and Q1865E in mouse. The analytical process can e.g. comprise sequencing the DNA section encoding the non-human Dock8 or the human DOCK8, including the section encoding the amino acids of DHR2, preferably of the β4 region encoding section, preferably exon 44.

It has further been found that the diabetes-prone rat strain LEW.1AR1*-iddm* carries an SNP (single nucleotide polymorphism) in exon 11 (amino acids No. 523..707 in SEQ ID NO: 3) of the von Willebrand factor Vwa2 encoding gene, at position 263216228 of the RGSC Genome Assembly, version 3.4 of RNO, resulting in a change of the amino acid sequence (SEQ ID NO: 3) of Vwa2 from the wild-type arginine (R) to tryptophan (W) at amino acid position 681 (R681 W).

Accordingly, in a preferred embodiment, in the processes and non-human mammals according to the invention, the Vwa2 encoding gene has a sequence causing a mutation R681 W in the Vwa2 encoding gene, corresponding to amino acid position 159 of the amino acids encoded by exon 11 of the Vwa2 encoding gene.

The invention is now described in greater detail with reference to the figures, which show in
- Fig. 1 a schematic drawing of rat Dock8 including the mutation in exon 44,
- Fig. 2 a graphic model of the DHR2 region of mouse Dock8 in the complex with Cdc42,
- Fig. 3 a sequence alignment of amino acid sequences of DOCK8 of human origin (Query) and of rat origin (Sbjct), both wild-type, with the centre line showing the consensus,
- Fig. 4 a schematic drawing of the structure of the gene encoding Vwa2 and the mutation in exon 11,
- Fig. 5 a sequence alignment of amino acid sequences of exon 11 of Vwa2 of rat origin, of mouse origin and of human origin, wild-type each,
- Fig. 6 micrographs of immune cells of pancreas sections from rat with HE staining, and in
- Fig. 7 schematically the cloning strategy in the process of generating a transgenic mouse according to the invention.

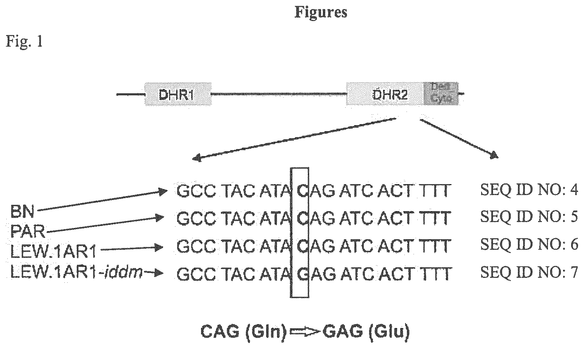

A section of the structure of the gene encoding rat Dock8 is schematically shown in Fig. 1. The gene contains 48 exons, and the mutation that causes a change in the binding of Dock8 to Cdc42 is located in exon 44. For comparison, a section from the nucleotide sequence of exon 44 of the Brown Norway rat (BN, SEQ ID NO: 4), the Paris rat (PAR, SEQ ID NO: 5), the wild-type strain LEW.1AR1 (SEQ ID NO: 6), and the mutant strain LEW.1AR1-*iddm* (SEQ ID NO: 7) are aligned. This sectional alignment shows that only the T1D phenotypic rat of the invention carries the mutation of C to G, resulting in the change from encoding glutamine (Gln) to glutamate (Glu) at amino acid position 1864 of Dock8 in human (DOCK8) and rat, and respectively at corresponding amino acid position 1865 in mouse Dock8.

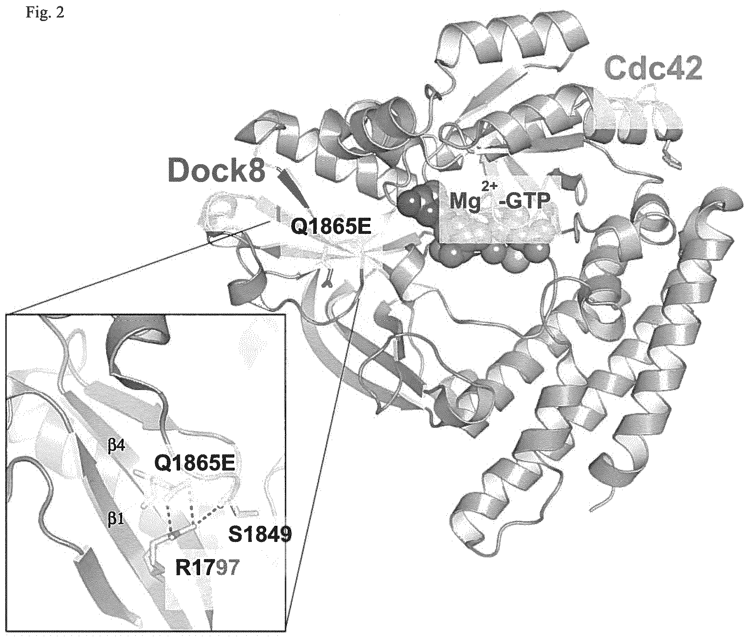

Fig. 2 shows a graphic model of the mouse Q1865E mutated DHR2 region of Dock8 in complex with Cdc42, with Mg²⁺ and GTP bound. The graphic model was prepared using the PyMOL Molecular Graphics System, version 1.5.0.4, Schrödinger LLC using the PDB-code 3VHL. Due to the high homology of the mouse Dock8 protein to the rat Dock8 protein and the mouse Dock8 carrying the mutation encoding Q1865E and the mouse carrying the mutation encoding Q1864E, the graphic model of the mouse Dock8 was used.

The glutamate residue at position 1865 (Q1865E) of mouse Dock8 is shown as a stick model within the β4 section of the DHR2 region. The enlarged inset shows that according to this model, the mutation at position 1865 to glutamate can result in formation of a salt bridge with the adjacent arginine in position 1797 (R1797), located in β1, and to interact with the serine at position 1849 (S1849), which is part of a flexible loop emerging from the α6 helix. The loop of the α6 helix is critically involved in the interaction with Cdc42, which interaction can be influenced by the stronger interaction of the glutamate residue at position 1865.

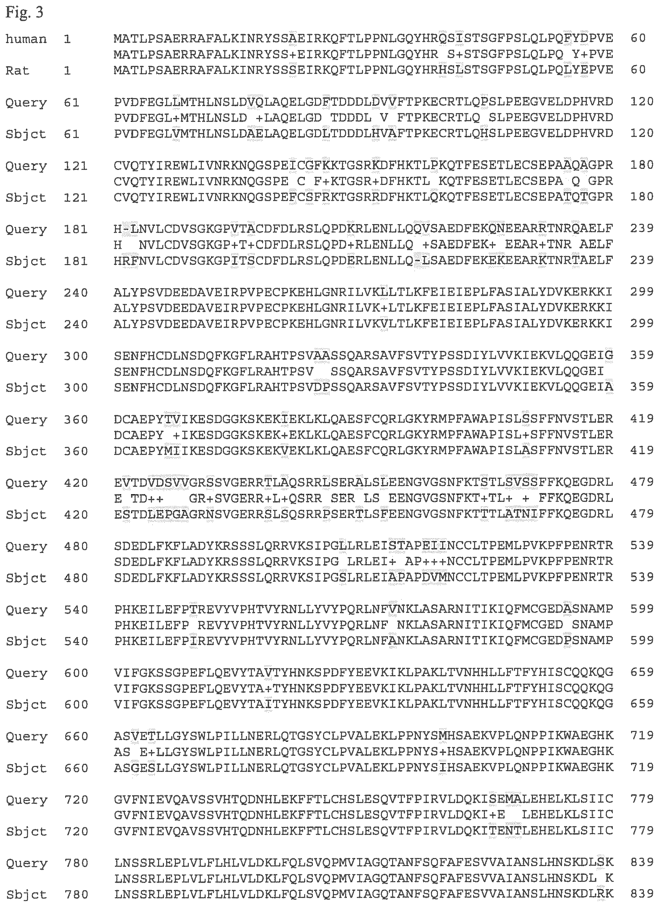

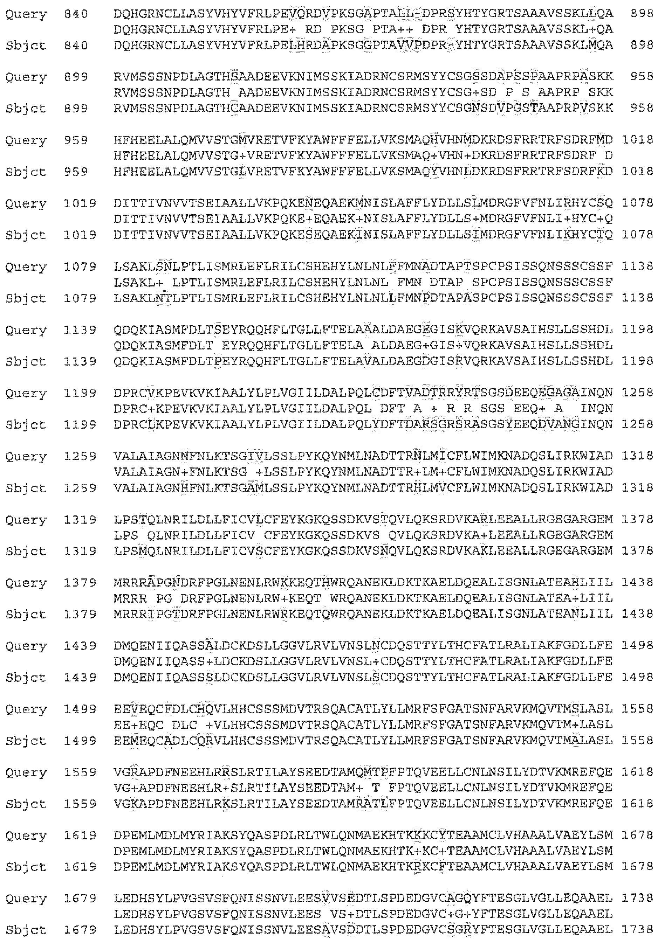

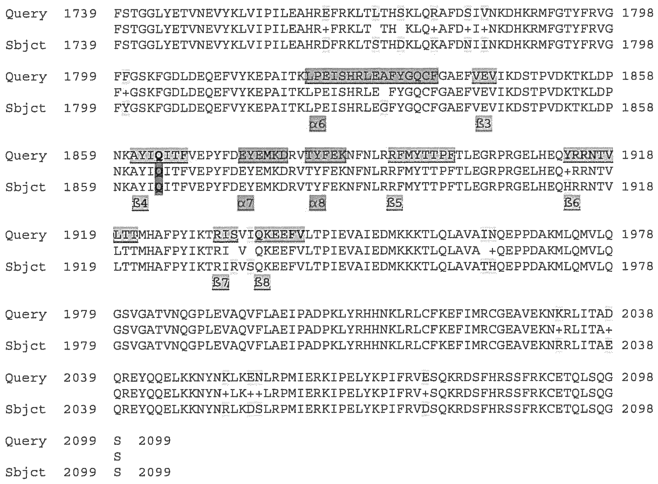

Fig. 3 shows a sequence alignment between the wild-type amino acid sequences of Dock8 from rat (lower row, SEQ ID NO: 10) and human origin (upper row, SEQ ID NO: 8) with the consensus sequence (centre row, SEQ ID NO: 9). Differences in amino acids are marked by shading individual amino acids. The wild-type Q in position 1864 is also marked. The high homology between Dock8 of rat origin and DOCK8 of human origin as well as of mouse origin shows that a non-human mammal carrying the mutation in the DHR2 region, exemplified by the Q1864E mutation in the rat, is a valuable laboratory model representing human T1D. Further, the high homology shows that an analytical process for determining mutations in the DHR2 region in human beings, which mutations affect the interaction of the DHR2 region with Cdc42, especially mutations in the β4 region, e.g. in exon 44, e.g. a Q1864E mutation, is suitable for identifying the genetic predisposition towards T1D, especially combination with MHC *RT1-B*/*D^{u}* in the rat, MHC *H²G⁷* haplotype in the mouse, and MHC HLA-DR3 or HLA-DR4 in a human respectively, and/or each in combination with a mutation in the von Willebrand factor Vwa2, e.g. in its exon 11, exemplified by the R681W mutation, e.g. in the rat SEQ ID NO: 3. In Fig. 3, α-helix and β-folding sheet regions are indicated below the amino acids, and amino acids forming the α-helix and β-folding sheet regions are underlined.

The amino acid sequence of mouse Dock8 wild-type is given as SEQ ID NO: 11, the mouse Q1865E mutant Dock8 as SEQ ID NO: 12. In SEQ ID NO: 11 and SEQ ID NO: 12, respectively, the DHR2 region is at amino acids No. 1633 - 2071, amino acids encoded or corresponding to exon 44 are at No. 1862 - 1940, and the β4 region is at amino acids No. 1862 - 1868.

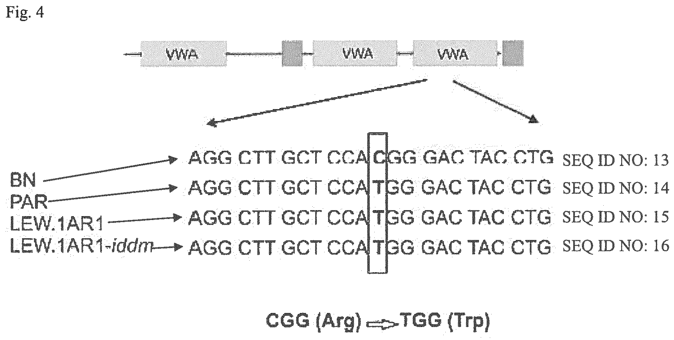

Fig. 4 shows the structure of the gene encoding Vwa2, including the T of the SNP in exon (ex) 11, resulting in a change of arginine (Arg, R) to tryptophan (Trp, W) at amino acid position 681 (R681W). The amino acid exchange is located in the third Vwa domain of Vwa2. The sectional sequence comparison shows that this mutation is not found in the Brown Norway (BN) but also in the Paris (PAR) and the LEW.1AR1 rats as well as in the mutant LEW.1AR1-*iddm* rat.

This mutation of Vwa2 is assumed to contribute to a diabetic T1D phenotype in combination with the mutation of Dock8.

Accordingly, a mutation in the Vwa2 encoding gene, especially in its third VWA domain, preferably resulting in a tryptophan in amino acid position 681, is preferred in the non-human mammals of the invention and in the processes of the invention for generating non-human mammals and for analysing a sample of mammalian origin, including human origin, in respect of predisposition towards T1D.

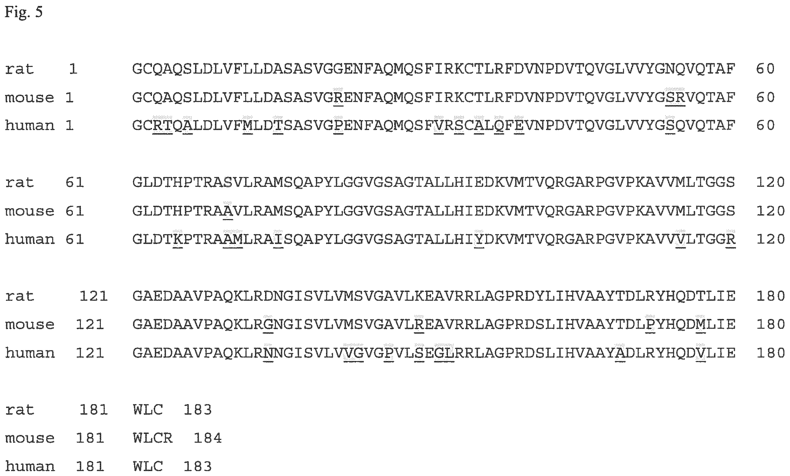

Fig. 5 shows an amino acid alignment of the wild-type Vwa2 amino acids encoded by exon 11 of Vwa2 of rat (SEQ ID NO: 17), of mouse (SEQ ID NO: 18), and of human origin (SEQ ID NO: 19), wild-type each. Amino acids encoded by exon 11 of Vwa2 that differ in mouse or human when compared to the amino acid sequence encoded by rat exon 11 of the *Vwa2* gene are indicated by underlining. The high degree of sequence identity and especially of the amino acid R in position 159 for each Vwa2 exon 11, corresponding to position 681 in mutant Vwa2 of rat in SEQ ID NO: 3 shows that a Vwa2 having a mutation in amino acid position 159 of exon 11 of Vwa2 also in mouse causes a genetic predisposition for T1D. Accordingly, the process for producing a non-human mammal preferably comprises introducing a mutation in amino acid position 159 in the amino acid sequence encoded by exon 11 of the Vwa2 encoding gene, especially a mutation R159W, and the non-human mammal preferably contains a mutation in amino acid position 159 in the amino acid sequence encoded by exon 11 of Vwa2, especially a mutation R159W in the amino acid sequence encoded by exon 11 of the Vwa2 encoding gene. In rat, the exon 11 of the Vwa2 encoding gene preferably encodes SEQ ID NO: 17 in exon 11, in mouse preferably SEQ ID NO: 18 in exon 11, each with the mutation R159W. The wild-type amino acid sequences of Vwa2 of rat is given as SEQ ID NO: 20 (exon 11 encoding amino acids No. 522 - 706), of mouse as SEQ ID NO: 21 (exon 11 encoding amino acids No. 522 - 706), and of human VWA2 as SEQ ID NO: 22 (exon 11 encoding amino acids No. 523 - 707). In the rat and mouse Vwa2, amino acid position 159 of exon 11 corresponds to amino acid position 681 in SEQ ID NO: 20 and SEQ ID NO: 21, respectively, and to amino acid position 682 in SEQ ID NO: 22 in human VWA2.

Accordingly, the analytical process for the genetic predisposition for T1D, both in a human sample and in a non-human mammalian sample, preferably comprises determining a mutation in amino acid position 159 encoded by exon 11 of Vwa2, especially determining a mutation R159W in the amino acid sequence encoded by exon 11 of Vwa2.

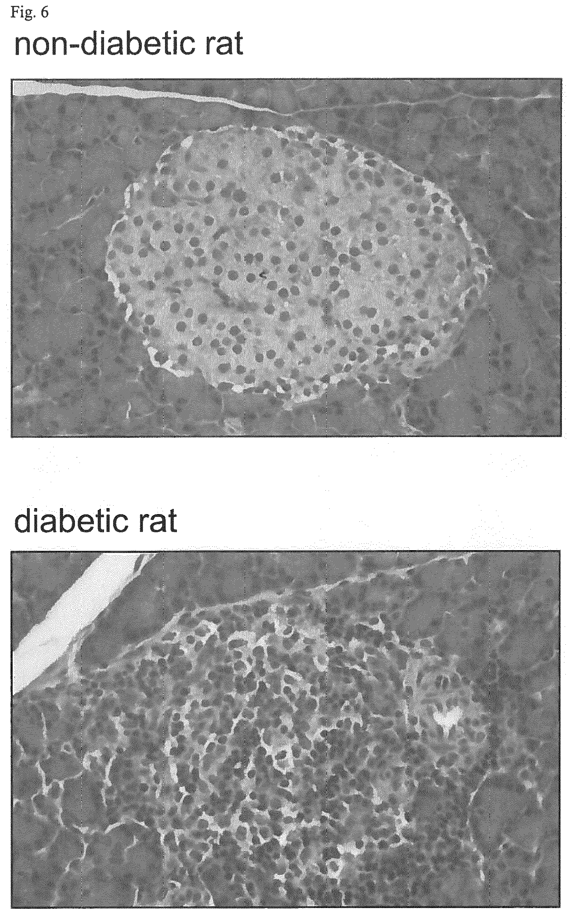

Fig. 6 shows micrographs of pancreatic islets with beta cells with haematoxylin (HE) staining from a non-diabetic LEW.1AR1 rat and of a diabetic rat, exemplified by LEW.1AR1-*iddm*. The micrographs show that the only known genetic difference between these rats, namely the Q1864E mutation of SEQ ID NO: 1, results in the disturbed morphology of the islets in the diabetic rat and in apoptosis of beta cells.

Accordingly, it is preferred, that the mutation of the DHR2 region of Dock8 causing a changed interaction with Cdc42 in the non-human mammal is homozygous. Further, it is preferred in the analytical process that in the non-human mammal and in the human, respectively, the haplotype is determined in respect of mutations in the Dock8 encoding gene.

The DNA sequence of the wild-type mouse Dock8 encoding gene is given at SEQ ID NO: 23, based on the mouse chromosome 19 (MMU19, GRCm38 genome assembly), positions 24,999,529 - 25,202,432, containing the DHR2 region at nucleotides No. 177307 - 200987, exon 44 at nucleotides No. 188908 - 189045, and the wild-type C at position No. 188818, which according to the invention is mutated to G to encode the amino acid exchange Q1865E in Dock8 protein.

Further analysis of pancreatic islets with beta cells from diabetic animals, exemplified by LEW.1AR1-*iddm* rats in comparison to those form non-diabetic LEW.1AR1-*iddm* rats by DAB immunohistochemistry with haematoxylin counterstaining with immunostaining for CD4+ (CD4+ T-cells), for CD8+ (CD8+ T-cells), for insulin, and for CD68+ (macrophages) shows that CD8+ T-cells and CD68+ macrophages with a smaller number of CD4+ T-cells infiltrate pancreatic islets as well as a severely reduced insulin production in the diabetic rats. The direct comparison between diabetic LEW.1AR1-*iddm* rats carrying the Q1864E mutation of Dock8 protein and non-diabetic LEW.1AR1 rats which otherwise have the same genetic background shows that a mutation within the DHR2 region of Dock8 protein to change the interaction with Cdc42 causes T1D, at least in the genetic background of MHC II RT1-B/D^{u} haplotype, preferably additionally with a mutation in the von Willebrandt factor Vwa2, especially in exon 11 of Vwa2, encoding e.g. R681W in the Vwa2 protein (SEQ ID NOs: 20, 21, 22), corresponding to R159W in the amino acid sequence section encoded by exon 11 (SEQ ID NOs: 17, 18, 19).

### Example: Transgenic rodent

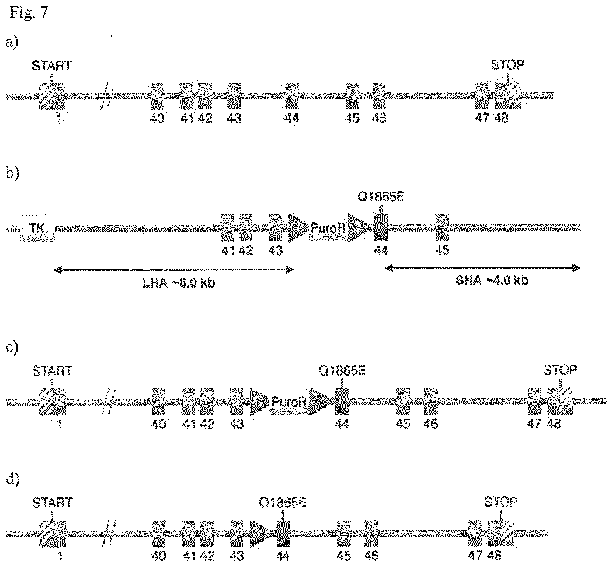

A transgenic mouse carrying the Q1865E mutant DOCK8 encoding gene can be generated using targeted mutagenesis in mouse embryonic stem (ES) cells. ES cells of the mouse strain C57Bl/6 N Tac were transfected using electroporation with a linearized nucleic acid vector having homologous sections at both the 5'- end and the 3'-end for recombination within the DOCK8 encoding gene, a resistance gene (Puromycin) flanked by frt sites for later removal of the resistance gene. A schematic representation of the mouse genomic locus is shown in Fig. 7 a), and of the vector in Fig. 7 b), wherein TK designates the selection marker thymidine kinase, LHA designates the 5' long homology arm, SHA designates the 3' short homology arm, and numbers indicate exons. The mutation Q1865E to be introduced in exon 44 is contained in the vector. Fig. 7 c) shows the integrated vector. The Puromycin resistance gene (PuroR) is flanked by FRT sites is located in intron 43 and allows for in vivo Flp-mediated removal of the selection marker following selection, as shown in Fig. 7 d).

Transfected cells were selected for the resistance carried by the vector and insertion of the vector at the target site was controlled by Southern blotting after restriction of total DNA or by PCR on total DNA spanning the insertion site. Homologous recombinant ES cells were grown on a mitotically inactivated feeder layer of mouse embryonic fibroblasts in ES cell culture medium containing leukemia inhibitory factor and fetal bovine serum for expansion and frozen in liquid nitrogen. Subsequently, the transgenic cells are injected into blastocysts and implanted into a foster mouse. Chimeric offspring are crossed, and the mice that are homozygous for the mutation encoding Dock8 having SEQ ID NO: 12 with the mutation Q1865E are selected.

## Claims

1. Process for generating a rodent having the MHC haplotype *H²G⁷* in the mouse or haplotype *RT1-B*/*D^{u}* in the rat predisposing towards type 1 diabetes mellitus, **characterized by** introducing into the rodent a mutation within the Dock homology region-2 (DHR2) region of the dedicator of cytokinesis 8 (Dock8) encoding gene, the mutation encoding an amino acid exchange in the Dock8 encoding gene corresponding to Q1864E of SEQ ID NO: 1 or Q1865E of SEQ ID NO: 12, altering the binding of the DHR2 region of Dock8 to Cdc42.

2. Process according to claim 1, **characterized in that** the rodent is a mouse having the MHC haplotype *H²G⁷.*

3. Process according to claim 1, **characterized in that** the rodent is a rat having the MHC haplotype *RT1-B*/*D^{u}.*

4. Process according to one of the preceding claims, **characterized in that** the rodent carries a mutation in the von Willebrand factor A2 (Vwa2) encoding gene encoding an amino acid exchange corresponding to amino acid position 159 of the amino acid sequence encoded by exon 11 of the Vwa2 (SEQ ID NO: 17 or SEQ ID NO: 18) encoding gene.

5. Nucleic acid molecule comprising the section of the gene encoding the DHR2 region of the Dock8 protein, **characterized by** comprising a mutation within the section encoding the DHR2 region, wherein the Dock8 protein is according to SEQ ID NO: 1 having the amino acid exchange Q1864E, or the Dock8 protein is according to SEQ ID NO: 12 having the amino acid exchange Q1865E, the mutation encoding an amino acid alteration altering binding of the DHR2 region of Dock8 to Cdc42.

6. Rodent having an MHC haplotype *H²G⁷* in the mouse or haplotype *RT1-B*/*D^{u}* in the rat predisposing for type 1 diabetes, **characterized by** having a mutation in the section encoding the DHR2 region of the Dock8 protein encoding gene, the mutation encoding an amino acid exchange in the Dock8 encoding gene corresponding to Q1864E of SEQ ID NO: 1 or Q1865E of SEQ ID NO: 12, the amino acid alteration altering binding of the DHR2 region of Dock8 to Cdc42.

7. Rodent according to claim 6, **characterized in that** the rodent is a mouse having the MHC haplotype H²G⁷.

8. Rodent according to claim 6, **characterized in that** the rodent is a rat having the MHC haplotype RT1-B/D^{u}

9. Rodent according to one of claims 6 to 8, **characterized in that** the rodent has a mutation encoding an amino acid in amino acid position 159 other than arginine (R) in exon 11 having SEQ ID NO: 17 or SEQ ID NO: 18 of the Willebrand factor A2 (Vwa2) protein.

10. Process for analysis of the activity of a compound by administration of the compound to a rodent, **characterized in that** the rodent is one according to one of claims 6 to 9 which is homozygous for the mutation Q1864E of SEQ ID NO: 1 or Q1865E of SEQ ID NO: 12.

11. Process for analysis of a sample obtained from a human or from a non-human mammal for genetic predisposition towards type 1 diabetes mellitus, **characterized by** determining the presence of mutations within the DHR2 region of the DOCK8 encoding gene, the mutation encoding an amino acid exchange of Q1864 in the Dock8 encoding gene of SEQ ID NO: 1 or an amino acid exchange of Q1865 in the Dock8 encoding gene of SEQ ID NO: 11 or in the amino acid No. Q1864 in the human gene encoding Dock8 of SEQ ID NO: 8, the amino acid alteration altering binding of the DHR2 region of DOCK8 to Cdc42.

12. Process according to claim 11, **characterized by** comprising determining the presence of a mutation encoding an amino acid at position 681 in the rat gene encoding the von Willebrand factor A2 (Vwa2) of SEQ ID NO: 20 or in the mouse gene encoding the von Willebrand factor A2 (Vwa2) of SEQ ID NO: 21 or in amino acid at position 682 of the human gene encoding the von Willebrand factor A2 (Vwa2) of SEQ ID NO: 22.

13. Process according to one of claims 11 to 12, **characterized by** in addition analysing for the presence of an MHC predisposing towards type 1 diabetes mellitus.

## Patentansprüche

1. Verfahren zur Erzeugung eines Nagetiers, das in der Maus den MHC-Haplotyp *H²G⁷* oder in der Ratte den Haplotyp *RT1-B*/*D^{U}* aufweist, der für den Typ 1 Diabetes mellitus veranlagt, **gekennzeichnet durch** das Einführen einer Mutation in das Nagetier innerhalb der Dock-Homologie-Region-2 (DHR2)-Region des den Dedicator-of-Cytokinesis-8 (Dock8) kodierenden Gens, wobei die Mutation einen Aminosäureaustausch in dem das Dock8 kodierenden Gen entsprechend Q1864E von SEQ ID NO: 1 oder Q1865E von SEQ ID NO: 12 kodiert, die die Bindung der DHR2-Region von Dock8 an Cdc42 verändert.

2. Verfahren nach Anspruch 1, **dadurch gekennzeichnet, dass** das Nagetier eine Maus ist, die den MHC-Haplotyp *H²G⁷* aufweist.

3. Verfahren nach Anspruch 1, **dadurch gekennzeichnet, dass** das Nagetier eine Ratte ist, die den MHC-Haplotyp *RT1-B*/*D^{U}* aufweist.

4. Verfahren nach einem der voranstehenden Ansprüche, **dadurch gekennzeichnet, dass** das Nagetier eine Mutation in dem den von-Willebrand-Faktor-A2 (Vwa2) kodierenden Gen trägt, die an dem Aminosäureaustausch entsprechend der Aminosäurestellung 159 der Aminosäuresequenz kodiert, die durch Exon 11 des Vwa2 (SEQ ID NO: 17 oder SEQ ID NO: 18) kodierenden Gens kodiert ist.

5. Nukleinsäuremolekül, das einen Abschnitt des Gens umfasst, das die DHR2-Region des Dock8-Proteins kodiert, **gekennzeichnet dadurch, dass** es eine Mutation innerhalb des die DRH2-Region kodieren Abschnitts enthält, wobei das Dock8-Protein gemäß SEQ ID NO: 1 ist mit dem Aminosäureaustausch Q1864E oder das Dock8-Protein gemäß SEQ ID NO: 12 ist mit dem Aminosäureaustausch Q1865E, wobei die Mutation eine Aminosäureveränderung kodiert, die das Binden der DRH2-Region von Dock8 an Cdc42 verändert.

6. Nagetier, das den MHC-Haplotyp *H²G⁷* in der Maus oder den Haplotyp *RT1-B*/*D^{U}* in der Ratte aufweist, der für Typ 1 Diabetes veranlagt, **dadurch gekennzeichnet, dass** es eine Mutation in dem Abschnitt, der die DRH2-Region des das Dock8-Protein kodierenden Gens kodiert, wobei die Mutation einen Aminosäureaustausch in dem das Dock8 kodierenden Gen kodiert, die Q1864E von SEQ ID NO: 1 oder Q1865E von SEQ ID NO: 12 entspricht, wobei die Aminosäureänderung die Bindung der DHR2-Region von Dock8 an Cdc42 verändert.

7. Nagetier nach Anspruch 6, **dadurch gekennzeichnet, dass** das Nagetier eine Maus ist, die den MHC-Haplotyp *H²G⁷* aufweist.

8. Nagetier nach Anspruch 6, **dadurch gekennzeichnet, dass** das Nagetier eine Ratte ist, die den MHC-Haplotyp *RT1-B*/*D^{U}* aufweist.

9. Nagetier nach einem der Ansprüche 6 bis 8, **dadurch gekennzeichnet, dass** das Nagetier eine Mutation aufweist, die eine Aminosäure außer Arginin (R) in der Aminosäurestellung 159 in Exon 11 mit SEQ ID NO: 17 oder SEQ ID NO: 18 des von-Willebrand-Faktor-A2 (Vwa2)-Proteins aufweist.

10. Verfahren zur Analyse der Aktivität einer Verbindung durch Verabreichen der Verbindung an ein Nagetier, **dadurch gekennzeichnet, dass** das Nagetier eines gemäß einem der Ansprüche 6 bis 9 ist, das homozygot für die Mutation Q1864E von SEQ ID NO: 1 oder Q1865E von SEQ ID NO: 12 ist.

11. Verfahren zur Analyse einer Probe, die von einem Menschen oder von einem nichtmenschlichen Säugetier erhalten wurde, für die genetische Veranlagung zu Typ 1 Diabetes mellitus, **gekennzeichnet durch** das Bestimmen des Vorliegens von Mutationen innerhalb der DHR2-Region des Dock8 kodierenden Gens, wobei die Mutation einen Aminosäureaustausch von Q1864 in dem Dock8 von SEQ ID NO: 1 kodierenden Gen oder einen Aminosäureaustausch von Q1865 in dem Dock8 von SEQ ID NO: 11 kodierenden Gen oder in der Aminosäure Nr. Q1864 in dem menschlichen Dock8 von SEQ ID NO: 8 kodierenden Gen kodiert, wobei die Aminosäureänderung das Binden der DHR2-Region von Dock8 an Cdc42 verändert.

12. Verfahren nach Anspruch 11, **dadurch gekennzeichnet, dass** es das Bestimmen des Vorliegens einer Mutation umfasst, die eine Aminosäure in Stellung 681 in dem Rattengen kodiert, das den von-Willebrand-Faktor-A2 (Vwa2) von SEQ ID NO: 12 kodiert, oder in dem Mausgen, das den von-Willebrand-Faktor-A2 (Vwa2) von SEQ ID NO: 21 kodiert, oder in der Aminosäure in Stellung 682 des menschlichen Gens, das den von-Willebrand-Faktor-A2 (Vwa2) von SEQ ID NO: 22 kodiert.

13. Verfahren nach einem der Ansprüche 11 bis 12, **dadurch gekennzeichnet**, das zusätzlich auf die Gegenwart eines MHCs analysiert wird, der für Typ 1 Diabetes mellitus veranlagt.

## Revendications

1. Procédé de génération d'un rongeur présentant l'haplotype du CMH *H²*/*G⁷* chez la souris ou l'haplotype *RT1-B*/*D^{U}* chez le rat prédisposant au diabète mellitus de type 1, **caractérisé par** l'introduction dans le rongeur d'une mutation dans la région-2 d'homologie Dock (DHR2), région du dédicataire du gène codant pour la cytokinèse 8 (Dock8),
la mutation étant codante pour un échange d'acides aminés dans le gène codant pour Dock8 correspondant à Q1864E de SEQ ID NO: 1 ou Q1865E de SEQ ID NO: 12, modifiant la liaison de la région DHR2 de Dock8 à Cdc42.

2. Procédé selon la revendication 1, **caractérisé en ce que** le rongeur est une souris présentant l'haplotype du CMH *H²G⁷.*

3. Procédé selon la revendication 1, **caractérisé en ce que** le rongeur est un rat présentant l'haplotype du CMH *RT1-B*/*D^{U}.*

4. Procédé selon l'une quelconque des revendications précédentes, **caractérisé en ce que** le rongeur porte une mutation dans le gène codant pour le facteur A2 de von Willebrand (Vwa2), mutation codante pour un échange d'acides aminés correspondant à la position des acides position d'acide 159 de la séquence d'acides aminés codée par l'exon 11 du gène codant Vwa2 (SEQ ID NO: 17 ou SEQ ID NO: 18).

5. Molécule d'acide nucléique comprenant la section du gène codant pour la région DHR2 de la protéine Dock8, **caractérisée par** une mutation dans la section codante pour la région DHR2, dans laquelle la protéine Dock8 est conforme à SEQ ID NO:1 présentant l'échange d'acides aminés Q1864E, ou la protéine Dock8 est conforme à SEQ ID NO: 12 présentant l'échange d'acides aminés Q1865E, la mutation codante pour une modification des acides aminés modifiant la liaison de la région DHR2 de Dock8 à Cdc42.

6. Rongeur présentant un haplotype du CMH *H²G⁷* chez la souris ou un haplotype *RT1-B*/*D^{U}* chez le rat prédisposant au diabète de type 1, **caractérisé par** une mutation dans la section codante pour la région DHR2 du gène codant pour la protéine Dock8, la mutation étant codante pour un échange d'acides aminés dans le gène codant pour Dock8 correspondant à Q1864E de SEQ ID NO: 1 ou Q1865E de SEQ ID NO: 12, la modification des acides aminés modifiant la liaison de la région DHR2 de Dock8 à Cdc42.

7. Rongeur selon la revendication 6, **caractérisé en ce que** le rongeur est une souris présentant l'haplotype du CMH H²G⁷.

8. Rongeur selon la revendication 6, **caractérisé en ce que** le rongeur est un rat présentant l'haplotype du CMH RT1-B/D^{U}.

9. Rongeur selon l'une des revendications 6 à 8, **caractérisé en ce que** le rongeur possède une mutation codante pour un acide aminé à la position d'acides aminés 159 autre que l'arginine (R) à l'exon 11 présentant la SEQ ID NO: 17 ou SEQ ID NO: 18 de la protéine du facteur A2 de von Willebrand (Vwa2).

10. Procédé d'analyse de l'activité d'un composé par administration du composé à un rongeur, **caractérisé en ce que** le rongeur est un rongeur selon l'une des revendications 6 à 9 qui est homozygote pour la mutation Q1864E de SEQ ID NO: 1 ou Q1865E de SEQ ID NO: 12.

11. Procédé d'analyse d'un échantillon obtenu à partir d'un humain ou d'un mammifère non humain pour rechercher une prédisposition génétique au diabète mellitus de type 1, **caractérisé par** la détermination de la présence de mutations dans la région DHR2 du gène codant DOCK8, la mutation codante pour un échange d'acide aminé Q1864 dans le gène codant pour Dock8 de SEQ ID NO: 1 ou un échange d'acide aminé de Q1865 dans le gène codant pour Dock8 de SEQ ID NO: 11 ou dans l'acide aminé N° Q1864 dans le gène humain codant pour Dock8 de SEQ ID NO: 8, la modification d'acide aminé modifiant la liaison de la région DHR2 de DOCK8 à Cdc42.

12. Procédé selon la revendication 11, **caractérisé en ce qu'**il comprend la détermination de la présence d'une mutation codante pour un acide aminé en position 681 dans le gène du rat codant pour le facteur A2 de von Willebrand (Vwa2) de SEQ ID NO: 20 ou dans le gène de souris codant pour le facteur A2 de von Willebrand (Vwa2) de SEQ ID NO: 21 ou dans un acide aminé en position 682 du gène humain codant pour le facteur A2 de von Willebrand (Vwa2) de SEQ ID NO: 22.

13. Procédé selon l'une des revendications 11 ou 12, **caractérisé en outre par** l'analyse de la présence du CMH prédisposant au diabète mellitus de type 1.


### SEQUENCE LISTING

<110> Medizinische Hochschule Hannover
<120> Mutation for type 1 diabetes mellitus and animal model
<130> M1061-N-EP
<150> EP14167082.8
   <151> 2014-05-05
<160> 23
<170> BiSSAP 1.2
<210> 1
   <211> 2099
   <212> PRT
   <213> Rattus norvegicus
<220>
   <223> mutant Dock8
<220>
   <221> SITE
   <222> 1864
   <223> Q1864E
<220>
   <221> CHAIN
   <222> 1860..1867
   <223> Ã\2374 region
<220>
   <221> CHAIN
   <222> 1861..1939
   <223> amino acids of exon 44
<220>
   <221> CHAIN
   <222> 1620..2070
   <223> DHR2 region
<220>
   <221> CHAIN
   <222> 1632..2070
   <223> DHR2 region
<220>
   <221> CHAIN
   <222> 1861..1867
   <223> Ã\2374 region
<400> 1
   Met Ala Thr Leu Pro Ser Ala Glu Arg Arg Ala Phe Ala Leu Lys Ile
   1               5                   10                  15
   Asn Arg Tyr Ser Ser Ser Glu Ile Arg Lys Gln Phe Thr Leu Pro Pro
               20                  25                  30
   Asn Leu Gly Gln Tyr His Arg His Ser Leu Ser Thr Ser Gly Phe Pro
           35                  40                  45
   Ser Leu Gln Leu Pro Gln Leu Tyr Glu Pro Val Glu Pro Val Asp Phe
       50                  55                  60
   Glu Gly Leu Val Met Thr His Leu Asn Ser Leu Asp Ala Glu Leu Ala
   65 70 75 80
   Gln Glu Leu Gly Asp Leu Thr Asp Asp Asp Leu His Val Ala Phe Thr
   85 90 95
   Pro Lys Glu Cys Arg Thr Leu Gln His Ser Leu Pro Glu Glu Gly Val
   100 105 110
   Glu Leu Asp Pro His Val Arg Asp Cys Val Gln Thr Tyr Ile Arg Glu
   115 120 125
   Trp Leu Ile Val Asn Arg Lys Asn Gln Gly Ser Pro Glu Phe Cys Ser
   130 135 140
   Phe Arg Lys Thr Gly Ser Arg Arg Asp Phe His Lys Thr Leu Gln Lys
   145 150 155 160
   Gln Thr Phe Glu Ser Glu Thr Leu Glu Cys Ser Glu Pro Ala Thr Gln
   165 170 175
   Thr Gly Pro Arg His Arg Phe Asn Val Leu Cys Asp Val Ser Gly Lys
   180 185 190
   Gly Pro Ile Thr Ser Cys Asp Phe Asp Leu Arg Ser Leu Gln Pro Asp
   195 200 205
   Glu Arg Leu Glu Asn Leu Leu Gln Leu Ser Ala Glu Asp Phe Glu Lys
   210 215 220
   Glu Lys Glu Glu Ala Arg Lys Thr Asn Arg Thr Ala Glu Leu Phe Ala
   225 230 235 240
   Leu Tyr Pro Ser Val Asp Glu Glu Asp Ala Val Glu Ile Arg Pro Val
   245 250 255
   Pro Glu Cys Pro Lys Glu His Leu Gly Asn Arg Ile Leu Val Lys Val
   260 265 270
   Leu Thr Leu Lys Phe Glu Ile Glu Ile Glu Pro Leu Phe Ala Ser Ile
   275 280 285
   Ala Leu Tyr Asp Val Lys Glu Arg Lys Lys Ile Ser Glu Asn Phe His
   290 295 300
   Cys Asp Leu Asn Ser Asp Gln Phe Lys Gly Phe Leu Arg Ala His Thr
   305 310 315 320
   Pro Ser Val Asp Pro Ser Ser Gln Ala Arg Ser Ala Val Phe Ser Val
   325 330 335
   Thr Tyr Pro Ser Ser Asp Ile Tyr Leu Val Val Lys Ile Glu Lys Val
   340 345 350
   Leu Gln Gln Gly Glu Ile Ala Asp Cys Ala Glu Pro Tyr Met Ile Ile
   355 360 365
   Lys Glu Ser Asp Gly Gly Lys Ser Lys Glu Lys Val Glu Lys Leu Lys
   370 375 380
   Leu Gln Ala Glu Ser Phe Cys Gln Arg Leu Gly Lys Tyr Arg Met Pro
   385 390 395 400
   Phe Ala Trp Ala Pro Ile Ser Leu Ala Ser Phe Phe Asn Val Ser Thr
   405 410 415
   Leu Glu Arg Glu Ser Thr Asp Leu Glu Pro Gly Ala Gly Arg Asn Ser
   420 425 430
   Val Gly Glu Arg Arg Ser Leu Ser Gln Ser Arg Arg Pro Ser Glu Arg
   435 440 445
   Thr Leu Ser Phe Glu Glu Asn Gly Val Gly Ser Asn Phe Lys Thr Thr
   450 455 460
   Thr Leu Ala Thr Asn Ile Phe Phe Lys Gln Glu Gly Asp Arg Leu Ser
   465 470 475 480
   Asp Glu Asp Leu Phe Lys Phe Leu Ala Asp Tyr Lys Arg Ser Ser Ser
   485 490 495
   Leu Gln Arg Arg Val Lys Ser Ile Pro Gly Ser Leu Arg Leu Glu Ile
   500 505 510
   Ala Pro Ala Pro Asp Val Met Asn Cys Cys Leu Thr Pro Glu Met Leu
   515 520 525
   Pro Val Lys Pro Phe Pro Glu Asn Arg Thr Arg Pro His Lys Glu Ile
   530 535 540
   Leu Glu Phe Pro Ile Arg Glu Val Tyr Val Pro His Thr Val Tyr Arg
   545 550 555 560
   Asn Leu Leu Tyr Val Tyr Pro Gln Arg Leu Asn Phe Ala Asn Lys Leu
   565 570 575
   Ala Ser Ala Arg Asn Ile Thr Ile Lys Ile Gln Phe Met Cys Gly Glu
               580                 585                 590
   Asp Pro Ser Asn Ala Met Pro Val Ile Phe Gly Lys Ser Ser Gly Pro
           595                 600                 605
   Glu Phe Leu Gln Glu Val Tyr Thr Ala Ile Thr Tyr His Asn Lys Ser
       610                 615                 620
   Pro Asp Phe Tyr Glu Glu Val Lys Ile Lys Leu Pro Ala Lys Leu Thr
   625                 630                 635                 640
   Val Asn His His Leu Leu Phe Thr Phe Tyr His Ile Ser Cys Gln Gln
                   645                 650                 655
   Lys Gln Gly Ala Ser Gly Glu Ser Leu Leu Gly Tyr Ser Trp Leu Pro
               660                 665                 670
   Ile Leu Leu Asn Glu Arg Leu Gln Thr Gly Ser Tyr Cys Leu Pro Val
           675                 680                 685
   Ala Leu Glu Lys Leu Pro Pro Asn Tyr Ser Ile His Ser Ala Glu Lys
       690                 695                 700
   Val Pro Leu Gln Asn Pro Pro Ile Lys Trp Ala Glu Gly His Lys Gly
   705                 710                 715                 720
   Val Phe Asn Ile Glu Val Gln Ala Val Ser Ser Val His Thr Gln Asp
                   725                 730                 735
   Asn His Leu Glu Lys Phe Phe Thr Leu Cys His Ser Leu Glu Ser Gln
               740                 745                 750
   Val Thr Phe Pro Ile Arg Val Leu Asp Gln Lys Ile Thr Glu Asn Thr
           755                 760                 765
   Leu Glu His Glu Leu Lys Leu Ser Ile Ile Cys Leu Asn Ser Ser Arg
       770                 775                 780
   Leu Glu Pro Leu Val Leu Phe Leu His Leu Val Leu Asp Lys Leu Phe
   785                 790                 795                 800
   Gln Leu Ser Val Gln Pro Met Val Ile Ala Gly Gln Thr Ala Asn Phe
                   805                 810                 815
   Ser Gln Phe Ala Phe Glu Ser Val Val Ala Ile Ala Asn Ser Leu His
               820                 825                 830
   Asn Ser Lys Asp Leu Arg Lys Asp Gln His Gly Arg Asn Cys Leu Leu
           835                 840                 845
   Ala Ser Tyr Val His Tyr Val Phe Arg Leu Pro Glu Leu His Arg Asp
       850                 855                 860
   Ala Pro Lys Ser Gly Gly Pro Thr Ala Val Val Pro Asp Pro Arg Tyr
   865                 870                 875                 880
   His Thr Tyr Gly Arg Thr Ser Ala Ala Ala Val Ser Ser Lys Leu Met
                   885                 890                 895
   Gln Ala Arg Val Met Ser Ser Ser Asn Pro Asp Leu Ala Gly Thr His
               900                 905                 910
   Cys Ala Ala Asp Glu Glu Val Lys Asn Ile Met Ser Ser Lys Ile Ala
           915                 920                 925
   Asp Arg Asn Cys Ser Arg Met Ser Tyr Tyr Cys Ser Gly Asn Ser Asp
       930                 935                 940
   Val Pro Gly Ser Thr Ala Ala Pro Arg Pro Val Ser Lys Lys His Phe
   945                 950                 955                 960
   His Glu Glu Leu Ala Leu Gln Met Val Val Ser Thr Gly Leu Val Arg
                   965                 970                 975
   Glu Thr Val Phe Lys Tyr Ala Trp Phe Phe Phe Glu Leu Leu Val Lys
               980                 985                 990
   Ser Met Ala Gln Tyr Val His Asn Leu Asp Lys Arg Asp Ser Phe Arg
           995                 1000                1005
   Arg Thr Arg Phe Ser Asp Arg Phe Lys Asp Asp Ile Thr Thr Ile Val
       1010                1015                1020
   Asn Val Val Thr Ser Glu Ile Ala Ala Leu Leu Val Lys Pro Gln Lys
   1025                1030                1035                1040
   Glu Ser Glu Gln Ala Glu Lys Ile Asn Ile Ser Leu Ala Phe Phe Leu
                   1045                1050                1055
   Tyr Asp Leu Leu Ser Ile Met Asp Arg Gly Phe Val Phe Asn Leu Ile
               1060                1065                1070
   Lys His Tyr Cys Thr Gln Leu Ser Ala Lys Leu Asn Thr Leu Pro Thr
   1075 1080 1085
   Leu Ile Ser Met Arg Leu Glu Phe Leu Arg Ile Leu Cys Ser His Glu
   1090 1095 1100
   His Tyr Leu Asn Leu Asn Leu Leu Phe Met Asn Pro Asp Thr Ala Pro
   1105 1110 1115 1120
   Ala Ser Pro Cys Pro Ser Ile Ser Ser Gln Asn Ser Ser Ser Cys Ser
   1125 1130 1135
   Ser Phe Gln Asp Gln Lys Ile Ala Ser Met Phe Asp Leu Thr Pro Glu
   1140 1145 1150
   Tyr Arg Gln Gln His Phe Leu Thr Gly Leu Leu Phe Thr Glu Leu Ala
   1155 1160 1165
   Val Ala Leu Asp Ala Glu Gly Asp Gly Ile Ser Arg Val Gln Arg Lys
   1170 1175 1180
   Ala Val Ser Ala Ile His Ser Leu Leu Ser Ser His Asp Leu Asp Pro
   1185 1190 1195 1200
   Arg Cys Leu Lys Pro Glu Val Lys Val Lys Ile Ala Ala Leu Tyr Leu
   1205 1210 1215
   Pro Leu Val Gly Ile Ile Leu Asp Ala Leu Pro Gln Leu Tyr Asp Phe
   1220 1225 1230
   Thr Asp Ala Arg Ser Gly Arg Ser Arg Ala Ser Gly Ser Tyr Glu Glu
   1235 1240 1245
   Gln Asp Val Ala Asn Gly Ile Asn Gln Asn Val Ala Leu Ala Ile Ala
   1250 1255 1260
   Gly Asn His Phe Asn Leu Lys Thr Ser Gly Ala Met Leu Ser Ser Leu
   1265 1270 1275 1280
   Pro Tyr Lys Gln Tyr Asn Met Leu Asn Ala Asp Thr Thr Arg His Leu
   1285 1290 1295
   Met Val Cys Phe Leu Trp Ile Met Lys Asn Ala Asp Gln Ser Leu Ile
   1300 1305 1310
   Arg Lys Trp Ile Ala Asp Leu Pro Ser Met Gln Leu Asn Arg Ile Leu
   1315 1320 1325
   Asp Leu Leu Phe Ile Cys Val Ser Cys Phe Glu Tyr Lys Gly Lys Gln
   1330 1335 1340
   Ser Ser Asp Lys Val Ser Asn Gln Val Leu Gln Lys Ser Arg Asp Val
   1345 1350 1355 1360
   Lys Ala Lys Leu Glu Glu Ala Leu Leu Arg Gly Glu Gly Ala Arg Gly
   1365 1370 1375
   Glu Met Met Arg Arg Arg Ile Pro Gly Thr Asp Arg Phe Pro Gly Leu
   1380 1385 1390
   Asn Glu Asn Leu Arg Trp Arg Lys Glu Gln Thr Gln Trp Arg Gln Ala
   1395 1400 1405
   Asn Glu Lys Leu Asp Lys Thr Lys Ala Glu Leu Asp Gln Glu Ala Leu
   1410 1415 1420
   Ile Ser Gly Asn Leu Ala Thr Glu Ala Asn Leu Ile Ile Leu Asp Met
   1425 1430 1435 1440
   Gln Glu Asn Ile Ile Gln Ala Ser Ser Ser Leu Asp Cys Lys Asp Ser
   1445 1450 1455
   Leu Leu Gly Gly Val Leu Arg Val Leu Val Asn Ser Leu Ser Cys Asp
   1460 1465 1470
   Gln Ser Thr Thr Tyr Leu Thr His Cys Phe Ala Thr Leu Arg Ala Leu
   1475 1480 1485
   Ile Ala Lys Phe Gly Asp Leu Leu Phe Glu Glu Glu Met Glu Gln Cys
   1490 1495 1500
   Ala Asp Leu Cys Gln Arg Val Leu His His Cys Ser Ser Ser Met Asp
   1505 1510 1515 1520
   Val Thr Arg Ser Gln Ala Cys Ala Thr Leu Tyr Leu Leu Met Arg Phe
   1525 1530 1535
   Ser Phe Gly Ala Thr Ser Asn Phe Ala Arg Val Lys Met Gln Val Thr
   1540 1545 1550
   Met Ala Leu Ala Ser Leu Val Gly Lys Ala Pro Asp Phe Asn Glu Glu
   1555 1560 1565
   His Leu Arg Lys Ser Leu Arg Thr Ile Leu Ala Tyr Ser Glu Glu Asp
   1570 1575 1580
   Thr Ala Met Arg Ala Thr Leu Phe Pro Thr Gln Val Glu Glu Leu Leu
   1585 1590 1595 1600
   Cys Asn Leu Asn Ser Ile Leu Tyr Asp Thr Val Lys Met Arg Glu Phe
   1605 1610 1615
   Gln Glu Asp Pro Glu Met Leu Met Asp Leu Met Tyr Arg Ile Ala Lys
   1620 1625 1630
   Ser Tyr Gln Ala Ser Pro Asp Leu Arg Leu Thr Trp Leu Gln Asn Met
   1635 1640 1645
   Ala Glu Lys His Thr Lys Arg Lys Cys Phe Thr Glu Ala Ala Met Cys
   1650 1655 1660
   Leu Val His Ala Ala Ala Leu Val Ala Glu Tyr Leu Ser Met Leu Glu
   1665 1670 1675 1680
   Asp His Ser Tyr Leu Pro Val Gly Ser Val Ser Phe Gln Asn Ile Ser
   1685 1690 1695
   Ser Asn Val Leu Glu Glu Ser Ala Val Ser Asp Asp Thr Leu Ser Pro
   1700 1705 1710
   Asp Glu Asp Gly Val Cys Ser Gly Arg Tyr Phe Thr Glu Ser Gly Leu
   1715 1720 1725
   Val Gly Leu Leu Glu Gln Ala Ala Glu Leu Phe Ser Thr Gly Gly Leu
   1730 1735 1740
   Tyr Glu Thr Val Asn Glu Val Tyr Lys Leu Val Ile Pro Ile Leu Glu
   1745 1750 1755 1760
   Ala His Arg Asp Phe Arg Lys Leu Thr Ser Thr His Asp Lys Leu Gln
   1765 1770 1775
   Lys Ala Phe Asp Asn Ile Ile Asn Lys Asp His Lys Arg Met Phe Gly
   1780 1785 1790
   Thr Tyr Phe Arg Val Gly Phe Tyr Gly Ser Lys Phe Gly Asp Leu Asp
   1795 1800 1805
   Glu Gln Glu Phe Val Tyr Lys Glu Pro Ala Ile Thr Lys Leu Pro Glu
   1810 1815 1820
   Ile Ser His Arg Leu Glu Gly Phe Tyr Gly Gln Cys Phe Gly Ala Glu
   1825 1830 1835 1840
   Phe Val Glu Val Ile Lys Asp Ser Thr Pro Val Asp Lys Thr Lys Leu
   1845 1850 1855
   Asp Pro Asn Lys Ala Tyr Ile Glu Ile Thr Phe Val Glu Pro Tyr Phe
   1860 1865 1870
   Asp Glu Tyr Glu Met Lys Asp Arg Val Thr Tyr Phe Glu Lys Asn Phe
   1875 1880 1885
   Asn Leu Arg Arg Phe Met Tyr Thr Thr Pro Phe Thr Leu Glu Gly Arg
   1890 1895 1900
   Pro Arg Gly Glu Leu His Glu Gln His Arg Arg Asn Thr Val Leu Thr
   1905 1910 1915 1920
   Thr Met His Ala Phe Pro Tyr Ile Lys Thr Arg Ile Arg Val Ser Gln
   1925 1930 1935
   Lys Glu Glu Phe Val Leu Thr Pro Ile Glu Val Ala Ile Glu Asp Met
   1940 1945 1950
   Lys Lys Lys Thr Leu Gln Leu Ala Val Ala Thr His Gln Glu Pro Pro
   1955 1960 1965
   Asp Ala Lys Met Leu Gln Met Val Leu Gln Gly Ser Val Gly Ala Thr
   1970 1975 1980
   Val Asn Gln Gly Pro Leu Glu Val Ala Gln Val Phe Leu Ala Glu Ile
   1985 1990 1995 2000
   Pro Ala Asp Pro Lys Leu Tyr Arg His His Asn Lys Leu Arg Leu Cys
   2005 2010 2015
   Phe Lys Glu Phe Ile Met Arg Cys Gly Glu Ala Val Glu Lys Asn Arg
   2020 2025 2030
   Arg Leu Ile Thr Ala Glu Gln Arg Glu Tyr Gln Gln Glu Leu Lys Lys
   2035 2040 2045
   Asn Tyr Asn Arg Leu Lys Asp Ser Leu Arg Pro Met Ile Glu Arg Lys
   2050 2055 2060
   Ile Pro Glu Leu Tyr Lys Pro Ile Phe Arg Val Asp Ser Gln Lys Arg
   2065 2070 2075 2080
   Asp Ser Phe His Arg Ser Ser Phe Arg Lys Cys Glu Thr Gln Leu Ser
                       2085                2090                2095
   Gln Gly Ser
<210> 2
   <211> 194534
   <212> DNA
   <213> Rattus norvegicus
<220>
   <221> source
   <222> 1..194534
   <223> /mol_type="unassigned DNA" /note="mutant dock8 gene" /organism="Rattus norvegicus"
<220>
   <221> variation
   <222> 181980
   <223> /note="mutation of wild-type C to G"
<220>
   <221> exon
   <222> 181971..182207
   <223> /note="exon 44"
<220>
   <221> unsure
   <222> 91791..91799
   <223> /note="any nucleotide"
<220>
   <221> unsure
   <222> 91822..91898
   <223> /note="any nucleotide"
<220>
   <221> unsure
   <222> 91916..91921
   <223> /note="any nucleotide"
<220>
   <221> unsure
   <222> 137330..137336
   <223> /note="any nucleotide"
<220>
   <221> unsure
   <222> 137340..137373
   <223> /note="any nucleotide"
<220>
   <221> unsure
   <222> 137375
   <223> /note="any nucleotide"
<220>
   <221> unsure
   <222> 137377..137378
   <223> /note="any nucleotide"
<220>
   <221> unsure
   <222> 137380..138487
   <223> /note="any nucleotide"
<220>
   <221> unsure
   <222> 138489..138501
   <223> /note="any nucleotide"
<220>
   <221> unsure
   <222> 138503..138505
   <223> /note="any nucleotide"
<220>
   <221> unsure
   <222> 138509
   <223> /note="any nucleotide"
<220>
   <221> unsure
   <222> 138511..138526
   <223> /note="any nucleotide"
<220>
   <221> unsure
   <222> 138528..138559
   <223> /note="any nucleotide"
<220>
   <221> gene
   <222> 170118..192368
   <223> /note="encoding DHR2"
<400> 2
<210> 3
   <211> 715
   <212> PRT
   <213> Rattus norvegicus
<220>
   <223> Vwa2
<220>
   <221> CHAIN
   <222> 523..707
   <223> amino acids of exon 11
<220>
   <221> SITE
   <222> 681
   <223> R681W
<400> 3
   Met Pro Pro Tyr Leu Phe Leu Pro Ala Ile Tyr Thr Leu Leu Phe Ser
   1               5                   10                  15
   Arg Val Ser Leu Ser Ser Ser Leu Gln Glu Val His Val Ser Arg Glu
               20                  25                  30
   Thr Thr Gly Lys Ile Ala Val Ala Ser Lys Leu Ile Trp Cys Ser Ala
           35                  40                  45
   Ala Val Asp Ile Leu Phe Leu Leu Asp Gly Ser His Ser Ile Gly Lys
       50                  55                  60
   Gly Ser Phe Glu Arg Ser Lys Arg Phe Ala Thr Ala Leu Cys Asp Ala
   65                  70                  75                  80
   Leu Asp Ile Ser Pro Gly Arg Val Arg Val Gly Ala Leu Gln Phe Gly
                   85                  90                  95
   Ser Thr Pro His Leu Glu Phe Pro Leu Asp Ala Phe Ser Thr Arg Gln
   100 105 110
   Glu Val Lys Glu Ile Ile Lys Lys Leu Val Phe Lys Gly Gly Arg Thr
   115 120 125
   Glu Thr Gly Leu Ala Leu Lys His Leu Leu Arg Gly Leu Pro Gly Gly
   130 135 140
   Arg Asn Gly Ser Val Pro Gln Ile Leu Ile Ile Ile Thr Asp Gly Lys
   145 150 155 160
   Ser Gln Gly His Val Ala Leu Pro Ala Lys Gln Leu Lys Glu Arg Gly
   165 170 175
   Ile Val Val Phe Ala Val Gly Val Arg Phe Pro Arg Trp Asp Glu Leu
   180 185 190
   Leu Thr Leu Ala Ser Glu Pro Lys Glu Gln His Val Leu Leu Ala Glu
   195 200 205
   Gln Val Glu Asp Ala Thr Asn Gly Leu Leu Ser Thr Leu Ser Ser Ser
   210 215 220
   Leu Leu Cys Thr Thr Ala Asp Pro Asp Cys Arg Val Glu Ala Tyr Pro
   225 230 235 240
   Cys Glu Arg Arg Thr Leu Glu Thr Met Arg Glu Leu Ala Ser Asn Ala
   245 250 255
   Leu Cys Trp Arg Gly Ser Arg Gln Ala Asp Thr Val Leu Ala Val Pro
   260 265 270
   Cys Pro Phe Tyr Ser Trp Lys Arg Val Phe Gln Thr His Pro Ala Asn
   275 280 285
   Cys Tyr Arg Thr Ile Cys Pro Gly Pro Cys Asp Ser Gln Pro Cys Gln
   290 295 300
   Asn Gly Gly Thr Cys Ile Pro Glu Gly Val Asp Arg Tyr His Cys Leu
   305 310 315 320
   Cys Pro Leu Ala Phe Gly Gly Glu Val Asn Cys Ala Pro Lys Leu Ser
   325 330 335
   Leu Glu Cys Arg Ile Asp Val Leu Phe Leu Leu Asp Ser Ser Ala Gly
   340 345 350
   Thr Thr Pro Glu Gly Phe Arg Gln Ala Lys Ala Phe Val Lys Arg Phe
   355 360 365
   Val Gln Ala Val Leu Ser Glu Asp Ser Arg Ala Arg Val Gly Val Ala
   370 375 380
   Ser Tyr Ser Arg Asp Leu Met Val Ala Val Pro Val Gly Glu Tyr Gln
   385 390 395 400
   His Val Pro Asp Leu Val Arg Ile Leu Asp Ser Ile Pro Phe Ser Gly
   405 410 415
   Gly Pro Thr Leu Thr Gly Ser Ala Leu Leu Gln Val Thr Glu His Gly
   420 425 430
   Phe Gly Ser Ala Ser Arg Thr Gly Gln Asp Arg Pro Arg Arg Val Val
   435 440 445
   Val Leu Leu Thr Glu Ser His Ala Gln Asp Glu Val Ala Gly Pro Ala
   450 455 460
   Asp His Ala Arg Ala Arg Glu Leu Leu Leu Leu Gly Met Gly Asn Glu
   465 470 475 480
   Leu Met Arg Ala Glu Leu Glu Arg Ile Thr Gly Ser Arg Lys His Val
   485 490 495
   Ile Ile His Thr Asp Pro Arg Asp Met Phe Ser Gln Ile Pro Glu Leu
   500 505 510
   Gln Arg Arg Leu Cys Ser Gln Pro Arg Pro Gly Cys Gln Ala Gln Ser
   515 520 525
   Leu Asp Leu Val Phe Leu Leu Asp Ala Ser Ala Ser Val Gly Gly Glu
   530 535 540
   Asn Phe Ala Gln Met Gln Ser Phe Ile Arg Lys Cys Thr Leu Arg Phe
   545 550 555 560
   Asp Val Asn Pro Asp Val Thr Gln Val Gly Leu Val Val Tyr Gly Asn
   565 570 575
   Gln Val Gln Thr Ala Phe Gly Leu Asp Thr His Pro Thr Arg Ala Ser
   580 585 590
   Val Leu Arg Ala Met Ser Gln Ala Pro Tyr Leu Gly Gly Val Gly Ser
                   595                 600                 605
   Ala Gly Thr Ala Leu Leu His Ile Glu Asp Lys Val Met Thr Val Gln
       610                 615                 620
   Arg Gly Ala Arg Pro Gly Val Pro Lys Ala Val Val Met Leu Thr Gly
   625                 630                 635                 640
   Gly Ser Gly Ala Glu Asp Ala Ala Val Pro Ala Gln Lys Leu Arg Asp
                   645                 650                 655
   Asn Gly Ile Ser Val Leu Val Met Ser Val Gly Ala Val Leu Lys Glu
               660                 665                 670
   Ala Val Arg Arg Leu Ala Gly Pro Trp Asp Tyr Leu Ile His Val Ala
           675                 680                 685
   Ala Tyr Thr Asp Leu Arg Tyr His Gln Asp Thr Leu Ile Glu Trp Leu
       690                 695                 700
   Cys Arg Gly Gln Ala Ala Ser Gln Pro Leu Gln
   705                 710                 715
<210> 4
   <211> 21
   <212> DNA
   <213> Artificial Sequence
<220>
   <221> source
   <222> 1..21
   <223> /mol_type="unassigned DNA" /note="section of exon 44 of DOCK8 gene" /organism="Artificial Sequence"
<400> 4
   gcctacatac agatcacttt t 21
<210> 5
   <211> 21
   <212> DNA
   <213> Artificial Sequence
<220>
   <221> source
   <222> 1..21
   <223> /mol_type="unassigned DNA" /note="section of exon 44 of DOCK8 gene" /organism="Artificial Sequence"
<400> 5
   gcctacatac agatcacttt t 21
<210> 6
   <211> 21
   <212> DNA
   <213> Artificial Sequence
<220>
   <221> source
   <222> 1..21
   <223> /mol_type="unassigned DNA" /note="section of exon 44 of DOCK8 gene" /organism="Artificial Sequence"
<400> 6
   gcctacatac agatcacttt t 21
<210> 7
   <211> 21
   <212> DNA
   <213> Artificial Sequence
<220>
   <221> source
   <222> 1..21
   <223> /mol_type="unassigned DNA" /note="section of exon 44 of DOCK8 gene" /organism="Artificial Sequence"
<220>
   <221> variation
   <222> 10
   <223> /note="G replaces wild-type C"
<400> 7
   gcctacatag agatcacttt t 21
<210> 8
   <211> 2099
   <212> PRT
   <213> Homo sapiens
<220>
   <223> DOCK8 protein
<400> 8
<210> 9
   <211> 1932
   <212> PRT
   <213> Artificial Sequence
<220>
   <223> consensus sequence of human DOCK8 and rat Dock8
<400> 9
   Met Ala Thr Leu Pro Ser Ala Glu Arg Arg Ala Phe Ala Leu Lys Ile
   1               5                   10                  15
   Asn Arg Tyr Ser Ser Glu Ile Arg Lys Gln Phe Thr Leu Pro Pro Asn
               20                  25                  30
   Leu Gly Gln Tyr His Arg Ser Ser Thr Ser Gly Phe Pro Ser Leu Gln
           35                  40                  45
   Leu Pro Gln Tyr Pro Val Glu Pro Val Asp Phe Glu Gly Leu Met Thr
       50                  55                  60
   His Leu Asn Ser Leu Asp Leu Ala Gln Glu Leu Gly Asp Thr Asp Asp
   65                  70                  75                  80
   Asp Leu Val Phe Thr Pro Lys Glu Cys Arg Thr Leu Gln Ser Leu Pro
                   85                  90                  95
   Glu Glu Gly Val Glu Leu Asp Pro His Val Arg Asp Cys Val Gln Thr
               100                 105                 110
   Tyr Ile Arg Glu Trp Leu Ile Val Asn Arg Lys Asn Gln Gly Ser Pro
           115                 120                 125
   Glu Cys Phe Lys Thr Gly Ser Arg Asp Phe His Lys Thr Leu Lys Gln
       130                 135                 140
   Thr Phe Glu Ser Glu Thr Leu Glu Cys Ser Glu Pro Ala Gln Gly Pro
   145                 150                 155                 160
   Arg His Asn Val Leu Cys Asp Val Ser Gly Lys Gly Pro Thr Cys Asp
                   165                 170                 175
   Phe Asp Leu Arg Ser Leu Gln Pro Asp Arg Leu Glu Asn Leu Leu Gln
               180                 185                 190
   Ser Ala Glu Asp Phe Glu Lys Glu Glu Ala Arg Thr Asn Arg Ala Glu
           195                 200                 205
   Leu Phe Ala Leu Tyr Pro Ser Val Asp Glu Glu Asp Ala Val Glu Ile
       210                 215                 220
   Arg Pro Val Pro Glu Cys Pro Lys Glu His Leu Gly Asn Arg Ile Leu
   225                 230                 235                 240
   Val Lys Leu Thr Leu Lys Phe Glu Ile Glu Ile Glu Pro Leu Phe Ala
                   245                 250                 255
   Ser Ile Ala Leu Tyr Asp Val Lys Glu Arg Lys Lys Ile Ser Glu Asn
               260                 265                 270
   Phe His Cys Asp Leu Asn Ser Asp Gln Phe Lys Gly Phe Leu Arg Ala
           275                 280                 285
   His Thr Pro Ser Val Ser Ser Gln Ala Arg Ser Ala Val Phe Ser Val
       290                 295                 300
   Thr Tyr Pro Ser Ser Asp Ile Tyr Leu Val Val Lys Ile Glu Lys Val
   305                 310                 315                 320
   Leu Gln Gln Gly Glu Ile Asp Cys Ala Glu Pro Tyr Ile Lys Glu Ser
                   325                 330                 335
   Asp Gly Gly Lys Ser Lys Glu Lys Glu Lys Leu Lys Leu Gln Ala Glu
               340                 345                 350
   Ser Phe Cys Gln Arg Leu Gly Lys Tyr Arg Met Pro Phe Ala Trp Ala
           355                 360                 365
   Pro Ile Ser Leu Ser Phe Phe Asn Val Ser Thr Leu Glu Arg Glu Thr
       370                 375                 380
   Asp Gly Arg Ser Val Gly Glu Arg Arg Leu Gln Ser Arg Arg Ser Glu
   385                 390                 395                 400
   Arg Leu Ser Glu Glu Asn Gly Val Gly Ser Asn Phe Lys Thr Thr Leu
                   405                 410                 415
   Phe Phe Lys Gln Glu Gly Asp Arg Leu Ser Asp Glu Asp Leu Phe Lys
               420                 425                 430
   Phe Leu Ala Asp Tyr Lys Arg Ser Ser Ser Leu Gln Arg Arg Val Lys
           435                 440                 445
   Ser Ile Pro Gly Leu Arg Leu Glu Ile Ala Pro Asn Cys Cys Leu Thr
       450                 455                 460
   Pro Glu Met Leu Pro Val Lys Pro Phe Pro Glu Asn Arg Thr Arg Pro
   465                 470                 475                 480
   His Lys Glu Ile Leu Glu Phe Pro Arg Glu Val Tyr Val Pro His Thr
                   485                 490                 495
   Val Tyr Arg Asn Leu Leu Tyr Val Tyr Pro Gln Arg Leu Asn Phe Asn
               500                 505                 510
   Lys Leu Ala Ser Ala Arg Asn Ile Thr Ile Lys Ile Gln Phe Met Cys
           515                 520                 525
   Gly Glu Asp Ser Asn Ala Met Pro Val Ile Phe Gly Lys Ser Ser Gly
       530                 535                 540
   Pro Glu Phe Leu Gln Glu Val Tyr Thr Ala Thr Tyr His Asn Lys Ser
   545                 550                 555                 560
   Pro Asp Phe Tyr Glu Glu Val Lys Ile Lys Leu Pro Ala Lys Leu Thr
                   565                 570                 575
   Val Asn His His Leu Leu Phe Thr Phe Tyr His Ile Ser Cys Gln Gln
                   580                 585                 590
   Lys Gln Gly Ala Ser Glu Leu Leu Gly Tyr Ser Trp Leu Pro Ile Leu
               595                 600                 605
   Leu Asn Glu Arg Leu Gln Thr Gly Ser Tyr Cys Leu Pro Val Ala Leu
       610                 615                 620
   Glu Lys Leu Pro Pro Asn Tyr Ser His Ser Ala Glu Lys Val Pro Leu
   625                 630                 635                 640
   Gln Asn Pro Pro Ile Lys Trp Ala Glu Gly His Lys Gly Val Phe Asn
                   645                 650                 655
   Ile Glu Val Gln Ala Val Ser Ser Val His Thr Gln Asp Asn His Leu
               660                 665                 670
   Glu Lys Phe Phe Thr Leu Cys His Ser Leu Glu Ser Gln Val Thr Phe
           675                 680                 685
   Pro Ile Arg Val Leu Asp Gln Lys Ile Glu Leu Glu His Glu Leu Lys
       690                 695                 700
   Leu Ser Ile Ile Cys Leu Asn Ser Ser Arg Leu Glu Pro Leu Val Leu
   705                 710                 715                 720
   Phe Leu His Leu Val Leu Asp Lys Leu Phe Gln Leu Ser Val Gln Pro
                   725                 730                 735
   Met Val Ile Ala Gly Gln Thr Ala Asn Phe Ser Gln Phe Ala Phe Glu
               740                 745                 750
   Ser Val Val Ala Ile Ala Asn Ser Leu His Asn Ser Lys Asp Leu Lys
           755                 760                 765
   Asp Gln His Gly Arg Asn Cys Leu Leu Ala Ser Tyr Val His Tyr Val
       770                 775                 780
   Phe Arg Leu Pro Glu Arg Asp Pro Lys Ser Gly Pro Thr Ala Asp Pro
   785                 790                 795                 800
   Arg Tyr His Thr Tyr Gly Arg Thr Ser Ala Ala Ala Val Ser Ser Lys
                   805                 810                 815
   Leu Gln Ala Arg Val Met Ser Ser Ser Asn Pro Asp Leu Ala Gly Thr
               820                 825                 830
   His Ala Ala Asp Glu Glu Val Lys Asn Ile Met Ser Ser Lys Ile Ala
           835                 840                 845
   Asp Arg Asn Cys Ser Arg Met Ser Tyr Tyr Cys Ser Gly Ser Asp Pro
       850                 855                 860
   Ser Ala Ala Pro Arg Pro Ser Lys Lys His Phe His Glu Glu Leu Ala
   865                 870                 875                 880
   Leu Gln Met Val Val Ser Thr Gly Val Arg Glu Thr Val Phe Lys Tyr
                   885                 890                 895
   Ala Trp Phe Phe Phe Glu Leu Leu Val Lys Ser Met Ala Gln Val His
               900                 905                 910
   Asn Asp Lys Arg Asp Ser Phe Arg Arg Thr Arg Phe Ser Asp Arg Phe
           915                 920                 925
   Asp Asp Ile Thr Thr Ile Val Asn Val Val Thr Ser Glu Ile Ala Ala
       930                 935                 940
   Leu Leu Val Lys Pro Gln Lys Glu Glu Gln Ala Glu Lys Asn Ile Ser
   945                 950                 955                 960
   Leu Ala Phe Phe Leu Tyr Asp Leu Leu Ser Met Asp Arg Gly Phe Val
                   965                 970                 975
   Phe Asn Leu Ile His Tyr Cys Gln Leu Ser Ala Lys Leu Leu Pro Thr
               980                 985                 990
   Leu Ile Ser Met Arg Leu Glu Phe Leu Arg Ile Leu Cys Ser His Glu
           995                 1000                1005
   His Tyr Leu Asn Leu Asn Leu Phe Met Asn Asp Thr Ala Pro Ser Pro
       1010                1015                1020
   Cys Pro Ser Ile Ser Ser Gln Asn Ser Ser Ser Cys Ser Ser Phe Gln
   1025                1030                1035                1040
   Asp Gln Lys Ile Ala Ser Met Phe Asp Leu Thr Glu Tyr Arg Gln Gln
                   1045                1050                1055
   His Phe Leu Thr Gly Leu Leu Phe Thr Glu Leu Ala Ala Leu Asp Ala
               1060                1065                1070
   Glu Gly Gly Ile Ser Val Gln Arg Lys Ala Val Ser Ala Ile His Ser
           1075                1080                1085
   Leu Leu Ser Ser His Asp Leu Asp Pro Arg Cys Lys Pro Glu Val Lys
   1090 1095 1100
   Val Lys Ile Ala Ala Leu Tyr Leu Pro Leu Val Gly Ile Ile Leu Asp
   1105 1110 1115 1120
   Ala Leu Pro Gln Leu Asp Phe Thr Ala Arg Arg Ser Gly Ser Glu Glu
   1125 1130 1135
   Gln Ala Ile Asn Gln Asn Val Ala Leu Ala Ile Ala Gly Asn Phe Asn
   1140 1145 1150
   Leu Lys Thr Ser Gly Leu Ser Ser Leu Pro Tyr Lys Gln Tyr Asn Met
   1155 1160 1165
   Leu Asn Ala Asp Thr Thr Arg Leu Met Cys Phe Leu Trp Ile Met Lys
   1170 1175 1180
   Asn Ala Asp Gln Ser Leu Ile Arg Lys Trp Ile Ala Asp Leu Pro Ser
   1185 1190 1195 1200
   Gln Leu Asn Arg Ile Leu Asp Leu Leu Phe Ile Cys Val Cys Phe Glu
   1205 1210 1215
   Tyr Lys Gly Lys Gln Ser Ser Asp Lys Val Ser Gln Val Leu Gln Lys
   1220 1225 1230
   Ser Arg Asp Val Lys Ala Leu Glu Glu Ala Leu Leu Arg Gly Glu Gly
   1235 1240 1245
   Ala Arg Gly Glu Met Met Arg Arg Arg Pro Gly Asp Arg Phe Pro Gly
   1250 1255 1260
   Leu Asn Glu Asn Leu Arg Trp Lys Glu Gln Thr Trp Arg Gln Ala Asn
   1265 1270 1275 1280
   Glu Lys Leu Asp Lys Thr Lys Ala Glu Leu Asp Gln Glu Ala Leu Ile
   1285 1290 1295
   Ser Gly Asn Leu Ala Thr Glu Ala Leu Ile Ile Leu Asp Met Gln Glu
   1300 1305 1310
   Asn Ile Ile Gln Ala Ser Ser Leu Asp Cys Lys Asp Ser Leu Leu Gly
   1315 1320 1325
   Gly Val Leu Arg Val Leu Val Asn Ser Leu Cys Asp Gln Ser Thr Thr
   1330 1335 1340
   Tyr Leu Thr His Cys Phe Ala Thr Leu Arg Ala Leu Ile Ala Lys Phe
   1345 1350 1355 1360
   Gly Asp Leu Leu Phe Glu Glu Glu Glu Gln Cys Asp Leu Cys Val Leu
   1365 1370 1375
   His His Cys Ser Ser Ser Met Asp Val Thr Arg Ser Gln Ala Cys Ala
   1380 1385 1390
   Thr Leu Tyr Leu Leu Met Arg Phe Ser Phe Gly Ala Thr Ser Asn Phe
   1395 1400 1405
   Ala Arg Val Lys Met Gln Val Thr Met Leu Ala Ser Leu Val Gly Ala
   1410 1415 1420
   Pro Asp Phe Asn Glu Glu His Leu Arg Ser Leu Arg Thr Ile Leu Ala
   1425 1430 1435 1440
   Tyr Ser Glu Glu Asp Thr Ala Met Thr Phe Pro Thr Gln Val Glu Glu
   1445 1450 1455
   Leu Leu Cys Asn Leu Asn Ser Ile Leu Tyr Asp Thr Val Lys Met Arg
   1460 1465 1470
   Glu Phe Gln Glu Asp Pro Glu Met Leu Met Asp Leu Met Tyr Arg Ile
   1475 1480 1485
   Ala Lys Ser Tyr Gln Ala Ser Pro Asp Leu Arg Leu Thr Trp Leu Gln
   1490 1495 1500
   Asn Met Ala Glu Lys His Thr Lys Lys Cys Thr Glu Ala Ala Met Cys
   1505 1510 1515 1520
   Leu Val His Ala Ala Ala Leu Val Ala Glu Tyr Leu Ser Met Leu Glu
   1525 1530 1535
   Asp His Ser Tyr Leu Pro Val Gly Ser Val Ser Phe Gln Asn Ile Ser
   1540 1545 1550
   Ser Asn Val Leu Glu Glu Ser Val Ser Asp Thr Leu Ser Pro Asp Glu
   1555 1560 1565
   Asp Gly Val Cys Gly Tyr Phe Thr Glu Ser Gly Leu Val Gly Leu Leu
   1570 1575 1580
   Glu Gln Ala Ala Glu Leu Phe Ser Thr Gly Gly Leu Tyr Glu Thr Val
   1585 1590 1595 1600
   Asn Glu Val Tyr Lys Leu Val Ile Pro Ile Leu Glu Ala His Arg Phe
   1605 1610 1615
   Arg Lys Leu Thr Thr His Lys Leu Gln Ala Phe Asp Ile Asn Lys Asp
   1620 1625 1630
   His Lys Arg Met Phe Gly Thr Tyr Phe Arg Val Gly Phe Gly Ser Lys
   1635 1640 1645
   Phe Gly Asp Leu Asp Glu Gln Glu Phe Val Tyr Lys Glu Pro Ala Ile
   1650 1655 1660
   Thr Lys Leu Pro Glu Ile Ser His Arg Leu Glu Phe Tyr Gly Gln Cys
   1665 1670 1675 1680
   Phe Gly Ala Glu Phe Val Glu Val Ile Lys Asp Ser Thr Pro Val Asp
   1685 1690 1695
   Lys Thr Lys Leu Asp Pro Asn Lys Ala Tyr Ile Gln Ile Thr Phe Val
   1700 1705 1710
   Glu Pro Tyr Phe Asp Glu Tyr Glu Met Lys Asp Arg Val Thr Tyr Phe
   1715 1720 1725
   Glu Lys Asn Phe Asn Leu Arg Arg Phe Met Tyr Thr Thr Pro Phe Thr
   1730 1735 1740
   Leu Glu Gly Arg Pro Arg Gly Glu Leu His Glu Gln Arg Arg Asn Thr
   1745 1750 1755 1760
   Val Leu Thr Thr Met His Ala Phe Pro Tyr Ile Lys Thr Arg Ile Val
   1765 1770 1775
   Gln Lys Glu Glu Phe Val Leu Thr Pro Ile Glu Val Ala Ile Glu Asp
   1780 1785 1790
   Met Lys Lys Lys Thr Leu Gln Leu Ala Val Ala Gln Glu Pro Pro Asp
   1795 1800 1805
   Ala Lys Met Leu Gln Met Val Leu Gln Gly Ser Val Gly Ala Thr Val
   1810 1815 1820
   Asn Gln Gly Pro Leu Glu Val Ala Gln Val Phe Leu Ala Glu Ile Pro
   1825 1830 1835 1840
   Ala Asp Pro Lys Leu Tyr Arg His His Asn Lys Leu Arg Leu Cys Phe
   1845 1850 1855
   Lys Glu Phe Ile Met Arg Cys Gly Glu Ala Val Glu Lys Asn Arg Leu
   1860 1865 1870
   Ile Thr Ala Gln Arg Glu Tyr Gln Gln Glu Leu Lys Lys Asn Tyr Asn
   1875 1880 1885
   Leu Lys Leu Arg Pro Met Ile Glu Arg Lys Ile Pro Glu Leu Tyr Lys
   1890 1895 1900
   Pro Ile Phe Arg Val Ser Gln Lys Arg Asp Ser Phe His Arg Ser Ser
   1905 1910 1915 1920
   Phe Arg Lys Cys Glu Thr Gln Leu Ser Gln Gly Ser
   1925 1930
<210> 10
   <211> 2099
   <212> PRT
   <213> Rattus norvegicus
<220>
   <223> wild-type Dock8
<220>
   <221> CHAIN
   <222> 1860..1867
   <223> Ã\2374 region
<220>
   <221> CHAIN
   <222> 1861..1867
   <223> Ã\2374 region
<220>
   <221> CHAIN
   <222> 1620..2070
   <223> DHR2 region
<220>
   <221> CHAIN
   <222> 1632..2070
   <223> DHR2 region
<220>
   <221> CHAIN
   <222> 1861..1939
   <223> amino acids corresponding to exon 44
<400> 10
   Met Ala Thr Leu Pro Ser Ala Glu Arg Arg Ala Phe Ala Leu Lys Ile
   1               5                   10                  15
   Asn Arg Tyr Ser Ser Ser Glu Ile Arg Lys Gln Phe Thr Leu Pro Pro
               20                  25                  30
   Asn Leu Gly Gln Tyr His Arg His Ser Leu Ser Thr Ser Gly Phe Pro
           35                  40                  45
   Ser Leu Gln Leu Pro Gln Leu Tyr Glu Pro Val Glu Pro Val Asp Phe
       50                  55                  60
   Glu Gly Leu Val Met Thr His Leu Asn Ser Leu Asp Ala Glu Leu Ala
   65                  70                  75                  80
   Gln Glu Leu Gly Asp Leu Thr Asp Asp Asp Leu His Val Ala Phe Thr
                   85                  90                  95
   Pro Lys Glu Cys Arg Thr Leu Gln His Ser Leu Pro Glu Glu Gly Val
               100                 105                 110
   Glu Leu Asp Pro His Val Arg Asp Cys Val Gln Thr Tyr Ile Arg Glu
           115                 120                 125
   Trp Leu Ile Val Asn Arg Lys Asn Gln Gly Ser Pro Glu Phe Cys Ser
       130                 135                 140
   Phe Arg Lys Thr Gly Ser Arg Arg Asp Phe His Lys Thr Leu Gln Lys
   145                 150                 155                 160
   Gln Thr Phe Glu Ser Glu Thr Leu Glu Cys Ser Glu Pro Ala Thr Gln
                   165                 170                 175
   Thr Gly Pro Arg His Arg Phe Asn Val Leu Cys Asp Val Ser Gly Lys
               180                 185                 190
   Gly Pro Ile Thr Ser Cys Asp Phe Asp Leu Arg Ser Leu Gln Pro Asp
           195                 200                 205
   Glu Arg Leu Glu Asn Leu Leu Gln Leu Ser Ala Glu Asp Phe Glu Lys
       210                 215                 220
   Glu Lys Glu Glu Ala Arg Lys Thr Asn Arg Thr Ala Glu Leu Phe Ala
   225                 230                 235                 240
   Leu Tyr Pro Ser Val Asp Glu Glu Asp Ala Val Glu Ile Arg Pro Val
                   245                 250                 255
   Pro Glu Cys Pro Lys Glu His Leu Gly Asn Arg Ile Leu Val Lys Val
               260                 265                 270
   Leu Thr Leu Lys Phe Glu Ile Glu Ile Glu Pro Leu Phe Ala Ser Ile
           275                 280                 285
   Ala Leu Tyr Asp Val Lys Glu Arg Lys Lys Ile Ser Glu Asn Phe His
       290                 295                 300
   Cys Asp Leu Asn Ser Asp Gln Phe Lys Gly Phe Leu Arg Ala His Thr
   305                 310                 315                 320
   Pro Ser Val Asp Pro Ser Ser Gln Ala Arg Ser Ala Val Phe Ser Val
                   325                 330                 335
   Thr Tyr Pro Ser Ser Asp Ile Tyr Leu Val Val Lys Ile Glu Lys Val
               340                 345                 350
   Leu Gln Gln Gly Glu Ile Ala Asp Cys Ala Glu Pro Tyr Met Ile Ile
           355                 360                 365
   Lys Glu Ser Asp Gly Gly Lys Ser Lys Glu Lys Val Glu Lys Leu Lys
       370                 375                 380
   Leu Gln Ala Glu Ser Phe Cys Gln Arg Leu Gly Lys Tyr Arg Met Pro
   385 390 395 400
   Phe Ala Trp Ala Pro Ile Ser Leu Ala Ser Phe Phe Asn Val Ser Thr
   405 410 415
   Leu Glu Arg Glu Ser Thr Asp Leu Glu Pro Gly Ala Gly Arg Asn Ser
   420 425 430
   Val Gly Glu Arg Arg Ser Leu Ser Gln Ser Arg Arg Pro Ser Glu Arg
   435 440 445
   Thr Leu Ser Phe Glu Glu Asn Gly Val Gly Ser Asn Phe Lys Thr Thr
   450 455 460
   Thr Leu Ala Thr Asn Ile Phe Phe Lys Gln Glu Gly Asp Arg Leu Ser
   465 470 475 480
   Asp Glu Asp Leu Phe Lys Phe Leu Ala Asp Tyr Lys Arg Ser Ser Ser
   485 490 495
   Leu Gln Arg Arg Val Lys Ser Ile Pro Gly Ser Leu Arg Leu Glu Ile
   500 505 510
   Ala Pro Ala Pro Asp Val Met Asn Cys Cys Leu Thr Pro Glu Met Leu
   515 520 525
   Pro Val Lys Pro Phe Pro Glu Asn Arg Thr Arg Pro His Lys Glu Ile
   530 535 540
   Leu Glu Phe Pro Ile Arg Glu Val Tyr Val Pro His Thr Val Tyr Arg
   545 550 555 560
   Asn Leu Leu Tyr Val Tyr Pro Gln Arg Leu Asn Phe Ala Asn Lys Leu
   565 570 575
   Ala Ser Ala Arg Asn Ile Thr Ile Lys Ile Gln Phe Met Cys Gly Glu
   580 585 590
   Asp Pro Ser Asn Ala Met Pro Val Ile Phe Gly Lys Ser Ser Gly Pro
   595 600 605
   Glu Phe Leu Gln Glu Val Tyr Thr Ala Ile Thr Tyr His Asn Lys Ser
   610 615 620
   Pro Asp Phe Tyr Glu Glu Val Lys Ile Lys Leu Pro Ala Lys Leu Thr
   625 630 635 640
   Val Asn His His Leu Leu Phe Thr Phe Tyr His Ile Ser Cys Gln Gln
   645 650 655
   Lys Gln Gly Ala Ser Gly Glu Ser Leu Leu Gly Tyr Ser Trp Leu Pro
   660 665 670
   Ile Leu Leu Asn Glu Arg Leu Gln Thr Gly Ser Tyr Cys Leu Pro Val
   675 680 685
   Ala Leu Glu Lys Leu Pro Pro Asn Tyr Ser Ile His Ser Ala Glu Lys
   690 695 700
   Val Pro Leu Gln Asn Pro Pro Ile Lys Trp Ala Glu Gly His Lys Gly
   705 710 715 720
   Val Phe Asn Ile Glu Val Gln Ala Val Ser Ser Val His Thr Gln Asp
   725 730 735
   Asn His Leu Glu Lys Phe Phe Thr Leu Cys His Ser Leu Glu Ser Gln
   740 745 750
   Val Thr Phe Pro Ile Arg Val Leu Asp Gln Lys Ile Thr Glu Asn Thr
   755 760 765
   Leu Glu His Glu Leu Lys Leu Ser Ile Ile Cys Leu Asn Ser Ser Arg
   770 775 780
   Leu Glu Pro Leu Val Leu Phe Leu His Leu Val Leu Asp Lys Leu Phe
   785 790 795 800
   Gln Leu Ser Val Gln Pro Met Val Ile Ala Gly Gln Thr Ala Asn Phe
   805 810 815
   Ser Gln Phe Ala Phe Glu Ser Val Val Ala Ile Ala Asn Ser Leu His
   820 825 830
   Asn Ser Lys Asp Leu Arg Lys Asp Gln His Gly Arg Asn Cys Leu Leu
   835 840 845
   Ala Ser Tyr Val His Tyr Val Phe Arg Leu Pro Glu Leu His Arg Asp
   850 855 860
   Ala Pro Lys Ser Gly Gly Pro Thr Ala Val Val Pro Asp Pro Arg Tyr
   865 870 875 880
   His Thr Tyr Gly Arg Thr Ser Ala Ala Ala Val Ser Ser Lys Leu Met
                   885                 890                 895
   Gln Ala Arg Val Met Ser Ser Ser Asn Pro Asp Leu Ala Gly Thr His
               900                 905                 910
   Cys Ala Ala Asp Glu Glu Val Lys Asn Ile Met Ser Ser Lys Ile Ala
           915                 920                 925
   Asp Arg Asn Cys Ser Arg Met Ser Tyr Tyr Cys Ser Gly Asn Ser Asp
       930                 935                 940
   Val Pro Gly Ser Thr Ala Ala Pro Arg Pro Val Ser Lys Lys His Phe
   945                 950                 955                 960
   His Glu Glu Leu Ala Leu Gln Met Val Val Ser Thr Gly Leu Val Arg
                   965                 970                 975
   Glu Thr Val Phe Lys Tyr Ala Trp Phe Phe Phe Glu Leu Leu Val Lys
               980                 985                 990
   Ser Met Ala Gln Tyr Val His Asn Leu Asp Lys Arg Asp Ser Phe Arg
           995                 1000                1005
   Arg Thr Arg Phe Ser Asp Arg Phe Lys Asp Asp Ile Thr Thr Ile Val
       1010                1015                1020
   Asn Val Val Thr Ser Glu Ile Ala Ala Leu Leu Val Lys Pro Gln Lys
   1025                1030                1035                1040
   Glu Ser Glu Gln Ala Glu Lys Ile Asn Ile Ser Leu Ala Phe Phe Leu
                   1045                1050                1055
   Tyr Asp Leu Leu Ser Ile Met Asp Arg Gly Phe Val Phe Asn Leu Ile
               1060                1065                1070
   Lys His Tyr Cys Thr Gln Leu Ser Ala Lys Leu Asn Thr Leu Pro Thr
           1075                1080                1085
   Leu Ile Ser Met Arg Leu Glu Phe Leu Arg Ile Leu Cys Ser His Glu
       1090                1095                1100
   His Tyr Leu Asn Leu Asn Leu Leu Phe Met Asn Pro Asp Thr Ala Pro
   1105                1110                1115                1120
   Ala Ser Pro Cys Pro Ser Ile Ser Ser Gln Asn Ser Ser Ser Cys Ser
                   1125                1130                1135
   Ser Phe Gln Asp Gln Lys Ile Ala Ser Met Phe Asp Leu Thr Pro Glu
               1140                1145                1150
   Tyr Arg Gln Gln His Phe Leu Thr Gly Leu Leu Phe Thr Glu Leu Ala
           1155                1160                1165
   Val Ala Leu Asp Ala Glu Gly Asp Gly Ile Ser Arg Val Gln Arg Lys
       1170                1175                1180
   Ala Val Ser Ala Ile His Ser Leu Leu Ser Ser His Asp Leu Asp Pro
   1185                1190                1195                1200
   Arg Cys Leu Lys Pro Glu Val Lys Val Lys Ile Ala Ala Leu Tyr Leu
                   1205                1210                1215
   Pro Leu Val Gly Ile Ile Leu Asp Ala Leu Pro Gln Leu Tyr Asp Phe
               1220                1225                1230
   Thr Asp Ala Arg Ser Gly Arg Ser Arg Ala Ser Gly Ser Tyr Glu Glu
           1235                1240                1245
   Gln Asp Val Ala Asn Gly Ile Asn Gln Asn Val Ala Leu Ala Ile Ala
       1250                1255                1260
   Gly Asn His Phe Asn Leu Lys Thr Ser Gly Ala Met Leu Ser Ser Leu
   1265                1270                1275                1280
   Pro Tyr Lys Gln Tyr Asn Met Leu Asn Ala Asp Thr Thr Arg His Leu
                   1285                1290                1295
   Met Val Cys Phe Leu Trp Ile Met Lys Asn Ala Asp Gln Ser Leu Ile
               1300                1305                1310
   Arg Lys Trp Ile Ala Asp Leu Pro Ser Met Gln Leu Asn Arg Ile Leu
           1315                1320                1325
   Asp Leu Leu Phe Ile Cys Val Ser Cys Phe Glu Tyr Lys Gly Lys Gln
       1330                1335                1340
   Ser Ser Asp Lys Val Ser Asn Gln Val Leu Gln Lys Ser Arg Asp Val
   1345                1350                1355                1360
   Lys Ala Lys Leu Glu Glu Ala Leu Leu Arg Gly Glu Gly Ala Arg Gly
                   1365                1370                1375
   Glu Met Met Arg Arg Arg Ile Pro Gly Thr Asp Arg Phe Pro Gly Leu
               1380                1385                1390
   Asn Glu Asn Leu Arg Trp Arg Lys Glu Gln Thr Gln Trp Arg Gln Ala
   1395 1400 1405
   Asn Glu Lys Leu Asp Lys Thr Lys Ala Glu Leu Asp Gln Glu Ala Leu
   1410 1415 1420
   Ile Ser Gly Asn Leu Ala Thr Glu Ala Asn Leu Ile Ile Leu Asp Met
   1425 1430 1435 1440
   Gln Glu Asn Ile Ile Gln Ala Ser Ser Ser Leu Asp Cys Lys Asp Ser
   1445 1450 1455
   Leu Leu Gly Gly Val Leu Arg Val Leu Val Asn Ser Leu Ser Cys Asp
   1460 1465 1470
   Gln Ser Thr Thr Tyr Leu Thr His Cys Phe Ala Thr Leu Arg Ala Leu
   1475 1480 1485
   Ile Ala Lys Phe Gly Asp Leu Leu Phe Glu Glu Glu Met Glu Gln Cys
   1490 1495 1500
   Ala Asp Leu Cys Gln Arg Val Leu His His Cys Ser Ser Ser Met Asp
   1505 1510 1515 1520
   Val Thr Arg Ser Gln Ala Cys Ala Thr Leu Tyr Leu Leu Met Arg Phe
   1525 1530 1535
   Ser Phe Gly Ala Thr Ser Asn Phe Ala Arg Val Lys Met Gln Val Thr
   1540 1545 1550
   Met Ala Leu Ala Ser Leu Val Gly Lys Ala Pro Asp Phe Asn Glu Glu
   1555 1560 1565
   His Leu Arg Lys Ser Leu Arg Thr Ile Leu Ala Tyr Ser Glu Glu Asp
   1570 1575 1580
   Thr Ala Met Arg Ala Thr Leu Phe Pro Thr Gln Val Glu Glu Leu Leu
   1585 1590 1595 1600
   Cys Asn Leu Asn Ser Ile Leu Tyr Asp Thr Val Lys Met Arg Glu Phe
   1605 1610 1615
   Gln Glu Asp Pro Glu Met Leu Met Asp Leu Met Tyr Arg Ile Ala Lys
   1620 1625 1630
   Ser Tyr Gln Ala Ser Pro Asp Leu Arg Leu Thr Trp Leu Gln Asn Met
   1635 1640 1645
   Ala Glu Lys His Thr Lys Arg Lys Cys Phe Thr Glu Ala Ala Met Cys
   1650 1655 1660
   Leu Val His Ala Ala Ala Leu Val Ala Glu Tyr Leu Ser Met Leu Glu
   1665 1670 1675 1680
   Asp His Ser Tyr Leu Pro Val Gly Ser Val Ser Phe Gln Asn Ile Ser
   1685 1690 1695
   Ser Asn Val Leu Glu Glu Ser Ala Val Ser Asp Asp Thr Leu Ser Pro
   1700 1705 1710
   Asp Glu Asp Gly Val Cys Ser Gly Arg Tyr Phe Thr Glu Ser Gly Leu
   1715 1720 1725
   Val Gly Leu Leu Glu Gln Ala Ala Glu Leu Phe Ser Thr Gly Gly Leu
   1730 1735 1740
   Tyr Glu Thr Val Asn Glu Val Tyr Lys Leu Val Ile Pro Ile Leu Glu
   1745 1750 1755 1760
   Ala His Arg Asp Phe Arg Lys Leu Thr Ser Thr His Asp Lys Leu Gln
   1765 1770 1775
   Lys Ala Phe Asp Asn Ile Ile Asn Lys Asp His Lys Arg Met Phe Gly
   1780 1785 1790
   Thr Tyr Phe Arg Val Gly Phe Tyr Gly Ser Lys Phe Gly Asp Leu Asp
   1795 1800 1805
   Glu Gln Glu Phe Val Tyr Lys Glu Pro Ala Ile Thr Lys Leu Pro Glu
   1810 1815 1820
   Ile Ser His Arg Leu Glu Gly Phe Tyr Gly Gln Cys Phe Gly Ala Glu
   1825 1830 1835 1840
   Phe Val Glu Val Ile Lys Asp Ser Thr Pro Val Asp Lys Thr Lys Leu
   1845 1850 1855
   Asp Pro Asn Lys Ala Tyr Ile Gln Ile Thr Phe Val Glu Pro Tyr Phe
   1860 1865 1870
   Asp Glu Tyr Glu Met Lys Asp Arg Val Thr Tyr Phe Glu Lys Asn Phe
   1875 1880 1885
   Asn Leu Arg Arg Phe Met Tyr Thr Thr Pro Phe Thr Leu Glu Gly Arg
       1890                1895                1900
   Pro Arg Gly Glu Leu His Glu Gln His Arg Arg Asn Thr Val Leu Thr
   1905                1910                1915                1920
   Thr Met His Ala Phe Pro Tyr Ile Lys Thr Arg Ile Arg Val Ser Gln
                   1925                1930                1935
   Lys Glu Glu Phe Val Leu Thr Pro Ile Glu Val Ala Ile Glu Asp Met
               1940                1945                1950
   Lys Lys Lys Thr Leu Gln Leu Ala Val Ala Thr His Gln Glu Pro Pro
           1955                1960                1965
   Asp Ala Lys Met Leu Gln Met Val Leu Gln Gly Ser Val Gly Ala Thr
       1970                1975                1980
   Val Asn Gln Gly Pro Leu Glu Val Ala Gln Val Phe Leu Ala Glu Ile
   1985                1990                1995                2000
   Pro Ala Asp Pro Lys Leu Tyr Arg His His Asn Lys Leu Arg Leu Cys
                   2005                2010                2015
   Phe Lys Glu Phe Ile Met Arg Cys Gly Glu Ala Val Glu Lys Asn Arg
               2020                2025                2030
   Arg Leu Ile Thr Ala Glu Gln Arg Glu Tyr Gln Gln Glu Leu Lys Lys
           2035                2040                2045
   Asn Tyr Asn Arg Leu Lys Asp Ser Leu Arg Pro Met Ile Glu Arg Lys
       2050                2055                2060
   Ile Pro Glu Leu Tyr Lys Pro Ile Phe Arg Val Asp Ser Gln Lys Arg
   2065                2070                2075                2080
   Asp Ser Phe His Arg Ser Ser Phe Arg Lys Cys Glu Thr Gln Leu Ser
                   2085                2090                2095
   Gln Gly Ser
<210> 11
   <211> 2100
   <212> PRT
   <213> Mus musculus
<220>
   <223> Dock8 wild-type
<220>
   <221> SITE
   <222> 1865
   <223> wild-type Q
<220>
   <221> CHAIN
   <222> 1633..2071
   <223> DHR2 region
<220>
   <221> CHAIN
   <222> 1862..1940
   <223> amino acids of exon 44
<220>
   <221> CHAIN
   <222> 1862..1868
   <223> Î²4 region
<400> 11
   Met Ala Thr Leu Pro Ser Ala Glu Arg Arg Ala Phe Ala Leu Lys Ile
   1               5                   10                  15
   Asn Arg Tyr Ser Ser Ser Glu Ile Arg Lys Gln Phe Thr Leu Pro Pro
               20                  25                  30
   Asn Leu Gly Gln Tyr His Arg His Ser Leu Ser Thr Ser Gly Phe Pro
           35                  40                  45
   Ser Leu Gln Leu Pro Gln Leu Tyr Glu Pro Val Glu Pro Val Asp Phe
       50                  55                  60
   Glu Gly Leu Val Met Thr His Leu Asn Ser Leu Asp Ala Glu Leu Ala
   65                  70                  75                  80
   Gln Glu Leu Gly Asp Leu Thr Asp Asp Asp Leu His Val Ala Phe Thr
                   85                  90                  95
   Pro Lys Glu Cys Arg Thr Leu Gln His Ser Leu Pro Glu Glu Gly Val
               100                 105                 110
   Glu Leu Asp Pro His Val Arg Asp Cys Val Gln Thr Tyr Ile Arg Glu
           115                 120                 125
   Trp Leu Ile Val Asn Arg Lys Asn Gln Gly Ser Pro Glu Phe Cys Ser
       130                 135                 140
   Phe Arg Lys Thr Gly Ser Arg Arg Asp Phe His Lys Thr Leu Gln Lys
   145                 150                 155                 160
   Gln Thr Phe Glu Ser Glu Thr Leu Glu Cys Ser Glu Pro Ala Thr Gln
                   165                 170                 175
   Gln Thr Gly Pro Arg His Arg Phe Asn Val Leu Cys Asp Val Ser Gly
               180                 185                 190
   Lys Gly Pro Ile Thr Ser Cys Asp Phe Asp Leu Arg Ser Leu Gln Pro
           195                 200                 205
   Asp Glu Arg Leu Glu Asn Leu Leu Gln Leu Ser Ala Glu Asp Phe Glu
       210                 215                 220
   Lys Glu Lys Glu Glu Ala Arg Lys Thr Asn Arg Thr Ala Glu Leu Phe
   225                 230                 235                 240
   Ala Leu Tyr Pro Ser Val Asp Glu Glu Asp Ala Val Glu Ile Arg Pro
                   245                 250                 255
   Val Pro Glu Cys Pro Lys Glu His Leu Gly Asn Arg Ile Leu Val Lys
               260                 265                 270
   Val Leu Thr Leu Lys Phe Glu Ile Glu Ile Glu Pro Leu Phe Ala Ser
           275                 280                 285
   Ile Ala Leu Tyr Asp Val Lys Glu Arg Lys Lys Ile Ser Glu Asn Phe
       290                 295                 300
   His Cys Asp Leu Asn Ser Asp Gln Phe Lys Gly Phe Leu Arg Ala His
   305                 310                 315                 320
   Thr Pro Ser Val Asp Pro Ser Ser Gln Ala Arg Ser Ala Val Phe Ser
                   325                 330                 335
   Val Thr Tyr Pro Ser Ser Asp Ile Tyr Leu Val Val Lys Ile Glu Lys
               340                 345                 350
   Val Leu Gln Gln Gly Glu Ile Ala Asp Cys Ala Glu Pro Tyr Met Ile
           355                 360                 365
   Ile Lys Glu Ser Asp Gly Gly Lys Ser Lys Glu Lys Val Glu Lys Leu
       370                 375                 380
   Lys Leu Gln Ala Glu Ser Phe Cys Gln Arg Leu Gly Lys Tyr Arg Met
   385                 390                 395                 400
   Pro Phe Ala Trp Ala Pro Ile Ser Leu Ala Ser Phe Phe Asn Val Ser
                   405                 410                 415
   Thr Leu Glu Arg Glu Ser Thr Asp Leu Glu Pro Gly Ala Gly Arg Asn
               420                 425                 430
   Ser Val Gly Glu Arg Arg Ser Leu Ser Gln Ser Arg Arg Pro Ser Glu
           435                 440                 445
   Arg Thr Leu Ser Phe Glu Glu Asn Gly Val Gly Ser Asn Phe Lys Thr
       450                 455                 460
   Thr Thr Leu Ala Thr Asn Ile Phe Phe Lys Gln Glu Gly Asp Arg Leu
   465                 470                 475                 480
   Ser Asp Glu Asp Leu Phe Lys Phe Leu Ala Asp Tyr Lys Arg Ser Ser
                   485                 490                 495
   Ser Leu Gln Arg Arg Val Lys Ser Ile Pro Gly Ser Leu Arg Leu Glu
               500                 505                 510
   Ile Ala Pro Ala Pro Asp Val Met Asn Cys Cys Leu Thr Pro Glu Met
           515                 520                 525
   Leu Pro Val Lys Pro Phe Pro Glu Asn Arg Thr Arg Pro His Lys Glu
       530                 535                 540
   Ile Leu Glu Phe Pro Ile Arg Glu Val Tyr Val Pro His Thr Val Tyr
   545                 550                 555                 560
   Arg Asn Leu Leu Tyr Val Tyr Pro Gln Arg Leu Asn Phe Ala Asn Lys
                   565                 570                 575
   Leu Ala Ser Ala Arg Asn Ile Thr Ile Lys Ile Gln Phe Met Cys Gly
               580                 585                 590
   Glu Asp Pro Ser Asn Ala Met Pro Val Ile Phe Gly Lys Ser Ser Gly
           595                 600                 605
   Pro Glu Phe Leu Gln Glu Val Tyr Thr Ala Ile Thr Tyr His Asn Lys
       610                 615                 620
   Ser Pro Asp Phe Tyr Glu Glu Val Lys Ile Lys Leu Pro Ala Lys Leu
   625                 630                 635                 640
   Thr Val Asn His His Leu Leu Phe Thr Phe Tyr His Ile Ser Cys Gln
                   645                 650                 655
   Gln Lys Gln Gly Ala Ser Gly Glu Ser Leu Leu Gly Tyr Ser Trp Leu
               660                 665                 670
   Pro Ile Leu Leu Asn Glu Arg Leu Gln Thr Gly Ser Tyr Cys Leu Pro
           675                 680                 685
   Val Ala Leu Glu Lys Leu Pro Pro Asn Tyr Ser Ile His Ser Ala Glu
       690                 695                 700
   Lys Val Pro Leu Gln Asn Pro Pro Ile Lys Trp Ala Glu Gly His Lys
   705                 710                 715                 720
   Gly Val Phe Asn Ile Glu Val Gln Ala Val Ser Ser Val His Thr Gln
                   725                 730                 735
   Asp Asn His Leu Glu Lys Phe Phe Thr Leu Cys His Ser Leu Glu Ser
               740                 745                 750
   Gln Val Thr Phe Pro Ile Arg Val Leu Asp Gln Lys Ile Thr Glu Asn
           755                 760                 765
   Thr Leu Glu His Glu Leu Lys Leu Ser Ile Ile Cys Leu Asn Ser Ser
       770                 775                 780
   Arg Leu Glu Pro Leu Val Leu Phe Leu His Leu Val Leu Asp Lys Leu
   785                 790                 795                 800
   Phe Gln Leu Ser Val Gln Pro Met Val Ile Ala Gly Gln Thr Ala Asn
                   805                 810                 815
   Phe Ser Gln Phe Ala Phe Glu Ser Val Val Ala Ile Ala Asn Ser Leu
               820                 825                 830
   His Asn Ser Lys Asp Leu Arg Lys Asp Gln His Gly Arg Asn Cys Leu
           835                 840                 845
   Leu Ala Ser Tyr Val His Tyr Val Phe Arg Leu Pro Glu Leu His Arg
       850                 855                 860
   Asp Ala Pro Lys Ser Gly Gly Pro Thr Ala Val Val Pro Asp Pro Arg
   865                 870                 875                 880
   Tyr His Thr Tyr Gly Arg Thr Ser Ala Ala Ala Val Ser Ser Lys Leu
                   885                 890                 895
   Met Gln Ala Arg Val Met Ser Ser Ser Asn Pro Asp Leu Ala Gly Thr
               900                 905                 910
   His Cys Ala Ala Asp Glu Glu Val Lys Asn Ile Met Ser Ser Lys Ile
           915                 920                 925
   Ala Asp Arg Asn Cys Ser Arg Met Ser Tyr Tyr Cys Ser Gly Asn Ser
       930                 935                 940
   Asp Val Pro Gly Ser Thr Ala Ala Pro Arg Pro Val Ser Lys Lys His
   945                 950                 955                 960
   Phe His Glu Glu Leu Ala Leu Gln Met Val Val Ser Thr Gly Leu Val
                   965                 970                 975
   Arg Glu Thr Val Phe Lys Tyr Ala Trp Phe Phe Phe Glu Leu Leu Val
               980                 985                 990
   Lys Ser Met Ala Gln Tyr Val His Asn Leu Asp Lys Arg Asp Ser Phe
           995                 1000                1005
   Arg Arg Thr Arg Phe Ser Asp Arg Phe Lys Asp Asp Ile Thr Thr Ile
       1010                1015                1020
   Val Asn Val Val Thr Ser Glu Ile Ala Ala Leu Leu Val Lys Pro Gln
   1025                1030                1035                1040
   Lys Glu Ser Glu Gln Ala Glu Lys Ile Asn Ile Ser Leu Ala Phe Phe
                   1045                1050                1055
   Leu Tyr Asp Leu Leu Ser Ile Met Asp Arg Gly Phe Val Phe Asn Leu
               1060                1065                1070
   Ile Lys His Tyr Cys Thr Gln Leu Ser Ala Lys Leu Asn Thr Leu Pro
           1075                1080                1085
   Thr Leu Ile Ser Met Arg Leu Glu Phe Leu Arg Ile Leu Cys Ser His
       1090                1095                1100
   Glu His Tyr Leu Asn Leu Asn Leu Leu Phe Met Asn Pro Asp Thr Ala
   1105                1110                1115                1120
   Pro Ala Ser Pro Cys Pro Ser Ile Ser Ser Gln Asn Ser Ser Ser Cys
                   1125                1130                1135
   Ser Ser Phe Gln Asp Gln Lys Ile Ala Ser Met Phe Asp Leu Thr Pro
               1140                1145                1150
   Glu Tyr Arg Gln Gln His Phe Leu Thr Gly Leu Leu Phe Thr Glu Leu
           1155                1160                1165
   Ala Val Ala Leu Asp Ala Glu Gly Asp Gly Ile Ser Arg Val Gln Arg
       1170                1175                1180
   Lys Ala Val Ser Ala Ile His Ser Leu Leu Ser Ser His Asp Leu Asp
   1185                1190                1195                1200
   Pro Arg Cys Leu Lys Pro Glu Val Lys Val Lys Ile Ala Ala Leu Tyr
                   1205                1210                1215
   Leu Pro Leu Val Gly Ile Ile Leu Asp Ala Leu Pro Gln Leu Tyr Asp
               1220                1225                1230
   Phe Thr Asp Ala Arg Ser Gly Arg Ser Arg Ala Ser Gly Ser Tyr Glu
           1235                1240                1245
   Glu Gln Asp Val Ala Asn Gly Ile Asn Gln Asn Val Ala Leu Ala Ile
       1250                1255                1260
   Ala Gly Asn His Phe Asn Leu Lys Thr Ser Gly Ala Met Leu Ser Ser
   1265                1270                1275                1280
   Leu Pro Tyr Lys Gln Tyr Asn Met Leu Asn Ala Asp Thr Thr Arg His
                   1285                1290                1295
   Leu Met Val Cys Phe Leu Trp Ile Met Lys Asn Ala Asp Gln Ser Leu
               1300                1305                1310
   Ile Arg Lys Trp Ile Ala Asp Leu Pro Ser Met Gln Leu Asn Arg Ile
           1315                1320                1325
   Leu Asp Leu Leu Phe Ile Cys Val Ser Cys Phe Glu Tyr Lys Gly Lys
       1330                1335                1340
   Gln Ser Ser Asp Lys Val Ser Asn Gln Val Leu Gln Lys Ser Arg Asp
   1345                1350                1355                1360
   Val Lys Ala Lys Leu Glu Glu Ala Leu Leu Arg Gly Glu Gly Ala Arg
                   1365                1370                1375
   Gly Glu Met Met Arg Arg Arg Ile Pro Gly Thr Asp Arg Phe Pro Gly
               1380                1385                1390
   Leu Asn Glu Asn Leu Arg Trp Arg Lys Glu Gln Thr Gln Trp Arg Gln
           1395                1400                1405
   Ala Asn Glu Lys Leu Asp Lys Thr Lys Ala Glu Leu Asp Gln Glu Ala
       1410                1415                1420
   Leu Ile Ser Gly Asn Leu Ala Thr Glu Ala Asn Leu Ile Ile Leu Asp
   1425                1430                1435                1440
   Met Gln Glu Asn Ile Ile Gln Ala Ser Ser Ser Leu Asp Cys Lys Asp
                   1445                1450                1455
   Ser Leu Leu Gly Gly Val Leu Arg Val Leu Val Asn Ser Leu Ser Cys
               1460                1465                1470
   Asp Gln Ser Thr Thr Tyr Leu Thr His Cys Phe Ala Thr Leu Arg Ala
           1475                1480                1485
   Leu Ile Ala Lys Phe Gly Asp Leu Leu Phe Glu Glu Glu Met Glu Gln
       1490                1495                1500
   Cys Ala Asp Leu Cys Gln Arg Val Leu His His Cys Ser Ser Ser Met
   1505                1510                1515                1520
   Asp Val Thr Arg Ser Gln Ala Cys Ala Thr Leu Tyr Leu Leu Met Arg
                   1525                1530                1535
   Phe Ser Phe Gly Ala Thr Ser Asn Phe Ala Arg Val Lys Met Gln Val
               1540                1545                1550
   Thr Met Ala Leu Ala Ser Leu Val Gly Lys Ala Pro Asp Phe Asn Glu
   1555 1560 1565
   Glu His Leu Arg Lys Ser Leu Arg Thr Ile Leu Ala Tyr Ser Glu Glu
   1570 1575 1580
   Asp Thr Ala Met Arg Ala Thr Leu Phe Pro Thr Gln Val Glu Glu Leu
   1585 1590 1595 1600
   Leu Cys Asn Leu Asn Ser Ile Leu Tyr Asp Thr Val Lys Met Arg Glu
   1605 1610 1615
   Phe Gln Glu Asp Pro Glu Met Leu Met Asp Leu Met Tyr Arg Ile Ala
   1620 1625 1630
   Lys Ser Tyr Gln Ala Ser Pro Asp Leu Arg Leu Thr Trp Leu Gln Asn
   1635 1640 1645
   Met Ala Glu Lys His Thr Lys Arg Lys Cys Phe Thr Glu Ala Ala Met
   1650 1655 1660
   Cys Leu Val His Ala Ala Ala Leu Val Ala Glu Tyr Leu Ser Met Leu
   1665 1670 1675 1680
   Glu Asp His Ser Tyr Leu Pro Val Gly Ser Val Ser Phe Gln Asn Ile
   1685 1690 1695
   Ser Ser Asn Val Leu Glu Glu Ser Ala Val Ser Asp Asp Thr Leu Ser
   1700 1705 1710
   Pro Asp Glu Asp Gly Val Cys Ser Gly Arg Tyr Phe Thr Glu Ser Gly
   1715 1720 1725
   Leu Val Gly Leu Leu Glu Gln Ala Ala Glu Leu Phe Ser Thr Gly Gly
   1730 1735 1740
   Leu Tyr Glu Thr Val Asn Glu Val Tyr Lys Leu Val Ile Pro Ile Leu
   1745 1750 1755 1760
   Glu Ala His Arg Asp Phe Arg Lys Leu Thr Ser Thr His Asp Lys Leu
   1765 1770 1775
   Gln Lys Ala Phe Asp Asn Ile Ile Asn Lys Asp His Lys Arg Met Phe
   1780 1785 1790
   Gly Thr Tyr Phe Arg Val Gly Phe Tyr Gly Ser Lys Phe Gly Asp Leu
   1795 1800 1805
   Asp Glu Gln Glu Phe Val Tyr Lys Glu Pro Ala Ile Thr Lys Leu Pro
   1810 1815 1820
   Glu Ile Ser His Arg Leu Glu Gly Phe Tyr Gly Gln Cys Phe Gly Ala
   1825 1830 1835 1840
   Glu Phe Val Glu Val Ile Lys Asp Ser Thr Pro Val Asp Lys Thr Lys
   1845 1850 1855
   Leu Asp Pro Asn Lys Ala Tyr Ile Gln Ile Thr Phe Val Glu Pro Tyr
   1860 1865 1870
   Phe Asp Glu Tyr Glu Met Lys Asp Arg Val Thr Tyr Phe Glu Lys Asn
   1875 1880 1885
   Phe Asn Leu Arg Arg Phe Met Tyr Thr Thr Pro Phe Thr Leu Glu Gly
   1890 1895 1900
   Arg Pro Arg Gly Glu Leu His Glu Gln His Arg Arg Asn Thr Val Leu
   1905 1910 1915 1920
   Thr Thr Met His Ala Phe Pro Tyr Ile Lys Thr Arg Ile Arg Val Ser
   1925 1930 1935
   Gln Lys Glu Glu Phe Val Leu Thr Pro Ile Glu Val Ala Ile Glu Asp
   1940 1945 1950
   Met Lys Lys Lys Thr Leu Gln Leu Ala Val Ala Thr His Gln Glu Pro
   1955 1960 1965
   Pro Asp Ala Lys Met Leu Gln Met Val Leu Gln Gly Ser Val Gly Ala
   1970 1975 1980
   Thr Val Asn Gln Gly Pro Leu Glu Val Ala Gln Val Phe Leu Ala Glu
   1985 1990 1995 2000
   Ile Pro Ala Asp Pro Lys Leu Tyr Arg His His Asn Lys Leu Arg Leu
   2005 2010 2015
   Cys Phe Lys Glu Phe Ile Met Arg Cys Gly Glu Ala Val Glu Lys Asn
   2020 2025 2030
   Arg Arg Leu Ile Thr Ala Glu Gln Arg Glu Tyr Gln Gln Glu Leu Lys
   2035 2040 2045
   Lys Asn Tyr Asn Arg Leu Lys Asp Ser Leu Arg Pro Met Ile Glu Arg
   2050 2055 2060
   Lys Ile Pro Glu Leu Tyr Lys Pro Ile Phe Arg Val Asp Ser Gln Lys
   2065                2070                2075                2080
   Arg Asp Ser Phe His Arg Ser Ser Phe Arg Lys Cys Glu Thr Gln Leu
                   2085                2090                2095
   Ser Gln Gly Ser
               2100
<210> 12
   <211> 2100
   <212> PRT
   <213> Mus musculus
<220>
   <223> Dock8 Q1865E
<220>
   <221> SITE
   <222> 1865
   <223> Q1865E"
<220>
   <221> CHAIN
   <222> 1633..2071
   <223> DHR2 region
<220>
   <221> CHAIN
   <222> 1862..1940
   <223> amino acids corresponding to exon 44
<220>
   <221> CHAIN
   <222> 1862..1868
   <223> Î²4 region
<400> 12
   Met Ala Thr Leu Pro Ser Ala Glu Arg Arg Ala Phe Ala Leu Lys Ile
   1               5                   10                  15
   Asn Arg Tyr Ser Ser Ser Glu Ile Arg Lys Gln Phe Thr Leu Pro Pro
               20                  25                  30
   Asn Leu Gly Gln Tyr His Arg His Ser Ile Ser Thr Ser Gly Phe Pro
           35                  40                  45
   Ser Leu Gln Leu Pro Gln Leu Tyr Glu Pro Val Glu Pro Val Asp Phe
       50                  55                  60
   Glu Gly Leu Val Met Thr His Leu Asn Ser Leu Asp Ala Glu Leu Ala
   65                  70                  75                  80
   Gln Glu Leu Gly Asp Leu Thr Asp Asp Asp Leu His Val Ala Phe Thr
                   85                  90                  95
   Pro Lys Glu Cys Arg Thr Leu Gln His Ser Leu Pro Glu Glu Gly Val
               100                 105                 110
   Glu Leu Asp Pro His Val Arg Asp Cys Val Gln Thr Tyr Ile Arg Glu
           115                 120                 125
   Trp Leu Ile Val Asn Arg Lys Asn Gln Gly Ser Ser Glu Phe Cys Ser
       130                 135                 140
   Phe Lys Lys Thr Gly Ser Arg Arg Asp Phe Gln Lys Thr Leu Gln Lys
   145                 150                 155                 160
   Gln Thr Phe Glu Ser Glu Thr Leu Glu Cys Ser Glu Pro Asp Thr Gln
                   165                 170                 175
   Thr Gly Pro Arg His Pro Leu Asn Val Leu Cys Asp Val Ser Gly Lys
               180                 185                 190
   Gly Pro Leu Thr Ser Cys Asp Phe Asp Leu Arg Ser Leu Gln Pro Asp
           195                 200                 205
   Glu Arg Leu Glu Asn Leu Leu Gln Leu Val Ser Ala Glu Asp Phe Glu
       210                 215                 220
   Lys Glu Lys Glu Glu Ala Arg Lys Thr Asn Arg Pro Ala Glu Leu Phe
   225                 230                 235                 240
   Ala Leu Tyr Pro Pro Val Asp Glu Glu Asp Ala Val Glu Ile Arg Pro
                   245                 250                 255
   Val Pro Glu Cys Pro Lys Glu His Leu Gly Asn Arg Ile Leu Val Lys
               260                 265                 270
   Val Leu Thr Leu Lys Phe Glu Ile Glu Ile Glu Pro Leu Phe Ala Ser
           275                 280                 285
   Ile Ala Leu Tyr Asp Val Lys Glu Arg Lys Lys Ile Ser Glu Asn Phe
       290                 295                 300
   His Cys Asp Leu Asn Ser Asp Gln Phe Lys Gly Phe Leu Arg Ala His
   305                 310                 315                 320
   Thr Pro Ser Ile Asp Pro Ser Ser Gln Ala Arg Ser Ala Val Phe Ser
                   325                 330                 335
   Val Thr Tyr Pro Ser Ser Asp Ile Tyr Leu Val Val Lys Ile Glu Lys
               340                 345                 350
   Val Leu Gln Gln Gly Glu Ile Ala Asp Cys Ala Glu Pro Tyr Met Ile
           355                 360                 365
   Ile Lys Glu Ser Asp Gly Gly Lys Ser Lys Glu Lys Val Glu Lys Leu
       370                 375                 380
   Lys Leu Gln Ala Glu Ser Phe Cys Gln Arg Leu Gly Lys Tyr Arg Met
   385                 390                 395                 400
   Pro Phe Ala Trp Ala Pro Ile Ser Leu Ala Ser Phe Phe Asn Ile Ser
                   405                 410                 415
   Thr Leu Glu Arg Glu Ser Thr Asp Val Glu Pro Gly Val Gly Arg Asn
               420                 425                 430
   Ser Val Gly Glu Lys Arg Ser Leu Ser Gln Ser Arg Arg Pro Ser Glu
           435                 440                 445
   Arg Thr Leu Ser Leu Glu Glu Asn Gly Val Gly Ser Asn Phe Lys Ala
       450                 455                 460
   Thr Thr Leu Ala Thr Asn Ile Phe Phe Lys Gln Glu Gly Asp Arg Leu
   465                 470                 475                 480
   Ser Asp Glu Asp Leu Phe Lys Phe Leu Ala Asp Tyr Lys Arg Ser Ser
                   485                 490                 495
   Ser Leu Gln Arg Arg Val Lys Ser Ile Pro Gly Ser Leu Arg Leu Glu
               500                 505                 510
   Ile Ser Pro Ala Pro Asp Val Met Asn Cys Cys Leu Thr Pro Glu Met
           515                 520                 525
   Leu Pro Val Lys Pro Phe Pro Glu Asn Arg Thr Arg Pro His Lys Glu
       530                 535                 540
   Ile Leu Glu Phe Pro Ile Arg Glu Val Tyr Val Pro His Thr Val Tyr
   545                 550                 555                 560
   Arg Asn Leu Leu Tyr Val Tyr Pro Gln Arg Leu Asn Phe Ala Ser Lys
                   565                 570                 575
   Leu Ala Ser Ala Arg Asn Ile Thr Ile Lys Ile Gln Phe Met Cys Gly
               580                 585                 590
   Glu Asp Pro Ser Asn Ala Met Pro Val Ile Phe Gly Lys Ser Ser Gly
           595                 600                 605
   Pro Glu Phe Leu Gln Glu Val Tyr Thr Ala Ile Thr Tyr His Asn Lys
       610                 615                 620
   Ser Pro Asp Phe Tyr Glu Glu Val Lys Ile Lys Leu Pro Ala Lys Leu
   625                 630                 635                 640
   Thr Val Asn His His Leu Leu Phe Thr Phe Tyr His Ile Ser Cys Gln
                   645                 650                 655
   Gln Lys Gln Gly Ala Ser Gly Glu Ser Leu Leu Gly Tyr Ser Trp Leu
               660                 665                 670
   Pro Ile Leu Leu Asn Glu Arg Leu Gln Thr Gly Ser Tyr Cys Leu Pro
           675                 680                 685
   Val Ala Leu Glu Lys Leu Pro Pro Asn Tyr Ser Ile His Ser Ala Glu
       690                 695                 700
   Lys Val Pro Leu Gln Asn Pro Pro Ile Lys Trp Ala Glu Gly His Lys
   705                 710                 715                 720
   Gly Val Phe Asn Ile Glu Val Gln Ala Val Ser Ser Val His Thr Gln
                   725                 730                 735
   Asp Asn His Leu Glu Lys Phe Phe Thr Leu Cys His Ser Leu Glu Ser
               740                 745                 750
   Gln Val Ser Phe Pro Ile Arg Val Leu Asp Gln Lys Ile Thr Glu Ser
           755                 760                 765
   Thr Leu Glu His Glu Leu Lys Leu Ser Ile Ile Cys Leu Asn Ser Ser
       770                 775                 780
   Arg Leu Glu Pro Leu Val Leu Phe Leu His Leu Val Leu Asp Lys Leu
   785                 790                 795                 800
   Phe Gln Leu Ser Val Gln Pro Met Val Ile Ala Gly Gln Thr Ala Asn
                   805                 810                 815
   Phe Ser Gln Phe Ala Phe Glu Ser Val Val Ala Ile Ala Asn Ser Leu
               820                 825                 830
   His Asn Ser Lys Asp Leu Arg Lys Asp Gln His Gly Arg Asn Cys Leu
           835                 840                 845
   Leu Ala Ser Tyr Val His Tyr Val Phe Arg Leu Pro Glu Leu His Arg
       850                 855                 860
   Asp Thr Ser Lys Ser Gly Gly Pro Thr Thr Val Val Pro Asp Pro Arg
   865                 870                 875                 880
   Tyr His Thr Tyr Gly Arg Thr Ser Ala Ala Ala Val Ser Ser Lys Leu
                   885                 890                 895
   Met Gln Ala Arg Val Met Ser Ser Ser Asn Pro Asp Leu Thr Gly Ser
               900                 905                 910
   His Cys Ala Ala Asp Glu Glu Val Lys Asn Ile Met Ser Ser Lys Ile
           915                 920                 925
   Ala Asp Arg Asn Cys Ser Arg Met Ser Tyr Tyr Cys Ser Gly Asn Ser
       930                 935                 940
   Asp Ala Pro Gly Ser Thr Ala Ala Pro Arg Pro Val Ser Lys Lys His
   945                 950                 955                 960
   Phe His Glu Glu Leu Ala Leu Gln Met Val Val Ser Thr Gly Val Val
                   965                 970                 975
   Arg Glu Thr Val Phe Lys Tyr Ala Trp Phe Phe Phe Glu Leu Leu Val
               980                 985                 990
   Lys Ser Met Ala Gln Tyr Val His Asn Leu Asp Lys Arg Asp Ser Phe
           995                 1000                1005
   Arg Arg Thr Arg Phe Ser Asp Arg Phe Lys Asp Asp Ile Thr Thr Ile
       1010                1015                1020
   Val Asn Val Val Thr Ser Glu Ile Ala Ala Leu Leu Val Lys Pro Gln
   1025                1030                1035                1040
   Lys Glu Ser Glu Gln Ala Glu Lys Ile Asn Ile Ser Leu Ala Phe Phe
                   1045                1050                1055
   Leu Tyr Asp Leu Leu Ser Ile Met Asp Arg Gly Phe Val Phe Asn Leu
               1060                1065                1070
   Ile Lys His Tyr Cys Ser Gln Leu Ser Ala Lys Leu Asn Ile Leu Pro
           1075                1080                1085
   Thr Leu Ile Ser Met Arg Leu Glu Phe Leu Arg Ile Leu Cys Ser His
       1090                1095                1100
   Glu His Tyr Leu Asn Leu Asn Leu Leu Phe Met Asn Thr Asp Thr Ala
   1105                1110                1115                1120
   Pro Ala Ser Pro Cys Pro Ser Ile Ser Ser Gln Asn Ser Ser Ser Cys
                   1125                1130                1135
   Ser Ser Phe Gln Asp Gln Lys Ile Ala Ser Met Phe Asp Leu Thr Pro
               1140                1145                1150
   Glu Tyr Arg Gln Gln His Phe Leu Thr Gly Leu Leu Phe Thr Glu Leu
           1155                1160                1165
   Ala Val Ala Leu Asp Ala Glu Gly Asp Gly Ile Ser Arg Val Gln Arg
       1170                1175                1180
   Lys Ala Val Ser Ala Ile His Ser Leu Leu Cys Ser His Asp Leu Asp
   1185                1190                1195                1200
   Pro Arg Cys Arg Lys Pro Glu Val Lys Val Lys Ile Ala Ala Leu Tyr
                   1205                1210                1215
   Leu Pro Leu Val Gly Ile Ile Leu Asp Ala Leu Pro Gln Leu Tyr Asp
               1220                1225                1230
   Phe Thr Asp Ala Arg Ser Gly Arg Ser Arg Ala Ser Gly Ser Tyr Glu
           1235                1240                1245
   Glu Gln Asp Val Ala Asn Gly Ile Asn Gln Asn Val Ala Leu Ala Ile
       1250                1255                1260
   Ala Gly Asn His Phe Asn Leu Lys Thr Ser Gly Ala Met Leu Ser Ser
   1265                1270                1275                1280
   Leu Pro Tyr Lys Gln Tyr Asn Met Leu Asn Ala Asp Thr Thr Arg His
                   1285                1290                1295
   Leu Met Ile Cys Phe Leu Trp Ile Met Lys Asn Ala Asp Gln Ser Leu
               1300                1305                1310
   Ile Arg Lys Trp Ile Ala Asp Leu Pro Ser Met Gln Leu Asn Arg Ile
           1315                1320                1325
   Leu Asp Leu Leu Phe Ile Cys Val Ser Cys Phe Glu Tyr Lys Gly Lys
       1330                1335                1340
   Gln Ser Ser Asp Lys Val Ser Asn Gln Val Leu Gln Lys Ser Arg Asp
   1345                1350                1355                1360
   Val Lys Ala Lys Leu Glu Glu Ala Leu Leu Arg Gly Glu Gly Ala Arg
                   1365                1370                1375
   Gly Glu Met Met Arg Arg Arg Ile Pro Gly Thr Asp Arg Phe Pro Gly
               1380                1385                1390
   Ile Asn Glu Asn Leu Arg Trp Arg Lys Glu Gln Thr Gln Trp Arg Gln
           1395                1400                1405
   Ala Asn Glu Lys Leu Asp Lys Thr Lys Ala Glu Leu Asp Gln Glu Ala
       1410                1415                1420
   Leu Ile Ser Gly Asn Leu Ala Thr Glu Ala Asn Leu Ile Ile Leu Asp
   1425                1430                1435                1440
   Met Gln Glu Asn Ile Ile Gln Ala Ser Ser Ala Leu Asp Cys Lys Asp
                   1445                1450                1455
   Ser Leu Leu Gly Gly Val Leu Arg Val Leu Val Asn Ser Leu Ser Cys
               1460                1465                1470
   Asp Gln Ser Thr Thr Tyr Leu Thr His Cys Phe Ala Thr Leu Arg Ala
           1475                1480                1485
   Leu Ile Ala Lys Phe Gly Asp Leu Leu Phe Glu Glu Glu Met Glu Gln
       1490                1495                1500
   Cys Ala Asp Leu Cys Gln Arg Val Leu His His Cys Ser Ser Ser Met
   1505                1510                1515                1520
   Asp Val Thr Arg Ser Gln Ala Cys Ala Thr Leu Tyr Leu Leu Met Arg
                   1525                1530                1535
   Phe Ser Phe Gly Ala Thr Ser Asn Phe Ala Arg Val Lys Met Gln Val
               1540                1545                1550
   Thr Met Ala Leu Ala Ser Leu Val Gly Lys Ala Pro Asp Phe Asn Glu
           1555                1560                1565
   Glu His Leu Arg Arg Ser Leu Arg Thr Ile Leu Ala Tyr Ser Glu Glu
       1570                1575                1580
   Asp Thr Ala Met Gln Thr Thr Pro Phe Pro Met Gln Val Glu Glu Leu
   1585                1590                1595                1600
   Leu Cys Asn Leu Asn Ser Ile Leu Tyr Asp Thr Val Lys Met Arg Glu
                   1605                1610                1615
   Phe Gln Glu Asp Pro Glu Met Leu Met Asp Leu Met Tyr Arg Ile Ala
               1620                1625                1630
   Lys Ser Tyr Gln Ala Ser Pro Asp Leu Arg Leu Thr Trp Leu Gln Asn
           1635                1640                1645
   Met Ala Glu Lys His Thr Lys Lys Lys Cys Phe Thr Glu Ala Ala Met
       1650                1655                1660
   Cys Leu Val His Ala Ala Ala Leu Val Ala Glu Tyr Leu Ser Met Leu
   1665                1670                1675                1680
   Glu Asp His Ser Tyr Leu Pro Val Gly Ser Val Ser Phe Gln Asn Ile
                   1685                1690                1695
   Ser Ser Asn Val Leu Glu Glu Ser Ala Val Ser Asp Asp Thr Leu Ser
               1700                1705                1710
   Pro Asp Glu Asp Gly Val Cys Ser Gly Arg Tyr Phe Thr Glu Ser Gly
           1715                1720                1725
   Leu Val Gly Leu Leu Glu Gln Ala Ala Glu Leu Phe Ser Thr Gly Gly
       1730                1735                1740
   Leu Tyr Glu Thr Val Asn Glu Val Tyr Lys Leu Val Ile Pro Ile Leu
   1745                1750                1755                1760
   Glu Ala His Arg Asp Phe Arg Lys Leu Thr Ser Thr His Asp Lys Leu
                   1765                1770                1775
   Gln Lys Ala Phe Asp Asn Ile Ile Asn Lys Asp His Lys Arg Met Phe
               1780                1785                1790
   Gly Thr Tyr Phe Arg Val Gly Phe Tyr Gly Ser Arg Phe Gly Asp Leu
           1795                1800                1805
   Asp Glu Gln Glu Phe Val Tyr Lys Glu Pro Ala Ile Thr Lys Leu Pro
       1810                1815                1820
   Glu Ile Ser His Arg Leu Glu Gly Phe Tyr Gly Gln Cys Phe Gly Ala
   1825                1830                1835                1840
   Glu Phe Val Glu Val Ile Lys Asp Ser Thr Pro Val Asp Lys Thr Lys
                   1845                1850                1855
   Leu Asp Pro Asn Lys Ala Tyr Ile Glu Ile Thr Phe Val Glu Pro Tyr
               1860                1865                1870
   Phe Asp Glu Tyr Glu Met Lys Asp Arg Val Thr Tyr Phe Glu Lys Asn
           1875                1880                1885
   Phe Asn Leu Arg Arg Phe Met Tyr Thr Thr Pro Phe Thr Leu Glu Gly
       1890                1895                1900
   Arg Pro Arg Gly Glu Leu His Glu Gln His Arg Arg Asn Thr Val Leu
   1905                1910                1915                1920
   Thr Thr Met His Ala Phe Pro Tyr Ile Lys Thr Arg Ile Arg Val Ser
                   1925                1930                1935
   Gln Lys Glu Glu Phe Val Leu Thr Pro Ile Glu Val Ala Ile Glu Asp
               1940                1945                1950
   Met Lys Lys Lys Thr Leu Gln Leu Ala Val Ala Thr His Gln Glu Pro
           1955                1960                1965
   Pro Asp Ala Lys Met Leu Gln Met Val Leu Gln Gly Ser Val Gly Ala
       1970                1975                1980
   Thr Val Asn Gln Gly Pro Leu Glu Val Ala Gln Val Phe Leu Ala Glu
   1985                1990                1995                2000
   Ile Pro Ala Asp Pro Lys Leu Tyr Arg His His Asn Lys Leu Arg Leu
                   2005                2010                2015
   Cys Phe Lys Glu Phe Ile Met Arg Cys Gly Glu Ala Val Glu Lys Asn
               2020                2025                2030
   Arg Arg Leu Ile Thr Ala Glu Gln Arg Glu Tyr Gln Gln Glu Leu Lys
           2035                2040                2045
   Lys Asn Tyr Asn Lys Leu Arg Asp Ser Leu Arg Pro Met Ile Glu Arg
       2050                2055                2060
   Lys Ile Pro Glu Leu Tyr Lys Pro Ile Phe Arg Val Asp Ser Gln Lys
   2065                2070                2075                2080
   Arg Asp Ser Phe His Arg Ser Ser Phe Arg Lys Cys Glu Thr Gln Leu
                   2085                2090                2095
   Ser Gln Gly Ser
               2100
<210> 13
   <211> 24
   <212> DNA
   <213> Artificial Sequence
<220>
   <221> source
   <222> 1..24
   <223> /mol_type="unassigned DNA" /note="section of exon 11 of Vwa2 gene" /organism="Artificial Sequence"
<400> 13
   aggcttgctc cacgggacta cctg 24
<210> 14
   <211> 24
   <212> DNA
   <213> Artificial Sequence
<220>
   <221> source
   <222> 1..24
   <223> /mol_type="unassigned DNA" /note="section of exon 11 of Vwa2 gene" /organism="Artificial Sequence"
<400> 14
   aggcttgctc catgggacta cctg 24
<210> 15
   <211> 24
   <212> DNA
   <213> Artificial Sequence
<220>
   <221> source
   <222> 1..24
   <223> /mol_type="unassigned DNA" /note="section of exon 11 of Vwa2 gene" /organism="Artificial Sequence"
<400> 15
   aggcttgctc catgggacta cctg 24
<210> 16
   <211> 24
   <212> DNA
   <213> Artificial Sequence
<220>
   <221> source
   <222> 1..24
   <223> /mol_type="unassigned DNA" /note="section of exon 11 of Vwa2 gene" /organism="Artificial Sequence"
<400> 16
   aggcttgctc catgggacta cctg 24
<210> 17
   <211> 183
   <212> PRT
   <213> Artificial Sequence
<220>
   <223> amino acids encoded by exon 11 of Vwa2 of rat
<220>
   <221> SITE
   <222> 159
   <223> wild-type =R
<210> 18
   <211> 184
   <212> PRT
   <213> Artificial Sequence
<220>
   <223> amino acids encoded by exon 11 of Vwa2 of mouse
<220>
   <221> SITE
   <222> 159
   <223> wild-type = R
<400> 18
   Gly Cys Gln Ala Gln Ser Leu Asp Leu Val Phe Leu Leu Asp Ala Ser
   1               5                   10                  15
   Ala Ser Val Gly Arg Glu Asn Phe Ala Gln Met Gln Ser Phe Ile Arg
               20                  25                  30
   Lys Cys Thr Leu Arg Phe Asp Val Asn Pro Asp Val Thr Gln Val Gly
           35                  40                  45
   Leu Val Val Tyr Gly Ser Arg Val Gln Thr Ala Phe Gly Leu Asp Thr
       50                  55                  60
   His Pro Thr Arg Ala Ala Val Leu Arg Ala Met Ser Gln Ala Pro Tyr
   65                  70                  75                  80
   Leu Gly Gly Val Gly Ser Ala Gly Thr Ala Leu Leu His Ile Glu Asp
                   85                  90                  95
   Lys Val Met Thr Val Gln Arg Gly Ala Arg Pro Gly Val Pro Lys Ala
               100                 105                 110
   Val Val Met Leu Thr Gly Gly Ser Gly Ala Glu Asp Ala Ala Val Pro
           115                 120                 125
   Ala Gln Lys Leu Arg Gly Asn Gly Ile Ser Val Leu Val Met Ser Val
       130                 135                 140
   Gly Ala Val Leu Arg Glu Ala Val Arg Arg Leu Ala Gly Pro Arg Asp
   145                 150                 155                 160
   Ser Leu Ile His Val Ala Ala Tyr Thr Asp Leu Pro Tyr His Gln Asp
                   165                 170                 175
   Met Leu Ile Glu Trp Leu Cys Arg
               180
<210> 19
   <211> 183
   <212> PRT
   <213> Artificial Sequence
<220>
   <223> amino acids encoded by exon 11 of Vwa2 of human origin""
<220>
   <221> SITE
   <222> 159
   <223> wild-type = R
<210> 20
   <211> 715
   <212> PRT
   <213> Rattus
<220>
   <223> Vwa2
<220>
   <221> CHAIN
   <222> 522..706
   <223> amino acids encoded by exon 11
<220>
   <221> SITE
   <222> 681
   <223> wild-type = R
<400> 20
   Met Pro Pro Tyr Leu Phe Leu Pro Ala Ile Tyr Thr Leu Leu Phe Ser
   1               5                   10                  15
   Arg Val Ser Leu Ser Ser Ser Leu Gln Glu Val His Val Ser Arg Glu
   20 25 30
   Thr Thr Gly Lys Ile Ala Val Ala Ser Lys Leu Ile Trp Cys Ser Ala
   35 40 45
   Ala Val Asp Ile Leu Phe Leu Leu Asp Gly Ser His Ser Ile Gly Lys
   50 55 60
   Gly Ser Phe Glu Arg Ser Lys Arg Phe Ala Thr Ala Leu Cys Asp Ala
   65 70 75 80
   Leu Asp Ile Ser Pro Gly Arg Val Arg Val Gly Ala Leu Gln Phe Gly
   85 90 95
   Ser Thr Pro His Leu Glu Phe Pro Leu Asp Ala Phe Ser Thr Arg Gln
   100 105 110
   Glu Val Lys Glu Ile Ile Lys Lys Leu Val Phe Lys Gly Gly Arg Thr
   115 120 125
   Glu Thr Gly Leu Ala Leu Lys His Leu Leu Arg Gly Leu Pro Gly Gly
   130 135 140
   Arg Asn Gly Ser Val Pro Gln Ile Leu Ile Ile Ile Thr Asp Gly Lys
   145 150 155 160
   Ser Gln Gly His Val Ala Leu Pro Ala Lys Gln Leu Lys Glu Arg Gly
   165 170 175
   Ile Val Val Phe Ala Val Gly Val Arg Phe Pro Arg Trp Asp Glu Leu
   180 185 190
   Leu Thr Leu Ala Ser Glu Pro Lys Glu Gln His Val Leu Leu Ala Glu
   195 200 205
   Gln Val Glu Asp Ala Thr Asn Gly Leu Leu Ser Thr Leu Ser Ser Ser
   210 215 220
   Leu Leu Cys Thr Thr Ala Asp Pro Asp Cys Arg Val Glu Ala Tyr Pro
   225 230 235 240
   Cys Glu Arg Arg Thr Leu Glu Thr Met Arg Glu Leu Ala Ser Asn Ala
   245 250 255
   Leu Cys Trp Arg Gly Ser Arg Gln Ala Asp Thr Val Leu Ala Val Pro
   260 265 270
   Cys Pro Phe Tyr Ser Trp Lys Arg Val Phe Gln Thr His Pro Ala Asn
   275 280 285
   Cys Tyr Arg Thr Ile Cys Pro Gly Pro Cys Asp Ser Gln Pro Cys Gln
   290 295 300
   Asn Gly Gly Thr Cys Ile Pro Glu Gly Val Asp Arg Tyr His Cys Leu
   305 310 315 320
   Cys Pro Leu Ala Phe Gly Gly Glu Val Asn Cys Ala Pro Lys Leu Ser
   325 330 335
   Leu Glu Cys Arg Ile Asp Val Leu Phe Leu Leu Asp Ser Ser Ala Gly
   340 345 350
   Thr Thr Pro Glu Gly Phe Arg Gln Ala Lys Ala Phe Val Lys Arg Phe
   355 360 365
   Val Gln Ala Val Leu Ser Glu Asp Ser Arg Ala Arg Val Gly Val Ala
   370 375 380
   Ser Tyr Ser Arg Asp Leu Met Val Ala Val Pro Val Gly Glu Tyr Gln
   385 390 395 400
   His Val Pro Asp Leu Val Arg Ile Leu Asp Ser Ile Pro Phe Ser Gly
   405 410 415
   Gly Pro Thr Leu Thr Gly Ser Ala Leu Leu Gln Val Thr Glu His Gly
   420 425 430
   Phe Gly Ser Ala Ser Arg Thr Gly Gln Asp Arg Pro Arg Arg Val Val
   435 440 445
   Val Leu Leu Thr Glu Ser His Ala Gln Asp Glu Val Ala Gly Pro Ala
   450 455 460
   Asp His Ala Arg Ala Arg Glu Leu Leu Leu Leu Gly Met Gly Asn Glu
   465 470 475 480
   Leu Met Arg Ala Glu Leu Glu Arg Ile Thr Gly Ser Arg Lys His Val
   485 490 495
   Ile Ile His Thr Asp Pro Arg Asp Met Phe Ser Gln Ile Pro Glu Leu
   500 505 510
   Gln Arg Arg Leu Cys Ser Gln Pro Arg Pro Gly Cys Gln Ala Gln Ser
   515 520 525
   Leu Asp Leu Val Phe Leu Leu Asp Ala Ser Ala Ser Val Gly Gly Glu
       530                 535                 540
   Asn Phe Ala Gln Met Gln Ser Phe Ile Arg Lys Cys Thr Leu Arg Phe
   545                 550                 555                 560
   Asp Val Asn Pro Asp Val Thr Gln Val Gly Leu Val Val Tyr Gly Asn
                   565                 570                 575
   Gln Val Gln Thr Ala Phe Gly Leu Asp Thr His Pro Thr Arg Ala Ser
               580                 585                 590
   Val Leu Arg Ala Met Ser Gln Ala Pro Tyr Leu Gly Gly Val Gly Ser
           595                 600                 605
   Ala Gly Thr Ala Leu Leu His Ile Glu Asp Lys Val Met Thr Val Gln
       610                 615                 620
   Arg Gly Ala Arg Pro Gly Val Pro Lys Ala Val Val Met Leu Thr Gly
   625                 630                 635                 640
   Gly Ser Gly Ala Glu Asp Ala Ala Val Pro Ala Gln Lys Leu Arg Asp
                   645                 650                 655
   Asn Gly Ile Ser Val Leu Val Met Ser Val Gly Ala Val Leu Lys Glu
               660                 665                 670
   Ala Val Arg Arg Leu Ala Gly Pro Arg Asp Tyr Leu Ile His Val Ala
           675                 680                 685
   Ala Tyr Thr Asp Leu Arg Tyr His Gln Asp Thr Leu Ile Glu Trp Leu
       690                 695                 700
   Cys Arg Gly Gln Ala Ala Ser Gln Pro Leu Gln
   705                 710                 715
<210> 21
   <211> 791
   <212> PRT
   <213> Mus musculus
<220>
   <223> Vwa2
<220>
   <221> CHAIN
   <222> 522..706
   <223> amino acids encoded by exon 11
<220>
   <221> SITE
   <222> 681
   <223> wild-type = R
<400> 21
   Met Pro Pro Leu Leu Leu Leu Pro Ala Ile Tyr Met Leu Leu Phe Phe
   1               5                   10                  15
   Arg Val Ser Pro Thr Ile Ser Leu Gln Glu Val His Val Asn Arg Glu
               20                  25                  30
   Thr Met Gly Lys Ile Ala Val Ala Ser Lys Leu Met Trp Cys Ser Ala
           35                  40                  45
   Ala Val Asp Ile Leu Phe Leu Leu Asp Gly Ser His Ser Ile Gly Lys
       50                  55                  60
   Gly Ser Phe Glu Arg Ser Lys Arg Phe Ala Ile Ala Ala Cys Asp Ala
   65                  70                  75                  80
   Leu Asp Ile Ser Pro Gly Arg Val Arg Val Gly Ala Leu Gln Phe Gly
                   85                  90                  95
   Ser Thr Pro His Leu Glu Phe Pro Leu Asp Ser Phe Ser Thr Arg Gln
               100                 105                 110
   Glu Val Lys Glu Ser Ile Lys Gly Ile Val Phe Lys Gly Gly Arg Thr
           115                 120                 125
   Glu Thr Gly Leu Ala Leu Lys Arg Leu Ser Arg Gly Phe Pro Gly Gly
       130                 135                 140
   Arg Asn Gly Ser Val Pro Gln Ile Leu Ile Ile Val Thr Asp Gly Lys
   145                 150                 155                 160
   Ser Gln Gly Pro Val Ala Leu Pro Ala Lys Gln Leu Arg Glu Arg Gly
                   165                 170                 175
   Ile Val Val Phe Ala Val Gly Val Arg Phe Pro Arg Trp Asp Glu Leu
               180                 185                 190
   Leu Thr Leu Ala Ser Glu Pro Lys Asp Arg His Val Leu Leu Ala Glu
           195                 200                 205
   Gln Val Glu Asp Ala Thr Asn Gly Leu Leu Ser Thr Leu Ser Ser Ser
       210                 215                 220
   Ala Leu Cys Thr Thr Ala Asp Pro Asp Cys Arg Val Glu Pro His Pro
   225                 230                 235                 240
   Cys Glu Arg Arg Thr Leu Glu Thr Val Arg Glu Leu Ala Gly Asn Ala
                   245                 250                 255
   Leu Cys Trp Arg Gly Ser Arg Gln Ala Asp Thr Val Leu Ala Leu Pro
               260                 265                 270
   Cys Pro Phe Tyr Ser Trp Lys Arg Val Phe Gln Thr His Pro Ala Asn
           275                 280                 285
   Cys Tyr Arg Thr Ile Cys Pro Gly Pro Cys Asp Ser Gln Pro Cys Gln
       290                 295                 300
   Asn Gly Gly Thr Cys Ile Pro Glu Gly Val Asp Arg Tyr His Cys Leu
   305                 310                 315                 320
   Cys Pro Leu Ala Phe Gly Gly Glu Val Asn Cys Ala Pro Lys Leu Ser
                   325                 330                 335
   Leu Glu Cys Arg Ile Asp Val Leu Phe Leu Leu Asp Ser Ser Ala Gly
               340                 345                 350
   Thr Thr Leu Gly Gly Phe Arg Arg Ala Lys Ala Phe Val Lys Arg Phe
           355                 360                 365
   Val Gln Ala Val Leu Arg Glu Asp Ser Arg Ala Arg Val Gly Ile Ala
       370                 375                 380
   Ser Tyr Gly Arg Asn Leu Met Val Ala Val Pro Val Gly Glu Tyr Gln
   385                 390                 395                 400
   His Val Pro Asp Leu Ile Arg Ser Leu Asp Ser Ile Pro Phe Ser Gly
                   405                 410                 415
   Gly Pro Thr Leu Thr Gly Ser Ala Leu Leu Gln Val Ala Glu His Gly
               420                 425                 430
   Phe Gly Ser Ala Ser Arg Thr Gly Gln Asp Arg Pro Arg Arg Val Val
           435                 440                 445
   Val Leu Leu Thr Glu Ser Arg Ser Gln Asp Glu Val Ser Gly Pro Ala
       450                 455                 460
   Ala His Ala Arg Ala Arg Glu Leu Leu Leu Leu Gly Val Gly Ser Glu
   465                 470                 475                 480
   Ile Leu Gln Ala Glu Leu Val Lys Ile Thr Gly Ser Pro Lys His Val
                   485                 490                 495
   Met Val His Thr Asp Pro Gln Asp Leu Phe Ser Gln Ile Pro Glu Leu
               500                 505                 510
   Gln Arg Arg Leu Cys Ser Gln Pro Arg Pro Gly Cys Gln Ala Gln Ser
           515                 520                 525
   Leu Asp Leu Val Phe Leu Leu Asp Ala Ser Ala Ser Val Gly Arg Glu
       530                 535                 540
   Asn Phe Ala Gln Met Gln Ser Phe Ile Arg Lys Cys Thr Leu Arg Phe
   545                 550                 555                 560
   Asp Val Asn Pro Asp Val Thr Gln Val Gly Leu Val Val Tyr Gly Ser
                   565                 570                 575
   Arg Val Gln Thr Ala Phe Gly Leu Asp Thr His Pro Thr Arg Ala Ala
               580                 585                 590
   Val Leu Arg Ala Met Ser Gln Ala Pro Tyr Leu Gly Gly Val Gly Ser
           595                 600                 605
   Ala Gly Thr Ala Leu Leu His Ile Glu Asp Lys Val Met Thr Val Gln
       610                 615                 620
   Arg Gly Ala Arg Pro Gly Val Pro Lys Ala Val Val Met Leu Thr Gly
   625                 630                 635                 640
   Gly Ser Gly Ala Glu Asp Ala Ala Val Pro Ala Gln Lys Leu Arg Gly
                   645                 650                 655
   Asn Gly Ile Ser Val Leu Val Met Ser Val Gly Ala Val Leu Arg Glu
               660                 665                 670
   Ala Val Arg Arg Leu Ala Gly Pro Arg Asp Ser Leu Ile His Val Ala
           675                 680                 685
   Ala Tyr Thr Asp Leu Pro Tyr His Gln Asp Met Leu Ile Glu Trp Leu
       690                 695                 700
   Cys Arg Glu Ala Arg Leu Pro Val Asn Leu Cys Lys Pro Ser Pro Cys
   705                 710                 715                 720
   Met Asn Glu Gly Thr Cys Val Leu Lys Asn Gly Ser Tyr Arg Cys Glu
                   725                 730                 735
   Cys Arg Gly Gly Trp Glu Gly Pro His Cys Glu Asn Arg Ile Leu Arg
               740                 745                 750
   Gly Asp Ala Pro Met Ala Arg Ser Phe His Gln Glu Pro Ala Gly Leu
           755                 760                 765
   Gln Gly Pro Thr Pro Ser Gln Gln Ala Pro Lys His Leu Arg Ile Gly
       770                 775                 780
   Lys Ala Leu Ser Ser Ala Lys
   785                 790
<210> 22
   <211> 755
   <212> PRT
   <213> Homo sapiens
<220>
   <223> Vwa2
<220>
   <221> CHAIN
   <222> 523..707
   <223> amino acids encoded by exon 11
<220>
   <221> SITE
   <222> 682
   <223> wild-type = R
<400> 22
   Met Pro Pro Phe Leu Leu Leu Glu Ala Val Cys Val Phe Leu Phe Ser
   1               5                   10                  15
   Arg Val Pro Pro Ser Leu Pro Leu Gln Glu Val His Val Ser Lys Glu
               20                  25                  30
   Thr Ile Gly Lys Ile Ser Ala Ala Ser Lys Met Met Trp Cys Ser Ala
           35                  40                  45
   Ala Val Asp Ile Met Phe Leu Leu Asp Gly Ser Asn Ser Val Gly Lys
       50                  55                  60
   Gly Ser Phe Glu Arg Ser Lys His Phe Ala Ile Thr Val Cys Asp Gly
   65                  70                  75                  80
   Leu Asp Ile Ser Pro Glu Arg Val Arg Val Gly Ala Phe Gln Phe Ser
                   85                  90                  95
   Ser Thr Pro His Leu Glu Phe Pro Leu Asp Ser Phe Ser Thr Gln Gln
               100                 105                 110
   Glu Val Lys Ala Arg Ile Lys Arg Met Val Phe Lys Gly Gly Arg Thr
           115                 120                 125
   Glu Thr Glu Leu Ala Leu Lys Tyr Leu Leu His Arg Gly Leu Pro Gly
       130                 135                 140
   Gly Arg Asn Ala Ser Val Pro Gln Ile Leu Ile Ile Val Thr Asp Gly
   145                 150                 155                 160
   Lys Ser Gln Gly Asp Val Ala Leu Pro Ser Lys Gln Leu Lys Glu Arg
                   165                 170                 175
   Gly Val Thr Val Phe Ala Val Gly Val Arg Phe Pro Arg Trp Glu Glu
               180                 185                 190
   Leu His Ala Leu Ala Ser Glu Pro Arg Gly Gln His Val Leu Leu Ala
           195                 200                 205
   Glu Gln Val Glu Asp Ala Thr Asn Gly Leu Phe Ser Thr Leu Ser Ser
       210                 215                 220
   Ser Ala Ile Cys Ser Ser Ala Thr Pro Asp Cys Arg Val Glu Ala His
   225                 230                 235                 240
   Pro Cys Glu His Arg Thr Leu Glu Met Val Arg Glu Phe Ala Gly Asn
                   245                 250                 255
   Ala Pro Cys Trp Arg Gly Ser Arg Arg Thr Leu Ala Val Leu Ala Ala
               260                 265                 270
   His Cys Pro Phe Tyr Ser Trp Lys Arg Val Phe Leu Thr His Pro Ala
           275                 280                 285
   Thr Cys Tyr Arg Thr Thr Cys Pro Gly Pro Cys Asp Ser Gln Pro Cys
       290                 295                 300
   Gln Asn Gly Gly Thr Cys Val Pro Glu Gly Leu Asp Gly Tyr Gln Cys
   305                 310                 315                 320
   Leu Cys Pro Leu Ala Phe Gly Gly Glu Ala Asn Cys Ala Leu Lys Leu
                   325                 330                 335
   Ser Leu Glu Cys Arg Val Asp Leu Leu Phe Leu Leu Asp Ser Ser Ala
               340                 345                 350
   Gly Thr Thr Leu Asp Gly Phe Leu Arg Ala Lys Val Phe Val Lys Arg
           355                 360                 365
   Phe Val Arg Ala Val Leu Ser Glu Asp Ser Arg Ala Arg Val Gly Val
       370                 375                 380
   Ala Thr Tyr Ser Arg Glu Leu Leu Val Ala Val Pro Val Gly Glu Tyr
   385                 390                 395                 400
   Gln Asp Val Pro Asp Leu Val Trp Ser Leu Asp Gly Ile Pro Phe Arg
                   405                 410                 415
   Gly Gly Pro Thr Leu Thr Gly Ser Ala Leu Arg Gln Ala Ala Glu Arg
               420                 425                 430
   Gly Phe Gly Ser Ala Thr Arg Thr Gly Gln Asp Arg Pro Arg Arg Val
           435                 440                 445
   Val Val Leu Leu Thr Glu Ser His Ser Glu Asp Glu Val Ala Gly Pro
       450                 455                 460
   Ala Arg His Ala Arg Ala Arg Glu Leu Leu Leu Leu Gly Val Gly Ser
   465                 470                 475                 480
   Glu Ala Val Arg Ala Glu Leu Glu Glu Ile Thr Gly Ser Pro Lys His
                   485                 490                 495
   Val Met Val Tyr Ser Asp Pro Gln Asp Leu Phe Asn Gln Ile Pro Glu
               500                 505                 510
   Leu Gln Gly Lys Leu Cys Ser Arg Gln Arg Pro Gly Cys Arg Thr Gln
           515                 520                 525
   Ala Leu Asp Leu Val Phe Met Leu Asp Thr Ser Ala Ser Val Gly Pro
       530                 535                 540
   Glu Asn Phe Ala Gln Met Gln Ser Phe Val Arg Ser Cys Ala Leu Gln
   545                 550                 555                 560
   Phe Glu Val Asn Pro Asp Val Thr Gln Val Gly Leu Val Val Tyr Gly
                   565                 570                 575
   Ser Gln Val Gln Thr Ala Phe Gly Leu Asp Thr Lys Pro Thr Arg Ala
               580                 585                 590
   Ala Met Leu Arg Ala Ile Ser Gln Ala Pro Tyr Leu Gly Gly Val Gly
           595                 600                 605
   Ser Ala Gly Thr Ala Leu Leu His Ile Tyr Asp Lys Val Met Thr Val
       610                 615                 620
   Gln Arg Gly Ala Arg Pro Gly Val Pro Lys Ala Val Val Val Leu Thr
   625                 630                 635                 640
   Gly Gly Arg Gly Ala Glu Asp Ala Ala Val Pro Ala Gln Lys Leu Arg
                   645                 650                 655
   Asn Asn Gly Ile Ser Val Leu Val Val Gly Val Gly Pro Val Leu Ser
               660                 665                 670
   Glu Gly Leu Arg Arg Leu Ala Gly Pro Arg Asp Ser Leu Ile His Val
           675                 680                 685
   Ala Ala Tyr Ala Asp Leu Arg Tyr His Gln Asp Val Leu Ile Glu Trp
       690                 695                 700
   Leu Cys Gly Glu Ala Lys Gln Pro Val Asn Leu Cys Lys Pro Ser Pro
   705                 710                 715                 720
   Cys Met Asn Glu Gly Ser Cys Val Leu Gln Asn Gly Ser Tyr Arg Cys
                   725                 730                 735
   Lys Cys Arg Asp Gly Trp Glu Gly Pro His Cys Glu Asn Arg Phe Leu
               740                 745                 750
   Arg Arg Pro
           755
<210> 23
   <211> 202904
   <212> DNA
   <213> Mus musculus
<220>
   <221> source
   <222> 1..202904
   <223> /mol_type="unassigned DNA" /note="mutant Dock8 gene" /organism="Mus musculus"
<220>
   <221> variation
   <222> 188818
   <223> /note=" wild-type C"
<220>
   <221> exon
   <222> 188809..189045
   <223> /note="exon 44"
<220>
   <221> misc_feature
   <222> 177307..200987
   <223> /note="DHR2 region"
<400> 23
   gcggcgcggc tgacagatga ggctggcgcc gggcgcgccg ggctctgcag cggctgtgcg 60
   ttcgggtccc gtccccccgc agccccgcga gccccgaagc cgtggaaccg cgcgcgcgcg 120
   ccatggccac gctgccgagc gccgagcgcc gcgccttcgc gctcaagatc aacaggtaag 180
   acggcgcggt tcctggccag tccgcggcgg ggcgcgccgg tccccgtggc gactcattct 240
   cgtcctcttg tcacccgcgc tgctgtccgg accagcgtct tgcgccacct ccccaacccg 300
   gcgtggctcc cgccgccctc gctgttctga gacaggtggc ccgacccgga gcgcggggtc 360
   gcgcgcccga acttggctta acaaattgcg ccttccttcc agtcgggaga cagcggaaag 420
   aagctaagtg gctgaaaatc agccctgctt tgaagcagat cggtccgtgg tccagggtta 480
   gggctctttc aaccccctct ttccaaaaaa catcttggct ttggatggca gcgctatttt 540
   tataccttgg tccaaaagga gccaaccatg aagtggaaaa aatgatttta aaaaaattcc 600
   tcctttaaat aagcgaaagg gaatgagctg tagctgacgt ttaaagattt ctagcactct 660
   agaaaagtga agaacacagg gatttccaat ttataaacag agctgcacac gccacatctc 720
   tgtgagttga ctactcataa agttctcggt agactcaagc ggcggcgttt cctccactga 780
   aaactgctga caagtattca agggggcatt tttgccttga cctgggaaat ctagactgcc 840
   tggctaaaag ataatgtgga agggcagcgc tctggaggac ttccagggtt ggtgtttttt 900
   tcttgttctt gtttgtttgt ttttgcatgt gggacgctgg cccacagtaa ttcatctttg 960
   ggctgctagt gagtgaagct ggagtcacta ggaatcacca tccttgaata gtgagcggta 1020
   aggaccacag agggccagtg gagtttccct gtgcagttgt gagcaagacc tttcacttcc 1080
   ttcactattt catctgtcca attagtttaa ttattaatgt ggactgtata caaatgctaa 1140
   agggtaacta tatcataatt cagtcttcct atcggagcct ttcccctcat caggctgtcc 1200
   tggctgcagt ggggccgtag gggcatagga agttttgatg gctgtcatca ttgccttccc 1260
   cgcttctcct ctctttgtcc ctgtcatgag aaagaaaaaa agccaggaaa caaggaacaa 1320
   gggcaggaag ctgttaggga ttcaggaggg gtgagactga tctgaagtga ctgtctggga 1380
   cctaatgcaa gcctggagac gctcttgcaa ggagcttcgg ggttggtata acccaagggt 1440
   ggaggattaa agtccttagc cagaagtggt ggacttaccc atcatcccag ccctcaggag 1500
   gctgagacaa gacaatcaca agctggatgc cagtctaggc tgcatacaga gaccttgcaa 1560
   caaggaaaca aaacagaagc aaccacccaa gcaaaacagt gttaggaaaa ccaagagcca 1620
   ggctacattc aaatttcctt acaaagtttt gctgcaaact aagtatatcc cattgggcat 1680
   acacatttta tatttttctc atgtttggtc tcctccctcc tccccttctt tctgtctctc 1740
   caaggtctct cactttgttt cacaaactgg ccttgaactc cgagcacttg acctgcttca 1800
   actttctgat tgccagggtt gcaactgtgt accggctttt gtgtcagtgg gaataactag 1860
   tatccgaacc ctagtttttc cctcttctga ctgttaggag gactgagtag agtagagaca 1920
   gcgtgcttat gatgcctggc tcagaatgaa tgttaaatag ctgctagctg acacatcagt 1980
   accgctagct tcgtcgtggt caatgccatc tctaatatca tcaaatctga gatgatactt 2040
   cagaacctac gaagacaaag taccaagggc agctgaagtt ggactatttt tccaggcaga 2100
   ggccatttgg ggcagggtat ggggatgcca gtggccacct gcagctttct tggggttata 2160
   tctctcacat gtgcaacaca gtgctgagaa ccctaaactg gagccaaatg ttccaagaag 2220
   tacccattta ggaaagaaaa gtatgctttc taatcttctg gaaagagtct acattgtatt 2280
   tttcttttta aaaagagttt tgttctcccc cctgtaagcc actttgtgta ttgggagcaa 2340
   gagttacact gaggtcttgg ctggtataac tttcagaaga gtggatttct gagaatgtat 2400
   ttggtgtggc tataacattc aaacccgcca cttgcaggct gaatactgac ttgcagatgt 2460
   tgttgcagat gttgtgctca gtcgtcctgg acagatgtgg tcagctttca ttgtccactt 2520
   gacacaatcc agagtcacct gggaagagag tcagaaatga tgggttgtcg gcatcgggtt 2580
   tgcctgtgga catgtctctg ttagattctc ttcattgttg tttgatggag ggaagacata 2640
   gcccctgtgg gcagtgccat ttcacaagct tgatcctaag ctgcagaaga gtgaggaaag 2700
   tggacttgag caagccaggg tgtgtggggc ttctttagcg cttggctgtg gatacgatta 2760
   gctgcttgaa tccctgtttt gacttcccca aaatgatgca ctgtgatatg aaattgtaaa 2820
   ccaaataaac cctttcctcc gctaagctgg agatgttttt accctggcga gagaaatgaa 2880
   acttgaaaga aggggaaagt gaagcgtggg gtgtacttgc acaaggctgt gtaatttgta 2940
   aagatcacag tccgcgatag cagccaggac tacagatggc ccttacattg gccctctgca 3000
   ttaggcctgt actattttga tctacgtgtt ttaagaattt cctttaaaag caaagagtta 3060
   actttaaaaa aatcatttct gtaaacagca ttgacagctc tgcttctgag cagtacctgc 3120
   taggtcagca aggtgctgtt cagcagagtc aaacatggaa aaaaaacctt tttttaccta 3180
   catgagtgtc tattggtttt taaaataaat ccgattccaa aagtaacacc gtgtggaaag 3240
   gtggccatca ggactcttag tacactttca cattttcacc tttctattta acaatgatat 3300
   tccttaagac acttgttctt tctattaatg aaatattgta atgtgacata ttaaaaatga 3360
   acagttgcaa agagctcagc tctgggccct acagaggaat gagcagtgtc ttacatacag 3420
   tctgtgaaaa atgttcccca ctgcatgcaa atttctgtgg cttcttgcaa gcagcattgc 3480
   ccagtgcagg gccagcgcca aagccagagg aagtcgtcaa tgctggcagc agctgtctcg 3540
   ccatggttga tctgactctc tggtgctctc agaaccttat attcccatgc tgggtatcat 3600
   cagggaggac atgctaggag cagctagtgt gactgtaact ctagttgggt attttgaaac 3660
   atttctctag aaccagtaac tcagggagat agaagacaag actctagcgc cgaattgttc 3720
   tgacttggaa tgtctggtag gctaagatgc attgctcttg actttagttt ttccctgatt 3780
   gtcacgagca agaaagacta gatctgaact aggtagtcac aacaatggtt gtgtttggga 3840
   tttttaggaa ctgctgttat cagatgtatc ctagttcttt aaggaacatt cccctgttaa 3900
   tgaacgacct ctttgatgac tggaatatgc tgcttttgta gatgcaggaa attattacta 3960
   atgctgtgaa gattcatcag acaagacctt catattcttt ttcctcttat gcagtgttgg 4020
   ttattaaagc tagggccatc tgcacgctag gcaaatgttt taccactgag ccatatctca 4080
   gcccacttgc ttctgtcaaa cccagaataa gtatagaact tgatcaaaca tgaaagtaaa 4140
   acaagaactc tagataaata aaggaaaatc ataaactgtt gtaagccctg gacacctagg 4200
   attgaaagaa gtagattgta agacttacac ttttatattc cttactgtat tagttaactt 4260
   ttctgtggcc gtgacaagat ctcatgaccc aaagcaactt acatacagga gaaagaattt 4320
   agtttggctt atggttccag agggagagtt gataaagggg gagggggagg caacgcaaca 4380
   agctgtcaga gcagaaagcc aagaggtcac atcttcaact gcaaactgca aacagaaaca 4440
   gcagtctgga aatgggccaa ggctgtaagc tttcaagttt tccccaagtg acaagcctcc 4500
   tatagctagc ttacactacc tccctaacag gggaatcaag tgttcaaaca cttaagcttg 4560
   tgggaagcat ttctcattca aactaccaca cttactatgc ggcagatgcg aatctaaatg 4620
   tttatatcgc cattcatcca cttctatgaa ttaggaacaa ttactgcctt catttataag 4680
   tggggaaaca gaacacaggc aagttattca cgttttgata tcacacagct aattaaggcc 4740
   agagccggga ttgatattca ggcagtctgt ctccagaatc ctgccctcct actcactata 4800
   ccatgatgtg tcttggtttt cctttaaaac aaagaagtaa caatatttac tatctactaa 4860
   gtactgtgcc agacagccaa gattccggca ctagtgaggg aggggctctc cggtccccac 4920
   cccttaatga ggagctagcg gcagctgata gctgctggag cagggagaat cattctttct 4980
   tgaggttgtt tcccatacac cagtgggtga ccccacaccc atgtacatgt gggtggcgct 5040
   aattggattt agtgcaatta aaaaaagaaa cgcatgggat tggaggggga acgtgttgag 5100
   gggatatgag gagagtggga ggagggaaat gaatggctgt gatcatagtt tattgtacga 5160
   atatataaaa ttctcacaaa gaaaattatt ataataacga aatgagaaca gttgaaaatg 5220
   cttattattg atttgattcg tgcacattgt acatatattg aaataccaca ctgtactcaa 5280
   taaatatgta gttattattt gttaataatt aaactaaaac agttaaaaat tataattttc 5340
   taaaaagaga aagacatgcc agtcaaatgg cttagctggt aaaagaactt gtctgacaac 5400
   ataggaactg tctctcgatg gagagagaga actgatttcc caatgtcatc ctctgacctt 5460
   tacacatttg tcatagacac gcacccatgt tcatacacac acacacgcac acgcacacac 5520
   tcacacacac actcacacac actcacacac acacacacac acatgatatc atacaattaa 5580
   aaatgtttaa agtagaacaa agcataaagt attctactca tcattgggat gatttttata 5640
   caagataaaa ccagatgcta agaaagaata aagttacata aaggtttcat tcaaggataa 5700
   ccccaaggta ccaaaacctg taattcaagc tctttgggag gctgaggcag gagaatcaca 5760
   agaccaaatt tagcctctgc aacttggact ctgtctcaaa ataacatgta aaaagagggc 5820
   tggacacatg gttctttgac agagcacctg ttcagcatgt accaagccca gttctgagag 5880
   agaggtgggg gtggggggag acagagacag acagagacaa acagaggagg gatggggaga 5940
   tggctcagtg gtaaagggtc ttgctgttca tttgatgccc agaaccacat ggaggtggaa 6000
   ggagatttgg ggattacagt taattaagtt gaatgaatct cagaagagac cttgaacttt 6060
   ggacttttaa cattgttgtg actgctatag actatgagga ctttgaaagt tggactaaat 6120
   gcattttgta ttatgctatg tttaagtatg gcccccatag actcatgttt ttgaacaagt 6180
   ctatggggac cagggagtgg aatgtgatgg tttgtatatc cttggaccag ggagtggcac 6240
   catttgaagg tgtgaccttg ttggaatagg tgtgacctgg ttggaatggg tgtgtcactg 6300
   tgggtgtggg tataagatcc tcaccctagt tgcctggaag tcagtcttcc actagaagcc 6360
   tttggatgaa ggcatagtac tctcagctcc tcctgcacca tgtctgcctg gatactgcca 6420
   tgctcccacc ttgatgataa tggattgaac ctctgaacct gtaagccaac cccaattaaa 6480
   tgttgttttt tataagactt gccttggtca tggtgtctgt tcacagcagt aaaaccctaa 6540
   ctaagagaga ggtggaagga aagaactgac tccataaatt tgtcctctgg tatcatgcct 6600
   gcacccctcc cccacacata catatacaca ataataatat tcaataaaac aaagtttaaa 6660
   gaaaagcaat aactcaggac tggagattac tcagtggtta agagccagta ccttcttgca 6720
   gaggaccctt cacatgcttt tgtgaatgga gggaatggag gttttatgtc cctgttggct 6780
   catggagagg atactcagca tcatgtggct ccccagggag aggggcagag ctcagagaca 6840
   aggtctccta tctcagctct ccacatagag ctagagaacg gatgggatga tgtcacaaac 6900
   catctggttt tcatcttctt taacaggggc atgaaaagtc aggtcaccct actggtcctc 6960
   ttgctggaga actctttttt ttttttgaac tcagaaatct acctgcctct gcctcccaag 7020
   tgctgggatt aaaggcgtgc actaccacgc ccggctggag aactcttaag aagcagggcc 7080
   acttgagtct tttcaatagc aaacagccag ttactcaata atatctcagg caattttatt 7140
   aaaggttaaa gcaagagaca tatactattc ctggtcccat aagcatccag gcttatatat 7200
   agcccaggtt ggccttgaac tcatgatgat ctttatgttt catccaccca tgtgcttgga 7260
   ttagcataca tgactactac agccagcttc caggtctcca ttctttcagt agcatgcaca 7320
   tgggacccaa gtgcaactca atgaagtaac agcttgggga gttgaaagga gaacagaaat 7380
   tacaagcaga ggggaagaca ctgtgtgggc atttgcacga gctctgcatg cagatcagtg 7440
   gagaactttg gggagtcagt cctcattgtc tgccttacta atgtggggtc tcttgtttct 7500
   gctggagtgt actctaggct agctggttca tgagcttctg ctgtctctac ctcccatctc 7560
   accacatgaa gactgctggg attacagatg tgcaactctg catctggctt tttccttggg 7620
   ttgcaggcat tttgctgcac atggccgatc aggcttgtgc gagcaagtgc ttttatctgc 7680
   tgagcgtctc cctgactcac attcccttgt aaaatttcaa aaactaaatc aaaaagtaca 7740
   gtagcaaaat ctagtaaaca aaaagaaaaa aaaaactgct cacatttcaa ccactcataa 7800
   actagtaata acctgttaac atttggatac acccccttct agtttttaca aaaacaaaat 7860
   cgtcttgtgt gtgctacatt gtaatcttta aaatgtgtca acaactcgcc agtcaatgta 7920
   ggtctatatt ataaatactg tatatgtatt ataatttatg aaagtagtta ttattggaca 7980
   ttaagataat tcccaggtaa cttccagttt tgcagaccac gtcgagcttg agggaacagt 8040
   ctgaagcttg gtttctgtct agtagcagaa ttacttgttt aaaaaaaaat taaggttttt 8100
   ttttctagtt tgttttcttc ttggtgtgaa tgtatgcatg tgtgtacagt gttgagtctg 8160
   tgtgtgcacc catctcttca tgcagaagcc agaaggacat ttggtatgct ctcagttttt 8220
   tggttttggt taggctctgt ctagacctgg gcttaccgga gcatgtaatc actccaggct 8280
   ttttacctgg gtgatgacat ccgcactcgg gtctttagga ccttacccag ctttcgttct 8340
   gagccagctt tagttcttca tgcttttatt accacgttga ctactagaat aggtacatca 8400
   ctttctgctc ccgccgctgg ccctgcaaag gctcatttcc ttgcattcag atcccactgt 8460
   accccaacca tttaccactt atcaaaagtt tatcattctt taaagtgttt aacttttgac 8520
   tccttgcgtt tttgttttgt ttttgttttt ttgtttgttt tgtttgtttg tttgtttgtt 8580
   tgttttttga gacagggttt ctctgtgtag ccctggctgt cccggaactc actttgtaca 8640
   gcaagttggc cccgaactct gcctacctct gcctcccaag tgctgggatt aaaggcatgc 8700
   gccaccaccc cggctgactc cttgcctttt ttaagcaata aaaatggtca tgtatttctt 8760
   ctttttgcct aaagtgcaac ccaagatgac cagtgaaatg ctaccacctt cctaccacct 8820
   tattccttga ataatgtttt ctgtttgttt ttgcttttac tttgcttaac agtttaattc 8880
   agtcaaaatc tcctttgagt gtataaaccc caaccatttt aaataaaatt ctatcacaaa 8940
   atgagatttc aaatcatgtg acttattgca tgttttacct gaaatgagag aatagaaaat 9000
   gtgagatttt tttttttcat tttttttgtg ttccttttcc tgtggggttg gaatttgggg 9060
   caaaacatct ctctctgctt agaaacctct caaatcttcc tggcttcctc tttcattgtt 9120
   ctctcaccac agaggtaaaa cttactgaag cccaactaca agtagattaa gaaatgtagt 9180
   gaccacaacc tcttcctccc tctctctttt tcttctacat ttgtatgtgt gtatggttat 9240
   gtgtattcac atgtgttggg ataagtgtgt gtgtgtgtgt gtgtgtgtgt gtatatgcgt 9300
   gcacatgtgt gtgtagtctc tgggttgaca tcaggcgtca ctttctctca ctgaacccca 9360
   agcatgtcat tttggctagt ctggctagcc accttgccct aggggttcct tgtctctgcc 9420
   ttccactcat tgttagtata ggaaggtcac gagactcatc tggcctctac ctgtctcctg 9480
   gctgtctcaa ttctcctcac ccttgcatgg caacccattg aaccatcatc ccagaccttg 9540
   tcttctattc ccttttattc tcccccttct tcttcctctt cttcttcttc ctcctcttcc 9600
   ttctccttcc tctctgtctc tgtctctgtc tctgcctctc tctttgtctc tgtctctctg 9660
   tctttctctc cacctttgta tgtttatctc tatttttagt gtctttgtgc atgtatgtac 9720
   atgggtactc aagtgccatg gcacatgtgt ggaggtcaga gggccaccat gtgggttcta 9780
   gggaatcaaa catgggtttt cagacttggc agcaaaggct tttaccagct gagccatctt 9840
   tctcttgact cttgataaaa caaacaaaca aacaaacaaa caaacaaaaa ggctcttgct 9900
   gcagactcag ggtttccctg gcagaacact ttctaccagg gacccacccc cagcagccac 9960
   acagtctaag acttcccaat ggaaactctg aaagccagaa gggcttggat ggaagtttta 10020
   caaactttaa gggaccatag atggcagtcc agcaaaactt tcagtcacga tagatggaga 10080
   aagaaaaaca ttacatgata aaaaattaaa tttaagcagt ttctaccaac atatctctat 10140
   agaaggcact agaaggaaaa cctcggccta ggagattaac cacatccaag aaaacacaag 10200
   agtaaggaat cccagaccag aaaatcaaaa ctgggagact tacaccacag taccaaaata 10260
   gcaggaataa gtaaaaactg ttcattcata actctcaaaa ttaatggcct taattcccca 10320
   gtgaaaggac acagactaac atgaaattag gaaacaggat ccacagggct ggagagatgg 10380
   ctcagctgtt aaggtggaac aacaatatga actaaccagt acccaggagc tcttgtctct 10440
   agctgcatat gtatcaaaag atggcctaat aggtcatcac ttgaaagaga ggcccattgg 10500
   acttgcaaac tttatatgcc ctagtacagg ggaacaccag ggccaaaaag ggggagtggg 10560
   tgggtagggg agtgggggag tggggggggg tgggtatggg ggacttttgg tatagcattg 10620
   gaattgtaaa tgagctaaat acctaataaa aaatggaaaa aaaaaaaagc actgactgct 10680
   ctttcagagg tcctgagttc agttcccagc aaccacatgg tggctcacaa ccgtctgtaa 10740
   tgagatctga agtcctcttc tggtgtgtct gaaaacagca acagtgtact cactcataaa 10800
   ttgagagaga gagagagaga gaaagagaaa aacaagattc atctttctat ttcatctaag 10860
   aaacacacct tactatatta tatattacct tagcaaaaag catgggaaga aaggtattcc 10920
   aggaaaatgg acttatgaaa caagcatata tagctattct aatatctgat aaaatagact 10980
   aaataaaaaa ctagtcagaa gaggtaggga aggacagtac atattcatca aaagaaaatc 11040
   caagaggatt acgcaccaaa ttacaagggc acccaggttt gtaaaagaag cactaccata 11100
   gctaaagtca catgttggac ctcacacagt gatagtggat gacttcagta cctcactctt 11160
   gctgacaggt catctgggca aaacctaaac agaaatgctg gagttaaata atttcataaa 11220
   tcaaaggaca gataatgtag agcatttcat ataagcatgt acatgggctt gcgcacatgc 11280
   acacacacac acacacacac acacacgcac ccacacacac gtgtgcgcgc acacacttac 11340
   ctacactttc ttctcagcag ttcatggaat tttctccaaa aagcatggta ttggcatggt 11400
   atttctcaca cacttaaaca cacacacaaa taaatttctt ataatcaaat ataaggaaat 11460
   tgaaataaca ccctacacca cagattaaaa ctggatatta ataacagaaa taacagaaaa 11520
   tatacaaatt tgtggaaact gaacaaatga aaaatgggtc aagatagaaa tcaagaggga 11580
   aattaaactt tttttttata acggattaac agtgaaaaca taacataccc aaacctatgg 11640
   gacacaatga aggtaattct aagaggcaag ttcatagcac taagtaccta cacaaagaca 11700
   tggagatctt atattagtta ctaagcaaca cctgaaagtt ttataacaac agaaagaaat 11760
   aataagactc aggaattaaa gaaacaaact agagacaaac ttgtaatata aaaactttaa 11820
   gacattgaag aaagaaattg aagaagacac tggaagatgg aaagatctcc catgatcatg 11880
   gatgggtagg attaatgttt taaaacagtc ttctactaaa gcagtctaga ttcaatgcaa 11940
   tcctaatcaa aattacaaca caagactgaa aaaaaaaaaa ccttcaactt catatggaaa 12000
   cacacacaca aaaatcaaga cagctaaaac aatcctgaat agtaaaagga ctgatgaagg 12060
   tattatcatt ccagatttta attatatcat ctcagatttt tttgttacag agatataata 12120
   aaaaaaaaac ccagcatggt attggcataa aaacaggtat attgatcaat ggaataaaat 12180
   ggaagaccca gacagaaagc cacatctctg ttaacacagg actttttgat aaagaagcca 12240
   aaagtttgca gtggagaaaa atacagcatc ctcaataatt attgccagtc acactggatg 12300
   gctgcaggca aaagaatgaa aatggataca tatttactat tgccacgaac agaagtccaa 12360
   ctagatcaag gacttcaata taagaccaga taccctgagt ctgatagaag acaaagtagg 12420
   ggaataaact tgaactcact ggcacaggaa aagactttct gacaggatac tgatggcaca 12480
   gctactaaga ccaacaatta acaaacagag gctgaaaaac ttctgtatgc caagcgacac 12540
   catcactagg acaaggtagt agcctaagat ctagaaaaag atctttacca attatgcatc 12600
   tgatagaggt cttgaattta gaatatataa agaactcaaa aaaaaaaagg aaaaaacaaa 12660
   tgacccaatt taaaaattag gtgcagaact aaacagtgtt ctcaaaagat gaaataaaaa 12720
   tcactgagaa acagagaaat gttcaacact ttagccatca agaaaatgta agttatgact 12780
   actttaaagc ttatcttaca ccatatagaa tggccaagat caataaaata agtactaggg 12840
   catgctggcc agaccgaggg gaaatggaac cctaattcat tgctgctgtg agtgcaaact 12900
   ggcgcaacca ctgtggaagt cagtgtggct gttctccagg aagctgggaa tccatctccc 12960
   tcaaattcca ataaaccact ctggaactct ccattctgct gcagagatgc atgctcatcc 13020
   tggcttccct attcacaata agcagacata ggagacagca gagatgttag tcgaccgatg 13080
   agcggataat gaaaataggg gagaatttac acaacggaat gctagtcagc tattaagaca 13140
   gatgaaattc ctggcaaata gatgaagcta gaaaaaaaag tcaacctgag tgaggtaacc 13200
   tagacccaaa aagacatgtt tttctcttac atgtagatgt tagcttttaa gttttcaaaa 13260
   taatgtggtt tgaacaacca cagaagataa ttgcttagta agggactgga tgggaggaga 13320
   ggatcttaca agggaaggaa aatagaatat attgttctgg agagacaaag tagaaactag 13380
   actagaagga ttaagtaggg atgggatgag agagaaaggc gagaggagaa tattagctaa 13440
   cagcagaggc catttgaaaa accatatagg aagaagccca ctactataga aaattcctaa 13500
   aatctatcca tatatgagag aaacatgaat ggagtaatcc aatagtgggg gaaacaatgc 13560
   cacaactatg atttttcaga cgcaaggggt ggacgcaaag gtgagcttac agagattgtg 13620
   acagtccaca caggatcaag acatcctgag tctcagcact gagagtggaa agtggacaca 13680
   ggttctcacc cctaaccagg aaggtatctg cagttgtcag ctgctaggct gaaaatcagt 13740
   tttctccggt ggagtctcat taggtatgtc aactgtacat cagggcaggc cccatggcca 13800
   acgtacttgg ctaacccaaa aggtactcag tggcattttt gtggactttt gtttcattct 13860
   gctttgtttg ggcattttct gagctttcag tcttttgctt gcttgttact ttttatttgg 13920
   gggttgttgt gctccttgtt ttgttttttg aaagaggaga gcaaccctgg gtggatgggg 13980
   aggtgaggaa ggtctgggag gaatcgggag agaggaaaaa cataatgaaa atgcattgtt 14040
   tgtaaagaat ttttaaaatt agtcttgtgg ttccagggcc aagtagtgtg atgttttata 14100
   cattccccca gccagtcatt cactcactca ctccatccgc agggtgtcct acattgagtt 14160
   ccgtgctaga ctctgaaaat atgaagaaaa gtgattcatt ggcctttgcc ttccatgatc 14220
   atggatgcta accaaaattg agatccagga gaggagggag cctctcttgc cttttggcct 14280
   ttgtcctggc catgcagtgc ctacagaggc gccaacgctg ggttcatggc cctggggatg 14340
   ctgcagtagc attacccaaa acatttctgg aaatcctgtc agaaatcctt gcagagatca 14400
   gagaacactt cgtcttaaca gcagactcag tggtctgaga gcactttcct tacagggaga 14460
   acgagctcta tttttttcaa agtgttcaaa aatatagaaa gtacagataa gtttgtacca 14520
   aatatttgag agttctctgg atgtaaatga agagttgaca gcatcaagcc ctttactgct 14580
   tttgcttggg agattttaag ttagggaaag ggaattttta ctgaaaaaca aagtctctgt 14640
   tctgttccct cacgtgaccg atttcaggag gctgaggggt gtgtgtgtgt gtgtgtgtgt 14700
   gtgtgtgtgt gtgtgtatac acgtgtgaat gtgtgtgtgt acacaggcat gcatgcttat 14760
   ccataagcgg tgcattttgt gtgatcatta acattcaaac aggcccatta gattatgcat 14820
   cgtcagcttg aattaaatat gaggaagggg gcttgggaat ataggtccac gggagcactc 14880
   ctgctcactt gtacctaagt gaggccctag gtgcaatccg gggtgataac tataacgtct 14940
   tggctatgtt tgggggtgcc ttctcaaatt gttctgagaa cacatgccaa aagaagttct 15000
   gaaagtgatt tcagaaagac agctttatgt gggattataa agaacaatag tcgtttatat 15060
   aatatatgca acctgatagt attatccaat cagtccttaa cattataatc ttgctgctca 15120
   tttcctggag tggctgggca gacattaaaa aaaaaaaaaa aaaaaccact ttctttattt 15180
   ctccctcagc tggctgtacc gaatgatgca tttgagatct ggatctaaat cacagtaaca 15240
   tgtttatttt taacacacgt tttaaaaagc acccagtaca gatgtttgtc tccgtgccct 15300
   aggacttaga aagatctgtc cgttcacaaa gggcttgtgt ctgctcagca gtcggtttgg 15360
   atgttttgcc tgggtaacag aaccatcacc cctgcgctct ggatgtctgg tctatctgat 15420
   gcaccgcagc tcacaccaga gaaactcaga ggagtgacca tgggtacgga tgctttcttc 15480
   aaaggattct gacacacaca cacacacaca cacacacaca cacacacaca cagaggtttt 15540
   tttctgtgta acatgaatgt tcagtgaagc taaccactcc tcatgaacat gctcagctgg 15600
   gcccaggttg actgaggaga ctggagtcct gcaggttgaa caatcctgca ctaatttaca 15660
   ccacagtgag tgtcctttcc ttatgtgagt ggggacaact caggctcaag gtccactggc 15720
   aaagcagcac tccatatcct gctagttaaa atgaatgtct caggcctcca ttgcagcttt 15780
   tgtttctaga catgagactg ccctcttccc tttaccatta tggttggata ttagtggata 15840
   aaacccacca atatctttca ttccaaatca ctgtccgttt tcacagtatc ccgtagaagg 15900
   gcaactacac tgaccaattc tgcaatcaaa tgttatctat tttatcttgt tctctacttg 15960
   atactttcat tcagatcaac agaatttttt ttttattcct tatgagtttt ctaggcatta 16020
   tatgagattt aaaaacaaaa caacacatct tgtaattcat tcggatcagg caagctaaca 16080
   taaatgaaat gttagagaat atagacactg ttgttttgtg cggctggatg caggtgaagg 16140
   gtagaaccca gcttgagaga ggctgtggct ctggggtgca agggcacatt tctgaagagg 16200
   tgagatttca gccaggctct gaaggacaga gctgtggcgg acacttggaa caggtaaggt 16260
   gagaatgatt gtgagcagtg tgagaatgta ctaggggttt cctgagcagg gtggtgaggg 16320
   agccgaagcc cttctggtat gaagagatgt ttgggatgaa gaagggatct gatttataga 16380
   cattgaaagg gacccagtgg gacttgagaa gcaactaagt gtatattcac tcactcattt 16440
   ctgcatgctg atgtgacgat gccttgcact gttctaagaa tgggtggaat tgagaataag 16500
   ctaggttggg ttctgtgtta aaagaagaaa tattccaaag aggagacaat aaatgaagca 16560
   ttaactgaac aatgatgtgt cagttatact aaaggctatt taaagatatg aaagggcata 16620
   tgaggcatgg acacttacag catgcctaaa ctaaagtatc tgagagagca tctctgggtg 16680
   agagtgtgag aggagtaaat aagatgcaga tcacagcctt agaatcacag agagatcaag 16740
   aggcagcaga aaaaagccca agcattatta agtggaatag aagtaagaag tgagcgtggt 16800
   tcagcgtgca aagcacttgc tacacatgtg tgaggaccaa agttcggatc cccagtgaaa 16860
   gctaggtagg catggtggtc ctcctgtagt cataggacag gaggcaaaga caggaatctt 16920
   tggagcaagc tggctagcca gtctagcaac cagagatcct gcctcaattt atgatgtgta 16980
   gcatgcacac aaaaagcctc cagtgtcaac ctgtggcctc ttaattttat tcatgaacat 17040
   ttgcagcatc catgcatatg ggcatatgca atcacgtatg tacacacacc tcacacaatc 17100
   ttggaacaaa acaattccat tctactacac agagcagcaa ggaatggaag caaagagact 17160
   gggggaagtg gtatggggta ggtgaaactg atagggtaag agcagagaac tgactggagg 17220
   gagagagaag aggcagctgg ggctgacttt tggtatagtg taggccactt actgagagga 17280
   gaatcaaaat atctccatag ttggatgtac aggtgtttat tgacaattta aaaatattta 17340
   tttattttat gtatatgacc acactagggc atcagctccc attacagatg gttgtgagcc 17400
   accatgtgct ttctgggaat tgaactcagg tcctttggaa gagcagtcag tgatcttaac 17460
   cattgagcca tctctttatc cctatattga caattttaat agagatgaaa atgtctgaac 17520
   ataagtaggt ttacaggatg attaaaaatt gaattgggga tgaaatttag ctgatgtttg 17580
   tagtcctgtc acttggaaca tgaagatagg agaatgagga gttcaaggcc agcctgggct 17640
   acacaagatg ctgtttaaaa aaaaacacag aaattaattg ggaacacatg aagtctaatg 17700
   tgtttattga cattccctgc aggtttattg tatccaagag aggttgttac tggctatttt 17760
   catgtgaaag tctgcatgtc atatatattt atgctcataa agaaaagaaa gctttaaaaa 17820
   gaccttgata acattttaaa aggcaaaaaa ttatattatg acactgcttt attgaaaaat 17880
   aaacatgtaa atatctctct gtgacaatga ccagatgaat agaaagatgt gtcataagtg 17940
   ttatccctgg caggtgagat tgttcaaggt tttgacttgt attcggggca tgcagatatg 18000
   atgctctcaa gggtttcttc aaagtcttgt acatttaata ccatccggat tcctctatgg 18060
   acaaatgaga taaaaaggtg atagggatac atttatgaaa ttgtaatttt cataatttaa 18120
   attgaaaagt tagttttgag aggataagtt tgaatttctc ttatagggct tttctttaat 18180
   aatcctgccc tggggctggt gagatggctc agtgggtaag agcaccagac tgctcttccg 18240
   aaggtcagga gttcaaatcc cagcaaccac atggtggctc acaaccatcc gtaatgagat 18300
   ctgactccct cttctggagt gtctgaggac agctacagtg tacttacata taataaatag 18360
   ataaatagat aaataaatct taaaaaaaaa atcctgccct gaccaattca gatgaatgtc 18420
   aagaacccac catttccaca gggcagtctg agacacagtc ttgatgtttg agacttaagt 18480
   catggacagt gttgattagg aagtcatctt tgaggtggga ataggaactg caggatagat 18540
   acatttgggg ggaaccattg atatggccca gcaggtggca catgccacca aacctgatga 18600
   aatgagttca gcgcctagga ttcatttggt agatagaagg aaccgactcc tgcaagtttt 18660
   ctgactttca ttgtgacacc cacctaccca cccagaatac cttttcttct agagatggac 18720
   agtaggaaaa gaaaaggaat gggctcagaa cacgagccat accaaaagag aactgtgaag 18780
   aggaggaagc gcgtggccaa ggtgtggcaa actgctgcgc gcgctaggaa acggagctgg 18840
   cgcagcagtg cacggccagg tgccggattc caggggtgaa gcaggagcac tggctttccc 18900
   ttgttggttc ccggaatctc cagaggcagc agttttagga actgttcgag gaagtcaggt 18960
   ctcacagggt tggaggttga atcgatgcag aggaaacgga ggaagctgac cgaggccact 19020
   ccttttaaac atctgtacag acagaaagaa gtgggacaat ggtaaccaga aactccaagg 19080
   aggctgcagc gttacccagg cagagcacca cccagagagg atgttcggga gtgaggagga 19140
   ggaggttctt cagtccatta agtcaccagg tgtggatgaa ggtgcttaga gtacttgatg 19200
   ctggccagtg agtaacgaag gctagcgttt gtactagcat cgaggcttta aaaatctggc 19260
   tttcatgatg gcgttttcta atgaagtaca atgataaaca ttgtgcttta cgaacattca 19320
   tctcctcatt accttccctt gtcttctctc ttcacttttg ctgggcccct tcaaacatct 19380
   gcttcccctg cctctgcctc tgcctctgtc tctctctctc tctctctctc tctctctctc 19440
   tctctctctc tctctctctc tcacacacac acacacacac ctatattctt cccatgaaag 19500
   aaaatacgca acacttgtct ttcttgtccc aatacactct tcttgtgtaa ccctctcttt 19560
   gttcctttct tcccacacag ccccctccta ctttcacagc cctaagagtt ctcacggcaa 19620
   gtcataatgt tctacttatc tgtcttctac gtaggagatt ctgagacatg acactttcat 19680
   agacagcatt tcccttagga atggggtact tattttggtt ttcttatgtt tacatttagc 19740
   ttcccgagtc tggagagatg gctcagtgct taagaacacg ggctgctctt ccagaggacc 19800
   tcggtttgat tgctagaacc cacacggcac ctcacaatca tctataacta gaattccagg 19860
   ggacctgtct tagttacgtc tcaatatgct gacaaatcac catagccaag gcaactttta 19920
   aaaggaagtc tttaataggg tttgtggttg cagaaggttg gaacccgcca tggcagaaca 19980
   aaggcacggt agtggcagaa cagctcagag atcacatctt gattggcaag caggaggcag 20040
   aaaggacact gggagtgaca caagtcttaa gaaaccttag agcccagtga catgtcccca 20100
   tcaacaaggc cttatctcca aatctttccc aaacagtccc actaactaag gaccaagtat 20160
   ttaaacacat aaacccatag aggccattct tgttcaaacc agcacaagag ccaatgccct 20220
   cttctggcct tggtggacac caggcatgga tgtcagacac agacatacat ggaggcaaaa 20280
   cactcataag aataaataat cttgagaaaa aacaataaac atatcttcct gtgtttgagg 20340
   tagaaaatga cagtactttt tattctgaca cacacttgtg agttcatgtg tttcactgtt 20400
   aagagtccta agtcctggaa catgaaatcg tttgatttaa agagaagtgt tttcagatat 20460
   aattgagcta aagttatcct gttaggctag tctcaaatcc catcactggt atcccatggg 20520
   aagagcctcg gggtgtagga ggcagccatt gggatgactg aggcacagtg gctctgtcaa 20580
   ggatgccaga cagatgctac cagatgcaag aaggcttctt tgtggcacct tcccagcagg 20640
   tatggcctgg ctgacacttt gtttcagttg ttgtcctcca aagctgtaag agaatagtgt 20700
   ctatcatttt aggtccagtt tgtggcaaat ttctgtggct gccctaggaa actaatacat 20760
   tctaaagttt tgcaagttaa aacaacagct gttttttttt tttgttcgtt tgtttgtttc 20820
   tcctattgca gcagtctggc tgtttgggtc gggccagctg cacagctctt ctgcaggttt 20880
   cagtttgggg gagttatgtc acagttactt ggtggcaggg gtgaagtggg aagatggcct 20940
   cctgcaggag tccaggaact tgattctgac tgttggatac cctcccccca tctcccaggg 21000
   tctcttgatc acacctagct ttctagcatc cttacaagga gcatgagtat tctaagagga 21060
   ccgggtggag ggttcctgag gcttatgtct ggaactgagc tttggtcatc tcaggactca 21120
   agtaggatga tgatacatga tgcctggccc ttttactcta tcatgtaggc tatgggagag 21180
   atgatcctgt ccctgctatc acatatgcta gggggaagat gatcttgttg ccatgacaac 21240
   cttcagatgg tatacgctga aggtcttacc tttccctgtt cacatcatag tacccatttt 21300
   ttgggcagga agactgtgaa tgggtggcac tgactccctc aaagacacca gtgagtcagc 21360
   ctttgtgtga gactctttgg tccatgggag acatctttgt gtctggaaga acaaatcata 21420
   gctctgcctg gaggcaggtg aacacactta ataacttctt aggcttctct ttaaccctat 21480
   aattaccctg atggagccca gattgcacag ctgtctgaaa cagaatggag tttaggggca 21540
   tcagagagaa agaggcctga gaagtgccat agtcgctcaa aagatctgga tacggggcag 21600
   ggttaggctg gaatatgatc aatagctgct gtgagcaaca atcagactgt atgtagaggg 21660
   gttatctagt ttttcttttc atttttattt gttgtttttt tttttgagat agtttttaaa 21720
   aatatagtcc aggctggtct tgaactaact tatacctcag gctaacctca aattcatagt 21780
   gattctcctg atactataaa tgtgctgaca ttttaggtgt atgctgctct acctgactct 21840
   ggtttttgtt gtttgtcata ttgtattgta ttgtattata ttgtattgct ttcttctcaa 21900
   taagaatcac agtgtttggg ttcatgagat ccctcatcag gtagagttca atccaggaga 21960
   tgacaagaga aatgattcca cagagttgtc ttctgacctc cacacatggc acacatgcgc 22020
   acatccgtgc acacgagtgt atgcacatac acacatatat gagcgcacac acgggcattc 22080
   gtgtatgcac aatggggggt gaattcaaat tcaagcactt ggcagtgagc atacatacgt 22140
   catggttcat gtgtcgatgt ctgaggacac attaggaaat cggttcgctc cttccacagt 22200
   gtgggtttgg aactggagct ctcaagcttg agagcaagca agcaccttta ccagcctcac 22260
   catatggcca gcccagctgc ttctctgtaa tgctctgctg tgttgtggct ttcaggttac 22320
   tctggcatca ctcagcaggt gactaggatg gtgacatagt tcttaaattc ctcaacttga 22380
   aaatagtgta agaagtttcc tttaggctgc tagtcaggac tgctcccaag gtctgttgat 22440
   acagggtgca tgtgctgggg aggtggaggg gggcagtgga tggacagggg gtcatggtac 22500
   aagcctagca gccagctcca tcagccactc atctctgtga aaagagaacc tgaatcttag 22560
   gtagacatct gtgtcatgga ttacagtccc agggaaggag cagagtgttg tcctcatgta 22620
   taggcctccc tctggtttgc tgttggctga gaaataacag gggtatggtc cgcatcacag 22680
   ggtctgggtc aggtctacag tcagaggctc atggaaatgg tgcctaaggt gctggagaaa 22740
   aacagtgagg attcaagatg gatttgtaag tcaggagtgg atgaccagat tggggagggg 22800
   acagttcaaa tttgattaca gtcaagaatg ccagaagccc cagatgtaac tctaaaaatc 22860
   acaccaactg ctccggaacc aggtacttga ctgttctgtt tttgtattgt ttgtttgttt 22920
   gtttttccct tagaacatct gaatttaaaa tgttttaagg aaaccctaca accctgagat 22980
   gtttcttttt aaaatatttg tttatttgtg tattttacgt atctgagtgc tctgtcttca 23040
   tgcacaccag aagaaagcat cagatcccat tacaggtggt tgtgagccac tatgtggttg 23100
   ctgggaattg aactcgggac ctctggaaga gcagccaggt actcttaacc actgagccat 23160
   ctctccagcc cctcactgag atgtttctag cagcttctct gagattgatt ttgagttcct 23220
   acttgccagg agagggagcc tggagtactt acctgagagg aagagacatt ctaaaagttc 23280
   actcctgagt aacttttgct atagcctgtt tccggtctgc gacaagagag ctgagatgag 23340
   aaattgaatc acttagtgtc catgactggg tctccgtggg ttagctggct cccgctttag 23400
   tgacagttga gtcacctgct gctcagtctt cccagctggt gatagatgcc acagtgatag 23460
   taacaaccac agaaaactgt gtggcctcct gagtcagaat ccagggaggc agacatgctc 23520
   ctgccagctc cgttggcact cagagcaatg tttgcaagtt ctttgctttc ctccgtcttg 23580
   gtttttacca cctgtaaaat agagacagta actctcccag ctcagagggg tcctgtgatg 23640
   taggtctgaa aatgctgcga agaggggagc actgaacata gtgatgaggt acctgtaaga 23700
   tgggtttgtt tcagctgggt gttatggcat tcgcctttaa tcccagcact cgggaggcag 23760
   aggcaggcga atttttgagt tggaggccag tctggtctac aaagtgagtt ccaggacagc 23820
   cagggctata cagagaaacc ctgcctcaaa aaaaatgggt tttttctgtc attatgtaat 23880
   aaggtgatgt gtatagaaac ctgacctaca gcaagcagag ggtcgaaaca ggaagtcgat 23940
   taatatggac atgtcaatat accagaaatg gagtatgtaa aaatgtcaag atgacagcca 24000
   ccacaacatt aggagcatac agcagttgta gtgtcatgtc aacacactgg aggttgaggc 24060
   aagaggattc aaagttcaag gattcatagc tggggctatg cagtgagagc cttccagtaa 24120
   agaaagaaag aaaaatattt cacttcactt ataaatacag ctaatattta caatgggcat 24180
   atgtatacac atgaatatag aagcacattg cttgtacttt gttgtaaatt ttgatggaaa 24240
   gacatttaaa ggaaaaatga aaatttggat gttaaatttc tcaggaacgc tgatgttctg 24300
   aggactggaa gagatgaaag atcctaacta aagtttccta aaagtacctt tattggatgg 24360
   ttttagttat ggcaactctt ataaagaaaa atatttaact ggggccgact tacagtttca 24420
   gaggtgtact ggctagtttt gagtcaattt ggcacagctg ggtttatcac agagaaagga 24480
   gcttcagttg aggaaatgcc tccatgagat ccaactgtaa ggcattttct caattagtga 24540
   tcaaggggga aaggcccctt gtgggtggga ccatctctgg gctggtagtc ttgggttcta 24600
   taagagagca ggctgggcaa gccaggggaa gcaagccagt aaagaacatc cctccatggc 24660
   ctctgcatca gctcctgctt cctgacttcc tttggtgatg aacagcagta tggaagtgta 24720
   agctgaataa acccttccct ctccaacttg cttcttggtg tttgtgcagg aatagaaacc 24780
   ctgactacaa atagaggttt agtccattat caccacagta gaacatagtg gtatgcaggc 24840
   taacatgtgc tggagaagga gccgaaagtt ctacaacttg atctacaggc agcaacagga 24900
   gactgtgtgt cacactgggc atagctttgc ccgcctccac agtgacaaac ttcctctaac 24960
   aaggccatac ctcctccaat aaggccacac acttgcaaat agtgccactc cctatggcca 25020
   agcattcaaa cacatgcatc tatggggtca taactattca aacgaccaca gagggcatca 25080
   tatgaattat tctagctatt ttcaagaatc tataaagcag acatttgtgg gcaacttata 25140
   tatgcttgaa gagaatacag ttatatagaa tatgagagga gaagaataat catctgtcta 25200
   acccgctctt agccttgtga gctacaatca tgcctgggat agcaagacac gcccactggt 25260
   agtgtaatgg tagcatggac attgtgggag taaccgaaca ttttatgatt ggatttaagt 25320
   ccttcagtac aagacgaaaa aacaaagcca acacccaaaa cctgtgacta gacaagtcat 25380
   aggctgtagg ggaaaagttc actactatga ttctgttaaa tggatatgtt attaaactga 25440
   ttcctagtag cttaccatca cacccgtaga ctaatgtgtt tcttagccct catcagcaaa 25500
   gctccttatt gcagcagatg actattagtt caggccatgg tgcatagagt aatagactta 25560
   ggaatactca gccctgaagg tgacctctgg gtcctacccc acttttccag ggctcaggga 25620
   gtattgcgga agaggaaggg aagagcaaaa gccagaggtg gtgggtgact acaaggacac 25680
   gatgtctctg gacacagcag ggcatccaca catatggacc cacagcagtt gtgacagcac 25740
   acacaagacc tgctcaagca caagcctgaa aaacaagacc agaaaataag gatggtgaga 25800
   ggcagtcagc aggacgatcc accccaattc aggaactctt ggcagttgat aggttctggg 25860
   agaagaaggg tctgttttcc ttaagaatgt agcccctggt gaggcaacca cactgcggtg 25920
   gaaggtcact catccaggcc catatgggct gcatacatcg tacttgatag gtttggggaa 25980
   atggtgccga gttgtgcggg tagtgaagga aggtagattt gggaaggatt gggaagggga 26040
   atatgatcaa aactattgaa tgaagttctc aaagaactag cccaaatcac ttagaagaac 26100
   cattaattaa ttttgcataa tttcttcttt ataaccaaag ctgtacactg gttaaattta 26160
   agccttgatg tctttatggt gttcttagtt atcatattca ctcatgagag actcgttcct 26220
   gagtaccaat gtgtgccaga caccattaga gtttggtatg aaaagctttc acgaatctcc 26280
   tacttgaatg gtgacgtgtc cccagtacca gatctatacc caaggtaata tcaggtaatg 26340
   atgccctgtt ccttgtccct tctctgttaa gtagagagat aataagataa tagtctatga 26400
   tgttgatatg ggattgcaga agttatttgc attgtaaatt tttttttttt tgagacaggg 26460
   tttctctttg tagccctggc tgtcctggga ctcactttgt agaccaggct ggccttgaac 26520
   tcagaaatct gcctgcctct gcctcccaag tgctgggatt aaaggcgtgc accaccaccg 26580
   cccggcacat tgtaagtttt taatgagtat tattaaattt gtaaaaaaaa tcattaaatt 26640
   ctcacaagga agaaatcagt gaactatttt acattcaaag agggttttct ctgtgtcttt 26700
   cagtgtgtct cagtctctct ccttgtctct gtttctctga gcagcatttt ctcatttgca 26760
   aagctgtctc agagaaatgt ctgttcttgt gtaagttttt atcctgttag atcagcttag 26820
   aaggatgatt gccagcctcc aggttgtctc tgagaagaaa ctgggaccta ggcctaccaa 26880
   aaatgagagc tcagctgtga cttgtttgct tccttgcttt tttttctgtt tctttttcct 26940
   cttctccttc ctcctcctct gcctcctttt gaattaaagt cttggaatat ggcccaggct 27000
   ggcttgaatt gcctcagctt catcgatgtt gtagttgaat gtccaaatct cagtaaaaag 27060
   gtttttgttg ttgttgatga tgctggtgtt tgtttgttaa ggtgggaggg gagagagaga 27120
   gaaagagaga gagagaatga gtgagaatga atgaatatga atgagagagt tcaggttggc 27180
   cttgaactca caattctttt cttaagtcct ggaattacag gtgaatccca ccctactcag 27240
   ctgttaactg tggcctctca tttattttct gttttactga gaagtgaaaa tgtcagctgt 27300
   ggaaaggtca actatacttt gtcttcactg atagggaaac caaatctaaa gtaggaaacg 27360
   ctgaaaggaa ccaggggtat aattcagata gagacattga gtgggttcat tattttctta 27420
   agaccctgac atttattggg ggtttcttgt gtcatgtatg tgcattacag tgtatgatcc 27480
   ataacagcct aaaacgtgcc ttcactcctt gcaggcagag atctcatctc actcaagacc 27540
   tgaacacagc actgtggtca aacccaggtg tggctgagac gaagctgtga ttttttatat 27600
   atataaattt actctttaat cttttttttt ttttttttac agtccagact ttatcccttt 27660
   cccagtcgat cctctctctg ttcaacatcc cacacctccc cacaccccag cctctactcc 27720
   tatctccaag aggatgtccc caccccagcc cctactccac cagacctccc cactccctgg 27780
   ggctccaagt ctctcggggg ttaggtgcat cttctctgac taagtccaga ctgggcagtc 27840
   ctctgctgta tatgtattgg gggcctcata tcagctgatg aatgctgcct ggttggtggc 27900
   tcagtgtctg agagatcctg gggtccaggg ttagttgaga ctgctgatct tcttctaggg 27960
   tcgccctcct cctcaacttc ttccagcttt tccctaattc aaccacaggg gccaccaaca 28020
   tggtactcac taactcacta ataagtggat atcagacaaa aaagtacaga atacccggga 28080
   tacaatccac agaactcaag aaggctaaca agctgaaggg tccaagtgaa gacgcctcaa 28140
   tcccacttgg gagggagaag aaagcaatca caggagggga gttggggagg gagggaggga 28200
   ccaaagctgt aattttaaca actatttttg ctatcaccaa ctataggaat caaacagact 28260
   ttttggctga attgagtttt tgtcgtggct gtctcttggg gtttgattgt gaagtgccct 28320
   tcataggctc ttgtatttga acacttggtc tccaggtgat ggtgttgttt gagaggactg 28380
   tgaagccttg gggaggtgga gccttgcttg ctacaggaag taggtcactg gggatgtcca 28440
   taaagttttc cagcctggac ccacttccat tcttgctctg cttcatggct gcaggtgaag 28500
   tgcacccata tagcctctga ctcctctgcc tggccttccc tgccctgatg gattgtattc 28560
   cctgagagtg aacaccacag tagatcctcc ttccttaagt gagtaatacg ccatccatgt 28620
   attgatgttg tttttccaaa tgacagaaga tattcagttt tacctcagac ggtcagagga 28680
   ctaactgtgg ggagaggtgg actccgtcct ctctgaatga agcgagagaa tctcacagct 28740
   cctggcaatc ttggagaggt ctctccttat agaggcttgg aaaaaaaaaa tctaaagcct 28800
   tggaaccaac agactttttg gctgaaacaa gttcttgtca tggttatctc ttgtggtttg 28860
   aatgggaaat gtccttcata tgctcttgta tttgaatgct ttgtctccag ttgatgctgt 28920
   tgaattcctg tgtgaaaaac aaatgcagtt tttatcacta ttgctcggta gtacagtttg 28980
   aggtcaagga tggagattcc cccaaaagtt cttttattgt tgagaatagt ttttgttctc 29040
   ctggggtttt ttgtcattca agatgaattt ggaagttgct ctttctaact ctatgaagaa 29100
   ttgagttgga attttgatgg ggattgcatt gaatctgtag attgcttttg gcaagatggt 29160
   catttttact atattaatcc taccaatcca attccatctt ctgagacctt cttcaatttc 29220
   tttcttcaga gacttgacat tcttgttata cagactttca cttgcttagt cacacaaagg 29280
   tattttatat tatttgtgac tattgtgaag ggtattgttt cgctaatttc tttcccagcc 29340
   tgtttatcct ttgagtagag gaaggccact aatttggtta agtttatatc gagccacttt 29400
   gctgaagtta tctggtttag gagttctctg gtggaatttt tggggtcact taagtatact 29460
   atcatatcat ctgcaaatag tgatattttg acttcttcct ttccaatttg tatccctctg 29520
   acctcctttt gttttttaat tgctctgtct aggactttga gtactatatt gaacaggtag 29580
   ggagagggtg ggcaggcttg tctagtccct gattttagtg ggattgtttc aagtttctct 29640
   ccatttagtt tgatgttggc tactggtccg ctgtatattg cttttgctat gtgtaggtat 29700
   gggctttgaa ttcctgatct ttccaagact cttaccatga aggggtgttt gcttttgtca 29760
   aatgctttct cagcatctaa tgaaatgatc atgtggtagc gacaaaaact gtatggtatt 29820
   ggtacagaga caagcaagca tatcaataga atagaattga agatcttgaa atgaacccac 29880
   atacctatgg tcacttgatc tttgacaaag aagctaaaac catccagtgg gaaaaaagac 29940
   agcattttca acaaatggtg atggttcaac tggaggtcag catgtagaag aatgcaaatc 30000
   aatccattct tctctccttg tacaaagctc aagtccaggt ggatcaagga gctccacata 30060
   aaatcacata cactgaaact aatagaaaag aaagtgggaa agagcatcaa gcacaatgac 30120
   agaggggaaa ttttcctgaa cagaacatga atagcttatg ctctaagatc aagaattgac 30180
   aaatgggacc tcataaaatc acaaagcttc tgtaaggcaa aagacactgt caataggaca 30240
   aaacagcaac caaccaattg ggaaaagatc tttaccaatc ctatgtctga tagagagcta 30300
   atatcaaata tgtacaagga actcaagaag ttagactcca gagaaccaaa taaccctatt 30360
   aaaatggggt acagagctaa acaaagaatt ctcaactgag gaataccaaa tggccaagaa 30420
   gcacctaaag aaatgttcaa catccttagt catcaggaaa atgcaaatca aaacaaccct 30480
   gagatttcac ctcacatctg tcagaatggt taagatcaaa aactcaggtg acaacagatg 30540
   ctgtcaagga tgtggagaaa gaggaatact ccttcattgc tggtgggatt gcaagcttgt 30600
   acaaccactc tggaaatcag tttggcggtt cctcagaaag ttggacatag tactacctaa 30660
   agacccagct ataccactcc tgggcatata cccagaagat gctccaacat gtaataagga 30720
   tacatgctcc actatgttca tagaagcctt atttataata accagaagct ggaaagaacc 30780
   cagatatccc tcaatagagg aatggataca gaaaatgtgg tacatttaca caatggagta 30840
   ctactcagct attaaaaaca attaatacaa gaaattctta ggcaaatggt tggaactaga 30900
   aaatatcatc ctgagtgagg tgacccagtc acagaagaac acacatggta tgcactcact 30960
   gataagtggc tattagccta gaagttcaga atacccaaga tacaattcac agaccacatg 31020
   aagctcaaga agaaggaaga ccaaagtgtg ggtactttga tccttcttag aagggggagc 31080
   aaaataccca tgggaggaga tacagagaca aagtgtggag caggaactga aggaaaggct 31140
   atccatagac tgtcccatct ggagatatat cccatataca gttaccaaac ccagacacta 31200
   ctgtggatgc caacaagtgc ttgctgacag gagtctgtct cttgagaggc tctaccagtg 31260
   cctgacaaat acaaagatgg atgttctcag ccaaccattg gactgagcac agggtcccca 31320
   atggaggagc tagagaaagg acccaaggag ctgaaggggt ttgtatcccc ataggaggaa 31380
   taacaatatg aaccaaccag tacccccaga gctcccaggt actaaaccac caaccaaaga 31440
   gtacatggag ggacccatgg ctccagctgc atatgtaaca gaggatagcc ttgtcagaca 31500
   tcaatgagag gagaggccct tggacctgtg aaggcttgat gctccagtgt aggggaatgc 31560
   caagactggg aactgagagt gggtgggtta gtgagcaggg tgaggggaga tgggaatcag 31620
   ggattcagag gggaaccgag aaaaggggat aacatttgaa atgtaaataa agaaaatatc 31680
   taatgaaaaa agtaaaaaaa aaatacaaaa aagaaagaaa agcaaagcaa atgctgtctt 31740
   gactgtgaag aacaacaaca aaaaatgtgt tttcaaacta aggatcccta acctttgccc 31800
   tagcacatca tcagactaga acaaatactt agcaacacta ctgcctggcc tccaggggca 31860
   cagtcagttc agggaaatag tgcaagaagc tagcattttt gagatgagaa ggaaaatgtt 31920
   ttggctagtg ggaagtcccc atgtctgcat ttctgagtaa gaaatgcatc tccatgtaca 31980
   tctgaccctg catgaccagg gcgcatttct gaccctgacc ctaatgttgc taacattagg 32040
   actcaccttg caaacccact gaacatgagt tcctggggtt ttgctgttgt tgctgttggt 32100
   cggttggttg gttgggtttt ttatgtttgt ttgtttttgg tttttttttg ttgttttgtt 32160
   ttgttttgtt ttttaatctc agaagacata atgtggtgaa agacgtccta gttctcagag 32220
   ttgagttctg gtctctgtgt gccaccagtt taaggatcgt atcagcaccc taacagttca 32280
   ttatttgcta actgacactg tcatctcaag cgtccctggg caccctatga gtgccacttt 32340
   taggtccacc tcttcccctg actcccattc aagggctgca gatgaggtgc agggtatgta 32400
   gcacttgtac atcatgtgac ctgaatctcg agagctgata ccacatgtgt ttctcaccct 32460
   gccccttggg agcttcgggc tcacaacaaa ccattctctg cagctcccat cctcaaagat 32520
   ggcctgttgg gacctcctcc tgcaaacctt gatttgatct ttaatggtag ctttcaaatg 32580
   tttgtaccac aggtgatgga agaaaatata ttttatccag ttctcactct ctctgtccta 32640
   cccaccttcc cttgctcccc ccctccccac acaaacacac atcgtttgga gaacagtatt 32700
   taacatatac tatattcagt gtactttgat ctaatgtcaa agcttatatt tcccctttac 32760
   acacatagaa tactcccttt ttaccttagt acttgcctca agtttcaaca tgtgactggt 32820
   tactaacatc cctcactcgc tcccagtgga ctgtaaattt cggtagggta gaatcggttc 32880
   taccaggaca gacctccaga atacacaggg catctgggtt ttcagatcac tggtctcttt 32940
   gctcacaccg ggcaagttga tcgttctgga tcatcctcct ttcccgaggt tggtagctgg 33000
   tttccagggt tacctctgag gcctgggtgg cctctgaatt gttgctctgt ctatggtgct 33060
   gtgagctatc ttcagttagc aacctgtatt acgtagcctt ctaggataaa tccttttata 33120
   gtaggtagct catgaagaat ccagaaaaca acgtcttgta atgaagtcag agtcttgaac 33180
   actgatgatt ttttttccca aagccccagg gcggctgacc tctgccccgc tgtaggctga 33240
   catcatttgc tgtacgctgt gaatgaggag gaaaagcccg taactttgtt tatgcagatt 33300
   actttttgtc tgacaaagct gcaggagtag aaggagcagg aggtgattta gaagattgct 33360
   tccgtatggt gagatggcat ttcttcagga tgatcccagt ctccgagtgc tcatgctttt 33420
   gtatctggag gtgtggaccc gtgattgata agcagtttct ccattttcaa atgagaccct 33480
   caagaagccc aagaaaatta gacagaaggt tataccatgg agggaaaaat gagattaaca 33540
   ttctttcttt ctctttaaga caaacattga cattaatagt ccttaaaaac attgaaaatc 33600
   ccggggagtc aagatgtcgg gttccaagta taagcctgcg cccctggcca ctctccccag 33660
   taccctcgac cctgccgagt atgacgtgtc tccggagacc agaagggcgc aggtcgagcg 33720
   cttgagcata agggcccggc ttaaacggga gtatctgctt cagtacaacg accccaaacg 33780
   cgtgtcgcac atcgaggatc ctgccttgat tcgctggacc tatgcaagat cagcaaatat 33840
   ttatcctaat ttcaggccca ctcccaagaa ctcactttta ggagctgtgg cagggtttgg 33900
   gcccctcatc ttctggtatt atgttttcaa aacagacagg gacagaaagg aaagacttat 33960
   ccaggaagga aaactggaca gaaaatttaa catctcctac taagtttggc aaggatgact 34020
   attcttgcat aaataaatct attaattaat caccgaaaaa aaaaaaaaaa aaacattgaa 34080
   aatcccatgt aaattataga tgttgagtat ttaataggtt gggggttttt gatgtggtgt 34140
   attttgttta tgtgtatatg cacataaacg tgtgtgtgtg tgtgtgtgtg agacattagg 34200
   tgtcttcctc agtcaccttc tactgtattt tttttttttt gagacagcgt cccccctgaa 34260
   actggagctc acgtgtgggc taattcggcc aattagctcc aagaatcccc ctgtcctgta 34320
   cctaacgcta ttaatctgcc tcccagtgct aggaccatgg cctttccatt acgacaccca 34380
   gcttgtcttg tgggttccaa ggatccttgt atgtgtggca aacattttac caactgaccc 34440
   tctgttcaga ggctggttgg aattgtgctg aggaggcctt gctgtgtggt atagaccagg 34500
   gtggctcaaa cttgcaatcc tgcctcagtc ccctaagttc tggcatgata gacagacacc 34560
   agcatgcatg gctaaaaagg ttttacattt tcatattttc cagttagaag ttattaaaaa 34620
   tattttcatt cttaggtaga atagaccttt atacattttt aaatgttcat atttgataaa 34680
   tgcaatgaag ttctttttat tcaccatact cttacctgga attttctgct tcattttaaa 34740
   ttgtgtttaa agtcaggaga aataggaaat gattttttat tttattgtct ttactacttc 34800
   cacacttcac tactttaact gaagaagctt ttatttccta cagcaagcct ttggtagttt 34860
   ttgttttctt ttttcttttc ttttcttttt ttttttcatt taaaaagctt aaactagttt 34920
   cacaaagtgt ctgtttgaaa agtgcaatct tccttgggca atgatttcca gtctacctcc 34980
   agctggtaga gtcactgact ctaaatacaa aatgtctaat tgcttcactc cctagtgcgt 35040
   actgcactaa gagttcttga tgttaaacaa ggagagttgc ttaaacaaga tgtagaagct 35100
   gcgttgaagc cccagtagat ccaagaaaag aaattgcatt aagtacagtg cagtgtctct 35160
   gataatttgg agtagagatc ctcatttgag gtgatggaca gttgaactat ggtgtcacta 35220
   ccacctaagg tatgtgtcct cagagtttca gaagggctca gtgcccttgc tttggatctt 35280
   gtctctgctg tccttgagct gtgcctctag aacaagttga catctccctt cagtctccca 35340
   ctttgaacgt tcagtgtggc ctgactcctt actgatttta gtgtgttaat tttgtcttcc 35400
   ttcctttctg tagctccttt gttctttttc cttaaccccc tttaaagcca gctccgctcc 35460
   tccatccttc agcttggttt ggtgtgtggc gggtcagttc actgagttac tggtcataga 35520
   tttaattgca tttttactga ggtcatgctg ttacaacagg gttgctgcct gaggaagtgg 35580
   agccaattct ttgaggccga ggcttatgaa caggctcaac ttgtggacgg gcattttggt 35640
   gtggccgtac tgctgaatcc tgggtagctg agcgaggtgt gggcaaacat ggatgcacac 35700
   tgtaggacaa ggattgcccc tacatgaatg agttggtcac aggaggccac tctgtcccac 35760
   gtggaagcca aaaccatttc tcagttcttt ctttgtactg atgttgctgc attaaacacc 35820
   accacccccc accctccacc accacccccc ggcctgggac ttcatgggat ggtgggaagt 35880
   ttcccagaat cttggatcac tgctaaagga ctggcaaaca tccaggtccc gagatagccc 35940
   agtgacatga agttgagggt gtgctgcctg cagcagagcg ttcgccccat cctcagcttg 36000
   ctacaccgag ttccgatgcc cgacagtgaa ccctctgcag cagtgaagaa gtcactgtgc 36060
   aaagcccagc ccgcctcaga ccacagaaga cgattacctc ccagaggtaa ccgaaaaggg 36120
   aaaactcggc gtgtctcata tactgccagc accatgctga aggagattta atgtgtgata 36180
   ttaagccttt cttctctgtt agacttggct tgagtgcatc acatctgcca gcctacattt 36240
   agcccttcaa ggatattctt ttgcctttaa ttgtggcatc tagacagtca gcgtgaaacc 36300
   gctgtgaagc agtcgccggc ccaagggtca gtccttcatc tttagaatat gggcggtgat 36360
   aaggagaatc tgtgtcctga aaccatgtgc acccctgtct gtcctccaca agtaaaggtc 36420
   tttcctctgt catgtgtcct ggcgcaaaca tttaccatgc ctgcaggaac atttggggct 36480
   ctaggcatgc gggcacagga ggcctgtgct gtttaatatt aaaccaacag gaaatgaccc 36540
   aggctcatgg gctactgccc tcatagggca aaagaggaga aattaattgc tttatagaaa 36600
   ttcctttctg tgttctgtag agaaaatgtt tccattgggc ctaaatgacc aaaaggagta 36660
   gtttggggct aaatagcttt gacattgtgt cctgaaggaa tgagacaatt aaagaaacta 36720
   acttcctctg agaacaacta tttataataa tttatctgca caacatgttt ccagacttca 36780
   ttggcctggg gctccctgga attgcccgtg tgtgtgtgtg tgtgtgtgtg tgtgtgtgtg 36840
   tgtgtgtgtg tgtgtgtgtg cagggtctct cattggcctg ggaccctctg attctacttt 36900
   ctactaggtg gatccccctc cctggctctg ggaatatagg ctcgtgctag tgaagtgtct 36960
   gtttagatga atgctagtga tcgaattaag gccctccctc atgttaacat ggcaagcact 37020
   ttacccactg tccgtgctcc agttcccttc tttttccttt tctgtctttt cttttctttt 37080
   ttctttaatg ctttcccatg cttcaggaac aagaattacc tggaacccca ccccctcacc 37140
   cccaaatgta tgcaggtact gcgattggaa ggaaaaacac atttgaagga cttaaggggg 37200
   agaaaagtca gtttcagaaa cggggtcagt caagaggcag agctaagagc tgcagtgtcc 37260
   tcccaaggat gttttcatgg ggcttggaga ccaacagcat ctgatataac acacagtcaa 37320
   gtcagaacaa aaccagggct gtccagcgaa aggtcaccag tggttttatc ttgaacagct 37380
   actgcagaac attgaagtgg gggtaaggag ggcagagggg agtggacaga ggcgtgtaga 37440
   gcctgcttgt caggggaagt gggtcatgct gactttgagg catgctgcct gtggaggaaa 37500
   ggtttcttat gggagtgggg ggggtggtca tgggatgcag agaggaggct gggcgtagag 37560
   tatcttaaga taagatgtga aacccggggt aaggagccat cttgagttag gccgtaggta 37620
   cctcctcttc ctgcctttgc ccacattcca ggaaaagtat tcgctgcaat aacagatttt 37680
   tttggacctc agagggttca ctgaatttca tgatgtttgg catctatcca tagttgaaac 37740
   aagaagagac aaaattgtac acccggagaa catcagagct gctgagaaat gactctcctg 37800
   taattttttt tttttttgaa gcctgtgccc actgctgaca tgggtgtttg cattaccctt 37860
   ggagcatatt ccacagttat ttacccatga aggattaatg atactgaagc cgggttcaat 37920
   ggcttcgcag caggctaaac ctgttctttt caagggttgg gaaagccttc acatgcctcg 37980
   ctctctgtca ctgatctccc agggaaagga aaatacccaa agcccagctc ctgggacagg 38040
   cttctctgaa gtgttgctgg aatgactctc cagcagggct ttcctttctt caattacctg 38100
   taatgtcttg tgcaagtttc ttagcctttc ctggcctcag tttccccatt tgcagagatg 38160
   aatctttacc ctgtttggag cgtgcaaagc ttaaatgagc tcatcaaaac agtcctagtt 38220
   agacagcaag tactctaaat tattactgac gttcttttaa aaagtactgc ttaacatttt 38280
   ctatattttt aaattagaat ataatggcat cattcccctc tccaaaccct ccacgtacat 38340
   accttactct ttctcagatt catgcctttt tctttaatta ttgttataca ttatataaat 38400
   atgtattatt tatttgtttc tatatatttc ttaaatatat gactacagtt ttcttattct 38460
   gtatgttaca cacacacaca tattataatt tcagggctgg ccacttggga ttagataatc 38520
   agttgggggt ggggcttccc ttgggaagac tattcttctc tcagtattcc ttagttgtct 38580
   gtagttcttg tctgtagctt gcattcttaa ttactgtagt aatagtaggg atggtggtag 38640
   actcttctga tttataaaca tgataaataa tctcattact cagaatatga gattttaaag 38700
   gtacacctaa aaataagaat ggaacttggc tagcacctat ggtctgagaa ccctggggcc 38760
   attcttacag ccgtgacagt ttgggggact cttccatttc tctctcctcc actgctggcc 38820
   ccactgtggc catttacctc aggactcttc tcactgacag gttctctggc cttaccgcct 38880
   ctgcctgctg agccttctct gtgtcgccag tctttcccct tggtcatgtg cttagctgtg 38940
   ctaactcctt cgccctagac aacagggttt aaacaacagg cactgctcct ttcaaactta 39000
   agtggtttgt aaatgagaga gaatgtcgac gattatttct tcctacagtt gtttatttta 39060
   caaactgcct ttgtaaacac ccttacataa atgtatacaa gctttgtgac taatttgtgt 39120
   tggtagccag tgtgcatata aagtactgtt acctagcaat ttagcggaaa gtgaagctat 39180
   gggcagaatt agttctaggg tgttctaaaa cacaaacggt tttattctgt ccttgagaga 39240
   gggggttgat tcagagtctc tttttttccc cttagactag ctttgaaact tgacaccacc 39300
   cttattattc tgcattcctt ctggcagaga actatacccc aattttatgt ttactatttg 39360
   ggaactacac tggagagcag tttaataaaa ctcacgaggt ggagactaat agccataact 39420
   cggtagtaag aaccaggcat agttgtaggc tcttggtggg tatactgttg actaacaaag 39480
   aatacagatc tcacagagat gcaattctgt ttcagaattg attaggggtt aagaccatcc 39540
   ttctacattc tcctatgttc tgcaagtgtt tgaggacaca ggaagagcac acttccttct 39600
   aggcagtgct gctcaatgtg tcaggcttag ctgagcatgc tttctgaagg gaaccctaaa 39660
   agtagtcagc gtgcacagtc cagtttgttc actgtgtgag ggatacatat tacctttctt 39720
   tgattttccc cttcttttct ttttttcctc tttccttact ttttaaaagt tttttttact 39780
   tattctttga aaaatctcat aacaatgtat ttgattatat tcatctccct tttccaaatc 39840
   ctcctaaatt cttcctcacc ttcgtaccat tccacctttt gtctctattc tctccctcca 39900
   accctccccc ccccgccccc gcccctttcc ctcactcccc tctcgtagtt gatctagtcc 39960
   agtgtgtgtt gagtgactgc tcctatgtgt ggagcctgct ttggagtatg ggcagtataa 40020
   caaacatcac tcattgaaga aaaccgactt tccctctctc tccggcagta atcatgtcaa 40080
   cagctccatg ctgggatttt ctctggcttg agcttgttca ggtcttgcgc gtgccgacac 40140
   aatttctgta agctcacatg tgtgtctgcc ctgttgtgtc tggaaaacac catttccttg 40200
   aagtcaccca tcacttcagc ctcttaaaat gttcatactc cctcttccat acagatcctt 40260
   gaactctcag gggaaggatc gaaacagaca ttccgtttag ggctgaacgc tttaaaaaaa 40320
   aaatatgttc attttaaaaa ggttatcgtt gtcacaagaa tgtcagcact aaacaaagtc 40380
   aagaaggcac acagttgact acactgtgta agaaacttag gaaaccatgg gggacaacat 40440
   tttagatccg ctggctgccg ttccattgta tttcctgtgt attttcatgg taaaaaatgt 40500
   tccctatttt ctttttaatt ttcatttatt ttatttggag attataatga attacatcat 40560
   tttctccttc cctttcttct tctaaaccct cccatatact ccttcttgct ttctttcaag 40620
   cccatggtct gttttttttt taattaattg ttattataca cacacacaca catttttttt 40680
   cataactatg agagtacaac ctgctagggc caaggttttt gaagtctttt attctctgtt 40740
   cattgaagtt gctgttttct gttcctatct atcacaagcc ctgccactag tcagtgagtg 40800
   tagctatgag gcatgggact cacaccttta gcagaatgat agtggatttt accataggac 40860
   ccatccatgc cctatgtagc cataggcttt cggccttatt gactgtgtca ttttgtagag 40920
   caggccttac acccatcaaa tccaatctta aaaagtgatt ggctcttccc ataacattca 40980
   tgccacgttt gtactagtgg atatagtttt gctggtcaga tcgttattgt aattcccagg 41040
   gtgcgtagct gagtgagagt cctgattcct tttcttctcc aggagtgtat gtagtcaccc 41100
   cagtagggat ggagcttcca gttaagtgct gggtagattt ctctgcgctt tatgactaga 41160
   gtgtgtgatg tccttagcaa cagagttacc agcaagctct ggatgacagt caagaacatt 41220
   ggaaataact taaaaggttt ggaggaggag cgtccatgag ataccattga ccgacagctc 41280
   aaaagatgta acccattcct ggtactgggg ttttagttca tggcatttga tgtttagatg 41340
   agctgtttgt tctgacagtg tggggtaatc tgcatttaaa tgactatagt aggttttcac 41400
   acagctttgt caaaaggcct cagagttgtt cctctccata tttcctcccc tatcctgccc 41460
   tctcatccca accccattaa aatccttcca gttctaaagt accttgagaa acttgtttct 41520
   atagtataag gagccaaaca tttactttta agggcatttc aggaatttga ccttgtttaa 41580
   ttaaggatct aacatttcca tttccctgat tggggtgggg ttggtggggg tgaagagtta 41640
   catgcaaaac caaatgtgtt tgagatctgc tttcatcttc gtgagaacca agtgtgcctt 41700
   attcctgaga tttgaggtag gcttaatggt aaggtaggtt tctcagcaag cctttctgct 41760
   ggcactggat ccatagggca ggcttttcct cttctctaaa tgctcttagc tgcagggcac 41820
   ctgaacccct gcagttatta gagtgtgtgc tctgtctgtg ctctgtctga tgaatcacag 41880
   cctctggaga ggagcccaga catcttcctt gctgaacagc tccctcgctt atggatgctg 41940
   taatttgagg atcacttccg ggaagaggat cccaggacag actgcaaagt ggacttttta 42000
   acgtttgcta acgttagcct gttgatactg ccttagtttg aattctgagg agtttcgtgt 42060
   ctaagttctg ttgtctcgat atgtaaaagg aaagtgtcat gtgtattccc agctataagc 42120
   atcttgcata atcacctagt aacgtcatag aatagcttca ggtcatcctt ttgactatga 42180
   gactctgtta aatattttga atttaagatg gataataagt gcagaataag tcaatgtaaa 42240
   agaaataacc gttttgtata atcttcatac ttgccaaatg tctcctaaaa aatgtcccta 42300
   taattctgct acttttgtcc tctgagcact taccatttct aagtagtaag aataatttat 42360
   gggtagtcta taagttttgc tgttttaaga aaataagtat gacattactc aagtattgca 42420
   tttagagaaa gatactgcat aaaatactac tttccaaagg ggggaaaaat tggccaggat 42480
   tctggactta aaaataactt cttcgtccat aactcagatc ttaggacttt accaaaacaa 42540
   acatagctgg catcttgaat agctatttca tgcacagact gtatttccat ttgttaagtt 42600
   attgaagggg ttagtgtatt ttactaagtg accaatactt tagtagcagt gagtgaagag 42660
   cctgtcctat taaggacaga ggtaaagctt ttgcaggatg taggggaaca gaatcattag 42720
   gctctttacg caggatgaca ggacgtttcc actgatggta gtgatatcat tgctcttgga 42780
   ggccgttggt agaaattgag cctcgctttt ggaaaaggtt ctatgtaaag ctaagttagt 42840
   gtaactattc cccaagagag gcagcgtcag atccttagag caattgcttg ataacttagg 42900
   aagattgaca ttaagataac ttagacattg aagttatttc cagttctgcc tgccaagccc 42960
   atgttcccag aaataagact cagactcaaa atatatttac aaataccttg gacatatagc 43020
   taggttcttc tctgactagc tgttacttaa catatcccat ttattctagc ctaccttctg 43080
   ccacatggct ggttacccgt gttcaagtac catgtgttcc gctagtcaca tttctctgtc 43140
   tccttctctc ccagaatcca ttcttctctc gatgtctcac cctctatttc ctgcgtaagc 43200
   cataggccat aggcttttaa attggcaggt gatgcatcta tacaacacaa gaagatgttt 43260
   tctctacagt gtagcttctt ggtttccatc atttaagcca aagttttgag aaaagatgga 43320
   gctgttttgg aactaggtcc gacaaggatg gggaaagagg tgtttttaaa tcccaccccc 43380
   cctcgacccg cccaagcctc tttaggtact tatggaaaag gctgttcctt cctttaaaag 43440
   tctttccgac cattctggct gctggacgtg ccaacattta gtgctacaac ttcatttaca 43500
   catggcagtc agagagacgc ctctggccct gtggtttcct agtgcggctg atcctgtagg 43560
   ctgccgtcct gattgttcca cttggggatt ctcaccatgc ttatcagtca gcaccggaaa 43620
   ggagcccaga gatcacatag tccagccctc ctgtttacat gtaataaaag cccagcccag 43680
   cctcattgac cagcgtgctt gatggcagac agatggcaga cagcacacct ggggctgtgt 43740
   gtctttaggt tcgcactcta ccgcattcta gatgcacaca ttgcacggag atggcctagc 43800
   ggggttgtta aaaatgctgg aagcaggaag gaggacacct gatgtctcgt ggtctgactg 43860
   cactgttcac cctttctacc tgggttcctc ctactaatgg ttttagtcca aaaacctgaa 43920
   caaaagctgc ctcactgcag atttcccagg cactcctggg ccaagttatg tctctctatt 43980
   atctattccc taggaactgt ggacatcctc ctgttgtatt tctaaggctg tgattgacac 44040
   actgcccatc ggcctgcctc tcactcccga ttggatggat taggctttct aagaacacga 44100
   acacatcctc agcttcagcc tacaatgctt ggctgagggc actttgtagt tgccccagtg 44160
   tttgatgagc gagtgccgag ccagtcattt tcttacagtt ccagcaaata tttgaccttt 44220
   ttttaggttg taaaaacgac tcagaattgc tggctcaaac tgccattttt catgctgtct 44280
   cacgagagtc gattcagaac aggtgtgtca aggtagctac acatttatta aataactagc 44340
   ggggtggtaa aaatggctca gcagctgctc tgggcaagct ctgttctgca agtagttcat 44400
   ggagtcttgg tcccaaatgt ctgtcacggt catcattgat ctgcatgaaa acatagaact 44460
   tcttgcccca cccacagcca ccaatctttc atcactatgc tgactcccac gagagtgacc 44520
   tgttagacaa atccccagac tcactccagt taaccttagg aaaacaaaga actgatggaa 44580
   catgccttgc ccatgtaagc gtttttttag acatgaactt gaaagaaaac atccaaggga 44640
   agtagtaact acactctctt gcgatcgtga ccatgaggac tgctggcata gttccacact 44700
   gtactggggc ctctttatca gattaacaga aactagagtc acctgaggag agggaacctc 44760
   aaatgacaag tttcctccat aagactggcc tgtggaccta tctttggggc attttcttga 44820
   ttaatgattg atgagggaga acccagacct ctgtgggtag agccatgctc aggcaggtgg 44880
   tccctgttta tataagaaag caatgtgaac aagccatgga gagcaagcca gtgagcagtg 44940
   ttcctccaag atttctgcat caagctcctg ccttgagtgc ctttcctaac ttccctcagt 45000
   gatggactgt gacgtggagg agtaagccaa atgaaccctt tctttttcca gattcgtttc 45060
   aggccatggt ttatcacagc attataaagc aattaatacc cacacagaca cacacagaca 45120
   cacacacaga gttcatgttt tatcacagca tcaggaagta accagtaccg acacacaaca 45180
   tgtatacaca cacatacaca cacacacaca cacacacaca cacacacaca cacgagtaca 45240
   tgttttatca cacatcagga agtaaccaat acagacagac acacacacat acacacacac 45300
   acacacatac acacatacag agtacatgtt ttatcacaca tcaggaagta accaatacag 45360
   acagacagac acacacacac acacagagtc catgttctat cacagcatca ggaagtaacc 45420
   aatatacaca cacgtgcgcg cgcgcgcacg cgcacacaca cacacacaca cacacacata 45480
   cacatgagtc catattctat cacagcatca ggaagtaacc aatatagaca tagaaactta 45540
   caatgtcttg cctcacactt tttttttttt tactttacaa cactgcaaaa atgatatgtg 45600
   ctcagtaaaa actgtatttc aaggcttgaa attttttatc ttttcccggg ctagcagtag 45660
   cattaagatg ctcccagtgc cccacagcag cactaggcaa cagctcccac ttggctacag 45720
   gatcacaaag gggaactgtt ggtgttccac agtatgctgt gttgtccagc agtcatattt 45780
   tctctcctaa ccatgatttg ggtgggtatt ggcttcagtg tcttgcgcat gttaggtaag 45840
   cactctgata ccaaaataga tccctagccc ctctgagttt tcaatcttta gacaggaatg 45900
   agtggtcttg aacttaaaat tctactgcca gggtttcaca ggttgctgag gatataggca 45960
   tgtggcactg tgccaacctg aatgaaattt tcatttacag tgttttcaaa ttacaaatgt 46020
   atggaaaacc cagtcatatt agtataccgc tcggatttag atcctcatgt ggtttgtttg 46080
   tttgtttgtt tgttttcatc tgtagattaa ttcatagttt atctgtaatt gaatttgtgt 46140
   ttctgtcatt ttaaatgtca aaataatatg ccttatattt tctgctgtta cttgctcaat 46200
   agtctccctg atgttgagtg ctttggctat tttaaattgt tttctgtgat cctatcatat 46260
   agagttctta tatccactta tgcatgagaa ttccacaagc tatattctcc aaagtgagag 46320
   cattagatgt gaaagtaaag gcagttgcat agttgtcttg attgttatag tattactttc 46380
   aagagacggt atcctcatct accagctccc agcagtctgc ggcatgctgg gaaatgttat 46440
   ctctgggacc tgttttagga aagtttgttt ccaaggtttt taagtgctgg gaagtagttc 46500
   atttccttat aggatggctt attttcatat aaaggataaa catgtcctct ttatgttgga 46560
   gagttaagaa ggtaagaccc aactgccttg tatgattgac tatgtaggga caggggacag 46620
   catcatttta ggtgtgttag gaatgagaat tcttgatgtt atataaagaa acacaccacc 46680
   aaagagaaag ttatcaggta aggcaatctc tttttataga aaggatacgt cagaacccat 46740
   accagcatga gtggcccaaa aggccttggt cttttttatc cccgatgtgg gtggtacctg 46800
   catctcattc caacttcata atcttcacag tgcaacacag tggcttcttc ttcagggaag 46860
   aagaagaaga ctcgtccagt taactgcctt tgatagaagc aacagatttc tcacaggtca 46920
   gacctgaagt gccagggaag atcagttgtg tggccctgaa ctaggacttt tgggtttgtt 46980
   taagaactga agaagccaag catagttctc agctcactga aggctgagac aggaggatca 47040
   ccagacttag tcagcaggct acatagtaag actctgtatc aagaaacaaa aaaagccaat 47100
   caacagacct agaaaggagg gacattctga tttcatgata gaaagaaatg ctgaggctct 47160
   gctatggaga gcagcaagag tctgtcctgg tggacctttc cctccagagg gtcgtctggt 47220
   attcccgctt ctgaaagacc actatgtttg atggaaacag taatatggaa ttccagaagt 47280
   ttttggggga ggaactgaag gcagaaattt ctgaaacagc ctgtattgtc tgataaacct 47340
   cagcagggag gtcagaaaag gtcagatgag ggtgatgcct ggttgtctag gctctccttg 47400
   ttatatgttg gagcatcttt tgggtatatg cccaggagtg gtatatctgg gtcctcaggt 47460
   aatactatgt ccaatcctct gaggaactga cagactgatt tccagaatgg ttgtaccagc 47520
   ttgcaatccc accagcaatc gaggaacgtt tctgtttctc cacatcctcg ccagcatctg 47580
   ctatcaccag agttttttat cttagccatt ctgactggtg tgaggtggaa cctcagggtc 47640
   attttcattt gcatttcccc gatgactaag aatgttgaac atttctttag gtgcttctca 47700
   gccattcagt attcctcagt tgagaattct ttgtttagct ctgtacccct cttttaaata 47760
   tggttatttg attctctaga gtctacttct tgagttcatt atatatattt gatattagcc 47820
   ctctgtcaga tgtaggattg gtaaagatct ttccccaatc tgttggttgc tgttttgtcc 47880
   tattgacaaa cactgttctt gccttacaga agctttgcaa ttttttgagg tctcatttgt 47940
   ctattgttga tcttggacca taagccattg gtgttttgtt caggaagttt tcccctgtac 48000
   ccaagaattg ggctttcccc caatttctct tctattagag tcagtgtacc tggttttata 48060
   tggaggtcct tgatccactt ggacttgagc tttgtacagt gagataagaa tggatcaatt 48120
   tgcattcttc tacatgctga cctatagttg atccagcacc atttgttgaa agttctgtct 48180
   tttttccact ggatggtttt agcttctttg tcaaagatca agtaaccata ggtgtgtggg 48240
   ttcatttctg ggtcttcaat tctatcccat tgatctacct gcctgtctct gtaccaatac 48300
   catatagttt ttattactat tgctctgtag tacagcttga ggtcaaagat ggtgattccc 48360
   ccagaagctc ttttattatt gagaatagtt ttcactatcc tgtttttttt tttttttttg 48420
   gtcagacctt caaagaagac ctaataccaa tacccttcaa actattccac aaaatagaaa 48480
   cagaaggaac actacccaat tcattctatg aagccacaat taatgcttat acctaaacca 48540
   cacaaagacc caacaaagaa agagaactta agatcaaatt cccttgtgaa tattgatgca 48600
   aaaatactca ataaaattct tgaaaactga atctaagaac acatcaaaac aatcatccat 48660
   catgatcagg gatgcagcaa tggtgcaata tatggaaatc tatcaacata atccactgta 48720
   taaacaaact gaaaagccag gcagtggtgg cacacgcctt taatcccagc acttaggagg 48780
   cagaggcagg caaatttctg agtttgaggc cagctaggtc tacagaataa gttccaggac 48840
   agccagggca acacagagaa accctgtctc gaaaaccaaa taaaaaccct gaaagtaaaa 48900
   atccacatga tcatctcatt agatgctgcg aaatcgtttg ataaaattca acacccattc 48960
   atggtaaaag tcttggaaag atcaggaatt caaagcccat acctaaacat agtaaaagca 49020
   atatatagca aactagtagc caacatcaaa ctaaatggag agaaacttga agcaatctca 49080
   ctaaaatcag ggactagaca aggctgcaca ctatctccct acctcctcaa tatagtactc 49140
   gaagtcctag ccagagcaat tagacaacaa aaggaggtca aagggataca aattggaaag 49200
   gaagaagtca aaatatcact atttgcagat atatgatatt atacttaagt gacccccaaa 49260
   attccactag agaactccta aacctgataa acaacttcaa caaagtggct ggatataaac 49320
   ttaactcaac caaatcagtg gccttcctgt actcaaagga taaacaggct gagaacgaaa 49380
   atagggaaaa gacacccttc acaatggtca caaataatat aaaatacctt ggtgtgactc 49440
   ttactaagca agcaaaatat ctctatgata agaacgtcaa gtctctgaag aaagaaattg 49500
   aagatctcag aaaatggaaa gatctcccat gatcatggat tggcaggatt aatatagtaa 49560
   aaatggctat ttgctgaaag caatctacag attcaatgca atccccatca aaattccaac 49620
   tcaattcttc ataaagttag aaaaggcaat ttgcagattc atttggtatt ggctactgtg 49680
   ttaacatgag tccctgcagt gtgagcacat gctacattaa tactaacgca aggtaacttt 49740
   ggtttgggac aatctaatct gatttttttg aactggtgat gttacaattt ctgattagta 49800
   tcagtttctg attaaaatca cacagagata aaacctgagt tcccccaatt ttactctaaa 49860
   ctaggtaatt tctgcatcca acctgatttt gtactgacgc ttgtaacttt tacctagatc 49920
   tttcacagct tagatacagc acctacgctt cacgtgtatt taatgtgtat ggcctagtga 49980
   cttttaatgt agtcagaact gctgacctgt ccaacacaat tttagaacat tttcatctct 50040
   ttaaaaagaa accctgctca gaggcatttc cgttactctg tggtcccgtt cacccttttc 50100
   atctcgcaat ctaccttctg gtctgtagat tttactactc tgggaaattc atatggagaa 50160
   taatacctat gtggtctttt catttcgttt cttcccctta gaatgatgct cttggaattc 50220
   tcccaaatag taccatatac atagtactag ttggttcctt tccctgctga gtgtgtttcc 50280
   ttttatggca gatcactttt tgtgcagcca ttaactgatg gacatttgtg ttgtttacac 50340
   gtttagcata tatatcagaa gatggcctag tcgaccatca gtggaaagag aggcccattg 50400
   gacgcgcaaa ctttatatgc ctcagtacag ggaaattcca gggccaaaaa gtgggagtgg 50460
   gagggtgggg gagcgtatgg gggacttttg ggatagcatg gaaatgtaac tgaaataaat 50520
   acttaattaa aaaaaagaag aagaaatcag caaaaaaaaa aaaaagaata atgctggggg 50580
   ggttgtttgt tggtttgttg gttggttggg ttttggtttt gtttgttttg ttttgttttg 50640
   ttgttgtttt tcaggggttt tgttgtttgt tttgttttgt ttgctttttg agacagggtt 50700
   tctctgtgta gccttggctg tcctggaact cactctgtag accaggctgg cctcaaattc 50760
   agagatccac ctgcctctgt ctcccaagtg ctgagattaa agacacacat caccactcac 50820
   aactagctgt tgtggttttt gtgataataa tgttctgaat gttggtgttt taagttttat 50880
   gtgcatattt gttttcattt tttggggagg tgtagctatc agtggggaga tatatatata 50940
   tatatatata tatatatata tatatatata tatatacaca cacacacata tatacatata 51000
   tatgtatgta tatatacaca cacatatata catacacaca catatatata cacatatata 51060
   catatatagt tcagcttcct gaggaactgc cagattgttt ctggagagaa ctatactatt 51120
   tgtatcccta ccaacaatgc atgtgtgtgc ttctctgtca cctggcaaca cttgtgactg 51180
   acagtctagt gctctgttct ggccattgta ctgattgctt agaggcatac tattatgatc 51240
   tgctatacat tcctctaatg acttacagca tatagtagac tgctcactcc taacgctgtt 51300
   ttccttcact gttaaataaa ggcaatgctc tgggaactta aaggcagttc atactctcct 51360
   ggcgctgccc catctgggga cttccatgct ttatcagccc tagtacaagg gccagcagag 51420
   tatgaatgca tggcatgcag tctcttcttc ctcatgctga acactatgag gctgtcctga 51480
   tcggcctgcc ttggcttatg tgtctacctc atggcccaca tttctgcctg agcagtgcac 51540
   agatgagttg aggcattttg aaagaaaatg atgcttttgt ggaaagagaa gtgatgacca 51600
   caggcagcaa tctcctgccc atctctgtgc atcacacaga aggctgggaa ccaaataatt 51660
   gtgccctgta cattgtcatg acggacattt atgtcttaat tgggtttttt gttttttgtt 51720
   tgtcctggaa ctcactctgt agaccaggct ggcctaaaat tcagagatcc acctgcccct 51780
   gcctctggga tcaaaggggt gcaccaccac ctcccggcgt cttaattagt tataacccca 51840
   tagtacatgg aagtgctctg ctcacaactc agccaagaac tgcgttggga ttatagtgtg 51900
   ttgggttagt ttaaattaat cacttcatgg agattttttt tttctatttt aattctaggt 51960
   attcatcgtc agaaataagg aagcagttta cgctcccacc caacctcgga cagtaccatc 52020
   ggcacagtat cagtacatct ggtttcccct ctcttcagct agtaagtatg cattacaggt 52080
   ttggtgggtc agtggtcagg gactgaagta cccagcatct acacccctcc cccacaccag 52140
   ccttagctcc tgttgcacct agcgttctct gcccactgct tcgtaactct gctagccctt 52200
   agacatcaga catctccctc tggcgtgtat agaatgagac agcccagaac tgtggttttt 52260
   attgatgtcc tcaacttagg gggcttgcag gctaccccaa aaccatccca attacagttc 52320
   ataagtggga agacgaggta gcctagaaac tgaccatgat ttttagctta tgttagttag 52380
   attttttttt taattgaaaa atctcagaag cttgagatac agatcgttct tatgggataa 52440
   ttttcttgga agtaaattta catactatga aatttatcaa ttttaggatc agggttttgt 52500
   gagtatgggc aatttatgag tcacttaagc tcctcccaga aagagtgtag agcgctttcc 52560
   ctcccaccct tttgcatccc ctcgttccca tcccaggagg tccctgagcc ttgaacttgc 52620
   accagtgaaa ccattcacac cagtgaaacc attcacatgg gagagcaaat aacagcttta 52680
   taacataata tcccctttca aggtacctac agtttgacat taactaatca actggggagt 52740
   atggagggtt tgttacttta tttgactaat aatatgtgta tcatattatt taaggactgg 52800
   aggccagatg tgtgtctggt gaggccaaac tgtcttcaaa tgagactgtt tgcaacccag 52860
   aagtgggtgc cgcttcctct gagccagcag agcctgctct gccctcactt cctcatacaa 52920
   gcacaagcca cagccactct ctctggctga tctagtggtt atcttgagaa ggttaggttc 52980
   ttttttgttt ctacttatta cttcaaagcc acttctgctc ttcgaaattt tgtaaccttc 53040
   ccctgtttcc tgtagccgcc attttgtcgc ccgtgacagt ttacccaccg cgttaactgt 53100
   gtgtcttcag gtatgtttta ctggagcaat ttgtggttat ttcaggctag cttaatgggt 53160
   tgtgtcattg aggagacagg actaacagat gtgtggcacc ttcactgcca gaggctacct 53220
   tggtgacatc ttttggagag ccctgtttag gactttggag tcttcaggcc tccctttttt 53280
   atttgtcttt tcctgcctgc ttcaggttag gtaaaaaaac acagtaggtg cattgtgcct 53340
   gtgttctcgg agtaagtaaa cggagacctg ttgcatataa gctcggctga gtgatttctg 53400
   attcagatac atttcaaaat gtgcaccatt cggtggaaac gtgattggat gtgcttttca 53460
   attttaaact gtgtggggag ctttgctact tgccttcaaa gagtgtgaag aggagagctc 53520
   tctgtagcag ctgctgggga atagtgtgga cagtaaagag gttttcaggg aggtttaacc 53580
   cctcatctcc ctggcagcct ttgtgataat ccgatagact agatagagtt agtacatgcg 53640
   aaatcctgtt aagtatttgc aagtgccccc tcagtacttt tctgtctcct tagagagact 53700
   ctgacaacag tgaattttga cgagtcactt caacagctgg gaagtcaaat taacaagagg 53760
   cttagctgtc catgttgtca cgaacagaag gtatagagga agccggaagg ttaaaatgct 53820
   tgctacaaga accacgtctc catctgctgt ccttcaatcc tgatcctgtt tgtatctgac 53880
   tgatcctcac attttcttca tcctgcttgt ccgcatgtgc tttccttggt cttccccttc 53940
   cccagatctc ccttctaact tctgtgcaat cttgcctctg tttcttctcc caatgggagt 54000
   caagacgcct gctctacctt ctaaaaaaat ctcatgctct ctttagaaca acccagtgcc 54060
   ttggcctgtt ctcctgtgat gctgtgggaa tgtgtgactc tgcctccttg gtagtcacct 54120
   tctcagacaa ggctcctggg agaaatcacc aacttataag gtgattttcc ttttccttga 54180
   gatggattcc tttcctaaat agtggagcac ttcagtaaga gtcacttcta gcaaggcacg 54240
   ggagtatctt agacgggaaa gactctcaac ctaccagagc attagaaaat atcttaagtc 54300
   ggagactgca gtatgaaccg ctagttggat tatttttggc taattaaaaa ataagacaat 54360
   gggcattttg gactatttgt tttgctttaa gtaacataat cgtttccata acctcataca 54420
   aatggttgtt taaacatata tatttagttg aagtccaggc atttaaaacc actcatcaag 54480
   aggtcatttg ccatttgcaa gagtaaagag tttggggtca gatgaaccta aggtgaaagg 54540
   tggttgtcac ttattgtttg tataacctaa gagaagtcac tgatttcagc cccctctcat 54600
   gcaaagctgg gtccatgtag aactctccag tagcgcctgt gtaaatagtg ggtaatattg 54660
   ttgccttagg tacttggcac tggagagact ctgtggacat taaagataag tatcagattt 54720
   ttcctgctcg gggaggcttt gtgatctatt tgggaagaca agtaatccag agggcccaca 54780
   ctggcttcaa atgtgatgga cattcagaag aaaggtccat agtgtgatag gaggcaaagc 54840
   ttgccggagg agctgagaaa cccagggaga tggggcttca gccctgatga actgttagac 54900
   atgcctgact tgttcttata tttctagcca ggacccctgc aggacagtga ggctggaacc 54960
   agcagtgctc taacattgct gcaggatgtt gctaggtccc cagagtcacc taaagattct 55020
   tagaggcctg ggcaaattcc aaatagcagg aggtatatac agcaaggtgg tttctttggt 55080
   tgctctccat gctctgagct gtagatatgt ggtaagcaca gactacatgc tactcctgta 55140
   gataatcctc gcaaactaca gctcttcatc agacatctaa taaatgagta gaaggcagtg 55200
   catttgatat tgtttccctg ttttattcag ttttgtatgt gtgtgtgttt ttggttttgg 55260
   ggggtttgtt gttgttttat ttatttattt tttttgagat aggctttctc tatgtagccc 55320
   tggctggaac ttgctctgta cttggtcgag gctgacctcc aactcagagg tccacctacc 55380
   tctgccttcc aagtgttggg actaaaggtg tgtatcacca ctgcctggcc agtttttgtt 55440
   tgtaatgcta gctataaatg cctgccacga tgactgtgtg tacaaatatt ttcaaaatag 55500
   ataagctact tttttaaaaa aaaaaattca tttattttgt gtgtgcatgt gcacacaggt 55560
   gctacggcgt gtcggtggag gtcagagttc agcttgcaga agcttgtcgt cttctgccac 55620
   gtggattcta gagactgaac ttagggctcc tctacctgcc gagccctcgc accagctcaa 55680
   taagctgctt ttacaattag catgagtgtt ccaggcatga tagtggatgg tgcctgcctg 55740
   acactcttat cagaggtcct tctgaacaga tgcagagaga caggcctctg gccatcccat 55800
   gtcctagcct gagatgcaca ccagctttgg cattctctac tgccactgtc atgggagcag 55860
   gtttaagcag agatctggtg ccatccatct atgtaagcaa acaactctac acttgatgct 55920
   tgtagtaaaa acaaaaacaa aaccaacctg caaaatgcaa agcctttcct ctggttgtta 55980
   aaagtttaaa gtatagtaag tatgtaagtg aaagggctgg gatctgtctc taacgcagca 56040
   aagaggattg aagacctatg cagaggttaa taactttaag tgcattattt aataaaggaa 56100
   aatataacta ttttttttaa aaaatagaaa agtctgaaga gaaaacaaag aacagcctga 56160
   tcgtgacggc tctgggcgag gtgaaggatg taaaaagcca caccctgggc acgcacctga 56220
   gcaccatagc tacgggacct gtttatgtgt ctaacaattg tggcttacag atgtctcatg 56280
   cccaagaagc atttcttctt gtgcacacac tggcttctgt gaaatacagc acccctttct 56340
   ttgccagctg agggtgactt gagcacgggc tccctaactc ctgacctcct ttgtgccttg 56400
   gatgccattg ttatctctca cctaatttct tcccagactt ggctgtgctg ttttgttgat 56460
   tatacggaag ggacatggca gaaaatgcag cgcttggtgt agttcctata cacattctta 56520
   gcgtggatga aatagaaggt agggcaggca ttctgtatga agagcaggca cgtggttatg 56580
   gtcactgtac ccaggggatg gaaacaggcc agggagatgg ctcagtgtgg aaagtgtgtg 56640
   ccactcaggc atgagaacct gagtttagtc cccacaaccc tcataaaaag ccaagtatgc 56700
   tggcacctac ttacagttgc aacactggga aggtggagac aggaacgttc ctggggcaca 56760
   ctggctagcc agcctaatct agtagccccc atgtcccagg gagagcccca gcctccaaaa 56820
   ccaagacaga tggcttccga ggaaaacctc aggttgacac atgtgtatac ttgtgcgtgc 56880
   gcacgtgcgt aattgactta ttaagaacca gatcggtggt catggtagca tctcagcctc 56940
   tactgtggca tcagtggaag atttccgttg ctggaggcag ggaggcaagc ctcgtttagg 57000
   tcacgaagta ctttgaaatc actgaaggca gaggaaattt tctgccttaa gttttgctaa 57060
   gtgcagattg gtgactgtct ctttttattc attgaggaag tgtttattgg gtcttagtct 57120
   gtacctgcag ctgctgtaag agctacggat aacaggcatg aatgtaacaa agggtgcatc 57180
   atttgttccc atggaacact ttagaatagg agatgggtgt aaaccaatga atgaattggt 57240
   cagccgtgat gagggcagga agggttacaa agaatcaaac aggcagaggg aacggggaca 57300
   cagataagat gctgttgtag gcaaggtagc ctttgaggac ctctgtctgc aatggagata 57360
   cccaggaagt gcccagcatg agcctgacac cttgccaaaa aaaggcattc caggcaggag 57420
   gaagaagcat gtctagtctg caaggcaaga gtctttggcc ccagaaacag ccaggaagca 57480
   gagagggcaa caggggagac catagctaca gcagcatcgc caattatagc tgagaacaca 57540
   aaatgtccat tcatgcaggt aagaagagga ggttctggga gcactgggct aggctaacct 57600
   gaagtccatg gagctggttg ttgggggtcg gggggggggg gaagcagagc aggctggaac 57660
   cccatggcag gagtggagct gatgctttac ttcctgacca cacacacact cacacacaca 57720
   caacctcaca cacacacact cacacacacc ctcacacata cactcacaca ctcatacact 57780
   cacacacata cacacacact cacacacata cactcacaca cacacaaaca cacactcaca 57840
   caccctcaca cacacaaaca cacacacact ctcacacaca tacacactca cacacataca 57900
   cacacactca cacacacaaa cgcacatata cacacaccct cacacacaca cactcacaca 57960
   cacaaacaca cacacacaaa cacatatata cacacacacc ctcacacaca cacactcaca 58020
   cacacaaaca cacacacaca aacacatata tacacacaca ccctcacaca cacacacaca 58080
   cacactcaca catacaagca cacacacact ctctcacaca cacactctct cacacacata 58140
   cacactcaca cacatacaca catatacaca ctcacacaca cacaaacacc ctcacacaca 58200
   cacatacaca cacacacaca cacgaacacc ctccccctgc cacctaaaca aagataatct 58260
   tgaaatcgct ttggatcatg gcatttaaac tagggtcttc ttgttctctg ctgactaaga 58320
   agacagaaga gaggagaaag cagagtggtc aaggaaagac ttggaccctt gcaagccaaa 58380
   gagctgaaga tccaatgtaa ctgctacctt acctcatgct cagacttcat ccgtcatgca 58440
   gtacactgaa gctgtagctc ttcagtactt gacaaatgtg tgcgcgtgcg cgcgcacata 58500
   cttatgggtg ctgtgtaagc atttatttct tcctgtttgt ggtgattgcc tcgtggtaaa 58560
   acctgctcat gagattaaag cgatagatgc tctagagaac aaattcttgg ctattatttg 58620
   attgtttgat tcctggctat tatttgaact tgtaggaaga taaacagttt gaaggtctat 58680
   caagttaaga gaacttggaa tctgcacccc attaccactg gcaagacaat tttctatcac 58740
   actggaatga gaagactcgt taccttatca gtttctctat agaaataagg agagaggtcc 58800
   ccaagtgcca gctgatgtgt tctagagagg catggtctag ccatgattcc gtcctacagt 58860
   ttgagcttac agcctgctcc acttctggac tatgttcaca cataaagagt ttgagtttat 58920
   tgtctgtgcg tgtggatgta tgtttgagaa ggttaaacat cattatccag tggttgaata 58980
   gagggtggcc ccatgctctc tgtatcttaa taccaaattc agtagaggct cagtgagcaa 59040
   ctgcatatga tgggtcagac catcatctgc tcctggtctt ggtccatctg tcctataaca 59100
   gttgctgagt cttcaccttt acagctagct tcaagtttca ggaacatggc tgggttttat 59160
   tttagctaga ctggcctttc cttcctttcc tctccccccc cccccagtgc ttgttcatta 59220
   agcataactg tgttcctcag tgcttttaaa gcatatttca ttacccgtgc ttgaattcct 59280
   catcatatac ttataatgtc attcatttat tttctattac atctgagcat ccaatcccct 59340
   gagaagtttt gaggagaatt tgtgggaagt tgtagatgca atttttacct ggtttgatac 59400
   acacttaact attactttgt acttcagtta tcttaataaa tactgactct gtggaaggca 59460
   tatatgcaag aaaaataatc aaaattatag tggttaagaa gacagcctga ctatacttga 59520
   attccaaatg tgctacttcc tagctatatg atcaatggta aattattata tcatctgaaa 59580
   atggggatag ttaacatgca tattccactg gattctagtg agactgactt ggaaaggccc 59640
   atgaggactg tggtcccttc ctcggctgac ttcaggctat agtcagaacc aggggatact 59700
   ttcccatctt cttgccacct gatgatcgaa gggatctctt tgctctcagt tcaaaccgta 59760
   acgctttctt ctgcctccat tgcatcctgc tgacagtgag tgctatgtaa gtcagccatc 59820
   taaaagaatc tgggccagaa gtaggtagtg gatttacttg ggtttcttta gaggagcaga 59880
   actggtagaa taaatatcta tttatttaag ggggttatta aaccccctta caggctagtc 59940
   tggctagtcc accaatggct gtcttttgac agaaaggaca acagtccagc agttgttcgg 60000
   tccacaaggc tgggttctca actggacttt aatgtacttt ggaatcccaa agaagcaagc 60060
   tctaaacctg ataaagggat gcctcagcag caggagagat gagatcagag agagtgcggg 60120
   caagcagggg acaagaaaac tcttccttct tccatgccct tttctgtggg ctgctacccg 60180
   aaggtgtgac ccagatttag ggtgagtcat atgacctcaa atgatctgga tttataaggt 60240
   agctcttcct atctccaatg agccaatcag gaaaagcctc tcacgggtat gctcggctac 60300
   ttgagtttta gttgattcca gacatagtca agttgacaac taagaattgc catcaccggc 60360
   agctgtatct tctctctcag gagtaagttg ttcctgtctc agggaaaggg agacagtgaa 60420
   agccatacat agttaaattc tggcaccctt ttgccctttg cctcttcaac agcactattc 60480
   ctccacccca gcttaaggcc tgctggtatg tgggctcaag gggatgaaaa accagttttg 60540
   tcagcccttc cttcaggctg gccctcccat aggacaagtc cggaatctgt ggcttttgtt 60600
   ttgtgatgct ttggggattg ggaacagaac agcgaacgtt cacttgggct gtgtcagagg 60660
   gctgcagcct ctactgctgt tgttatttct gggccagtct ccttgatcta agtggctttc 60720
   actgccttaa ggcagtggtt cctggatctt tgcatgcatc gggctagacg tattttgttt 60780
   aatacgggac tggcaaaatg ttgcctattc ttaatactat caactgaaga gcaacattgt 60840
   ttcagttact ctgggtctct atcctgtcta ttccctcccc ctcccctccc ccctctctgt 60900
   gggagtatga aacgaagatg gggcagcttt cctcagcaga tcctcagggg ttgctcgccc 60960
   aggttcagtc ctgtgcgttt cttcccgact agtagtgttt ggaaacctca gccatagagg 61020
   ggtttagcca caagcctggc tccaggtcag ctgggactgt gtctaagctg ctggctttga 61080
   gcagcagtgt acagccgtct cggtccaagg gaaacactgc atctgagcgc acagccagag 61140
   gagtgctgcg tttgcctgag tttctggaga gttgccttct ttctcttttt cactgtgttt 61200
   gggtaaacaa aagcatagcc tccgagagag agcttttgca ggagaaatta taaaaattcc 61260
   tcaaagcctg tgatctcatt ttggatctcc tcaggattcc tcatcaggct gcaattaagc 61320
   cgagaaaaat tctgccttgg ggagggattt taattactca tcgtgttgtg acttgacttt 61380
   ctgctactgc ctttctttac ctctcctgtc acaattgcag tttgacagct cctccagtcg 61440
   cttcatttag gctaaaagtg aaaacgctgg gttgaagggg agggcagggc agatatccac 61500
   cgacttgcgt gcctgtgtgt ctgtctactc caccaggaag cctggtggga acgaccctct 61560
   cccctccttt cccctccagc ctcagcttta tgagcctgtc gagccagtgg actttgaagg 61620
   actcgtgatg acacacttaa acagcttgga tgcagagctg gcccaggagc tgggggacct 61680
   caccgatgac gacctgcatg tggccttcac acccaaagaa tgtaggactt tgcagcactc 61740
   tctgccagag gaagggtaaa ttattttttt gaaaatatga gatgggaagg gtgctgattc 61800
   tgaacccctg tctgtctgtc tgtctgtctg tctgtctctc tctctctctc tctctctctc 61860
   tctttctttc ttcttgaaac agccatgtac cctaaactga acgcagagtc acagccctcc 61920
   tgctcctgcc tccgtggtgc ctgagttaaa ggtgtgcact ggcatgccca gttttgaaat 61980
   actctggaaa agaagcaaac tttggattgg ggtggcgagg actaggggga agggctacag 62040
   agcagcagca aaatgcctgt gtaacatgtg caaggtctca gatttgatcc ctagaattac 62100
   aaagaagtta ataattattt taattaaaaa aaaaacattt tagggcccga ggagatgtca 62160
   tgaggacctg agctcagatt ccccagcacc cccagaaaaa gcaaggcatg gtgacaggca 62220
   tgtagtttaa ctccagtgct agagaggcag agggtatccc tggggcttgc tgagcagcca 62280
   gtttagccac ttagtaagct ccaggtccaa ctgaaagacc ctccctcaaa actaaagaat 62340
   tgaggaaggt acccaacatc tacttattgc ctcctcatgc acatgcacac actcaccagc 62400
   acacacacac acacacacac acacacatat tttaaaagga gcaaaactac tcaatccact 62460
   aaaatataca gtacatatag tattgctccc tagatgtaga gtttgtttgc ttgcttgttt 62520
   gtttgtttgt ttgtttgttt tgtggtgctg aggacctagc acaggcctca cacatggtag 62580
   gcagatgtac acgatttcta aattggttta gccaaaagct ttggaaagcc ctgtgggagc 62640
   taagatacag tttaagggat gcagtgggac agggtgggac agtccaaaaa aagtgaagat 62700
   tacaggccct agaattggac ttctgggtgc cagcttactg accataggct agtcacatga 62760
   cctcaagaag cctcatggtc tctatttaca gagtgtgaag agtgttcata acaagtatgg 62820
   aaatgcctct ctcaaaagca ttttatgggc caggaatagc tcagcaggaa agacatttcc 62880
   caggactgac tcattcaagt tgtcctctga ttgccacata taggtgtatg tgcacatgca 62940
   tgtatgcaca cagacacaca ctctcacaca cacaagcata cataagataa aggaatgtaa 63000
   aagaattgtt aagatctttt atttgtttct gtacgaaggc ctggatcaaa ttagtaactt 63060
   aagtttccgt gtacgtttat tgaacatctg ttgagttctg agattaaaag atgaggaagg 63120
   gaaactccct tcctctacag aagtttcata taagggagac acaacagaag cagtgtaaaa 63180
   tgaatattca aataaccgtt tagagctgcg ttgctgagca tggcgtggac gctagtgctc 63240
   gctgtccagc aaccgtagac tcatgtctga atggaatgtg agcagccagc cataggatga 63300
   acacccctgc ctcactggag atagtgagtg actttaattt tcactcattt taaaccaaaa 63360
   cgctaccttg aacatttttg agtcttctca tttctcccag aaagtagagt atgagacatg 63420
   gtgcctgccc tctttgctct gaggtgtttc tgtgttaggc tgtgtttcag cacaggtgtt 63480
   gacagctggg acatggaaag tcccattctc ttcttgagtc agttttcctt cacacagaaa 63540
   gctagccatg tgtgcagctc tactccatgt tacaaatact cgtttcccaa aaacctggtc 63600
   tctgtcatta ttggggaaca gttttcttag tgtcagccca tttcttcaag tgtggtttga 63660
   atattgacaa cacattttca tttttggtag agttacatta tttatttgtc tgaatattta 63720
   tctttaaatg tggccagaat cttagaagaa ctggtataga atacaggaag tttagaggta 63780
   gaaggacttt taaaactttg ataaagaagg ttttcctaga gccacttggt aggtgagata 63840
   ccattttgtt gctgtgactt tcagggaatg tgttacctct ctgatgactt tcaggcagcc 63900
   tgtaccatct cagttgttgc caaagtcttc tgtaataaga aaggcagaca gggaaagcat 63960
   tctggctacc atgggagtag ctactagtga ctctaagtaa ctgtcctgtg cttccctcgg 64020
   aagctatatt gagtagtggg agttatgtgt ctgggcctgc aggtcctgca ggtcctggtt 64080
   tggccatttg gtctatgatt tggattaatg tccttaacat ctccttcttc tgtacaacaa 64140
   gaagaacagc agtgtctgcc ttgttgaata gagacatgtt aagtacatag tgatgttatc 64200
   taagtcttag ctagcacaga gctcttaccg tgcaccagac accctttcaa gcactttgtg 64260
   tgttagcatc aaatctttgc catgattctc tgaaggaggt cctcttatta catcagcact 64320
   tgctggtgaa gaaactgaga cagagaaggg ctggatgata ggaatgcatc aggttacaga 64380
   gtcaatgagg aatggagctg agggttgggc agtcagggct aagagatctc ctatctagcc 64440
   tttgcagaat ttcatcttgg atggataaca gttccatggg tgagttccgt cctgcctcct 64500
   tgtgttagtc agtttccctt tactgtaaca aatatccatg ataaatcaac taaaaagaag 64560
   gaccatttat tttggctcac aattttgaag ctttcagccc ctggctgttt gggctgcatt 64620
   gctttggcca tttagtggcg cagagtattg tgatgagaga ccatagcaaa gaagtaattt 64680
   gtgacccctt catggccagg tgcaaaagac agagaggaag acagtagtgt cccacggtcc 64740
   ccttcaagac ctttcataag accccacatc ctgaagagtt accacttctc aattatgtca 64800
   tgccagagac taagacttta acaggtgagc ctttggaggc catccaacat ccaaattatt 64860
   ctatccctgt ttcataaatg caaaaattaa aacttatagt aaaacatgaa aggggagaaa 64920
   gcccagaggt ctcaacccta gacaaagaac tgcaggaggc taaggaatgc tgagggccag 64980
   agaaataatc ttctccaggg aagagcacac caatcgctta tccaatacca aatggccatc 65040
   ctggaaaaca tacgtataca agtaacacta tatggcctga gacagttgta tttatgaaca 65100
   tatatatgta tactcacata tacatttata ggtataacaa taattaaaga aaaagaggcc 65160
   atgagtttgc ctaagaacat gggagagttc agaggaagga aaagggaaaa taatatatta 65220
   taattgcaaa aatattcaaa taaaaatctt ttagctttta ggcatattta tgtctagtac 65280
   taatatattt ctaacaatac caagagcttt atactgtcat tatttctgat tgcaagactg 65340
   tggaacatat agaaaatatc tctaatgatg gattcctcaa caggtacgac agaaatccaa 65400
   cacttactaa gtcttcaaag ccaccaaagc ccaaggcagc tgttcttggc tttctaaatg 65460
   gaaaacacac atgcccaaac aaaaatcaca caaaagcaaa agaaaataaa ataactgtta 65520
   gaaatgacta tttgaaccta tttttcatgt ttggtgtgta attatagctg tttctcccta 65580
   ggaatgtctt aatgccttca ggcacagatg tgctttagca attctctata tcaaaggaac 65640
   tcaacagagc ataaaacaac aacatatttg tttaatgaaa acacaagtat cattgcctta 65700
   ggaatactcc atacacttga aacaagtggg caagaacatc actaatgttc ttattgacac 65760
   agatttgaga tggtggtgtg tcgcttgatt gtggaaatga cttattttat cacttttccc 65820
   agtaataact tctttttatc ttatttttca cctaattaga gttgaactgg atcctcacgt 65880
   cagagactgt gttcagacct atattcgaga gtggctgatt gtaaaccgga agtaagttta 65940
   tcccctcttt atatatataa atgctaattt ttataaatgt aaattttgta ttattggtat 66000
   ttttttcaaa tgaaatggct ttttggaggg gttttagggt tgcagacaaa ttgaacagaa 66060
   agctttgaga tctcccatat tctccctctc agagcgtcac tcatgctggg cacacactgg 66120
   gtggcattcc agtcctgcca ttgctttttg agctatatac ttgatggcct ttgacaaaag 66180
   tgtgacatgc ttctgccctt gcagcatcaa atgaaagggt tttgctgccg tgagagtccc 66240
   gacgctccac ctgtctctgc tccagccctc actggtctgt accagtcttc ttcatggtct 66300
   tgggcttcag cttgcttcat cttgtcccct tgtggcttca tcccttgttt tctatcatca 66360
   ctgaaaaaaa aaatcttgcg ttgtacagat gtactgcagt ctgtttatct cctcagttgg 66420
   gaagaaaaat cacattctaa acatgaagag tagagccttt ttagaagtta taaatagata 66480
   gagttagcca ggcacggaag aacataccta cagttacaac tactcaagaa gccgaggcag 66540
   gaggatatac tttggaccta aaatttcaaa gccagcctga gcagcaggac aagacgatgt 66600
   ctcataaaaa caaacaaact gactttctgt tttttttttt ttattaatct tcatttttta 66660
   ttttgatcaa ctcctagaga caagatagtt gatgaattga aacttggcct caggttcaaa 66720
   gatgccgttg gaatttgtga cccccaaaac aaagtacttg ctagtcttcc atggagagca 66780
   tttttatctc tgggttaatc aaacattttg tggcattttt ttcagaaggt tctgtctgtc 66840
   tccatggtaa cgaccttcac agcacagtct cacgagacgc atatgaacac acagaaatcc 66900
   acgccctgtg tccacacaca gaaatccacg ccctgtgtcc atgtgcacat atatgtggat 66960
   gtgtgtgctg ggaagtaggc tgcaggtgtc tacagattga ggctcaccac actagataga 67020
   tgccagcctc tggaacataa gcagcttgcg gaaggtagcc tccccctctc ccgacactag 67080
   tcggaattac tgttctcagg gatagataat aagactcttt tgggttttat ccagttcctg 67140
   taagtgtgcc attgaatctg tgctggatta ttttttcttt ttagtaaaga aaagtatgca 67200
   tcctgtttcc agtaacctca gaggaaagag actcactcct agaaggtagt gatgacaaag 67260
   ctgcatgcca gtccgtggtc ccttagaacg caggtggaaa gcaaagttcg ccagccaggc 67320
   aaataagatt aagccatgct tctgataaat gagagacttc agaacaagtt attttcagac 67380
   tccaaaatgt tatcatcaga ggcacttttc tgggtattta aagttctaaa aaagaaatcc 67440
   aagaaggaac aataaacaga aaggtccatt ggactaaatt tggtcttcag atttattttc 67500
   tatttgaaac cctgagcctg cccctcctcc caccagcaca gactctcgga atgcttttct 67560
   gtcttctgca aattttatta aaaaaaaaaa aaaagacttt agaatgctcc tggtagaaat 67620
   ttagaaacta tagaaagaca aaaccaaaat gaatggattt cattctttgt agataaccat 67680
   acttgacagt gatataccag cattgaatca aatctctttg ttgtgtatgt ttatataaat 67740
   actacatcaa actctagtac tattttttag cctgaatttt caaagtaagg gaagattccc 67800
   tgtctatatt tttaattatt cttcctcaac acagtaagta atgattgctt gatgtatgtg 67860
   tgtcacatgt ttaagcaaat cttatttttt ttttttttgc ttttgtactt atgttttgta 67920
   cagtgtagca atgtccttct ccctaaagcc ctgtgcacat ttattgtttt ttccctatgg 67980
   ggaaaaacta taatttctgt acctaagata tatgcacaaa tttaagactt aataatagac 68040
   cagagatgta atcaatatat gaattaaatg ttgaatatta gcaaatataa aatactttat 68100
   tttgtgaaag aattgtgaaa ggtttttgac attttctttg tttatctgat tgaacaatat 68160
   ttcctcatat atttgatggc cacgagtaat tttttcctta gtgtgtacta cttaagaaat 68220
   ttgaacaact ctattgagct atttggtttt gtaaaacctt agataacttt tttttttcct 68280
   ttttggggca atgatgatgc attcctataa tcccagtact tgggaggtag ggacagatgg 68340
   atctctgagt tcagggccag ccttgtctac tatgtgactt tcaggacagc cagggctaca 68400
   caaagaaacc ctattccaaa agccaaaacc aaaaccagaa cacaaacttt ttttcctttt 68460
   aatggtatgt tttgtgtgtg tgtgtgtgta tgtgtgtgtg tgtgattgtg tgtgtatgtg 68520
   tgtgtatatg tgtgtgtctg tgtgtgtgtg tatgtgtctg tgtgtgtgtg tatgtgtgtg 68580
   tctgtgtgtg tatgtgtatg tgtgtgtgta tgtgtgtgtg tatgtgtgtg tgtgtgtgat 68640
   tgtgtatgca catacgtgaa gatgcacaag gttggtgttt gaaatcttcc ttaattattt 68700
   gttcatctta tacattgaag cagagtccct cagtcaaacc cagggtttac aaatatggcc 68760
   agtgtcacta gcttgcttaa gggatcccct gtcctcacct tctgagactt ctaggtggat 68820
   tctgggaatc tcagctctga tcttcttgcc ttcacagcaa atgctctaat tgctgtgtaa 68880
   tctccccagc acctttcacc atataagttc cttaccaggt agagcttaga tattgtttat 68940
   attttagcat ttgggatgat ttatgtaatt aaatccatat ttgtattttc cttaaggttt 69000
   ttatctttgg tgtcatgttt aaatgtatgg tttggggctt gacctttagc caactcgagt 69060
   gttgtttctg ttccgatgcg tgtcccattt ctttttcaga agcagtgatt atccatgcag 69120
   ttatctctta taaaaacaat atctgtttct attctgtcaa gcagactggg gacggagatt 69180
   agcgttggct gacttctcag cgcactcatg tcctggatgc gtttccttcc ccacacaagc 69240
   tagtgggcag agataaggct ccaactctga cactcattgg cagtttctga cagtctttga 69300
   ctggtgtctt atagaagggc aatttttcta cccctacatt tgattctacg atactatgaa 69360
   tctgaaatac taagtatgaa aattacaatt tctatactca acttcttcca acacacactt 69420
   ctcccaggag tcgttcattc gctgtgtgta cccataatgc tttattcgtg gggaaaatcc 69480
   aggcaccctt catcactcgt cacctactat gttttcaata cagcatttaa aacaatgtat 69540
   ttttaattta ttctttggta atatatagtc aaacatatat acaatgtaac ttgatcatat 69600
   ttatacctta ttcccctgga cacctccccc agctaaggaa accccttcca acgtttattc 69660
   ctttcttttt cttttgcgct aattaccact cagtccattc ggtgctaatg gtgtgcacat 69720
   ggatgcaggt gaatccacca gggtatgggc aatgtattag ccacatcgtc cctaagaaaa 69780
   aggattctct ttcccagcag ccatcagcta cagtagttct taactaagag tagggcttca 69840
   tgagcccctt cctgtccgta gcaccgagag gtggctcagc agtgaggagt gcctgtgctc 69900
   ttcagaggac ttgtgtttgg ttcccagcat ctgttaggca tgtcatggcc actgtgggct 69960
   ttctccagtc cactttggca tgcctactga tgtcattgcc ggctcgtttt aggcagtcat 70020
   gttggtgaga ctttatgggg ggggggtagc tctgacgtta ctaagagatc aaattttata 70080
   acaaattccc tgatcctctg gcttttacag tcttttcatg ctccttttga agtgttctct 70140
   gagcattagg catgggagtt gttttgtaga tatatccatt ggaactggac ttcacaactc 70200
   tgttttgatt ggttgtggtt cgtctccatc atccccatgg tctccacctg tagtcctcat 70260
   gtctcaccaa tgtctctttt tgtaagttat aagtatgcat catggttttc aataatatct 70320
   ttgagatcat cttgtttcag agaactaggc catttttttt ttaaagaccg ccttgatttc 70380
   ctcagaggac aggaaggtcc catgagttat ggttctttct ctgttgatga atatgtaatt 70440
   gtctcctctt cccataatga aaaattctag aacatttgct ctacccatga ctgactattt 70500
   ctgaaggtag aaaagagacc catgcatcta atatgttctg attacttcta ccattcatat 70560
   atttggatct tattcctgta gtccaaaagc ccaactatat atacatattt atgtaaatgt 70620
   aaaatatcta tatatacaca ttatcccagt acataccatg aagtcccatc tctagggatt 70680
   tgactaaacg aagcaaaaac atatgttcac ataaaagtat gcaaatactt gtaacatttt 70740
   tatttgtagg tgccaacatt ataaataaca ccgaatgtat tcaatagggt aatgagaaaa 70800
   caaagtctgc taaatgcaga caatgatacc actcagcaag atgatgggat atttaaacaa 70860
   ctgcacagac aacactagat tcattatgct aagtaaagta agccagccag taagtagttc 70920
   tacttatatg acattctaag agaatgtgaa gacactccag ttactgtatc aaggaaataa 70980
   ccctcagacc acaccatata gttttctaag cttcggggat gggcggcagc ctgctttctg 71040
   cagaggtctg atcttagtga agccattttg ccttccatga acatctttgt catttaaaac 71100
   catgcagtca agcactggtt ccgtcagctc ctttatccct caattatctc cgggctgcta 71160
   ttacacctct ctgtaaaatc tagagatagt ttttttatgc ccttgttctc tccagtaatg 71220
   gctttgaatt ccacttaatg ggcagaggtg catgataatg gccactcctc cccctctcat 71280
   tctgtgggca tttgccactc aagtcctgtt gttggcacgt ttaaatcaga tagatggtgc 71340
   tgctgtaaag gacaaaagag aggtctggcc ttatttttat cactcaaaag ggaaagaatg 71400
   aattatgtaa aatgcatggt ctctttttgt agaaatatat tcaccttttt ctggttttct 71460
   acacatgtgc acgtgcatgt gtgtgtgtgt gtgtgtgtgt gtgtgtgtgt gtgtatttgt 71520
   gtgtctgtgt gtgtgcattt gtgtgcatac acatatatgg aagtcagtct gaggaacttg 71580
   caggaagaag ccctttcctt ccatcatgtg ggccccgggc attgaactca ggaggtcggg 71640
   cttagcagca agcaccttta cctactgacc tatctcacaa acccagagaa tatatgcttt 71700
   tcctagaact atttacctgt gattcttaga cttgcaaggc ctccttggat agagctcttt 71760
   ctatgtttcg tgcctaaact gccatgttta tgtgaagata acagttcttc agccctttgt 71820
   gttttgtgtg ctttgataca aggcacagaa gggatgattc atgtggggct gtgttcactt 71880
   cttatcctgt tctcatgtgc cttcatcatt ttgtgccaat ttatgcttga tagcatcctt 71940
   tgggggggga tagtagggat tttaagcgca atttcccctg tcgttttctc taaatgaagc 72000
   cggggtgatt aacttcagag tgttttcacg acttggtggt gagtcaaact ctccagtgat 72060
   taaatgtgtt tacgtgctgt ttatttttgg tgcattatcc atagcgacag tgaaacattt 72120
   catgggtgtt atgggcagca tcataaaagt ccctggactt tatctttcat ccccagtgat 72180
   taaggtgatg ctgcaggaag gtcacgcaca aggagagtgg gtacctcaag gagcagggca 72240
   tcatctgttt tatcactcca cttttactcc cagtgaactg ataaacaatt aaaaattgaa 72300
   cacctcagtg cctataattc agttactccc aaaacaccat aaaaatgtct ccccgatcaa 72360
   taattgctta ttatgacaca catttgttgt gccagctgtc aaggtgctct gctttcttaa 72420
   agcatggaga gcaagccagg ctcatcagcc tacatggtta ctaacaagca gtaacctggc 72480
   aggggacacc atcacagcct gctctagacc tcctcggcag aggccctggt caaaggcttc 72540
   ttctaccaag ccttcgctgg ggtggccaga agctgtcaca cacagccttc tgtgagtttt 72600
   taaaacgtct tgttgatatt tccctggaag cttttgtgtc agtgtgcatt aagttctggt 72660
   tacctgtgtc ctccctggtc attgtggcat cttttatggt agactgactt cattggtgcc 72720
   aggtgctctc aactgtatgt attctccttg gtaccttatt cccaagagaa ttaaagttat 72780
   aaacatttga cgaatgaaaa gaatgaatga aacttaacaa gcaagtatcc acctagcagt 72840
   acatgtagga cggacgagga tctcattaaa acctggagcg gcagggtgcc atgagtttaa 72900
   tcaggacaac agactcattc agggctttgt agcatcctac aagtggaatg ctaaaatggg 72960
   ttctttgcag agctctaccc ctgtttctga cagctaaatt acagatcccc tcccaaaaca 73020
   atgtcattct gcagaaatac taggagcctt caatgtgttt gtgaagtaag tgtctggacg 73080
   tagagagcat tcatcggcct agcagtgttg tcacggatgc tcacgatgac agctggcgtg 73140
   taactgtcac cattagcaga accctaaaag cagaagctag ggcagggatt cagacatctg 73200
   tggttttcaa gagttgggtc ttcagaaaaa gtctggggtg gagtctgctg aggatggggg 73260
   tggatctcag atcatattta gccttgggct gcatcagtgt tgcgaagagg aacgcgactg 73320
   taaagttatt gtgccatcct actgtgaagt acgggaccat tgtcttccaa tgccaatctt 73380
   tataaccacc atggagccaa agttataggt tggcatgggg gacaggaagg taagatgagt 73440
   tgacaattgc taagctcaca gcagctggag aacgtgtatg agttggtaaa ggggacttga 73500
   ttggggtccc tgtagtttca accacagtgc cacgtgtctg acacactgct ttccttgtat 73560
   ttggagacaa cctcaaattt gtagaaaaat tgtacatata gaacaaagac cccttcctcc 73620
   tcaccactaa tcatgtgaga ctaggttccc tacctgatat ccccatcacc ctgcctacat 73680
   tggtgtgtaa ttgttacaaa caagtacatt tgccattgta tagttgccat ataaccccca 73740
   cattctgcaa ataaatatta atttttcaat accactaatc ttcagacttc atttttcatc 73800
   aaatggccaa agaatgttcc ttcaaggatc cagttctgag ttgcacagtg tgtttaagta 73860
   ccccatgccc ttagccttgc tcacatgaga agacactctc agtatatagt tatatttctc 73920
   tgccttatga aattatgggc cagctatttt atagaatggt gctccctttg ggtttaattg 73980
   tcacgatata tccctttcct tatcaaacct tcagtttctt tctttcaata tggatctata 74040
   attttccact ttattgaatg atttcactca tgtctgatgc atatttcaat gatcaaacta 74100
   tcttcagtct gactggagcc atgctctttg actgacccct atgtcttgtt tacatggccc 74160
   tagcagtcat tgatggcatt cttcctccct gacacaaaaa tttccagaaa ctcttagatt 74220
   tccccatctt cctcctggag ttagccgtgg ctctcagagg ctgccatggt ttggtgggct 74280
   gtggtactca gcagccaagc tgtatgtatt aagtgtgctc atttttacta aggttttcct 74340
   cctcacgccc tttcagtgaa gagagtggaa accatgagtt cttgctggca tggacatctg 74400
   acttcacagt gttggtttta gcattctccc tttgatattt taattccttt gcttggcaaa 74460
   atatggctcc cgtgtccttc atgttcctac taagttgtct aatcctcctc tgtgtaccca 74520
   ttacccagat tctcttccac cccagtcatc ctcctcctcc tcctattctg gttctgacac 74580
   tggccttgtc acccctaccc agatgagcct cttcttgcac tgacacagta ccaggatgcc 74640
   caagcctgat acctcctgtc agatgtccct tcttataaat gcttctttat tagactcggg 74700
   ctcagtgatg ggcgtgctcc tggctgtcca tgctcagata tcttccttgc actatttaga 74760
   ctccaattca cccctctgtg agggtagcac tttcataata aatgctttct gtaccctatc 74820
   ctgagttcga gttccagttc tggatttccc aggctactct ccacctccaa gacagatggc 74880
   tgccttcttc caatgtatct taaggtttta acacctagtt ttatagggag agtaggaaag 74940
   aaaggagaga gagtgtttga gaagtgggaa ggaaagggag acggggagtg aagggacggg 75000
   agagaagaga aggacacaat tattctctga tcctcacaat aaactcttaa atgaacttca 75060
   gttgtcccct tttctatatc caaggaaact agctcagcga cgtgaggcca cctgcccaga 75120
   tcagcatctg agatcaggcc cttctcactc agccagattc tacctgctac acactctggc 75180
   ctctaaagag aaggctttca agaaatcttt gggtttgggg cgtttctgag tgtgtaagaa 75240
   aggtgtttaa gacagagttt ggcaagcgat ggctgtattc attctgtgtg gacagttgct 75300
   ctcccagtct gcctatttcc ccatcttcct gtgtcttcac acgggctcaa tgggaacttg 75360
   gctttgtttg agtcggttgc cagggtggga cgactaggct gagatgcctg tagccagagg 75420
   ctctgagcca gcggtctcac tacctaccca gtggtggctc cttccatgct ggcacttctg     75480
   gaatgagtca cctcagtgct tgccagctcc tgcctctccc cagccagcat tacccctgct     75540
   tccaagaaga gcactgtgac tttctgagat tttaagatgg ggaagtggat gctgaagggt 75600
   atgaggaagg agagaaacgt ttgtctgact ccaacctgaa cacaatggtc ttactgcttt 75660
   ccaaagtaca agtcccaaag gctaaagtgt gtgtgtgtgt gtgtgtgtgt gtgtgtgtgt 75720
   gtgtgtgtgt gtgtttgctt ataagtagac actcttagag atcgatgtag tttataagat 75780
   gggggaaaaa ggcaagctaa cagagtgcag ttctggttgc taagacagca acaccatgta 75840
   ggtcttttgg ctgcttctcc agttttgttt tgtttgttgg ttggtttgtt tgtttctcta 75900
   taaatccaat ctctccttga gaaggagctt ataatactga acctgttttt gaattcagtt 75960
   ctctttagag ggaatgggtc ctaaaaagta actgcagaca ccatcgtggg gagttagaat 76020
   ggattcagag ggactgctgt gagtatctag tctttttttt ttttttttct gctccacata 76080
   gggcattaca gagctaataa ccacaggcct agtgtgttct catttgggtc taattgcaag 76140
   acaacatttc ttatttaacc tatatttgcc actactttag tttaattatt tcctcttgcc 76200
   aggatattag accactcaca gtgtggccaa gtagacaagg tacaggacta catcacccgc 76260
   agctcttttc atcttttctg ttatttattt gtgagtagtg tggctgtttg ggaactggtc 76320
   acagggtgtg gcagaggcta caaaacaacc tgcgggagtg aattctctct ttcctgtatt 76380
   tgggttccag ggatcagact caagccgtaa gtcttggtgg caagcaccat ttcctgttga 76440
   gccaccttgc tacccaccca gggtgttttc ttcctttatg caccaaggac tgccatcccc 76500
   tccatgtggc tccctagact tgctggtgga gaataacttt caagaagttg gcagacttaa 76560
   ggacaggtct tttcccaaag agctctgtta aaagacagta cattttataa aataaatgtt 76620
   ggttagttat cattgactct tcatttacaa actcagaaca actgaaacta aaaatacaca 76680
   cacacataca cacacacaca cacacacctt tcatttttgt tttcagatat ttaggaatgc 76740
   caaatcagaa attattatgg tcatttttct tgattttctc ctaagtattc atatttgttt 76800
   atggtcactc tcttcttttt tagttcctaa ttcagcttgc tttttgccca gccttattag 76860
   attggattgg gcacacaaat ggccaaggga cactgaaggc aaccagtcat agtaacccag 76920
   gcttagaata gttgccaggt tgctcttgta gaaagcttca agactttttg tcactttttc 76980
   tcaaaaactt tttttttttt tttttttgtc ttctgagcac cataaaatca agagaagtcc 77040
   tggcctccct gctaaagcac tggggcatct cctccctcct taactaatac agtgaactac 77100
   cacacctaac acagtgaact actataccta acacagtgaa ctactatacc tagtacaatg 77160
   aactgccata cctaacacag tgaactacta tacctaacac agtgaacctt cagtacctgc 77220
   tgtgtgtacc tctcttcctg cagtgcccat ccctttcaga gccttacaaa gggtgtttga 77280
   atccttgcta gcttcctaca ggtctgtctt tacctgtagg ctatttgacc tgcctaataa 77340
   attctaacct cactgcatgc ccctttacca gcctgcactg tggtctttac agtaaaaaca 77400
   gaaagcatgg gctggagaga tggctcagcc attacaagca atatctgctc ttgcagaggt 77460
   cctgagttta attcccagca accacatggt ggctcatgac catctataat gtgatctgat 77520
   gctctcttct ggcatgcagg tgttcatgca gatagagcac tcatatacat aaagcaaaat 77580
   aaataaaagt ctttaaaaaa aataaagcac acaaattagg tgtgaatata cgttttccaa 77640
   gaagcctttc agtcagaaaa ataggcaatg tatgtgttgg tttccatagc tctaaaatga 77700
   caaattagag ctcctgcttt ttctattatg gtcttacttc tccagtaaat gtcttggcca 77760
   ttctatttta gactcttccc ggagagagag tggtggagga gacaggtaga cagacagaga 77820
   cagagagata gacaggaagg agaaaaggaa gggagggagg gagggaggga gggagggagg 77880
   gagggaggga gggagggaga agagagggag ggagggagaa tgggaacaca caccagtgaa 77940
   tctaggccct cgtgcatgct gggcaagagc ctgaccactg accctgacct atattcccat 78000
   cctgtagaca gcattctcct gaagcatcca gagtgggtct gtgagtgtta gcaacgaatg 78060
   tcggtgcata cattattttt gtatattttt tccttcgcgg tgtccggctt ttcttactca 78120
   ttattaagaa cattgatgtg gagttgaaat ggcttcctct atttttatct tgaaacattc 78180
   tctgtttgct gcttctgcac actgctaggt ttatttcagc ctcttacact gcagtcacag 78240
   ttcaacttca gcttggagcc aagtgcctca cagctgaagt gaccacacct ctaccaatgg 78300
   cattttccca ggcgtccctg cctttcataa tgggccagcc aggaggccag ttcctatgtg 78360
   gctacaacag agccttgtgt gagggctggg tgcggcattc gatggaagcc tcacaagcca 78420
   tcctctgtgg agcctccgcc gctgcttccc cccttgtgtc gttactttat ttttaatcta 78480
   atgcgttgcc tctttctccc tctctcctgc tccccgtctc cctccaaatc aaatcatgcc 78540
   aacactgaaa gaaaccaagg aagttcagag ttttgcagct ttaaaaagac gggatctcgc 78600
   agagattttc agaagacgct tcagaaacag acgtttgagt cagaaacctt ggagtgcagt 78660
   gaaccggaca ctcaggtact tcctgtcaac aaacacggtt accaggtgac agatggctct 78720
   gcccaaggcg tgctgtcagg ttcttaaaaa ctagaataaa agtcttcaag aaggaggaac 78780
   tctaccattg agtcagtggc tgttttcccc aaactggttt gtagctagct gcagaaacca 78840
   ggattcaaat agaaacccca aagcacagtg attaaaatgg aaaaagtgaa ggggctgtag 78900
   ctggggaaga gaccgacagt gggccccgcc ctgttagagc caggatgagt caaagccttc 78960
   atcttcagag tgccaggcct ttcttagtga tccctcggct ccatcattaa cctctctggg 79020
   ttttagtttt cttccctgga aaacactaag tgttttccca aggatgacac taagtatctc 79080
   atgagatgac atgagtatgg agtgagtgag tcagaatgtg taaagataat aaatgcctag 79140
   tgtccaataa atcctagcta ctatgactgc tgttcccact attttcataa ggtggtgata 79200
   gtcgattaag tccatgctgc atatacccat gtccatagaa acattactcc cagtagccaa 79260
   aagatggaag caatcaagtg tccattgtgg aagaatgggg catcaagcgt ggttgccggg 79320
   cgactgggga ggaatgggga gttactgttt agtgcagagt ttcagtttta caagatgtaa 79380
   agaatcatat ggaaagaaag gatggttgct ctatagaatg atagtactta accctaccaa 79440
   aagatggctg agatgatgta ttttaggctg cgtgtatttt agaacagaaa tgtagaagag 79500
   ctagcaatga tgcactgttt tttaagttgg tgtgctcatc cctcaggccg gtgttgtagg 79560
   tgagaaaaca gagaaaacag aggtggatga gtttccgttg cacctagaag ctgttgccga 79620
   gctggtgtgg tttttctttg atgcttgctc ccatagccat tagtaaaacc ctgtgatagg 79680
   tgtatcccaa tcccgtatgt ctgtatttgt aaacctagca ctcagcctta gagtgtatat 79740
   acattttttt gaaggcgcct tagagttatt ttctagtctc gaaactcaag ctctgaagct 79800
   gattagaaaa atgtccctcc gtccattcga agtccaggct cttatgtgct gtgccttgtc 79860
   ctagctgttt gctccagcct gagacatgaa cacggttcct aagaaacttg gcttcagcca 79920
   atgactgata ggaacacagt gatgttttca agtcactgct caggactggc ttacctaacc 79980
   ggtgaggaat gacctgcttc caggagagaa agtacatcgc acttcaggat agtcccacca 80040
   gaaagccaaa cacagttttg tcaagccttc agtttgttaa aacaagtaaa caaacaaaca 80100
   aacaaaaatt ccttgagact ggagctgctg tagatccctg ctggaacagc cttgagacat 80160
   ccctgagtct agttctcatt gacaaatgag aatcttgacc ctcatataga ccgattgttt 80220
   tctctaacag acaggacccc gtcatccctt aaacgtgctg tgtgacgtgt ctgggaaggg 80280
   ccccctcact tcttgtgact tcgacctccg cagcctgcag cctgatgagc ggctggaaaa 80340
   cctgctccag cttgtgagcg ctgaggactt tgagaaggag aaggaggagg cccgcaagac 80400
   caatcggccg gctgagctct ttgccctcta tccgcccgtg gatgaggtga gtgagagttt 80460
   ccatagggga ggatattggt gacaacaggg aatccaagta ttgttctgct ctctgacact 80520
   gtagaacagc gtgaatatct ggatgcttac ttcgtggctc ttttcactta agcacacttg 80580
   tattattgga gagcccctta tttatcatca taccaatggg atttctatag caataactag 80640
   ctagaatttt catagtattt taatttcctg aacatttcaa gggtctgcat acagtatatc 80700
   attcagagcc aaccgtccag ttacttaggg gtgtgtttca caactaatcc aacaggaaca 80760
   cactgttact taaagcttgg tgttattaag tgctttgtta agatgtttcc cccgtggccc 80820
   taatgagcaa gtagagacac ttgctttgaa tacgctctgg gcagagataa tcacagccat 80880
   gctcctagtg tgtgaagctt tcgaatccag ttttcccacc tcttcattta ctgaagttgg 80940
   cccagagcag tttagatgtt gcttgcggtt gcttgctgga gcactttgga tggcctgaga 81000
   ggctgtcatc tttcaagcct tctgtgcttt gactccttgt gtgctgtgtg ctcttttcag 81060
   cctttccctt tgtctgtcag gatccctaca ctcccgggtc tcaccaagac agcagctgtc 81120
   ctgtctctgc tgctcacgtt ttatataaag ttcagttgca agaaccaaga gctggaggct 81180
   agagagatga tggctcagtg gacaagagtc cttagcaggc aaacctggag acctgagttg 81240
   gatccctaga ttcgcataaa agtgatggga gagatctggt tccacagaat ggttcgttca 81300
   cctccacatc cccactgagg cacacgcctg cccaggtgtc ctcataaaaa catgcatgga 81360
   caaatgataa aaatgaatag gatttggggg attttttttt gagtgaaaga taaatggtac 81420
   ctgtctatag atgtaaatct ttgaagtttg gtccctggac caagcaagac atcagaaagc 81480
   ctgagcctgc tctgctgagg ctgctgctcc tgggagctct cagctcccca ctcctcccca 81540
   cccaactccc tttgattttc ttcccattga cctgaaagga aaccacacca agcagcagga 81600
   aggagatgca gcatcaagtc catggtggca ctgtcatcca gtaccacccc agtccctcaa 81660
   gccctcagcg tcccttgcct ggagtcacac ttctgatgac cagggagcag ggcagcttgc 81720
   ttttaccacc cccaccccca ccccagtcac ttctctttta tctttatttt ccaatcctct 81780
   tagtttctga gctctttgtc ttagttgcag ttttgtctga agcagattaa aggtaaatat 81840
   gttgggaccg gaacgatgga gcacactgat gatcagagtt cagtccccag agaccaacac 81900
   ccaaaggttg ttctctgtcc tcccccacct cctgccccca gcacaaacaa acagataaat 81960
   aaatggaaaa gtgaaagagt aagctgacac attgaaaaat agattgttcc tcattaaata 82020
   aatcactaag tgccttgggg ggctggggag accgttcagt gggtaaagtg ttggttgcat 82080
   aggtgtaagg accccagcac ctacatgact ctgcacactg tggcgcatgt ctatacccct 82140
   ggccctgggc acagagacaa atggagcctg gggtcactgc cgatctaacc gaaatggttc 82200
   agtgaaagag tatacacaca cacatacata catacacaca cacatacaca cacacacata 82260
   cacatacata cacacacata cacacacata tacacacaca tacacacaca catatacaca 82320
   cacacataca tacatacaca cacacacaca cacacacaca cacacacaca caggggggca 82380
   tttcataaag agaaagtaaa gaggaggtga ctaaaacttg tgggtggtca gataggaaga 82440
   gagattgatc aaaacaaaga aagtttctgt taaactggaa ccctggattt tactgtatgg 82500
   taagcactat tagtgatatg gtagtataca tttttaaatt gttaatcaga gtaaagttaa 82560
   ctttcttacc accaaacaaa atgataaact ggcaaggtaa tgcataagac tcttctgctg 82620
   ccggctggtg atacacacac ctttactccc agcacttggg aggcagaggc aggcgatctc 82680
   tatgagttcg aggccagcct ggtctaaaga tggacagcca ggcttacaca gggaaaccct 82740
   gtcttgaaaa ccaaacaaca accaaacaaa aatgtttcta caaagaacac atatgacatc 82800
   acattttatc ccataaatat aattttatta tcaattttaa atatttattt taaagctaat 82860
   taggaagaat ttgagcaata ttatcatcac catagtttgt ttgaactagg gtctaagaat 82920
   ggaagtgttt aagcaatttc tccaagattc agcagatgaa ctaaattaga aagcagaggc 82980
   cttaaccaat ctgagtgctt gcttatatgc ctgcatacaa caaccgggtg gtagcacaca 83040
   ctgttgaaac acagacgtgt tttaaaagtc agttcttcta atgtttcaag tttattcaca 83100
   ttaagacttt aaaaaatcag atttgtcaag aaatattgga gctaacagat ttgaccttta 83160
   cttccttgct ttctgcccat ctccatgatg gagatgctcg cttggttgta acacaagaca 83220
   ctagagtggc catcccgtga gagaagaaaa tgcaggaggg gacaggagga tgactcaaat 83280
   gtttaccgtc aagagtctcc ctgcaccaag tgggaggatg tccagtccaa ctggatccaa 83340
   gaatgaacca tcaactgtgt gtggagcttt gttttatggc ttatgagaca gtgcagaaat 83400
   tcagatgggg gctggaagtt atcttacctg aggtcccagt ggctgctttt attgagtgga 83460
   gaggaatgcg gtgtaattag cataccaagc cacttgtaaa ctagaatgtg taaaatgctt 83520
   gagagcctct acaccaccct ttaatatttt gaagtcattc tcaaagaaac agtgtttgtg 83580
   ctggtcactt gaacttttgc cgagacagtc ctatggccca gaggtgaatt ctgcctcctc 83640
   taaaagaatt ataaatgttg atatttttaa tgacatgaga tatttatgtt acactatttt 83700
   atttcctagt agagaacatt agaagggcaa actctttcac tgaaaattta agagtctgta 83760
   gaaaaggtca aaggatttag ctaccaagtt ttcaccatgt aagcccttgc ctaattcaga 83820
   cccatccttt gataggcatc ctctgtgtta aagatggcct tcttccaccc cacatcaccg 83880
   agtagcctat tggtagtcca tttggttaca agtgatggaa cccaactaaa aaagacttta 83940
   aacagaggag gagggtactc actggacctc ataacaaaat cacaggagtc agacgtggag 84000
   ttggccacta gatgcacagg atcagcaaat ggaggactac catgctgtct ttctctcccc 84060
   acctcacccc accctctctt ctcttaacac aaacacacag acacaggcaa acacacacaa 84120
   acacgtagca cacatatacc catccataca cataaacaca cacacaggca aacacatatg 84180
   cacatacatg aacacacacc cacccaggat gatgaaaacc ccaggaatcc actaaagcgt 84240
   cctatttcct ccttctgcta cttgggaatt gtctccgatt tctgatacac gtgaagcctc 84300
   aataaattca gaaataagaa gcttaggaat cctgacttag caaactgata ccaaatattt 84360
   tgtgacagta aaacttagac ttattttttt aggtcaataa tgttactact gatgttttta 84420
   aaagttccca tgtgtactgt aatagtttca tatccccata ctgatgtgtg ggaacttgct 84480
   tcccagtatt taataatcca gtattaaatg tgaggaactg gcaagggagc atataggcaa 84540
   aaccaggcag cccaagctgg aattgttgga gccgagggag caaaaatggt ggctcctttt 84600
   actagtgtat ctacctctct gtgtttaaat tttcctcaac gaatgattat ttaaaaagaa 84660
   tattttaaaa gttctagttc aaaccaaagc tactattaga aatactaagt gtaatgtttt 84720
   ctccagagag tgggtcttcc ctgcagaggt atctgctctg gtcatgctga cagtcccagc 84780
   ttcctctgca ggtacccagc tcacctcggc tgaggggaag tcagtagtta cactcaatgg 84840
   gagctggagg gagggggtgg ggtggagtgg gggtgagttt accacttggg tcatcccacc 84900
   aaacggaata cagacttacc ctgaggtgac aactgctact gggaaagggc actgctgagc 84960
   tgtactgggg tgagagaacc ctgaccgttt attgcctagg aaaagagagc tagacaatga 85020
   cccagtgttt tggcttcctg tgagcattta gcacagtagg gttggacagc ctactgtggg 85080
   ggtgtgaatt aaagctttta tttttttatt agttattttc ctcatttaca tttccaatgc 85140
   tatcccaaaa gtcccccata ccccctcccc cactctccta cccacccact cccacttttt 85200
   ggccatggca ttcccctgta ctggggcata taaagtttgc aagtccaatg ggcctctctt 85260
   tccagtgatg gccgactagg ccatcttttg atacatatgc agctagagtc aagagctccg 85320
   gggtactggt tagttcataa tgttgttcca cctatagggt tgcagatccc tttagctcct 85380
   tgggtatttt ctctagctct tccattgggg gccctgtgat ccatccaata gctgactgtg 85440
   agcatccact tctgtgtttg ctaggccctg gcgtagtctc acaagagacg aattaaagct 85500
   tttaaagatg aacattactt gtcttggcat aataggaaca cagttaagat accatggtcc 85560
   acggtgcaga gtgtaaagtt gggatagacc ttgatggttg gtagctcttg ttactactca 85620
   gttgtcttac acctagactg tcagcatatg ttagctgtgt atgtgtatat aagttataat 85680
   tcaactttaa gcctctagca cctatttaag ataaaaaaac aaagacaagg ctctatttat 85740
   aaggatcgat agttattttg actcatattt caagtctgaa acttagcaca tttacttatt 85800
   aaaactcttg acaagaggct agtgggatgg ctcagaggtt aaaggtgctc ctcaccagac 85860
   ccaatgacct gaattgatca ctggagacca catagtggaa gaagagaccc aactcctgaa 85920
   aatttcctct catatctaca tacaaaccat ggtgtgtgtg tgtgtgtgtg tgtgtgtgtg 85980
   tgtgtgtgtg tgtgccaaac ataatataca agcaaacaag cacctccccc accacacaca 86040
   gtaaatataa ataaaatatg tacattaaaa atttggaaag agaagtgggt tttgaatgtg 86100
   tatttagagc ttcagagtag ggagaaagac taggcacaaa gttttgcaat gctgtctggt 86160
   agatgtcact aggccatagc tatgtttgca tttccttcct tccttccttc cttccttcct 86220
   tccttccttc cttccttcct tccttccttc ctccctccct ccctccctcc ctccctccct 86280
   ccctccctcc ctccctcttt cccttcccct tccttccttc cttccttcct tccttccttc 86340
   cttccttcct tccttccttc cttccttctt tccttccttc ctaacatctt ccctccattc 86400
   ctcctttccc ctctttctcc ctcagtttct ttctcctaaa tgttcctttt ccgaggggag 86460
   aaaatgtttg ctttcgtgct ggcttgaaat gttggcaggt ggatggcaaa ctctcttctc 86520
   aagacagagc attgctctgc agctggaaat aaagcaggga cacggagggc atcccttcct 86580
   cccctggctt aactccgact acaaggccct agggaaagtt gctgacttct ttacgatgtg 86640
   attatcaaca ggaggatgct gtggaaatac gtccagtacc tgaatgtcct aaggaacatc 86700
   tgggcaacag aatattggtc aaggtgctga ctctgaagta agtgtcagac tcacagccac 86760
   tgtactaacg tccaccttgt gtttagatga aagctatgtg gcatcatcaa tgctggggtc 86820
   gagagggttc tcccagcatc ttttcaatag aggggagcat tggggtaaca gaaagagcag 86880
   agattcagtt gattccctgg actctgtttc tccaaagcct ttaacaacca gacttctata 86940
   acatggaaat caaacacccc gtgtctagga cagttaagtg tataaggtaa tgacgaactt 87000
   tctaacatta agaaaaacaa cagtaggttg gatatgtaac taggtggtag aatagttgcc 87060
   tggcatgtgt gaattgagac ccatagattg acccttgtct tagattgggt tactactgct 87120
   gttatgaaac gccatcacca aaagcaagtt gggaaggaaa gggcttattc cccttaggct 87180
   tccacaccca tcacggaatg aaatcagcac agcaactcaa agagggcagg accctggagg 87240
   caggagctga tgcagaggcc atagaggggt gccgcttact ggcttgctcc ccgtgctttg 87300
   cctgacctgc tttgttatag aaaccaggac cacaagccca gggctggcac tacccacagt 87360
   gtgctaagcc cttccccaat caaacactag ttaagaaaat gctctgatct tatggaggca 87420
   ttttctcagt tggtgctcca tcctttcaga tgactctagc ctgttgtctc ccctggttct 87480
   gcccttcaat acgtgctaat accactactc atgcagggac cagtgctggc tctgttgtgt 87540
   cctttttcag tctagccagt tccttgggat gggtgtcagg aattgcatat gttgagataa 87600
   ccagcctata aaccagattt gtcttcagta atgggcacag aaaggcaaag cctagtcctt 87660
   gcaagttaaa aatcagcagg aggactggct gagagaggtt gttcccggtg ttcagaaagt 87720
   ctgtactact gtctgccacg cctagcccca ttcagttggg ctactgtcag atgcagttta 87780
   gatttggaac taagagaaca ttctccccgg gctaattgtt acaagccttc cagatttatc 87840
   taacacataa agccacggca gctgttctta taaaccggaa acacgtcctg ccgagctaga 87900
   gatgaattat aaaacaaaac aaaacaaaac atggagtctg gactttttca ttaacactca 87960
   taattttccc attcatatct gtgtgtgtct cctatttgtg gtgttatcct tgtgtttctg 88020
   gaatcatgtt cacattttta atgatttgta taaatccatg cacttttcag acattcagac 88080
   atagacaggc tagaaccata ttgattttct ccatccctcc ctccctcccc gtctctgaca 88140
   ggtttgagat tgaaattgaa cctctgtttg ccagtattgc cctctatgac gttaaagaaa 88200
   ggaaaaaggt atgtttaaaa aaacaacagc ccatcctata aatcccatat gattttagag 88260
   ttcacgtttc caacctcatt tctggctgta agaaagcaca acaggtgttg tgtgtgttgt 88320
   gtgtgtgtgt gtgtgtgtgt gtacaggttg tgctgcatgg gtgagtgtgt atatgtattt 88380
   acaggtatgt gcacatatgc atgtgtatgt gtgtggaggc ccaaagttga atcgtgtttt 88440
   tcttagccca tttgcacgtt acctgtgcag gcaggctcat ccggttggac ccagagcttc 88500
   cgggctgggc ttgctccagg ggttcccagg ctctgccttt cagacgctgc atttacagcc 88560
   ggcctctgtc catccggcat ttgtgtgcat tcctgggatt cgaactcaga cacccaggcg 88620
   tgtgtgtcag ttgcaccccg ctgagcccca gcatagacat caaactggaa aacatattaa 88680
   ccctgatagc tccagatttt cttccttggc catgtttgaa gaaagctgat gtggcaatgc 88740
   atcatctgtg ttctctgaag aatctttaag ggaaaggcca gctggaggct cactggttgg 88800
   atagagaaag aaataagtaa tacttgaggc tatgtagggt gtaaaataat gttgtcattt 88860
   taacaggtca gcaatgcgtg atttttgtcc agaagtttgt tgttgttgtt gttttgagtg 88920
   ttttgctttt tgccgtgaga tagattcatt gtggggctgg ggagatgatt cggtgggtaa 88980
   gagctcttgc caggcaagca tgagggcctg aatctgaacc cctaacaccg acataaaaag 89040
   cctggcatga ttgcatgttt ctaaatgctg gagatatgtt gtcttaagac aataaaacag 89100
   aaagcaatag aagtagacat ccaccatcct gctcttactc ctgcatgtaa cacacacaca 89160
   cacagagaca gagagacaga gagagacaga gacagagaga cagagaggaa attaaatagc 89220
   ttcattgtga atggatctag tgacatcttg ttagtgacat agtcattaca tagtaagagg 89280
   tccgtgtctt ttttccttaa tagttcaagg tggtaagggg tgaatggcag gttagtaggg 89340
   tctctatacc agtcagtcat tcagaagtcc aggctgccaa gagtccgtcc ttccttaata 89400
   tgtcctctcc ccaggcactc cgcttagggc cattggtgct tgcaatatga aatatgtgag 89460
   atggtgaagt ggaggaggcc tggagatgta atgtctcact tatgctcccc attcactgca 89520
   gacgagttag tcacgtgatt gctctaactg tccatgtgct gacaataaac tacctcagca 89580
   tgtagctttc actaacccac tcttagcaag gacagaaaga acccagtaat ggctgccacc 89640
   tcaccctaaa ttagaaagcc ggagagcgct gggcatgtca ggatgaggat gcattgcctg 89700
   taccaacctc ctcctcccac gagtaacaac gtccttaggt ctttagtgtg catgccaggc 89760
   tgcctcaggg ttgctctctt ccttcttccc tctctctctg atgcagagtc aagccaggct 89820
   gcactgctcg ctagactcag tccaggcctt ccagcctctg agatccttgc ctgtgctttc 89880
   ttctctgccc tctgtgcctt gcatttctgc ctgtgagaag cgctcatctg tcgctaacgt 89940
   cttctccata aatagttttc ctcattaccc caatcatcgt gtactttcta aaacttccct 90000
   ggcgcctctg tgcctgagtg tagtggctta acttgctatt actgtagctt gttgtgtcgg 90060
   cttttccctc actgtgactg taaaccaggc agaggtgtgt gcctgtcttg ttcagtgcaa 90120
   taccttgcat ttcactgata tccagaaaat actggtgagt taataaaact taagtgttgc 90180
   tcctatggct ccaaactgtg tagaaattgt aacaaaatgt ccgaatttct agcagccact 90240
   aaccctatcc acagttatac ttcttttagc agaatttccc tatgactttt caactcctgt 90300
   tgcatttggg accaagaata caatacctct aactgacttt tccctgtgtt gatcatttac 90360
   agatctcaga aaatttccac tgtgacctga actccgacca gttcaaaggg tttctgcgag 90420
   ctcacacacc ctcgattgac ccatcgagtc aggctaggtc tgccgtgttc tctgtcacct 90480
   acccatcttc agacatctac ctggttgtca aggtgatcac atacagtcca agatgcatgt 90540
   tctgatattc atgttgttat cttactcggc aacactgaca aaggtcacat gacaggcttg 90600
   tgtgtcctct gtgatagttg ggttgtttcc ttctcaccaa cacttgccct tacaagtaac 90660
   ttctgccgga agactagaaa ggtggggggc ttgcagggcc tgtagtcaga ggttgcaatc 90720
   ctgactttgg agtcagacac tgtgtgacct gctgtgactc aaacttcctc agacttggct 90780
   tcctacctct ggtgatcata ctgacctcca caagttgtct caacttcaaa caaagcctga 90840
   gccttcctaa ctgaggaaag ccccctcagc agttcttgtc tgggaggttg ccatgatgga 90900
   aggtctccta gcaatgttcg ggtgtctcta gcaccctcgg cccagcatcc cctgatctgc 90960
   ctctgacacc ctgagatatg gctgcatttt cccacctcac tctctcctct attacagatc 91020
   atgtgtctag tgagagtcaa aatccatggg gccattacaa ataggatgac actccactgg 91080
   agactgtcca tgcaagaaaa ccttagaaga ccagagcctt tctctacata ttaaagttac 91140
   tagtttgaat ctaacaacaa acattaggta ttgaacaacc tggccaaata attgtactta 91200
   gtaactctcg aaggcaaaca agttaaatag ctcaagacaa gtaaatccaa gtgtctgatc 91260
   tcaagggcag tcagacttct gtgtttgagc acttttccgt ttagtttggt acccctgtct 91320
   gcatcacttg gattctcatg tggttaatag tggcgaacag ggtctaaaga atccgggcag 91380
   cctggagttt gcttctttta tattatttca caaagacggc gatagctcaa aggcacctgt 91440
   ttaaggatct agaattttga aatcggtggc tgggactgga aggcctacct gagggttctc 91500
   agctgtagct cagtaatact agcctaacat ggaccaggta ctgggctcag tccccaggaa 91560
   ctagaaagta taaaaataaa atgataaaat gataaaataa gtcttttgtt aagactgtgt 91620
   atctgttgct attattgtaa actctccgtg cgtctcactc tggtgtgtta acatacagga 91680
   aagcataatt tgagctataa cagcactgtc gttaaatata cagttctctc aatgggcaac 91740
   caacaaaacc acactgattc tagagctaat tttatttcca gcgctacttg attctattaa 91800
   ccttcagtaa ttacctaaca atggtaatct gttggctgct accacggaca acatcctctc 91860
   agatttttta atgtggagct gtacttgttt gacaggaaca aacacattgt aaggtgtctg 91920
   gatatcatta agaaacttag gagcccaaga gatggctcag tggttaagaa cacaggctgc 91980
   tcccccagag gacccaggtt caattcccag cacccacgtg gcagttcaca cctgtctgta 92040
   acttcagttt cagggaatct gacaccctca tgcagacata caggcaggca aaacacaaat 92100
   agacataaaa taaaaaataa tgaattatta agaaatttag atctaccctg gaaagccctc 92160
   tccctttggt gtctctttgc cttgattaca agaccatcat gaagctgctt tcctgcaaat 92220
   acatactaag tggtattgta acatttaggc aacccaggta aatgtcatgt gataaagaaa 92280
   gggcttctca ctttcttatt tttattccca aaatgaacat caagatcttg catgtattct 92340
   aggctgagtg tcgcctcatt ccgctatcag atatattact tcaagggata atttcaggct 92400
   gagacattta gttcaagatt ttgaggacat gaaagaattt gattgacaaa ggaggattgg 92460
   gaagtgagga tgggtgtgaa ccctcaagct tttttaaaaa aggtttaaaa atagatcagc 92520
   tatagccggg cagtggtgag acatgtcttt aatcccagta cttgggaggc agtgacaggt 92580
   ggatttctaa gtttgaggcc agcttggtct ccagagtgag ttccaggaca gccagggcta 92640
   cacagagaaa ccctgtctca aaaaacaaaa caacaaacaa acaaacaaac agatcagcta 92700
   ggcattaaac ctctgtggct ttaatgggga ctttggaaag tagcctcttt gccttaaagc 92760
   tcccagctgt tccttcctaa tgtgcccctc tgccggtgcc catgcttaag agggctcaca 92820
   gtcacacaca tgcttacaca tcagccagga aggaccaggc acgagggctt catctcagac 92880
   tggagttctc agaaagtcac cctgaccaat gcagcaaagg ggatagtagt tcctgattct 92940
   gtctgatctc gactcttgat tctagaatgt gaggggcctt gccatgggtt tcaaataaag 93000
   gcaagatgtg gccattcctt ttggcagagt cccagcttac cacaatccat ttcttcttct 93060
   ctttttcctt ttctcttttc ttttctctct tctttttctt cccttctccc tccctccctc 93120
   cctccctccc tccctctttc cttccttcct cccttccttc cttcctccct tccttccttc 93180
   ctccctccct tccttcctcc ctccctccct tccttccttc cttccttttc ttcctgtgat 93240
   gctggttatg gaacccacag ccttgcacat gaaaagcagg tgctctgcca ttaggctaca 93300
   gtcccagccc tgaaggagtc ttaataacta ctcaaggttc actttaaaga ccatcatgtc 93360
   tttatttgtt gaattcatgg gaaacttagc ctctcttcag ttctttctac ggagccttgg 93420
   tggtgagctt tgggaggaga gaagccatgt ttcatgtgct cacagctctt ctgcccagca 93480
   gccacctcat gcagaccttt ggaataaatg atgaatgagt gaaggaacga atgctggtgg 93540
   tcaagaacac tgctcagtgt ctggctccag cccttggtgc ccatgtttcc agtgtgcttc 93600
   atctctcagg gccagccttc tcttaacgct gctggccatg gcataagctt tcaaaccgct 93660
   tttgttgaaa gagactttct aatggggcta cccatgaaac cgttgtccat tgagcagctc 93720
   tgtgagactt cctttgaaac tccattgcat ttacctctgt cagtggaata gtcagagaaa 93780
   tagtttctgc ttcacttggt gcctgttaag aatgtgggcc cagggtctgt gttgaccaga 93840
   tttctgcttc aggcgtctca gctaatgaca caggctaagg gggctaagtc ttagcatcta 93900
   agttcacttc agttttcttt gagtaaagaa ttggaacatt tcagactttt ccctttcttt 93960
   gccctaacca cggtgcccat agccatgaaa atcaattgtc agctccttaa cgtgacgcaa 94020
   actggcagct atggctagcg ttccttagca gaccgaagag acgaaacgtc tcaaggcatt 94080
   tctttctgtc tgtcaaattc agagaagcag ccctaacagg aagtacaagt gcacacttac 94140
   aggccattta atctgttcat ttatttactg tgtggaaggt tttagttcca ggcctgactt 94200
   ttacagagac catgtctcat ttttactttg taagcaataa taaagaaaat gagcttttca 94260
   cttcctacat gtatctcctg ggtttggttt tataaaataa cttttattgc atttttattg 94320
   tggatataat acatgtttat tgtttaaaaa atagaaactt ctcatcagca gtaggaagac 94380
   aataaaaaac acacataatc ccatcacata aggaaaccac agttagcatt tgctgttatc 94440
   ttctttttaa tatgccttgt gccttttcta ctgtgaaact ctgtggtagt ctccacagtg 94500
   tgtgtgtgtg tgtgtgtggt tgtttttttt tttttttttt tgagacaaat tttctctgta 94560
   gctctggctg tcttggaact cactctgtag aacaggctgg cctcaaactc agagatcttc 94620
   cttccagtgt ctctcgagta ctggaattaa aggcatgcac taccatgcta tattttaaaa 94680
   tctgagagca tatgtaaaaa acaccctata tcattaaata ttgtcattaa acatgatttt 94740
   catgtttgca tagatcccat tatataacta taactcactc actttgtttt aatgctattt 94800
   ttgttggtag attgaaaagg tccttcagca aggagagatt gcagactgtg cagaacccta 94860
   catgatcatc aaagaaagcg atggtggaaa ggtatggcca tttaagtact gggaacatct 94920
   taggatgaac taatttacaa acaaataatt ttcaacaaaa tagtggagtt agactaccgt 94980
   attttccatt tgaaaacatg ggtaaattgt atttttatga aagaagaaag agaaaccagt 95040
   tactgacaaa aaaaaagaag ttttaaaaca aatacatgat tgacacccta aggatgtcac 95100
   ttgccagtgt gaactctggc ttagatcaag ccacaccggc tgccttggtt ctgaggctcc 95160
   aacttgagag ggttgccaag gccccataag cagaagaggg caagagctct tggttttcct 95220
   caacagcaca aggtattgtc ttatgcagca ttcatagctg gtcaccagcc gctgctcctg 95280
   ctcatcataa attaattagc acattataag tcctaagaca tgactaagac tcttctcagt 95340
   gccaatttac aaaagtttcc agttttaaaa acatggccct tttggaatca gtcagtcttg 95400
   taaacttcaa ttaaaagtag ttgaactcaa taaagacaga tactggcgtt ttaaaatctg 95460
   ttagtatgat tttaggaaga actcctttgg aagcattatc aacaacaagg acattgttag 95520
   gaataattaa gactcttttt agaatttcaa agtagctttc gtaaagtaga ttctcttggg 95580
   actttgatag ataacatact aacctttctt tccccagttt ttctcaggag aattgaagtt 95640
   atctaggtaa acggtgtgtt tgcatgttgg ctgctgtaac aagccatgta atgaagcatg 95700
   tcttcataac aaaaaaaaaa aagtcatctg ctaactgatt gctgtccttt aggggggaaa 95760
   aatctcttcc ttttgtaacc tttgcttcct tatcagctag acttcagcct aaactgttgc 95820
   ttcctgttga ctcatggccc gcccccctcc cctctccccc caccaggccc atcagagtag 95880
   ctactgtcac cccatgcagg cagacaactt ggtgctgacg ctttgtttca tcttgtctct 95940
   ttactgtctt cctccagagt aaagaaaagg ttgaaaaact aaaacttcaa gctgaatcct 96000
   tctgccaacg tttggggaaa taccggatgc ccttcgcctg ggcccccatt agcttagcaa 96060
   gcttcttcaa catctccacc cttgaaaggg agagcacaga tgtggagcct ggggttggta 96120
   agatctccat ctgggtcggg aggcagaggg ctcccccata tgccaccttt gcggttatct 96180
   gctcattagt aaggacttga gaaaaccaga gggtgtgagt aggtgagagg aagatcataa 96240
   atagacgact aaactgcaca tgcttggtca gtgaccccga aacaattact gtgagcagct 96300
   atggagccta agcacataca ctcttctact aagaactttg tatggagttt taagccctta 96360
   aaaagttaat tgaaacaatt gccatgagcc gttatggaac tttaaataca ctcttctact 96420
   aagaacttcc tatggagttt tgagccttta aaaagttaat tttgagtagc catcccatct 96480
   caaaaattaa aatgcttgaa aatgacaaga acatgggtga gcaggcacag taaacaaaga 96540
   cactgtgact ggctcccaca cagagggatg ggcactgtcc aaagctctga gagcaaaact 96600
   ctggctctga gagcaaaact ctggctcctc agcaaaatct ctggcctcct aaagtctact 96660
   ttgcccagta caatgagagt aagcatcgaa agtagccagc agttaactct tctaacttac 96720
   cgcaccgagg ccccttttcg tcgcctcttc ataaatgtga accagtcatg tctacagttt 96780
   cagggtggaa atgtcccagt gagtttagtt ttcaagtgga cccacgccca ccagctgtgg 96840
   tggattttct gttttcctaa acaccttcgt attccgcaaa tatccgagtc ggcccttgga 96900
   tgtttcgtgc attccttggc tctgccagtt ctgaaatgtt gccttctgct gggagtggag 96960
   gccacagagg ctgctactgc tgctgccctc ctggagttac tgttgcataa tcagcagaca 97020
   gaactgcatt ctttttgatt tgtcagtaaa cttcacccct tctgaccttc acatccagtt 97080
   tttctaaggc agttgttcta tgtattgact tattacaaag ggtgggggag ctccgtgggc 97140
   atctctagtc cacgtaattc aggcagaggg cttgccgaga tgttggttag gtctaagttg 97200
   aggatagttc atcattagca ctgaagctga tttcctaagt agtagccaga gagccagcac 97260
   attacacagt ttaggtagcc caatggtagc tctcatcagc atgagagttg ggcatagcaa 97320
   cactcaagag ctgtgctttc taggcagtgg actatacaga acagaaccct taggcttgca 97380
   gaatataaga cacaaggtta caacctggtt cttcaaaaga aataagaaat gaggaagcta 97440
   ttgagagcct tagccctgtc tggtttctag tgaaaaggtc agccagggca cttctgctgc 97500
   ctgctccaca ctagagcaga gacaaactgg atccagaatg tgcacaagct ggtttgcatc 97560
   cccggtgcct tcaggggtgt ctgagtgcgt gctctcccag aggaacacaa gtgtaaaaag 97620
   caaagcaacc accgggctga ctcaacagcc tatcagcaga cccagtactg gctgagctcc 97680
   agattcttgc agggtgacac ctagccacct ctgttctcca gcaggaagga cttagagcca 97740
   ctagggttct ctctgctcta gtaagaatat tagggacttt agcaggagga ctaaggaagg 97800
   ttgagccccg tggagggagg gctgggttca gcaataattg ctgtgtgttc tgacatgtaa 97860
   gaataacaac tgctttcctc aagggaggaa ctctgtgggt gagaagagga gcttgtccca 97920
   atccaggagg ccctctgagc gaaccctctc cttggaggaa aatggagttg gatccaactt 97980
   caaagccacc accttggcca ccaacatctt cttcaaacag gtatctgtgt ctgttacagt 98040
   gcagttggat tcggttgtat tgatgagggt ggtagatcca agggagtagt gaaatgtgat 98100
   cagagaagtc aattagtgtc gtttgttcag ctggattgtt gcgaagatta gagcgcatgc 98160
   aagcgtgcac aagaccagct gccacaggtc aggaactgtg tggagctcgt agctaatcac 98220
   acagcagacc ttttctctga gggagcaaaa cttaattctc tggggcctgt gaaaaggggc 98280
   gagggaacac acacacacac acaaaactta attctctggg acctgtgaaa aggggcaagg 98340
   gaacacacac acacacacac tggataaagc aaggctccaa caactttgtt ttttctctta 98400
   gcctttttat accacctaaa acaaaacttc tctatacaag aaaaaagatt acctcatacc 98460
   cacaagtaac atattctcta aaaacaatca tcacaaaaaa acatattctt caaaaacagg 98520
   ttaaaaccat gacccaaggg gagattacaa catgattact gattaaatat tcttttgaaa 98580
   cccacaccca cctgaacagt tctcatctat ctttctcccc acaaatccat cctgacctca 98640
   aggcaatgat tcttctgtca ttgattaacg aagtttaatc aagctgtcca ctgtagccag 98700
   ttttccctat gctagctgac ggcagtttgt atttaccaag aaacatatct cttatcttgc 98760
   atctcctagt catctgttct ctattgttat actcataata ggaccgttta cattttattt 98820
   ctaaccctgc cttttcatgg tgcacaccga aacctgtgat ggtatcctta gatcacaaga 98880
   tcaagagaca cagagccaac ctagagtgtt ttcttgaagc attaatctgt ctcagacctt 98940
   cctttccaca agccagacca tgagcttgta gtgcatgaga gctcaccgtg cccctttcct 99000
   agcctgtaca gccctcacta ctctacacag agggggcata ttctactctt agttctgtct 99060
   ttgttagatg ttcctggcaa aacccaccta aaccatctcc tgaatcttcc ttgtcaactc 99120
   ccctaacttc ccaaaaccat ttaaatcaaa tcaggagtct gtaaaattac caactcatca 99180
   aaacgacatt gttctgtgaa agtttatctt catattgatc tgtagaaaaa ttgcccaata 99240
   gagtgggctg tctcccataa agcagtcaca ccagactatg ggcagtgggg gtcttgccaa 99300
   gcccaatcac accatgctag atacaggagg aaatatggca atgacagact cgaaaaggca 99360
   cagcagtagc aagaagcaag ccagggacaa ccccttgggg ctgtgcctct ctgtgttaaa 99420
   tccatcacag ctgtgcaaaa acagtggccc atctccaggg agctctcttg catctgccac 99480
   aggtgtggcc gtggccggac acctcccgtg gtgggcatgt agagggtggg gcttacacat 99540
   accttcctta ttcaagacaa aggtgaagac aatgaaatca gaaagaaaga tgattttccc 99600
   atctccatat ctctgaagaa gtgagctagt tcagcattgt ttcagggact aaaaagagct 99660
   tctgttagtt gacatttaca tgacagccac atgtgacctt gggtcctcaa ggtcagatgg 99720
   ctgtctcaga gagctgtaga ggttctgtct gcagcaatgc tgacgagagg ttggctgagg 99780
   agagcatggg gtgctagtgc ttgcactaac tgatttcaga gcggcccctc acgctcatct 99840
   ctatgagaaa cttgttaaaa atcaaataga taagccaagg gtgggagcac tcaggagact 99900
   gagacaggag gatttgcaag tttgaggtca gcttgagcta cctagtcgaa tacacaaaag 99960
   taatcatttt aaaaaatggc tgtcaggggg aaatcttaga aatagctggt ccaagtccag 100020
   gaatcagcag aggctcccca ctaaccctaa acatctctcg gcaggaggga gatcgcctta 100080
   gtgatgaaga cttgttcaag tttttagctg actacaagag atcttcatcc ctacagcgaa 100140
   gagtcaaatc catcccaggt gtgcttggct tctccttagc atcttcggtc caggctgctt 100200
   ctgttgctat gataccatac tgaccaaagg ccactgggaa gagggaaggg ctcacttggc 100260
   tttactctat cacattgtgg tccatccttg agggaaggca gggtaggagc ccaggatgat 100320
   cctcctgtca ggccctgaag cagcaagcat ggaagaatgc tgctcactgg ctcgctcctg 100380
   ggatcacatt tggctgcctt cctcctaccg ctcagcctca cctgcctaag ggtgttgccg 100440
   cccaccctca atggggtgtg ccctcccaca ctaattagca atcaagaaaa tacttcgaag 100500
   actgtttgat cataggccag tttgagcctg acagttcttc agttgaggtt tccactcccc 100560
   aggtgattgt agattcatgt gaagttgtct gtcaactgca gcgagctatg acactcttca 100620
   actatgccca agccatttgt gattgacatt tcttgtttaa tgctttcatt tataaaaact 100680
   gctgagggca atgtggattt ttgcatttga agttaagaat ctaaactgct gtgcattttt 100740
   gacaaatcta ttaatgtttt cttattcata aagggcatat tctacagata gtataattta 100800
   gatttggtct ctcaagaaag tctgataatc atttgaacat atcaacacac cccttatcac 100860
   acaaaatgtt atatggaagt ctggcattta aagagagtgg gatgatttgg cggacgaaga 100920
   cacacttacc ctcaagcgaa tgcccatgtg ctatttctgt atagcatatg ttgtgtctat 100980
   atttctctga cacccaaggt gagagacctc aacccaaagc ccaccaattt ttactgctcc 101040
   caatacctga aagttttttt taaccatgta tagctgtagt atggtggatc tgggccttct 101100
   agtcagctcc ttctcattat atagctcctt actttggtgt tataaaatgc tcctctcaaa 101160
   agttatcatg gcaggctttt acaatctcaa attttaggaa attatctggg aactccaaaa 101220
   tgcatgtgct cctgcaggtc atccaacaga gtatccctgg gctccttctt gatgtgacag 101280
   atttcagtaa ctccccaccc ccgaccccca tggcttgctc taggctcact gaggctggag 101340
   atatccccag ctcccgacgt gatgaactgc tgcctgacgc ccgagatgct gccagtcaaa 101400
   ccttttcctg aaaatcggac gcgtccacac aaggagattt tggaatttcc gatccgggag 101460
   gtgtacgtcc ctcacactgt gtacaggtga gaaatgtggg cccttgtttg gataatacac 101520
   tcgtctctcc agaaccagcc cctcttccgt cttcctgagt atgaggggga aactaaacgt 101580
   gacagcttag aaccacagag aacatttagg tcgcttctag agatgccttg cctaatgctt 101640
   tggaataagg atgtattttt ttcagtcata gttgtaatag attctactgg atgtattctt 101700
   accagaaatg aaaaaaaaaa aaaaaaaaag aaaaatggaa agaaaaccaa atctatcacc 101760
   atggctttct tccatgcaga cagtctttaa ggacgagagg aacatgactg gaatgtccta 101820
   ggtgtctaag gtttcacaca ggcatggtgt cacatgccta attccagtgc ttaggaggcc 101880
   agtgtgggct tcatggtgag ctccaggcca gtctggatta caggcaaaac aattccaaat 101940
   gaagcacagg ttttcagctg aaaacccagc tctattcagg aaacgttagt cagtttagac 102000
   agacatagtc ctcagacgct gctgcccagt gctttagtgt gtggttgtta ggctgatgca 102060
   ataggcaaaa ttaaagtgcc atcttatttg gtttccaatt aatttccttg taagtgtcac 102120
   ggctaccata ctgaatggca aaagagtaag ataccgtaga aatgaaggtg aggctgctca 102180
   gctttcctag tggcctcgtg cccctgactc ttagggaatt tgaagtggcc acccaaacaa 102240
   agcattgttg ctcatgagct ctcgcttccg aaatgtgggg ggagagtgct gaatcgggcg 102300
   tcatttctca acttatccat caatgacact ttgtggtttg ctttccgtat aaaatatctc 102360
   atcaggtctt actgatctca caaagtcacc cttgtgcctt gctgcctgtg gcgcttcctt 102420
   tgcttgtggc agaaaccaaa cacctgccaa gaagcaactt aaggaaggag ggagggaggg 102480
   aggaatgggg agaggaagcg agggactctg ttaactcaca gtttgagggc atacagcccg 102540
   tttgagagag ggagacatga cggcagtggc gcatggtaga gtgctgtaca gctctacaga 102600
   ggaccacctc acagcctggc tgagtgggaa gcagaccggg agtcgggctg gctcccaatg 102660
   acaaatgcac ttccagtgga gatgctccac ccactaaaga ccacgccttc caaccagcac 102720
   cacttgctga ggaccatgtg ctcaaataca ggagcttgtg agggacattt taccttcata 102780
   ccacagccat gctcagcaca acttatatgt gccttcctat gtgtgcgtgt gtgtgtgcgc 102840
   gcgcgcatat ctctgcatac agatgctgtt atgtttatat tcatgtatat atattgtcta 102900
   tatattgtgt actttggctt ttttcactcg atagtgtttt cttatctctt ttgagtatta 102960
   caatgcagct acacattgta ttaactccgt gcattatgct catcgttagt catggtgttg 103020
   tgtgtttaac cagtggaaac ttctaaaaag cttgtatttt tttttaaata tgaatactga 103080
   tctatgtaga tcagtattgg ttttcttgtt ctgtattatt cctttgtcta aagaggccat 103140
   attgtattta ttcagttcaa ttgggaaaca tttggggatt tttttttccc ctgcttgcct 103200
   tttgttaagc atttatggtt ttgagtgtac gtgtgtgtgt gtgtgtgtgt gtgtgtgtgt 103260
   gtgtaggcca gaggtcaatt tcaggagcta catacgttgc ttattgagat aaggtctttc 103320
   actgggagct gggctcacaa attagactag actttctgct caatggctct cccatgtctg 103380
   aattcccagc attaggacta taaacaaatg atgccgtgct tgggttttcc atggtaggga 103440
   gagggtgtca agcccgggtt tttgtgttag catggcaagg gcttagtcta tgagctctct 103500
   ctagatgagc cagctctcca ccctaacatt tgtggctttc cttttaaatt aattgagttt 103560
   tatgtgtatt tgtgtcctgc ctacatatat atctatgcac cacgtgtatg tctggtgttc 103620
   atgaagggca gaaaagggca gttccgccaa gctagagtga ccaagcgttt ggagctgcca 103680
   cgtgggtgct gggacttgaa catggtttgt ctggaagagc aacccgtgct tttaactgca 103740
   tctcacctgg cctcagcatt tgtggttttg actcatcctg cccagtgaca gattttttta 103800
   tcttgtccct ctgtgagtgt gtgtgaaaag tttccctacg agtatgccta acggtgaaat 103860
   tactgggaca ggtgatattt gtttttagtg actttcatat tgtttcttta cccccctaca 103920
   cacacacaca aaagaaatgg atggttatct attctcccag caatgtgtgg gagcccccat 103980
   ctctccagtt cctcacttcc tcactcgtgc tttcctatgt gacatgcaag gagtgttaga 104040
   gccagcccac ctgtccccac cgcacatgca ctaacctgag tcctggaaag gacaggtgtc 104100
   cagtgaacca agtaaaaagt taccatgaga aacacactca ggtgccccac agtaagacat 104160
   ggcgcgcttc cttctggtgc ttgtgaggca acaggacccc caactttatc cttcctgaag 104220
   caactcctca ttttcttcaa caccaaatca gcctttggtc tccctcctta aagagccaca 104280
   agtatttata ggaagtgctc attccgttcc ccactccatg aaggacgacg gaggcatgac 104340
   catgtttgct tacagttgcc atctccatct ctgtggaaac cctatccttt aattggaaga 104400
   ggaagcttgt ggcatgggaa taaacttgta ccacatgaat tctatagcta tccaactgtt 104460
   cccaccaccc cagcacttaa ccacccgcag gagtcattta ctacagacac tatcctttaa 104520
   tcacatcgct gcaggatttg gagtgcatcc gtgcactgac tgagccattc agaatgtacc 104580
   agtgctgagg acaagtctag gcagcttact ctgcaccctg catggcgacc tgagaaacag 104640
   gcctgcaggc gtagagctgc tgttctttgg ttgtgccata gaatggttac agccgctcct 104700
   ctggaaccag gtcttcactc tggcaagtga tggaaggcaa ttctttccag ggtggtgaag 104760
   gagggacctg ttcttggctc taggctattg tgtcttaaca gcatgtttgt ggaaagatac 104820
   acgtaacaca cttgacccag aatctttcag cgtgaaaact tcaaggaatt tagaaagcat 104880
   gttgtcttaa cttcttgtgg tgttagaatt tagtattgct tctatggtcc tcatgtgctg 104940
   agagctcaca tctgatccac aagcaaacaa aaagagaaac ggatagctgg gaatggaacg 105000
   ggcttttgga accttaaagc ccacttccag tgacacagct ccccaacaag gccacacctc 105060
   ctaatccttc ccaaacagtt tcaccaagtg cagaccaagc actcaaatgt atgaacttag 105120
   gggagacatt ttcattcaat catcacaaaa tacaggaaaa tgggctctga agagcacatc 105180
   aattcaagga taaatccttg acacttaatc tcacgaatgt gatatatgac ttaacttata 105240
   aatactttgt atagctatcc cctacaaagg ataaatgcat ctggtatgcc aaacaataaa 105300
   tgcttccatg gccaaagcca gaagtttagg gtcagatttg tcattgtcac cagactgttc 105360
   agggaagggg gttaatggca gacatcaacc cttgcttcct ccctcctcct caggtaactc 105420
   cgttgttagc tgtgtctctt cagtgtcttc tgccaagctc actgtggatg accattctag 105480
   ctgagtcctg tgtggactct acccccaggt acctctacca tctacaagtt gctatgagaa 105540
   aagggcccaa ggcccatcat gacagaacca ttcaggtttt ccaagcgtta agcattcata 105600
   gaatgaacgc ttgctcagac agtcgaaatc acttcacata ttgagaagtg ctactccgaa 105660
   aacagactag atcgctgacg tcagagaccc cggtcttctg aaaccacatt cagcctcttg 105720
   ctaccggcag gaaggataga tttgacttac agtatcagtg cctgtgaagg gactgaacta 105780
   gaaaggtgtc ctctgagaca gacccaggca agcagctcta agaagtcacc aagtaggaag 105840
   ctaaggttga caaaaaaaaa aaaaaaaaaa agacagtcca agtctgagct tgcattcctg 105900
   tctccataac cagacttttc ctttttaaag actagacatt tgcctaagaa tgcattggaa 105960
   caactcctgg catatttttc ctgatgccca acacagctgg caatagaggt accagatgcc 106020
   tcgtggtagg agcagaataa aaataatgtg agttggggcc cagcctgggc aagaccttca 106080
   aggccatctt atcaggtggc agccatgggg ggggggggtg tgcagagatc acaggctcag 106140
   agtttgtaat gagcttgtta actccatgtc gaccttttaa ctagtgatta cactaaagca 106200
   agccacataa agtacgtttt catctctcgg cccagcctcc gaagaagtct tgtttatcta 106260
   attattggac tgctttggag gactgtgctt gccgagaaca aggctgtcag tcggaacaca 106320
   attgctttga tggataatta gctgtttcca ggcctcctcc tattcagctg cgtgttctca 106380
   gaccagaaag cctgatctgg cctaacaagg aggagcctct ggtgcccctg tgatgtcttg 106440
   ctggattctg tgaggcttgt ggcatagcag agaagagaaa ggctcggggt aggtataggg 106500
   ctgtttccaa atgatcatca gggagctgga gtttagtgac tgtgatcggt aggaacccaa 106560
   agtactcaga ggaaccgtct gcggcagagg gtttgccctt gagtgggact tgattgtttt 106620
   taataaaata gactttcagt ttaaacccag ttatcgttat ttctaaagtg gatcttttca 106680
   gtaccggaag gaagtgtggg catgtcacac aatgtctggt ctcactctgg agggccctgc 106740
   tcaggagccc caggaactgt gctaggttct ccactatatg acgtcatcca aaggtctgtt 106800
   gtgacttaga gtggcccacc ccttcatcca cttgatttgg gaagcgatcc cagtgttcat 106860
   ctagatttgc agtcaagtgc gtttgtcaaa gaacacgtag tagtctctaa gcctcacatc 106920
   tcacgtgagg gtggtgcttg ctctccgtgc tgtggtgcgg cccacttgcc tgtgtgctga 106980
   ctcagcatca tcaccaagga atcttggtca tataaaaaag cttagacctc atttctttaa 107040
   atctccataa atgtgcctct actgaaatct ctatcaaaag caccctccac tcttcattgt 107100
   tccattagtt gttcgagttt tatttcctaa ggaataggta acctcattca caggttccaa 107160
   atattaaagg taccgaaggc agagtgaaaa ttccttctgc cactcctgac ttgcagccac 107220
   ttccagttct cctcgttgga aacagctcgt ttatgtatgt ggagtggctc acttataaaa 107280
   gccccgtgcc gcaagctggt gcttgctatg cctgctgaaa ctaagcagtg ggcttctaag 107340
   agatttacca gagaaacagc agtgaacaga agagatggac agagtgtatg cgccatacct 107400
   caggccggct gtggaccagt ggattcaatt ttgcttcttt gcagatgtga actggaccta 107460
   tgcacacgtt acagagcatg acccccacaa actgttcctc ctgagaagcc gggtgtgcag 107520
   ggcactcttt atgaacacac agaactctct cttgcttttt cagagctgtg cttaatctaa 107580
   ccatacgttt tcctgagtga atcttgtgct gaggaaggaa ccttttttgc tttatagtta 107640
   taatggaata gcttagccta cacacttcat gcttgtctag ttattggacg gttttgtgct 107700
   tttcaccctt tggagaagtg tttaaggagg tccaccttaa gagagaacgg ttaggcattt 107760
   gttttactgg gcttagcaag cagtggccag aactccctaa caaaccattt acttcagatg 107820
   agcagtgggg aaaaaaaaaa aaaaaaaaat caagctgttt gaaaaagcca agaaatggaa 107880
   acaaaaatgc ttccagctca ggcctcgtgc aaaaaaggga cacagagccc acatcctgat 107940
   gtctggctcc caaactcaac aataaacaat cctctttttt tctaagaatg gaaaaattca 108000
   gaagagagcc gacaacgtcc tttacagcct tttattttat ttttattcct gagctatgat 108060
   tttagtgcac cattcagcca cttgagggca cagttctctc acttctggac tcaggcaagc 108120
   agctaccttt tgcattagaa aacagataca ctgttttgtt acagaggccc acacatttta 108180
   tggctcatct cttaaacgca agaccaagaa aaaatgagaa ggaccccagc acttcaggag 108240
   gagcttctgt tccttttttt tttttttttt ttttttttgc aaaggatttt attcttacca 108300
   ccagctccat cttcttcctc ggtatttagc gcttttttgg gaaggtggag tggaaagagg 108360
   ccaaggtgtg catcccatga cacagagtga agtctccttg gagggcggta gttcactata 108420
   taacccactc ctttggggca gtagtgccct gtacaaacct acacttctac caacgctctc 108480
   tggtacagga aactgaagca gagccaagga ttaggtggga ggtacagggg aggcagtgag 108540
   gaatggtgca tgaagacagg ctatgtgcat agtcgttggc tgtacagttc aggcttatgc 108600
   tcatctggac ctaccatgct tcaattgctg ctccctcccg tttgactgtt ctaattaagt 108660
   cttgcttttt aatgttatag atgctatttt taccctatag gtcctattta tagaacgcag 108720
   caagaggttc acaagaccct cttttcagat agcctcaggt caaggctcac tggggcagaa 108780
   ggtatgggga ggtaattaga tgaaggcaaa tcccggcttg tagttcacta tgatgcatgg 108840
   tttgtctaga ctggagagaa ctccctgaaa tccaggaggc agtctttggt ttctgtattt 108900
   tgattaacct ttgtcgtcat tgtttccaga cagggtctca ctgatctgct cgggttggct 108960
   ccagacttct gggctgaagt gaccctcctg cctctgcctc ccaggaaata gggaatcaat 109020
   gctcctttgt ttgtttgttt gtttcaaaac tggtcttcag cagggctaac tcctctgatg 109080
   gatcagccct caggactatt cctcttactt cttgttctga tgtaggagag aatggggacc 109140
   ttctatcttt aagtgttttc atcctcagtt tcaactctcc tatctactga ggtccccaaa 109200
   agtcaacatt catttactcc tctctagagc agaggtattt aacagctctc catgaatgga 109260
   tggatgccct gtggtttcag gcctcaaaca gcctgacctt ggaccctcac agcaggaatc 109320
   agggctaacc aagagatgct gtgtcagggt tttctgaggg gaagagtgga tgtcggtggg 109380
   agaggcagac aaacaccaca ggtacaaatc cacataacca ggcctgcagg atcaagcggg 109440
   aggcctgaat tctgacagcg tagtaagaca atgtgttttt acttttagtt ttattttggt 109500
   gctggagatg gaagttaagg cctcccgctg ctaagcatac actctacaac tgagctatat 109560
   acgcatcagc ccccacttaa gaaaccatgg ctaactgatt acacatatat ttttagattg 109620
   aaaagtaaaa ttatttgccc actagcttaa aataccagag agattataag gatgcaagcg 109680
   atgggtctat cctacctctt ttacttcata tttttgctaa ataacttcca aggtatgagt 109740
   ttagatctat gcaactattc gtggaaatat aaatgggata gtttaattag gataaaatca 109800
   gagggcctca acgtattggt tcgctgtgtt ctaaaggatt tgtaccctga ggctaatagt 109860
   tatgtttagt ggtgagaaaa tcctgtgcat atttctcagc ttttcttcaa agtttaaaag 109920
   taagcctggg ctgcggcagg gcgatttatc atgctgactc ccaggtgatg cagatgtcaa 109980
   ttgtcccttg ctcccctatg gacagctggc agtagcagaa ttatagagca tttgtaagtg 110040
   tgtgtttaca aagcagactt ccacttaaaa aaagacagac acattagatt ggtaggtttc 110100
   ctaagtccag aacacataaa aaactgagct ctgaaggcag ggcctgatgg cacccatcta 110160
   caaggccaaa aggctaccta aaagaaacac tgtcaaaaac caaaaccaat ccaagttctg 110220
   agatttccga ctttactcct ttgttctttg tatatttaaa acccctaaag cttctctctg 110280
   acactgaggg gcagcacctg tacatagtaa atgtggaata acgtcagcca ttcctattac 110340
   atctaaaata gatctccctg gaagtgaaat gtgaatggca aacaagaatc acctcctccg 110400
   ctatctgcct tgcttaaaga gtggagctag ccactttcct ttgcaggctt gtatcaggcc 110460
   atcagctcac cacagatgct ggcattagca ggcatctggg catctgctgg catcagcctt 110520
   gccctctggc tgctgaagag gtgaagggca cccacttttc tgtttgcagt gaatcctctt 110580
   gggggggggg gcggttagct cacctgcatt tccactgccg cagcacctca gctctttgac 110640
   tttgcctctg caaattagaa ttagaagcta agatccacat atgagagaag gatttagcat 110700
   tcgtctttct gagtctggga cacctcactt aaatatgttt tacagttcat ctgttttcct 110760
   gatgatttca taatatattt tatagctgaa taaaattctg catagatacc atattttcat 110820
   tatttattca tctgttgatg gacatctggg ttcattccat ttccttccca ttgtgaatag 110880
   ggcctctatg aacacagatg agcaagcgtc tctgtagtaa gatatagaga cgtttaggtg 110940
   tgcggatgct ttagcagcaa agctgactca gggagatcta tttctagggc agaaaaacct 111000
   atttttctgt cccccgcttc tagaatccgt atctaagatc taggatttcc ttaggctgta 111060
   agcatctgag catgtgttac ttaacacata ttaatatgta ctgacttcct cacttattgt 111120
   atatactcta tataacatta taacagcctg tctcaaacca ccaccaacaa caacaaaaag 111180
   tgttaaaaaa caggtttcaa gagccgggtg tggtggcgca cacctttaat tccagcactc 111240
   gagaggcaga ggcaggtgga tttctgagtt cgaggccagc ctggtctaca aagtgagctc 111300
   caggacagcc agggctatac agagaaaccc tgtctcgaaa aaccaaaaaa gaaaagaaaa 111360
   gaaacgggtt tcagttgtca cagaggcagt gacagagtga ggggtggtat tcagaaacac 111420
   acagagatgg ggagttggga agaggcagga agacatacac ctgcaaatta aagatgcaga 111480
   gaaagcaaga gagacacaga agctagagac agtgagaggt ggggagagca atccggaggg 111540
   tttgatccag cctccacatc cccaagctct gcacttctgc tgagatctag acttggggat 111600
   gtcaccgtgt tcacaggcat cctcgtgcac gacggttgac agcaaccctt ttacaacagg 111660
   tcaggaaatg aacttaaaac ccagtttgct ctgctacaga aacacgtaat ttaccagcag 111720
   tgccagtgta cagaagacag cccatacaat agaaaatcag tacttttttt tcctccatta 111780
   aaaaaaagtg catttagata tcattccttc atcccaggct gcatattgaa ataacagaaa 111840
   gaaagctaaa attggcacca attccatttc aaagtcaaac ctactgcttt tcatttccca 111900
   gtcttttcca gcccctgctt tcataggtcc tcatgttctg cctaactttt agccctgtca 111960
   gagtctttgt gttttgtttg atttggtttg gtgtggtttt tttttttttt tttttttttt 112020
   tttttttttt tttggttttt ggcggggttt gtttgttttg tttgtttgtt tgtttgttta 112080
   gttgcattct gggtttttac ccaacatcgt gtgtggaaag aaagcctttg actctcctca 112140
   gctgcacttt caaagagaca cacacaggga agaagcgtcc agggtttgcc atgaacagtg 112200
   atcgctgatc cgtggtgctt ttgttgatcc gtggtgcttg ttgggatctc taagcctgcg 112260
   gtgaagtctt cccagagttc cagaggctgc attctggctc tgctggctgc ccttccttgt 112320
   gtaccttggc aagtgcagaa gtaagaaaca caaatatatc ttattcaaac cgtggtctag 112380
   gagacccggg tcaggagtgg aagacgtggg aacaccgcat gaagggttgg cttgctctct 112440
   actctcacta accagaaaac ggtctctgtc accgctatca tagactttcc tccctgtggc 112500
   atagtttcca tcccgctgaa gtcgtggtcc cctgacctga tcagtgataa ggtattcact 112560
   gtgacatcat ctgtcacctt tgcacagaaa gcgcattcgc tctaaggagc tcagcagcag 112620
   ctggtgctcc aggaatgtca ctggctcatg aaacttctcc gtggacctca ctcttgtggc 112680
   cagctcgtcc ttctgacaga gggtttattc caccctttcg cctgtttggc tttcagtgtg 112740
   ctctgcactt tgttcccttc ctgctggctg atgtgatctc gaagctcagc tacagagcca 112800
   tttaagttgt ccagttgttc caacagaagc taacatgctt tctatgagag ctgacttgct 112860
   ccttgatcct aacagagctg tagaagcctc tggaaagtgc ttgagtgttg tcctggggaa 112920
   aggggtaagg gtgagctttt ctcttccttt ccttgccctt ttagataagg gatagactaa 112980
   acttaggaaa agccaaacta atgtgttttt atgctgtggc tttaaatgtt cctttgggct 113040
   aacctgttct ccacaaagga aataaaggac agtgccaaga tggtaagaaa aaaagagttc 113100
   aatgtttgat ctatgaatgg ctgcagttcc acagttagtc ctggacctca aatgagtttt 113160
   aaactaagct gagggctagg gacaaagctc aatgcttgcc tagcatgtgt aagaccttag 113220
   gctcgacttc tagcactgga agatgataac cacatatata tacacacagt tatcccgagc 113280
   tagtatctcg ctaccaagaa ttcactcgga caaccggatt cttctacggc aaagctttat 113340
   tgcttcatct gaaggaagac cccgaacaag gaaaatggtg ccgcctatat accccttcgt 113400
   gtgacatgtc agctcctgat tagctgctca ctcatcaccc cactactacg ccccgggatg 113460
   ggcagtgact tatttactct tgcacctgcg cacattactt gtttaccagt taggcacagc 113520
   ggaggctcat gatggcgatt gctcatggct ctccacacac agtcataagt atataaatgg 113580
   catattcctg aatctgtggg aagaattaac aacctataga gctgctaatc aagagaatcc 113640
   atctgaagga tgcttaaaac actctttgga agggtaacat ccaactttct ccttctctaa 113700
   gctgggatgc atataagtaa aaaacatctc tttgattaca tttctccaaa tgtgattaaa 113760
   aaaaataaga tgaggctcag atgccttgca cattcatgag gcctctagtt attacagaac 113820
   ctgtcaaaac agcttcattc ttcatatgag tgattcctgt ttacaatatg gactgtcttc 113880
   ttacaggctc ttcaataatg taatttttac aattttcctt aatactaact tatactataa 113940
   attacatgag tctgttgacc agttgactaa gattactctt ctctgataca agctctggat 114000
   tacagactag acatagagat cttccctgtc aaaaggaaag cattattctc ttctgttcat 114060
   tctgtaagag atgctaatct gccaaagaca ttacttagca ttcttgtcag taaggaatat 114120
   ggtggtcctt aggaaaacaa tcttctcaaa aacattttag gcaaactcac tctacttttc 114180
   tgggagtcgg tttcatggtc tgcaaaaagg gatattaaac caaattctct aagacttttt 114240
   cttctgttgt gtgtgtgcag acagtatcaa cataaccttg acaggtatgc tggtgacact 114300
   cgaaaaggtc tagcaacccc ttcagaagca ctgtgcaaaa cagttgactg gtgttgggtc 114360
   ataggggaca ggtctgtggt gcctgcttac tggggtcaaa gacgagagtg gtaacagcga 114420
   accacactgt ccagtccagc aggccactgc tagagtgact tacacgccct tcatccagct 114480
   gcttcaaagc attttcaaac cctagctcat cagtcctcca agcacagcat taaaggatga 114540
   tcagtggcta tcaattaatc taaacatgaa aatcggagca ctccagggtt taaaagaatt 114600
   ctttcaaaag tctactgcat ggcaaaagat caagatacaa ggtgttcatc acagctctat 114660
   aatcaatgaa agggagaaaa atcacataaa tgtgctttgg gggctattat atactgcagc 114720
   acattcatta tatgaagtag tgttcagtaa ttaaaatcat gattatgaag aatatctaat 114780
   aacattggaa atggctataa ttgaacagta ttagacacta aaacacgaaa ttgtatacac 114840
   agtttgattt ttaaaaaata tttattgtat gatggaaaag actaaatgga aacatgctaa 114900
   agtttttacc atagttcgcc tcacacagga ataatgaatg gttgctgttt tcttctaaag 114960
   aatcctttag tgttaaatac tttcctgtgc ttttcagcta ttcattggta aaaacctgtg 115020
   cacattatac agctgtggtc atgtattact ttcacaatca taatcatgta tagatagaat 115080
   tatgtgacta taaagtaaca tgagcctgta tttgtatgtg agagagagat gcaggatgtg 115140
   gcatagatca gttagatgag atgttaggag aaaactggga ggctgtgcca tatgatctga 115200
   agccccagtg ttcagaaagc tgagaaagct gtgggaactg ctggagagaa ggcagtgggg 115260
   tcaggagtat tgtttaaatg tgctaaccga aaaaagtcaa tgtggaaaaa gcaatgaaaa 115320
   ataaagtcag agaactagag aatttgctca gaggttaaga gtttgtctta gtcagggttt 115380
   ctattcctgc acaaacatca tgaccaagaa gcaaattggg ggaggaaagg gtttattcag 115440
   cttacatttc tacattgctg ttcatcactt aaggaagtca ggactggaac tcaagcaggt 115500
   caggaagcag gagctgatgc agaggccatg gagggttgtt ctttactggc ttgcctcccc 115560
   tggctttctc agcctgctct cttatagaac ccaagactac cagcccagag atagcaccac 115620
   ccacaagggg cctttccccc ttgatcacta attgagaaaa tgccttacag ttggatctca 115680
   tggaggcatt tcctcaactg aagctccttt ctctgtgata actccagctg tgtcaagttg 115740
   acacaaaact agccagtaca gagtctttgt tctagatgtg gtggcccagg cctttaaaac 115800
   ttgggaggca gagtcaggtg ggtgtttata agtttgaagc cagtctggtc tatatagttc 115860
   caggtcagcc agggctgtct cacaaacaaa caaacaatac gtattgctct tgcagagtcg 115920
   tcaggtttgg ttccagtacc tgcatggtgg ctctcaagca tccctaattc attttagggg 115980
   agctgacatc ctcttctgac cttcaagaac agcaggcgtg cccacagagc acacacatgc 116040
   acacaggcaa aatggtcaca tataaataaa tcataaaaaa aaaagatcca caataaaagt 116100
   ttgggaaacc atggtaggga ggggccagat acagctcttt atatttttca aagatccctc 116160
   tctaggaagc aaaagtgaat tgagaaagaa ctgctgtgtt agaaagatgc ctccgtggtt 116220
   aagaccactg gatgctcttc caaagggccc tcgcacccac atgatggctc acaagcatct 116280
   attaactcca atccccaggg ctctaacacc cttctccggc ttctgcatgc atcaggcaca 116340
   catgcataca tatatacata catatacata catatctact cagacatatg tgtagataaa 116400
   acatccatac acagaaaaat aataagtaat tctttttaaa gaatgaaaga aggaggctct 116460
   gtgctccaag tgacaggtga gggtggtctg gagtggagag aatgaggatg ggtggagggg 116520
   agaggggagt gcacctctag tttgtccact ttgtggaaga acctgtcact tgaatagcca 116580
   ttgttagtga gttctcggtt ttcctttcta ttctttcctt ccctccctct ccctgtcttt 116640
   ggctgtgtga aatagggtct cttgctgtgt agccaaggtt gactaagaac ttgcagcagg 116700
   cctctgccca tcccaaagcc tattggttgg tgcctttatt agtcccctga tgctttggga 116760
   agcctaactc tccagaaaca gaggctgtca aagccataca ggtagaagcc aagatggatt 116820
   ccaatctctg ctctctggct ccagtactaa ccctttctaa gatatcctat cccagctgtg 116880
   caataaacac taacctgctt atggacatgg ctgcagtgag gataagcaag aaggagcaat 116940
   gggcagagga gtgcatactc cctagacaga catgtttatg tgttcgtacc catgcatgtg 117000
   tgcatttgcg tgcacataca cacacagtac attaatatgt cagtgtccac tcttgatggc 117060
   acctgttcct cgaccgtact agatttactc tgactaatga atgagatcgg caggtccact 117120
   gagacagcag agggcagaga agacgcagtg gatgttgggt cttctgctct cagtggctga 117180
   ctcattgagg agcccacctc agctcacaag gctgtgaact tctcatctta gaaagtattg 117240
   gcttgcactc ggtacctcgt cttgaaggac actcccccca ccccacccca ccccacccca 117300
   ctcccgttct acaccaatcc ttttgttgta ccatgacctg ccatctgcat tcctggatag 117360
   gctgtgtaca ctccaatatt tgaagcacat tgaatattct ggccattgaa attaagttta 117420
   gattgttgtc ttacattcct tttcatttga gtgtgagaag tattcagtgt agacaggaat 117480
   aagagccaac aagaggacgg gataaagaca gattaaaacc aagggcaaaa atccagaggt 117540
   gagtcaggga ggtgactatt gtgtgaagtc aggatggcct agaaaggcca tgcaactcta 117600
   ccagccccta gactctcacc cccgtcctgt ctccagggag cacccagttt agagaaacag 117660
   ggccaacaaa gcataggaat tctgtgcatt tgcccctgtg acctttgaac tttacacaca 117720
   agtcaacagc tcctgtgttc ttcagagttc caaatattga ttttcttctt gaattacctg 117780
   aaagcattcc ttactacaag atatttgagg agaaaatcct tgggttttgt tctgtcttgt 117840
   cttttgattt ttttttttgt tttgttttca aggcagaatt gtgctatggg acccctggct 117900
   ggtctgcatt tccttctgta catcaagctg gcctcaaact cgcagtgagc ctcttgcctg 117960
   tgccactcga gctctgggaa tcgcatgtat tacagttctg cgcccagctc agaagaatcc 118020
   ttgggttgta atttcttcct ggatatgaca tgttcttctg aagccagata cctttatttt 118080
   ggtgtttctc ctagtgattt cctaggatgt aaaagttcta aaatatagtg agaaaaccca 118140
   gccaaataaa atgagtgctg aggtctaaat atttggggaa aggagtagag agagcccttt 118200
   tccttctaag gcagtgtttc tcagcctgtg ggtctcgact ccttcctttc acagggtcct 118260
   cttaagacca tggaagacat agatggttgc attgtgcatc ataatggtag caaaattaca 118320
   gttatgaagt agcagtgaaa ataactttct gggggggggg tattgctgta acatgaggaa 118380
   ctgtataaag ggtctcagca ttaggaaggt caagaatcac tgctgtaagg cattcaggct 118440
   acacagtagc ctgttcatct taatttgccc caagcttccc cagcagtttg gctaccccgc 118500
   caccttcttt tctccaaata cctgttaaca ctacatatag cactgggatt agaaagagta 118560
   gagcagactg gagagtacag gcataggggg cagtgttaac cacctcaaag ctaatggact 118620
   aggccttatt ctgaaatggg ggacggggag gggagttagg cctagttccc aaatctggct 118680
   tctctctaat gtgctatcta cttagttcag aaatctgcac gttttgaaac acttagaaat 118740
   ctcatctcat cctctttcca gaccagggca gggattctga acatgagctt gcctctcccg 118800
   agtggtgact gagtggttca ccaggattag gcttataaca tgactttctg tgctcatttg 118860
   atccaaagtg actactgtgg ggctctcttc acagtccacc agaagcaggt ccactcagcc 118920
   gttcattcac ctcacacgtg actactaaat tcatgaagtc ttttctagtg aaacatactt 118980
   tacattttaa tgtagaccca attgataaca ttaataattt aataatatat agtaagaatg 119040
   aaaaatatgc taaatcttta aagaaattta ttcagatagt gtgtgtgtgt gtctatatga 119100
   acatgttagt gtaagtgcat gctcactgag atgaagatga agaagatgat gacaggtgtc 119160
   ctccagctat tccacagagg cagggtctag ttctcaatcg agagctcagg tctgccaggc 119220
   tggaagccag caagctccag agagcctcgt ttctctgcct ccccctcaga tatgaggtta 119280
   caagcattct ccaggtgccc agtttgttgc ctggatactg gtatccaaac tttgttcttc 119340
   atggttgtga ttcaagtgtt cttaaccata gagtcatgta aacagagaca gcccggcagc 119400
   atttgtcatc atcccaaaac aacatacggg atcatcgcag tagtatggac acgggttgca 119460
   gaagacacct tgggaaataa caaagccata ttgccttccg gatgactcct catctcatca 119520
   gtcatttcca gcgggagaca ctttatggca gcgtagcatt acctactttt agtgtgaccc 119580
   aaacccgtcg aatgcttgtc ttctgcatct gctaatacaa ggttcatgtc gcagccgccg 119640
   acacagccct ctgtcctcat agtcaccagc ttgtagtgca gattctgaac tatggtctgg 119700
   actatggttg ttgagctgcg ccatctcctg cccctgtggt cctggccact gtaataccat 119760
   ggtggtgtct cacaaaaccc acacaggtct gccatcacct tagagagcaa cttctggact 119820
   gaaacaaaag ccgaagtgaa ctccagacat catctgttga tcattggtct ctctcttttc 119880
   cagaaacctt ctgtacgtat acccccagcg actgaacttc gctagcaagc tagcatctgc 119940
   ccggaacatc acaataaaga ttcagtttat gtgcggagaa gaccccagca atgctatgcc 120000
   ggtaagtaga tgggtagtgt ttgcctccag ttgggtggcc tgtgctccct ctgccaggtc 120060
   acagggatga aatcaagagt tgctcagcat acagagtacg tccaccacac acacccaagg 120120
   tctcttggtg atgctaggcc agggctttac cagtgagcca catctggctc aatacacaga 120180
   gtatttttgt tccagcatag cctactcaac acatgcagga acctttctgg ctagtccagg 120240
   attgtccaca ggaccatggt agtgaaacag caccagtctt aagtctcttc ccacattata 120300
   cctttgtgag gtgccacacc ttggaggtga tggtcatttt aaaggactga aaggtggtgt 120360
   ggggtctcgc cacattgccc atagagcgag gccattctaa cactgaagag ttagagggtg 120420
   acttggtatt cattctttgg ctgaatttct tcaacacatg cacgtaggtg agcatgaata 120480
   gagccatgga gaacagtcct gaagcaaagc ccagtgataa taaacacgcc agcgcaacgc 120540
   ctttgggaaa tagacttgta tcctgagtca aattataaat taataataca gactagacta 120600
   gtataaccaa agaggcctct aacaccagaa tttcctagta aatgacatgg tcgattgcta 120660
   ttactattca cacacatcct ggaattcagc tactattcag ctggggttgt agctactgag 120720
   agcatttgtt tagcacgtgt ggaggtcctg gtttgatccc ccgcagcaca tggggggatg 120780
   gggagaatcc ttacgtatta agtgagagag ggagaggttt gagctttctc cttcacatca 120840
   ttttctggtg aagtcatttc ttccttccct gtgtatctac ttcctagaaa gtccaccctc 120900
   tctcccacct cttcgctgat gcaaggtgac atcttgcgac agtggtggga aaggcagaaa 120960
   agagggtgct tggaaggaga gaggtctgga ggactcgcct agtcctctct gccctagccc 121020
   tggcaggcca ccactcaggc ttcccagtgt gtatgtgcac atcctgcgtg tggccaacgt 121080
   tctcagccaa aggacagtgg tggtaaatgt aagccagttc tccactcacc aagccctggg 121140
   ccaggtctat ggttgctgca agttcactag acagatcatg taccatcggg gctgcagcag 121200
   actccatgag cttctgcata caggtggcca gggcattgga ggctacaatc actcacatgg 121260
   ctaagccctt gtctggaggg gaaaagatgc tggtgtctca ctggaaacaa atgaatgtga 121320
   tgtcatgtat atccatgcac agaggcagta aagagacctc tggctttcca gtctcactag 121380
   cctgtgccct tggtcactca ttatcccctt aaagccccag tttacccttc aacccacttc 121440
   tggtccatat ggttgggcca gtgataggac ttagccttaa cttataaact acagctctgg 121500
   gagagcagtg tcctgttggt gtggtagcac atgttcatag agccccaaat tcctctaact 121560
   ttgttccttc catgttgaca aacaggtcat ctttggcaag tccagtgggc ctgaatttct 121620
   gcaggaagta tatacagcta ttacatacca taataagtaa gtatatttca gagtcctgaa 121680
   catatactac aaccatttct gtattcccta gattttcggt ttttaactcc acaagtgctt 121740
   tttccaaaag tttttgcaac atggccacag agcagaggat tttacgcctg ttctgtgttg 121800
   ctaatgcata atttttataa atgactaagg ctttgataga atatatctat ctaatataat 121860
   tctatttaca aaatgatgat agggacactt tttaaaaaag atttattcat tttatgtata 121920
   tgagtacact gtagctgtac agatggttgt gagctatcat gtggttgcta ggaattgaac 121980
   tcaggacctc tgttcactct ggcccagctg gctccagccc atatatttat tattatatgt 122040
   aagtacactg tagctgtctt cagaaacacc agaagagggc attagatctc attatggatg 122100
   gttgtgagcc accatgtggt tgctgggaat tgaactcagg acctttggaa gagcaataca 122160
   atgctcttaa ccactgagcc atctctccag ccccgatagg gacactttta taatgttaaa 122220
   taaagaagag tattttctca cttggtctga aatccagagg gccagaaaca aagccccaac 122280
   tctctatggt catcttgtca tctgccaacc aaaggaagaa tggtatattc aatcctgtca 122340
   gtaaagtgag tcacaggact ggctatctaa cctcatttac acatcacagg gaaggaggga 122400
   catccctgtg ggtttcactc tgacctgata accagactcc tctaggggct tcctgtgaac 122460
   cttggccaag cttgtcgagg agactaagtg ggtgatgttg taaagcctta gcacagtcca 122520
   ctgatgaggc agccacatgc caggcctgtt gttgaggctg agatcatact cagaatctcg 122580
   tcctcttcag actgacttgt gtttctttat catgtaggga aagtcctttc tgtgtaacaa 122640
   agccttgacg gtttcatgta ggtggaatgt ttctcatagt atcagcatgg ccgcaaggcc 122700
   ttaatggcca aggggcagga taaagtgaaa taaaaagccc cacagcatgc aaggcacatg 122760
   ggagcagagg gtcggcaggc agcaggggct ccgtctcgca cccagggact catccttttg 122820
   ccgagagtga cctgcctaca ccgtgcatct gatacaggtc tcctgacttt tacgaagaag 122880
   tgaaaattaa gctccctgcc aagctcacag tgaatcatca cctcctcttc accttctacc 122940
   acatcagctg tcagcagaag caaggggcct ccggagaaag ccttctgggg tactcggtga 123000
   gtcgctgtca gcgagctgct tccccctgcg gttgctggtg cgcaccagcc agggtctctg 123060
   cagaggccgt gtgcagtctg ctcacttgct tctctcctgg ggctgccaaa tacgtggtgc 123120
   atattacagc agagagtaga aacagcttta cctagagagt gccaggctcc tcccatggaa 123180
   agctggtggg tggtaaatct ctaaagagca tttaagaaat gaagcattac aatatctgtt 123240
   atttccaaat ttaatgtaag tgcttcacat gtagaattag ctcttcagag gggggggtgg 123300
   gagttttttt ttttttcttt aaaaaccatg taaggtaaat aaatgaacat tggtaatcag 123360
   acaggagctg gcagtggcca taaagaaaaa atgctactcc gtgggcccag aaagcggcaa 123420
   atcattggcc ttaccatctc agaaatgctc agggaattta caactcaggg aacgggacgc 123480
   aaaccgctgc caggagaccc ggtcaatatc taatcattgt ttagttttgt ttgctggctg 123540
   tgagctagat aaatctcccg agtttcgtgc agccgtaaag ccctctctgc tcttgacacc 123600
   atctgtttca gtggctgccg attctgttaa acgaacgtct tcaaaccgga tcctactgtc 123660
   tgcctgttgc cttggaaaaa ctaccaccca actactccat acattctgct gaggtaacca 123720
   gcgctgcctg gcagctgctt ccgtcctgct gtggtctggg gttcctttcc cacacccact 123780
   tcccattccc caggatgtta ctcgctgggt tctccagggc tgtcatctcc aagcacgctg 123840
   tgtttttctg agagcatgtt gttccgacag gatgggagga atatcttgct catttagacg 123900
   tcaccacacc agaatgttct gtaggtgcaa cgtgcacctg tgctgcatgg agttagctgc 123960
   gctgcacagg gttagggttc tgtagatggg gaggcagggt gaccccactt gtgacagaca 124020
   ccgtacttgt caagaattct ttcagaatgt ttccaggcct gtaccagcag gttcctcttg 124080
   tggtcacagc cagatggatg gactgtaaag atgccaatgc cagagccagt gcccttcctg 124140
   acctggctgt tctggctttt gcaggctgta gcaatacccc agtctaaaga tagtcggtcc 124200
   catggctcct agctccgtat cacatttctc cacccgttag cagaggtcta catcaatgag 124260
   tagtgttttt aagcagatca tcttactcat aaatatttta tcatgcatct tgaagatatg 124320
   actctttcta tcacaataca atccgaggac ctttatattt aatgtaatct tgaatatcaa 124380
   actatctcca ttccatgacc acctttctag ttccccacca ggaatcatgt ttttatgctc 124440
   atctgaattg gggctcagat cactcatcgc tcttccttga attctgttgg gttttaagtt 124500
   gaagaaattt ccaggacctt gagtttagat agacccttca tggtctttct cccctgccat 124560
   tttctgatac cctcaggttg gccctttccc tttgttccct aaagctgttc cccttgacac 124620
   cacaggctag cttaaggatg gagacattcc ggctcatctt tatgtcctag tcatacagtg 124680
   tctcacagta cccctcacaa cagcttttgc agagccaagc aaggtggtgt gtgctgtaat 124740
   cccagtctga gtgaagttgg ggcaggaaga tcactgagtg tacgacctag tgagccattg 124800
   ttaaaaaaac aagaacaaac aagaagacgt acacacacac acacacacag ctgattttca 124860
   atttttaagc tccaccttcc aatttaaaag aactcctgta ttgtgttgtg tggtgttttg 124920
   ttttgttttg ttttcaaaag catggataaa acctgagttt gtgttgttgc tttgttgaat 124980
   tttttaaaat gttttttaag acagggactt aaaatatata tagcctaggc tgtccacaaa 125040
   ttcaccaagt agccaagaac gactgtgaac tcccctgatc cccctgcctc tgcctctcta 125100
   caagtatgca ccaccacgcc aatcccaacc tgagctttaa ggcgaatcca aatcccaaat 125160
   aacaactgta tgagtgtggt cacactgctg gcatgtaatg agtgtggtca caccgctggc 125220
   atgtaatgag tgtggtcaca ccgctggcat gtaatgagtg tggtcacacc gctggcatgt 125280
   aatgagtgtg gtcccaccgc tggcatgtaa tgagtgtggt cacaccactg gcatgtaatg 125340
   agtgtggtca caccactggc atgtaatgag tgtagtcaca ccgctggcat gtaatgagtg 125400
   tggtcacacc gctggcataa tgagtgtagt cacaccgctg gcatgtaatg agtgtggtca 125460
   cactgctggc ataatgagtg tagtcacacc gctggcatgt agtgagtgtg gtcacaccgc 125520
   tggcatgtag tacaaagccc atgataaaat gtttatcctc tgattctcaa gggtaaaagg 125580
   aagtccacat ccgactctaa agtgcagtga gcattaactg agctggttct tatccagcag 125640
   gaaatagcgt taaagtaaat agtagcttct ttttcctacc tatagactca ccctcagccc 125700
   tcccctcccc attgctgttc cctgtcaaat acagcagaag caagtggcta gcgagctctt 125760
   gaatgggcta gttctgacgc agcgctgtca gtgtaaaata tgagctgaat ttcgaagact 125820
   tggtattgaa aaaaatctat tttttattaa taatttatat tgacctgtca ttgaggaaat 125880
   aggccatact gtgttaaatg agatagtctg ctgaaattgc cctccatgtt ctttaatgtg 125940
   gcctccctaa aacttaaaaa acttgggctg gtgagatggc tcagtgggta agagcacccg 126000
   actgctcttc tgaaggtcca gagttcgaat cccagcagcc acatggtggc tcacaaccat 126060
   ccataacaag atatgactcc ctcttctggt gtgtctgaag acagctacag tgtatttaca 126120
   tataatgaat aaataaatct ttaaaaaaaa aaaaaaaaaa aaaaacttga ggccagtgaa 126180
   gtggctcaat gggtaaggtg ctcgctacca agtttgatga cctgagtttg atcttgacgt 126240
   ccaggatcct catggtaggg ggagagaacc aacttcctaa agctattctc aggcctctat 126300
   gtgtgcatat acacatgcat gttctctctc tatagccctc tctctccaat gaaaaagtaa 126360
   tgttgattcc cgttgggttt ctattgaatg tcactcctcc agatcataaa tgcgaacgaa 126420
   ctccagtgtc tgcaggaatc cagcagggcg cataagtgag gctggtcctg gtggaacgtt 126480
   tcactctggg ggttgtgcac aggtttcaaa ggctatggct gcctctgttc cacagactgg 126540
   caccgtgctg ggagatcttc cagctctcaa gtcagaaatg agcctgttgg ggctggagag 126600
   atggctcagc agttaagagg tcctaagttc aattcccagc aacagggtgg ctcacaacca 126660
   tctgtaatgg gatccgatgt cctcttctag tgtgtttgaa gacagctgca atgtactcct 126720
   ataaataaaa agtaaataaa tcttttttta aaaaagaaat gagcctgtta tatgaacgtt 126780
   gctggctttt aaacattaac aacccattta caacaggccc aataccctgt gggccaacta 126840
   gagctctgtg agcttgggtt ccagcttgtg ccattcagag acgtccatcc gtctctgccc 126900
   ccttgcctgt ccttacaagc ataacatgct tcctgccatc atgatgaaag aggcgcagct 126960
   cccgtgctag gccctctttg ctccagcacc ctctcatctc tctagaggaa catagtaccg 127020
   atgggaggtc gagagaggag ttatttctaa gacctaagga gtaggtatcg gaggatgcat 127080
   aatgagcatg tgggagccaa gcaggactgc cacttggaaa cactttgcta aaagggaagt 127140
   catttctggc attcaaaccc attcggagtc ccagtctctg tcctgatatc tctgattgat 127200
   aggaagatca catgactcca ttcgtagcct caaagacctt cggtgttgaa taagaggttg 127260
   gaggtagatc acccagctaa ctcttccacc atttgaacaa atgctaaggc cccgtgtgtg 127320
   tgtgtgtgtg tgtgtgtgtg tatgtgtgct tatcagtgtg aaaataacaa gtgtagactt 127380
   gaacttgaga caaaggcctc tgactcccaa gtctagctat ggacgttctc catctgtaga 127440
   gccttgccat tcctgacatg ttgcttgtac cgcttgctgc taacagttga gaagggaaat 127500
   tgcattgcta atctttctct tcctctctct ttaacacaga aagtcccttt acagaatcct 127560
   cccattaagt gggccgaggg ccataagggg gtatttaata ttgaagtgca agctgtttct 127620
   tccgtccaca cccaggtaag ggacatcaag gtctgccacc gtccgggtta gccatctgct 127680
   ttgtgctctc tcttgacagt gtgataccta gtacatcacc tacctacagc atgagttgga 127740
   gcctggttta ttgtttcttt aattaatagt tgtcaaaagg tcctatgttg tataccacat 127800
   tctacaaata agcccactgc ctttgctgga agcatctgag atttcgagtt ctaatccaga 127860
   gcactggcta agggattgca ccaagctcct gggtggatgg tgaaagaata acacctcctg 127920
   agagtcttcc accctctgcc ctcccaggat aaccacctgg agaagttctt caccctttgc 127980
   cactccctgg agagccaggt gagcttccct atccgtgtgc tggaccagaa gatcaccgag 128040
   agcacgctgg agcacgagct gaaactcagc atcatctgcc tcaactcctc ccgcctggag 128100
   cccctcgtgc tcttcctcca cctggtgctt gacaagctgt tccagctttc cgtgcagccc 128160
   atggtcattg ctggccaaac aggtagaggc ctgtgggtag ggaggaggca tggctagatc 128220
   aggcgggcga gtgggcaggc aagcaggaag gcaggcagac aggcagcagg cgggcagaca 128280
   ggcagcaggt gagtagacag gcaggcaggc agcagacaga caggcagaca ggaagcaggc 128340
   aggcgggcag gcaagcaggc agacagatag gcaggcaggc agacgggggg gggggggggg 128400
   gggcaggcaa gcagacaggc agcaggtggg taggcagtcg ggcaggcagg cagacaggca 128460
   gtaagcaggc aggcaggcaa gcaggcaggc aggcaggcag gcaggcaggc aagcaagcag 128520
   acaggcagca ggcaggcaga tgtactttgc ctaatgaatc atggctattc acagacccac 128580
   aaatgctggg agcaactttt gcaacacttg acatgaagac aggtccggta ctgttttgtc 128640
   agactcccta catgtttaca aaaaaggaag aagggctgga gagatgactt agtcgttaga 128700
   agcacttgct gctcttgcaa aaggcccagg ttcagttccc agaactcaca cagttcacac 128760
   tgtgtgcatc tccagtttaa aggtacctga tgccctattt ttttcaattt tttattagat 128820
   attttctaca tttacatttc aaatgctatc ccgaaagtcc cctataccct ccccctgccc 128880
   tgctccccta cccacccact cccacttctt ggccctagcg ttcccctgta ctggggcgta 128940
   taaagatttc aagacctagg ggcctctttt cccaatggtg gatgactagg tcatcttctg 129000
   atacatatgc agctagagat acgagctctg agggtactgg ttagttcata ttgttgttcc 129060
   acctataggg ttgcagaccc cttcagctcc ttgggtactt tctctagcta ctccattgtg 129120
   ggccctgtga tccatccaat agctgactgt gagcatccac ttctgtattt gccaggcact 129180
   ggcatagcct cataggagac agctatatca gggtcctttc agcaaaatct tgctggcatg 129240
   tgcaatggtg cctgcctgat gccctctttt gacccccaca gatactgcac tgacatggca 129300
   cgcatgcata catgcaggca aaacgctcat acacatccaa aaataaataa aataagtatt 129360
   ttcaaaagtg tttttgaaag aaaagcaggg gactagaggg atgagaaaga aggagagaga 129420
   aagaacatta agatgggctc ttaggactga tgagatgctc agcaagtaaa gatgcttgtc 129480
   tccaagcctg agcgatgcca tgaattagat gctcgagatc cacatggtag aaagagagta 129540
   ctggcttcta gaagttgtct gaactcaaga tttgccccat gacacacgaa cacacacaca 129600
   ttaaaacgtt ttcaaatgtt tgtggttcag gtacttggtg gtgagacttg gggctcactg 129660
   gccaggcctg aatcaaaaca atatggtgga gaacaattga gggcaatctg ccgtcagcat 129720
   ctggcctctg catacacatg catacaggca cgcacgtaca caaatatgca catgaagaca 129780
   ctgaaggact tagtcggcag ccattcattt ggaacctgct tctgctctgt ttagtactat 129840
   ggaaggcaca atatgggaag gaggaggcac aaatgattgt gagaaatgca ctaggcctag 129900
   ggctggtctc cagccttcat agggaggcta catttaccac caggcatagc tgcaaaatga 129960
   tacagtttgg cttcctatca ctgtggggca ttgcaggctt cgtgcaggag tctgagcatg 130020
   gaagggtggt ctttgtggtc agtgtaattt tgtagcaaga agcaaggaag tcactccagg 130080
   tatggaggac agcatgagaa aggcaggaag gaggaaggat cacagaatgg gatgagctga 130140
   tggctacctc attaggacat agctggatct gagcagaaac aggcacaaag ttggctaaag 130200
   ccaaattatg atgagctagg agcaccagac aaatgaggta tagacagttt gcatggccct 130260
   gctgtgggga atgacataac ctgtgtatgt tttaaagatg tacctctatg agaatctgtt 130320
   gggggacaga tttatgcatt gctctcacat ctgttgggca tcctgttaag atgcagattc 130380
   tagttcagct ggtctgaggt agggccaaag gctcaagccc aaggtgatgc tgataatgtc 130440
   catctggaga agccacccag ttttcagctg agtttagaaa gtaatggcat aaagacatgg 130500
   gtatgtcttg actgtgggaa actgtgtcct ttgcagtggg gagaaaaaaa aaaggtgtaa 130560
   gccaggcatg gtgggctatg ctaccgtacc tataattcta gctgaagcag gaggattgca 130620
   gtgagttctg ggccagcttg agctacacag agggaacttg agaatgtctg tagtgagatt 130680
   atctggaaca ctggatctgg ctagaacaca cctgcctcca tgggcgtttc ttttgcctgg 130740
   ctaaggtggt cagtgatcga gacgtgctat tacaagcctg agaacgtcta cctcagtttc 130800
   tcttctcttc ttggctcctc agcaaacttc tcccagtttg ccttcgagtc tgtggtggcc 130860
   attgccaata gcctgcacaa cagcaaggac ctgaggaagg accagcacgg gaggaactgc 130920
   cttctggcct cctacgtgca ctacgtgttc cggctgccgg aactgcacag ggatacatcc 130980
   aagtcaggta gtgcttcctg gagcaggggt agggatgggg atggagatgg agatggggat 131040
   aggggcaggt atcagcaggg taccacgtca gaccactgcc actggattaa aggctcagtt 131100
   ttggttttca gagtaaaact tgtatacgtg ctgaaattaa aagtttgtag atgctttgcc 131160
   ctctcttcct tcctccctcc ctctttttta tgttgtactt actatgtctg cctagttggt 131220
   aacttctcag tgattacccc actgccaatt tgtgatccac ttgagggtct gctttaaaag 131280
   acggcgaact gtcaactgta agattctaga ataacagcaa ggaaaacaca acctatgcca 131340
   catcagtgac accattgaaa gttcttaaac ttttcatcac ataaaatagt cttcattccc 131400
   ctgaactatg ttaataataa tccattttgg tattcttctt agttatttac gtagaccata 131460
   aaccacaatg cctgggtagc ccggtatata aaaatatcat cagtagactt tttttttatt 131520
   acgtattttc ctcaattaca tttccaatgc tagcccaaaa gtcccccata cccccccgca 131580
   ctcccctacc cacccattcc cactttttgg ccctggcatt cccctgtact ggggcatata 131640
   aagtttgcgt gtccaatggg cctctctttc cagtgatggc cgactaggcc atcttttgat 131700
   acatatgcag ctagagacaa gagctccagg gtactggtta gtccataatg ttgttccacc 131760
   tatagggttg cagatccctt tagctccttg ggtactttct ctagatcctc cattgggggc 131820
   cctgtgatcc atccaatagc tgactgtgag catccacttc tgtgtttgct aggccccggc 131880
   ctagtctcac aagagacagc tatatcaggg tcctttcagc aaatgcttgc tagtatatgc 131940
   aatggtgtca tcatttggag gctaattatg gaatggatcc ctggatatgg cagtctctag 132000
   atggtccatc ctattgtctc agctccaaac tttgtaactc catcagtaga cttttaaaaa 132060
   ttccacatcc aaataaattt ggcacctttt aagtctctgc agtcataatt tacataaatc 132120
   tgtagttcca atccaatttt taaaccagag ttggacttcc tttgaaatat tgcatctgcc 132180
   tgtcctcaaa agatgactct gggtgactca attctgaaca actttcctcc aaaacttaca 132240
   agacttgtgt cattcaaata ttgagcacac aaaaatattt gtgtttctcg ttttccttga 132300
   gaattcagaa actctggcaa ttggtgacag actagtacca agtgtatgcc tccagtcccc 132360
   ccacagccct ccccactacc ctccacctca caccctgccc ctccgagctg cagaatactc 132420
   aaggccccat cttaggttca catttcccca ccattcctcc cttagtcagt ctttcttgag 132480
   ctccgtaact gactctgtcc tgtctgttga catgtctctg gctgctgggg tacaggtggc 132540
   cccaccaccg tagtccccga cccccgatac cacacatatg gacgcacatc tgccgctgca 132600
   gtgagttcaa agctgatgca ggcccgtgtg atgagcagca gcaacccaga cctgactggg 132660
   tcacactgtg cagccgatga ggaagttaag aacatcatgt cttcaaaggt agggatgggg 132720
   gtggggaggg ggcacatgta ctcttgtttt gcttcttagc tgatgtctac tgtatgcact 132780
   tctaggtaca gcttgatatt ttgatatgta agtattttgt atgttgatca ggtcagggta 132840
   cttaggatgt ttagcaccac atacatccat ctttccttgg tggttagaat gctaacatac 132900
   tctttttata gagattttga agtatttgag aaagagtttt gaccattgat gttttggtgt 132960
   agtaccgtat taaaacacgg tggggcactg ttctcatatt tttttcctgc tttcttagca 133020
   cactaaagga actgttggta cctgaatctt tttggcgttg ttttaacttg gggttctttt 133080
   aagacggggt ttctttgtgt aaacctgact ctcctggaac ttgctctgta gaccaggttt 133140
   ggccttcaac tcagagatcc accagcttct gcctcctgtg tgctgggatt acagtcatgt 133200
   gccaccactg cctggttctt ttaggtgtct tgacatagtg tctcactctg taacccagat 133260
   tagcctagaa tttagtaggt aactcagacc aaccttgaac tcacggatat tctcctgact 133320
   cagcctcccg agtgctaagt ttacagttgt gagccaccat gcctagctag gtactcagtt 133380
   gccctccatc gactttgcca aatactttca taaaacactt atttaacttg catgtagaag 133440
   catttctgca tgtatgcatg tatgtacact atgagcatac cgtgtgccag aagataccag 133500
   caaaggtgtc ggttgtgagc cattgtgtga gtgctgagaa ccaaacccgg gtcctctgca 133560
   agaacggcaa gtgcttttga gcatcacttc agctctctac ctctgtcggg tgctttgatg 133620
   gtacttttcc agaacggaac tattagtgtg gggtggttat tttgtgtctt gtctgtttgt 133680
   ttaattgtcc atattcattg tacatgcgat ggttttatag tttcaagtag accgagtacg 133740
   tttaccctca cccctcaatg cccttcactg gtccccttcc cttctgcttt cattttgtat 133800
   gcatttaatg gactcaagtg tccctataca acgtgtgcac cacaaaccag agaacgcatg 133860
   caatatttgt ctacgtctgg cttgcttcac ttaacatgat ggtctcccag tatgcccatt 133920
   tcctagcaaa cagcataacc ttgctctctg tgactgaata acactccgtt gtgtgtataa 133980
   aatatcttct ctacctcttt atttgctgat agacacttaa gatctttcca tagcgtgact 134040
   actgtgaata gtgatgcaat aatatggatg tgcacatgtc tctccagtgt gtgactgata 134100
   gcctttcagg tacctagcca catatggttg gctgggtcag agggtagtgc tatttgcagt 134160
   gcctgagcac ccaccatact gatttctata aggattggac aatttacgtt cccaacaagg 134220
   agtctgtcca ccttcactag cactttatta caactattct cttttccgtc tttattaatt 134280
   atttgagaat tttaggcagg ccctttcatg atattcacac caactcttcc aagatctatc 134340
   tctctgccca caccaaactt catgccctta ctttctggtg gttgttgttg ttgttgttgt 134400
   tttctttaat aacccacaga gtcaaatttg tgcagcctag atagatagat agatagatag 134460
   atagatagat agatagatag ataatattat gtattatata ttacatatat aatacacaca 134520
   ctcatggccc agggtcatcc actggagtgt ggttgaccta cccgaggcca ctcccagaaa 134580
   gaaaactaac tcttgccccc aaagccagca gctgtccata gctcctcata gaggaactca 134640
   tgaaccacac ccttcctata acgatggctc tcctcagtga gggtgagata taatcagtgg 134700
   ctgtttcatt aatgctttta ttttcctctt ttttttttct ggtgggggag gatctgctgt 134760
   ccttactcta tatatgatat tcagttttcc cagccccacc taaagaggtt gtgtttatcc 134820
   agtatgtttc aggctggcct tgaactcctg acctacctgc ctccacttca agagtatcgg 134880
   agttgccttt tcctgctggc taaagatccc acatatgttt cacgtgtttc ttggccaact 134940
   attcttcatc ttttgagaac catctgttct ctttattgcc tgagtagttc cattcttcca 135000
   tatttagtgc ccttagtata ttctaggtat taaccctctg tctgccacag gtttcccgtg 135060
   ggccgcctct cgttgcctcc tttgctgtgc agaagatttt tcgtttcacg caatcccatt 135120
   cgccagttct tggggttctt tgcacagagc atttgctgcc attttgaagt gttttctctg 135180
   tcttacacta accgtcttgg agtttcttac gttgaactct tccgcccgtt tttagttgac 135240
   gctcatacac tgtgagcaat gagagatgaa gatcaatttc cttattcctt actctgtatg 135300
   tgatgcccag gtttcccagg ccgcttactg aagaggctac ttgtatccaa cgtacatttc 135360
   tggcatcttt gtccagcttc acgtggctgt agctttgtga gtttaattct ggctcttgta 135420
   tccattctat tggtaaggct ttctactgag atttttgttg ttgtctgatt tattgagatc 135480
   ttcattccca aacttcagtt tattattttt catcatttct acattgctat tgagttctct 135540
   ttcctatccc ctattgactt ccttctttat acaattgttt agtgctgtct ccttaaatct 135600
   atttagaaat gtacttgtgt tgtccttagt gtcattgatc attcttataa atagtctttt 135660
   taaactcttc tgggatctca tcttatttcc ttccattgga atccgttatt atagaactgg 135720
   tcatttttgg aggagtcatg ttgctttgat ttttcaagtc tcttatgttt ctgcagtggg 135780
   acttgtacat ctgggattac gtcatggttg gatttgttga ttatctattt catttaaaat 135840
   attctaatat ccatcctccc cgctagttgt taaatcaaat cctcacagca catgtttagg 135900
   gcattgagca taacctacag caagggaaaa aaatggtctt tgtgtgactt gtacgaacac 135960
   aagtgcagat tgtctctatg tagctagcta tgtatggaag caaactggct ttgtggtcac 136020
   atacctggaa gctggtgcac agtaccttga tgcagtcgct tgtctgactg caggtgctga 136080
   tcccatccct gtgtagcatc tcactgtccc ttctctggtg tatgggtttt gagccttagc 136140
   accctgtgtt gctgcgtgtt tgtttcctac atctatcttt tcgttgtttg ccacacagcc 136200
   tgtctggttt atgggccatg tagtgagtat tttaactaga ggagtctttc agtcagtggg 136260
   tactggaagg gactgtcacc aagctttctc ctggcactca ccctagtacc tttaagcatt 136320
   tgctactatg ttggaacaaa gagctctaca gtcccgttta ctttagacaa catggtataa 136380
   gatgggtggt tgccatggac caaggtagct agctaatgct gggcatctga gggactgtga 136440
   gtcagcgcaa gatggctagc aagggatgtc cagggaactt agaagtaagt ggtgatgctt 136500
   tttagcagaa tgatactttt atgtcactct gtgtaaatat gaggacttct cttttgactt 136560
   tgctttcacc agcctgccta tatgtgtgtg tctgtccata ggcctgtgtg ggtgtctgtc 136620
   cataggcctg tgtgcctacc ttcatgtggc ccctatgcag gtttgccttt gtgcctgtgc 136680
   ctgtgcctaa gtgtagcccg tgtctgtcac tatgcttggg tgcctgcagc agctgcctac 136740
   ctagcatgga taacatgcat cttatctgtc agtcacttca gccctctggc ctcctaggac 136800
   tctcctcttt gccccacccc acccccacac caagtcttgg tcctcacaca ctgcttgtac 136860
   actcctgggg agctgatgac tctcactcca gactgtggag tttcttccat gtagaactct 136920
   gcctagttct gagtcttcag agtgagctat gcagaggaca ggcctgtaag ggtcccttgc 136980
   ccctgactcc cagcagtctg cttaaaatgg cttgtctctg tgcatgtcag cttccaagaa 137040
   cagagaattt tcctagaacc agtctgctgt acacagcaca gggtttctgt ctggactgca 137100
   catgctcttg gcgctgtgtc ctgtgttgat taagcttttt gaagctcagg tgtccccttc 137160
   ttcttagaac tcaatctctc ctctttgcgg gtacgtattt gtctccccca acccttcata 137220
   aattacctgg ctcttgagag taagagaatt ttcctggctc tctctttatc ttaaccccag 137280
   gttccgactt gctgggttac tctgagagta gagcacttgc cagtcaggag cacttccaga 137340
   atgacctctg aactctcccc tgttctgttt agtgagcggt ctttcttctt tgtcacttaa 137400
   gctagtgttt gtttgtttgt ctgtctgttt gtttgtctgt ctgtctgtct ttgtcagcac 137460
   ccttgtagga tggccgctca acaagcaccc cgctttatcc cctaagggaa tccagtagtc 137520
   tccgcttggg ttttaccatc ctccctgagt tggttcctgt gtttcaacta tacttaactc 137580
   catgttactg cacatcaatc tattaacagc aactatctca ggaacaagta acatcattta 137640
   ctttctttgc ttggttttat atttatatgt ggctgttatt ttatatttat atgtggttcc 137700
   agtacagaaa aggcctaaca tagaatcaaa acgaattctg cggttgtgat gtccaggaat 137760
   tttcctcatc acagtttgta tgtttcagat tgccgatcgc aactgcagcc ggatgtctta 137820
   ctattgctct ggcaatagtg atgcgccagg ttcaactgca gccccaagac cagtcagcaa 137880
   aaaggtatag accggggcgg aataagggta atctcagccc gagaaaggac tcactggtgt 137940
   cttcatgtta caactgaaaa cagacactaa gtgcttgctt tctgtcccct gcccagctaa 138000
   catccacaca tccttaacaa aagcaagtgg caaaattaat attattttaa cactatatga 138060
   agtaatatgc cttcttgact acgtgttttt tggctgagac caagtgcatc cagaaagaac 138120
   ctgagccatt cctcaaggtt gacctgcaac cctcctcaat tctgagttca gaaaacatgc 138180
   aaagtagggg attcctgaaa ccaacaaaac cccctggtca ggacagtgag tgtggtccag 138240
   agagacatag gctatcaaac cagttgtctg tcatgtgtcc ctctctgcta ggcatccaag 138300
   aggttgatgt catgattacc attcaggatc cttaaagtgc agatacacta actgcagtta 138360
   tacatgaagc tgtttgcagt ccccaacagc acggaaagat tttattacag acaaggagag 138420
   catgcgcctc gaatggctgt ctttggcctg gaggcgtgaa ggatagggaa cgggaggtgg 138480
   cactgagctg acgctgtaga ccgtacagta atccaggctc tagatgtcgg caagatcttc 138540
   caggtgttaa ctctgagagg gaaagtgaca ggaacttgtg gttaggccca aaactaactt 138600
   gtcacctacc ctaacattta gcattaggct gtcacagcca ctggtcccaa gttctgcttt 138660
   cctttaaaaa ctgtattata aaaggctgaa catgtgacac aatccttagc ccccatcccc 138720
   cagtactgga gaggcaaaga gatctctgtg agttcaagcc agcctgatct gcttagagag 138780
   ttctagaaaa caagagctac atagtgagtc ctgtctgaaa ataaaataaa actgaagcaa 138840
   tcagcatatt tgcctaaagt ccgacttcct tagtgtacat cctgtttggt tttgcggttg 138900
   gcttgccttg acccggaatg accaggaggg agttggtttt atttgggggg gggggagtta 138960
   gccagtgtgt tgccttcata gcttagcaac cgcttttgct acttgcctgt cttctctaac 139020
   ctctggagca aggtgttcct acactaacac tgtccagtaa gcaatgtgaa ttacattgcg 139080
   aagcagctta tccttgtgta atctcttcct gccctaagac tctggcagac aggctgacct 139140
   atttctagcg gctacctcca cggctggtgg agcgtttcct tcttcccatg ggagggtgga 139200
   aagggcagca tgctgtcatg tgcctggttg gcaaccagtg gggttaacac tcaactccaa 139260
   cccctcccag atggcacagc ctcacttaag actacttcag caaacagatg tgctcccgtg 139320
   cactaagata actaaggttt aaactaccct atagaatggg tgaaaccagc caatgagcag 139380
   aacttaaccg cagaaagtag tcggaacact gtaacatctg ctcagagatt tctccgtttg 139440
   tctccttccg atctagataa gtagccctca aattaaaagt ctataagagc tagtaaatgc 139500
   cattgtgagt accccaatcc tgtggatggt ttaaaagttt gtcatttgca atcattatca 139560
   ccattcagtt tttgctttgg actgactttg aaactaactt ccacacaaag atgtgaaatt 139620
   tgagtgttga aatagttctc actctcctgg ctgcggaaaa accacagaag agtctatctg 139680
   tgtgcgtgtg ttctaggctt tgcacacatg tgtaagatga gtgaacgcca aaagaatcct 139740
   ggccattgct tttgccttta gttggtatgg cactgtcttg atttgcccag caccgagcag 139800
   gtcccccaag acaaagggag ttcgttctat tttgagagca tagaagtatc aagcaaacca 139860
   aaatgagtta ttagttttat ttttgatcct cagcttcttc cctttttccc ctttaaaaaa 139920
   ttttcatgtg tgtgtgtgtg tgttgtaatt cagagtttca ctcataaact catccatgac 139980
   atttccctca tgaagcaata atgggatgat gcaaagactt gtccaacctt gaaggcatct 140040
   gaatatatca tccagacagt ggttctcaga gtgtggcatc ccagtgccag caggagcttg 140100
   acagaatgca gatcctcagc ccctgctcta gaccttctgc ctcagactct aaggatggca 140160
   ccagccatct gagtgttact tgaggatcat agcgaccaac aactcagttt tgtggaagag 140220
   caaactaaga gcagagagag caaacgcatt gtgtggttgg tggggctttg aaagtccttt 140280
   ctctctagca tataccacca gtaccaccca ggagagctgc tccacatatg caactgttat 140340
   tgtaaggctg gggccatggt gcagtgccac ccgctgccct cctatgtgtt atttctgtac 140400
   tgattttgcc acctgtcatg gcctggggta ccttggacac aagcctctca gctgttgagt 140460
   cagaagagaa ggaaagaggg tttagtgggt attaatcaac cttgccgtca cagcctggtt 140520
   cttgttggtt tgttgtttag catttccatg aggagcttgc cctgcagatg gtggtcagca 140580
   ctggagtagt gagagaaacg gtcttcaagt acgcctggtt cttctttgag cttctggtaa 140640
   gattcttgtg tttgtatcta tgctccggct gggcgagcaa agcacgcctg gggaaggcag 140700
   agtttcctgt gtcaagacct aatccgaagc ctagggttgc tccttgaaat gcagctgtct 140760
   ctcatcctcc atccatcttt caccaaactt tatcgactct accttaaaag tcaacccccg 140820
   tctttgtatc tcctcatggt tcccacttga ccaagccacc attgttcctc atgtacttct 140880
   tgaggtatct ttgctctcag tccatcccgc ccaccccagg cagccagtgt catacagact 140940
   gtgtcagcct ccttgtggtc ttcagaatac tagaagtaaa gtgccatgaa aagaaaccat 141000
   agctaaaatg tccttgtata atcctaatgt gtacctgtgt ctcatgccat ggtcccctca 141060
   tttagccaca ttggacccct ttttagttcc taaaaaatac caggcctgga ctttttaacc 141120
   taaagccttt ttaaaagttt cctctcacaa gctacctttt actactcacc ctcaacacac 141180
   tttctgctct gtctcctggc accacctcct acagagccct tctctggcca ccatactcag 141240
   taatggtgtc tcaactttac tagcctcctc ttggagcccc cattcactcc ttacgagcac 141300
   tgtatgcaat ttgccattgt tttctgtctt acctactgga tttctaggat gtgatccgtc 141360
   tttattactc aacgcttgat atcgtacaag cagtaactag caagactaac agtggttggg 141420
   aggaatatgg aatctgtata tctatttacc tacctatgga gtcagtctcc cagcagaaca 141480
   gccaagggta catttattgc tcctccatgt tatcatgaga agagtgccga tacctccttc 141540
   tacttcacta ttgacagaca tggaaagatt ttgtcataaa gaccattaag aacagaggct 141600
   gggaagatga tgactcagtg ggtgagagca cacatgctct gcaagatgcg aacctgagtc 141660
   cgaatcccca gagcccacat acaaaactag gcggggctgc atgtgcatgt aaccccagca 141720
   ttggaggaca gataggtggg tgccagaagc tcagtggcca gccagcctag ccaactctgg 141780
   aaccttctgg ttcagtaaga gtccctaggt gggggcagga atggtggtgt actcctttaa 141840
   tcacagcact ccaggaggca gaggcaggag gatctctgtg agttcaaggc cagcatggtc 141900
   taaaaaaatc aagttctggg acaggcaaca gagaaaccct gtctcaaaaa caaaacaaaa 141960
   acaaataaaa acaaacaaat aaaaacagaa tccctgggta ataaggcaga aagcaataga 142020
   gggatatagc atgtagtttc tagtacccct ctttaattaa gctctgggca gatagcacta 142080
   ccagcccctg ccctggttct cttggatcct ccaatgaaag gcacacacac gcacacacca 142140
   gcttattttt aatatgcttt tagctcaatg gctgggtact tctaagcctc ccttggctag 142200
   catgtccttt tcctcatgcc cagctcaaca tctctaaatc tgccctcagc ttagttgcat 142260
   ttctaatctt gcctgctacc ccaggcccag ttggggaagc agcccgatgg tcactccctc 142320
   caaagcatgg ctaatctctc ttgcatctcc tagcctggct tttgggaata tggaagtcct 142380
   gcctcttcta cccagccatt ggccactagt atcttgattg atcagtcaag aaccaattgg 142440
   ggaacaggac cttcagcatt ctcacccaca cattccccat cgaagcatca gaaccatcct 142500
   cctacagagg aagacatcca aaacccagtc cttcgtgttt actgggttcc ttcctctcac 142560
   actaactgaa gaatatgttt aaggaaaagg agtagaattc ctacctgagc tctgtttcct 142620
   ccattgaaat ccgaggcaga gaaaacagca atgctgaaca gacagaaaga aaagggccaa 142680
   aggaaaggaa gataccacac atctggagga agcaaggcat ggcgggcagc agagttaaga 142740
   atcttgtctt ttaccatgga aacagtttta atccctgaga ggctcagcgt cccaaggcct 142800
   agtctgagga tgaccccagt gcactgactc agatcccaca atcccatggg agcgtttgat 142860
   agaatgaatg agggtcctgt tgggagtgga ggaaactgta cgtatgtacc tgtcttccta 142920
   gctgacttca aggcacacct acagtgtgct agtccccacc acctctattt tgtgagtcct 142980
   aaccatagct acagggctct ggaattttga ccaattcaac accagatctt aatcttcaag 143040
   ccaagctggt taaggcctgt ggctacagct tgctagcctt tggttaatag catcatggta 143100
   tacatgagct ctgtggtctg gcacaaaatg gactggtaac aaaactcatc cctttttttt 143160
   ttcaggagac acagaaagta tttttttttt ttaatgtctc gaaaaacaaa acaagcaaaa 143220
   aacctgtcct gtatgtcctg ttctgcccgc cgtcaagctt cccctcagag aagatccact 143280
   tgggccatga cccatctatc aataattcat aaaataatcc caaattcaag tcaaaattgc 143340
   agacgggggg gggggggaag aactaaattt gccttagtga aaggtgtcct aactattaac 143400
   tcgcacattt acacacactt ccttaaccac tcttagatct taggcaacac cctgcttgtg 143460
   gaaaaagcac cgagatccct agggcaggga ctcaagccca acttcatgag ggatccggta 143520
   gcaactctgc gggctctcag gtccatcctt gaatcctgtg agaacttctc ttctccccat 143580
   aacccgcacc agggctcgag agatacgcca ccttcctcag ctggggacag gtggagcctc 143640
   cttctctaac tctcaagtct aggttctttc tgttccctgt tttgcctctt ggagaagttc 143700
   agagtttaaa tgtctcagtc cactatactg actgacagta ggctaaccca tgccatccag 143760
   ttagaacatt gtgtgactta tttggttccc cgtcatcatg agctcagctt gagtggttcc 143820
   aaagagcatc ttgtagctcc attttgaagg caagtttgta ttctgggaag aaataacatt 143880
   gactctgtct gttgagtaac gtcagtagca gttggcaccc ctgaaaaaag aaaaataaca 143940
   tcctaagtga attgagcatc ttacttctgc atgccagcta ttgtcttaca tatttttaaa 144000
   gcattgtctc gtttaatctt cctacagcct tgactccagg tctttgtgat tctgagtttg 144060
   caggtggggt tagaggaaga atagccacag aagagccccc agatctgtct agggttggtg 144120
   gcattacttc attgtgtatg actacaagga gctaagaatg acagagaagg tgaaaattgc 144180
   ttctaactgg tataattgtt cacttagact tgtttggccc aagagaaggg gacatctaaa 144240
   gttaatatgt ccctcttacc tctaggaccc ccttatgcta cacagatgac atagtttaga 144300
   gaagttccca tggatcaaag cactttgatg gggttggtcc aaatgcaggc ccaccaggac 144360
   agcagataca cagggctcta cagactgtag cctctctgtc agctgaaaag catttatcac 144420
   aaggcctctc cccagggtgt ctgaggtgat gcagaagcca ataaagacta gagagacacc 144480
   cagggtgccg cagctgcctg aatcctttgg ttacctagcc tcgtttgttc tgaagaatca 144540
   cagagaagga gcaggcattc agggcataga gaaatctaga gacagagaag aggatccgct 144600
   ccgcaggggt tggagccata gaaaaacgtt tacactgcca ggactactca gtggcagcag 144660
   gagtgacaaa tggctaaaca gttcaccagg agctacagtc tcctggtcac cagtccagcc 144720
   agaagccaaa aggaaaatag ggaaggggcc attgctacag tatacagaca cctcctgagc 144780
   attcacacac ggagaagggc aagaagccat gtgggggttg ggggctgtgg tgggcataga 144840
   acgaccattg atctcagacc caggcagcac cttagtcctc cgtgcactgt acctgaatgg 144900
   tgctcagaaa gcctgaagag gagaacaact tctccttggc agcagagggg cagtttgaca 144960
   cctccagctg tgctggccac cgggtgctaa tgaacaataa ctttgtcaca ggagccagta 145020
   cacttagtcc tcgggttcgt ttcacacatt ttaatgaatg agccctgtgc agttagaagt 145080
   ctctgattca gaccttggag gcttcgaggt aaataggtga cctagttcac ttcctccgga 145140
   catgccatcc tgcacgaggg ggttctgaaa gttgtccctt ctcagaatgg gccacagagg 145200
   ccaatgtggg agagattctt tttctgtccc caccaaaccc tacatatggg tcaagcccca 145260
   ctgatatttt tgctgggttc ttctgtgagg acccagggac acaagagctg gcaggttgct 145320
   ggcagtctgg acgccggcca tcagtgggga gtactattgt gagaattatt tatttggttg 145380
   tgggatggcc aaactcagca ggcaggagcc gaatgtaaca ataagttcaa caggctggcg 145440
   ctagggtggg tcagccagcc gaggatgggg attggggtca cctgcacaaa gagcccttgt 145500
   cttccctggc cctaggctgg aagtacagtg agagagatac ttttgtctaa caaaaaactg 145560
   caccctgttc ctacccctcg ttcaggtcgt ctccctgtgc gggtgtgcat ctccgggact 145620
   atgactatgt ctgtgtgacc tgggctagaa gggaccttta ggttaggaaa aaccatggtt 145680
   cttacattgg cttttctgtg gttgggtctg ccctgagcta gtcagggcat tatccgaaac 145740
   atgtatcaga aagctgcctc tgtccttcct tcttgcattg ccctctgtgt ttgcaccttg 145800
   tttgtctcgg ccttgacagg tttatgtttg gagaaacaga actctgctgt cagtgaacaa 145860
   agggttccat tttgctcaga gtggaagcca gtttccaaga caccagatgg ggcagatgtg 145920
   ggtgggtcag cttcaaagga aattgctttt ctgttgttgg attgtttact tgcttgtttg 145980
   tgtgtgtgtg tgtgtgtgtg tgtgtgtgtg tgtgtgttac ttgcttgtat tggctgatgt 146040
   ttttgcttgc tggctttgag gcagggtctc actttgtagc cctggctgac ctggaactta 146100
   ctatggagac cagtcagact ttaaactgtt agtaattctc ctgcagccag ccatctcttg 146160
   ggtactggga ttacaggtag gtgccagcag gcccagctga caattgcttt ttgctgccat 146220
   gctctaacta gtctgtacaa attcatgaat atgatttcag gatgtcagca cacagccatg 146280
   aaaaatccca gtgtagtgga cacttgacct atttctctga taaaatagtc tgaggaaaag 146340
   aaggaaggat gggaggggta agggagggaa ggagggagaa aggaaggaag gaagggagga 146400
   agaaggaaga aagaaaggaa gggagggagg gagggaggga gggaggagga aggaaggaag 146460
   gaaggaagga aggaaggaag gaaggaagga aggaaggaag gaaggaagga aggaaggaaa 146520
   aaaggaagga agacggacag gtaggtttat attggctcac agttcactga tgcagtccat 146580
   cagggtaagg gtgtcatggc ggcaggagca tgggacagct ggccacattg catcagcggt 146640
   cagcaaacag acagttgact gatactgttc tcagctgcct tttcccattt tgttcagcct 146700
   ggaaagcagc cacagaacag tgctactcac ggcagacagg acctcctcac tccagttaat 146760
   ctaatcctgc ccagaggttt ggctcctagg tgattttctg atcctgtcaa gttgccagct 146820
   ggcagaaact accaccccaa tcttgccagt caaactatca caaatagctc tcagcaaact 146880
   cacccaactg taagactcca cctctggtga tgtcaaagga tgacgccaga atctcctggc 146940
   ccaggaggaa cccaaaaatt atacatgatc tagattttaa aatagtaggc ataaaactgt 147000
   tattatacta gctaccagtt gatcaataga ttggttaact ataagttagg cttaaaaaac 147060
   tcaaaacata attttcccta gaagatgagc caacatgggc ttcataatga gactcatcta 147120
   aaaacacgta tttatatcct tcagaggcaa cattctatat aatggttaaa tttctagata 147180
   gccaacaaaa tcctgatcat tccatgatat agtttgcagt ttaatttcta gaagcagaga 147240
   ttaactccct gtgaactatg gaattgcttt aggaaaaaaa aaagtttgat ttttttaaac 147300
   tcaatatagg aggggccttt ctttcttgtt ctccttactg tttcagggaa agaaaagaca 147360
   agccaagaca gcattataaa gtagttcctg agcgtcttgt atcatgtact gtaacagcct 147420
   atgtgcttct gaggatccag cctcactctg tctccaggat ggaggctgtc ggggaggggg 147480
   gtgtcttctg acaccaggct aatcagttaa gcttcaggtt ttgccagcag catgaaagga 147540
   gcccctgact tgggctcaag aggaggacac actgcccacc tccagccacc gtggcaacca 147600
   ttccatgcat cagaagcatt ttctagttca gagccatctt ggacaccatc agcaccaatc 147660
   aggaagtcca tcatgaaaga tttccttcaa gccagtcagt ccccttttgt atgttgtaca 147720
   atcgcatcac caacctctgt gctgacattg tcccattcct ccgtaggtga aaagcatggc 147780
   acagtatgtc cataacctgg ataaacggga cagttttcgg aggactcgtt tttctgaccg 147840
   cttcaaagat gacataacta ccattgttaa tgtggtcacc tcggagatag cagccctttt 147900
   agtgaaacct cagaaggtag ctgtgtgatt ctgatttata aattgtttgc cttgactgac 147960
   cgtgttacag aacaaaaata aaaacctcat gggaaaacta gttaaaatat aacaccagtt 148020
   aaaatatgct ggcctattct ccggggtgtg gctggagatt gctgtaaaga ctttgcaatg 148080
   tgattggtca tttaaatgat tttatcagag aaaacactgc tgctctcaag aaattcggtc 148140
   tgcagtttaa tggagtacat agtggatatt tgccacctac ttgaaaatgt tttcaaaaac 148200
   acatttatgt acagaaagaa aaataacaat ggtttgtaaa atgtaaagta ttgatgtgta 148260
   gggtatgatt tttataccaa tctttaactt gaaatcattc cacaattaaa atgtaaaaac 148320
   atatgtaact aagaacgcca tgggccagca gaatggctct gagaatagag tacttgttac 148380
   caggcctgag ggacctaaat tcaatcccca aaacctgtgt ggtcaaagtg gagaaccagc 148440
   ccttgcaatt gtctcctgac ctttgcatat tcgccataac tcatatttat caccgccaca 148500
   caaatatatg taatttagat tgttttattt atttatattt attttattta catattttga 148560
   tgttttattt attttatgta ttttatttat ttatttttga gacagggtct tactatatag 148620
   ctctgactgt cctggaactc actatgtaaa catctcataa ataagagcac tgtgaaataa 148680
   gaagaaaaat aaaacagaaa aatcatacaa aaacaaaaaa ttagcctggc ttcgattttt 148740
   gaaaaaaatt cactctccca agtatgactc taacatcttt cagaatgaga accttgccta 148800
   attggaaaag aaaaaattcc taagccttct tatcctgact ggatttttaa aaataatcct 148860
   tataaattaa gacgctatct cctgggagct ggggggtggc tcagtagtta tgtgcacgtg 148920
   ctgctcttac agagaagacc aggattggtt ctctctctca cacacacaca cacagacaca 148980
   cacacacaga cacacacaca gacatacaca gacacacaga cacacacaca cagacacaca 149040
   gacacacaga cacagacaca cagagacaca cacacagaca cacacacaga cacacacaca 149100
   gacacagaca cacacacaca gacacacaca cagacacaca cacacataca cacagacaca 149160
   cagacataca cacccacaga cacacagaca cacacacaga cacacacaca cagacacaca 149220
   cacagacaga cacacacaga catacacaca gacacacaca cacagacaca cagacataca 149280
   cacacagaca cacagacaca cagacacaca cagacataca cacacagaca cacagacaca 149340
   cacacacata cacacagaca cacacacaga cacacacaca cacagacata cacacacaga 149400
   cacacagaca cccacagaca cacagacaca cccacccaca gacacacaga cacacacacc 149460
   cacagacaca cacccacaga cacacacaca caaagacata cacacataca cagacacacc 149520
   cacagacaca cacacacacc aaaagaaaca tgtgatgtct tatgaatact cactcataca 149580
   cataaaataa ataaacctag aatatgtttt aaatctactt ctttgaagat agtgaaagca 149640
   tttcaagtta gtctgcacac tccaccccaa ccaaaaaaaa aaaaaaaaaa aaaatcttta 149700
   gccagaaact gagtgttggt ggcgcacgtc tttaatccca gcagtagaag gcagagcagg 149760
   cagatctctg agtttaagcc tagccttatc tacagagtga gttctaagac agctaggact 149820
   acacagagaa accctgtctc aaaaaacaaa aacaaaattt gaaaatttaa aaaattatct 149880
   aggtttttat attctcagaa ttcagaaggc tgagaccaga aattctgagg atcagaaatt 149940
   caaagccagc ctgagctaga tagtaaaagg tttgtttttt tttttaagtt aaaaatattt 150000
   taaattattt ctgttaaata aataaaattt taaaacgtaa aaattcacag cccaaaattg 150060
   tatatatgga atggggttga ttacatacct ctaattttgc atgtacagaa caagaccgat 150120
   ggggaaaaaa aaatacatct agaattaaac ttcacccaga aggatggtgg gccaatgtaa 150180
   tagtctcttc ctctcccagt gtagttccag tccagggcta aacagtatgg tgtgtgcctc 150240
   tgctgctctt acaggaaagc gagcaggcag aaaagatcaa catcagcctt gccttcttcc 150300
   tgtatgacct cctgtcaatc atggacagag gcttcgtgtt caacctcatc aagcattact 150360
   gcagccaggt gagcgccccg gagctgcggc tcagcccctt cccacccact gcttatgtga 150420
   cagagtattg ccctcttcag tgtgaccccc cacacatctt ggactttcac catctgtaga 150480
   gtgtgcgggt cctttggcta ccctggatgg cctgaacccc tggagcatgc atcaaagctg 150540
   tcagcaagtc agtctgagga aactgaattt tctcaggagc gtgacttgct ccagttaaca 150600
   gagaaatcac aggactgtgc atttaatctt ggatcagggc atccagagtt tgagacgggg 150660
   acgatggctt catgtgcgag tgtgaggacc tgagtttaga gctgcggcac ccacctaaaa 150720
   gctggtgacg taagcctctc accccggtgt tcctggagac agacattgga agtggagcta 150780
   aggcagatcc caagggcttg ctggaaagcc acagaagaaa gcatttgaca ttctcctgac 150840
   ctgcagttct cacacataca cacacacaca cacacacaca cacacacaca cacacacaca 150900
   gaggaaagga gaaaccaaaa tataaggttt cttttcagtc attagaatta ttaattaaat 150960
   tgtggtttga ggttctaatc ttaaaaatct gaagaactcg gattgaattt gcaggagtat 151020
   aaagcacatg gttttttaat gcggcacaag tgataactac cccagagggt tgttgttgtt 151080
   gctgctgctg ctgctgctgc tgttgttgct gttgttgttt tgagccagag tctcactata 151140
   taactctagc tgccctagaa ctatgtaaac caggcactca gagaccagcc tacccctgtg 151200
   tcccgagtga tgggattaaa tgcatgcacc accttgccca gctttctccc cgaaaatttt 151260
   gaacataatt ctttcttgat tgtctcctat cagaatatcc tggcaataag aacataatat 151320
   tgttactaca cctcttcttc catatttctt ctcttcacca tggccaccat gtctgccact 151380
   attaccacaa ccacaacaac caccaccccc tctcccaggc tgccatcacc gccaccatca 151440
   ctgagactcg gaacagttag taatgggatg gctttaaatg tcatctctgt gtcttcttgg 151500
   tttccttggt tacaaaatgc aagggttctc tgctgggctt tgaggggtgt ggtaccttat 151560
   cgaggagaaa ggtgtgacca gaggagacac aggagcctca ctgggctgcg ccagagaaaa 151620
   aagctctgcc cagtcaacca tccagaggtt tcccgaaagg agctgccatc caggggaacc 151680
   aggagtggcc aaagttcagg cttgctcgtg tgtgtgtgtg tgtgtgtgtg tgtgtgtgtg 151740
   tctgtctgtc tgtctgtctg tctgtctgtc tgtctatgta gtgggtaact ggtgaggttg 151800
   ctctgcaggc aaagttggaa tctgggtcag attactggag gggctgtagg ctctaccttc 151860
   tcagtaggaa acagctatgc attgagggct cctgagctct gcaaagtaga aactaaaggc 151920
   tggaattctg tttgggtttg agctgctctt gacattttgg ttgtgagcct gggctttaac 151980
   aactgagtcg tcatctccac aacccgttga gctgctcttt aaaggggaat acgagaagac 152040
   ccttgaattt gtgaacccat tgctgttgct cagcatcaca tagacaacgt gagatgcagc 152100
   tgcacaggtc tgtctgattc ttgcttggga caactactgc acagaactgc acagtgagct 152160
   cttagcagat actgttggtg cttatgtaat ttaaaacagt taacaacttg ttaattaagt 152220
   agccatacat tctgactaaa gccataaagt ggctaacttt aatcccagca ttctcggaga 152280
   cagaagtatg tggatgtctg tgagttcaag gccagcctgg tctatataga ctttctggcc 152340
   agccaaggct acatagtaca accttttctt taaaaagaca gacgatccct atcaagtgat 152400
   ttcttcttaa ctaaaaattg taagagtgta ttatgtgaaa ctagagagag agcattctag 152460
   caatattatt aaataattta gctacaatgc ttttaagtat taaattatat atcaaacagc 152520
   taatgtgcat ttgaggttat aaggaaggca tattttagag atatattaat aatttcttaa 152580
   tttgcagatt tcttatttgt ggagttaaaa taatttgaat tattctgtat gttttcttgt 152640
   gacttcttcc aatacaaaaa tgcattattt tataattaca aaataaaaaa ggtttaatgc 152700
   tttcaaaaat tagttttaaa catggttcta gctgcatatg tagcagagga tgaccttgtc 152760
   aggcatcaat gggaggagag gtccttggtc ctatgaaggc tcgatagata ccccagtgta 152820
   ggggaatcaa gggcagggag gcaggagtga atgggtgggt ggaagagcac cctcatagaa 152880
   gcagggggag ggggagtgga atagggggtt tctgggaggg gggacactgg gaaagggata 152940
   acattttaaa tgtaaataaa taaaatatcc aattaaaaaa atggggtggg gtagacctac 153000
   ttccacaaga attatgcaat gaattagcct atattcaaat aaggcttaat acccacagta 153060
   ataaattcac atatagaagt atctgttcta tcaaaagctt catattttaa acttaatatt 153120
   gttaatagct aataaaatag atctgagacc atttttagct ggaaaaatag tttttaaata 153180
   gatagatcta ttattaatat ttatattaaa ctatttgacc cacaatactt tgtctgtatt 153240
   cacttagtgt attttttttc aaatatctga agttaacatt tctgtcctag tttaacaaaa 153300
   ttatctgttt agaaagtttc ttagagagtc tattcataaa cttccatgcc ctaaaaatgt 153360
   gaagattaat ccaaactgac aaattttggt atagtcaaga aaagataact gattttaata 153420
   caagcccttt aaattctatg gagattttgg taagaaatat tagtatatat cataaacatt 153480
   gatgcattga aaattctttc aagaaaatat gaaatactcg ctgtctaaat ccactcaggg 153540
   cttgggagat cagaattaac attaaaatgt gttgtgtttc ataggctacc atgttcaaaa 153600
   cacaaatata gcacatatct gtcaacacac tttcataaaa gggaatccta aatgaccaag 153660
   cttggtatta ctttattctg tttctcatac gctctcaaat catcagaggg aattaattct 153720
   tttgtattgg aaattgcctt aagtgctcaa aaatctttta gggggctgat tttcaaataa 153780
   tcgaatgcat agaatttcag ttttggtgta tgctggtgtg gtagacgtgt cagtagtaag 153840
   gaaccacagg agttagagcc aacacagaga gaggtggtcc ctggcttgtt tttataacac 153900
   ccgtcatctc caagccaaat cgacccctaa gtttcccaaa agatgtcctg gtaagaagag 153960
   actcgtaggg aggcggtccc atcctccatg tccacctgtc tgccctgtgg cagatcccat 154020
   cagagtgaac aacagcaagg acttcagaac tgctgggctc cagagcactg tgtgcctatc 154080
   acgtacaagc ccatctgttt tcaaagcttc agaatggacg aggggaaagg tgatctcaga 154140
   gcagcagggc atgggtgact gtgcggggtg gggccacttg ccttccaaaa caaaagtaag 154200
   gtcacctaca gcacacagcc tcctgccatg cacaatgctg tcttccacgg tcagtttgtc 154260
   acctcttggt ggcagtttgt agtttcgcct cttagacaca gttgaatttg caggaaatgt 154320
   gttccgttgc gctatgccga agctggtatc tcaagcttat tttctgcata ggttggctgt 154380
   agcgttgatt agccaccata aggttggagc aagagttccc atttggggcc cgctgttaag 154440
   aatagtagta aataaaagtc ggctggagct cccggaatgt catttgttgc tctctcaggt 154500
   taacctttcc tccaatggat gtctgtctgt gcagaggaag agtgtgtcag tgtctgttga 154560
   gctccgctcc cgttatccta cactaaacat tctttcctct gcctctttcc ctccagctgt 154620
   cagccaagct gaatatcctt ccaacgctca tctccatgcg gctggaattc ctgaggatcc 154680
   tctgcagcca tgagcactac ctcaacttga acctcctctt catgaatacc gacaccgcac 154740
   cagcatctcc ctgcccctcc atatcctccc aggtagtctg ctaactacag gaaaggggcg 154800
   agctgctttg ttaattagct tagttcaatg gggcatctcc tatcttacta attagaggaa 154860
   aatcacacta ttcaaaccag atcagttgat ccagatatgg gctctacggt tccctgaagc 154920
   tgcagcatct aaattgacca ttttcaaatc aagtaatttg gcacaagggt ccctaaggag 154980
   gttaactatg taggaagaaa tctgtaagcc taggaaaatg aagaaacaca ggttaagtct 155040
   caagtcctcc actgtcgaca taaaccaatt attgtacaat atagtgtgca aatacaggtt 155100
   tagtatattt gcaaatacag gtttgcaatg tctagcaaga ttcagaagca ctgtgttagc 155160
   ctagtctctt tgtccgacag cttacaaagg aagaactgca gagggggagg ggcacgagat 155220
   gaaatgattc ccaataggat ttgactgtgg caaatgtcct tactgcgtcg gcaccagagg 155280
   tttctcaagg agttagtgtc ctctagaaaa cccttaaaga aatgttattt tatagcccta 155340
   atccagatgt gggaaacaag gcagatgttg acagcagtag gcaagatagc tcaggatggt 155400
   attagattct attctgctgc atatccttgg tactagtaca gagggaaagg cttgacagtg 155460
   ataagcgcct taagttccag gactaagagg acgggggtgg ggatcacatg ggcccaggag 155520
   ttagttccag atcagcctga gcattatagt gaagcctcat ttaaaaataa taataataat 155580
   aataagtcat agtagtagct atttgtgcct gacaatatac ctgaagttag ctccatgacc 155640
   attataatgt gatctgggac acacgtgact tacaaggacc aattccaaat ggacttatta 155700
   ttgtctgctg tcgtctgttc tgtgggctga atcctataac ttccccatgc tgaccttgcc 155760
   ctgggtcctc tccccatgct cctcttctgg ctgccctaaa gagttgacag acccagggcc 155820
   cctactccac agagctccag gacttgcagg gacacttact tctgtccccg gtgaaggatg 155880
   tcacgctccc ggagtaagaa cacgggacag gaatggccta gggctgccaa ctctggcatt 155940
   gtcgtagcag acaccacttc tggttccagg ccagctaaca tccagacact gtgctcagat 156000
   gaccttcaga aaggattctg gggagacaga ccacagcgag ggactgggta cagtccttat 156060
   ttctctgtcc acgtgtaccc tgggctgttc atctgcaact tttaatacca gacaggcaag 156120
   ggagggatta gctagttttc tttgttctgt ttttcccatt ttgctgttct ggggacctgg 156180
   tcatatcagg cacatttccc accactaagc ttgaccccta gcctagtttt catagtcatg 156240
   ttcatgttgg ccagtcagtg catgcgccgt ccggccagag ctccgtaaat gcaagccagc 156300
   actgttttaa ttccagctct tcatactcat aaagctgccg ttggtatttc tgtagaactc 156360
   gagttcctgc tccagtttcc aggaccaaaa gattgccagc atgttcgatc tgaccccgga 156420
   gtaccggcag cagcacttcc ttacagggct gctcttcacg gagctggctg ttgccctgga 156480
   tgctgagggg gatgggtgag tatctgacgc ctaaaatgga acctgaaggg aaagatgtta 156540
   acagcattcc cagttcaact tcttatgtgt acagcaagac ctcagaactg taccacttac 156600
   cagttccaag aagaagcact cctgtcttaa gaaagtggta ctggtggagt atagagaggc 156660
   aaggtgatta gaagatccat gaaatgggct tctttttaca gcactggcag ttgaactgag 156720
   agcctctcac actgtaggcc agtactgtat caatgagcca cattcccagc cccaaatgtc 156780
   tattgtttgt ttgtttttta ttttcgtgac agtttctctg tgtagtcctg gctatcctag 156840
   aactcactct gtagaccagg cttgcctcaa actcagaaat ctgcctgcct ctgccttcca 156900
   agtgctagaa ttaatggtgt gcaccaccac catacctgcc tcagaaagag gttttgtttt 156960
   gttttgtttt gttttgtttt gttttgtttt gttttgtttt gttttgtttt tcgagacagg 157020
   gtttctctgt atagccctgg ctgtcctgga actcactttg tagaccaggc tggcctcgaa 157080
   cttagaaatc tgcctgcctc tgcctcccga gtgctgggat taaaggcgtg tgccaccacg 157140
   cccggctcag aaagactttt taaagtccct tgtttgagca gaatttgcag agaatgcttt 157200
   gctgagtgaa ttccctaagg gcaaacccat tttgcagggg aggctgctga ggtggaaccc 157260
   agggctttgc acacactaag caggcgctcc tccactaagc tatcccgtca gccctaacaa 157320
   ggtcacctct gacaaagtga gcacagagac gaaataaata ccagcatgcc actgtccgag 157380
   gctggcaaag gaagtgacgg tgaagaagcc agctgtcttg ggtctagatg acgcttctta 157440
   tgggcaagcc ttctcaggca aaggcagatg ccataggcat gggtctcgtg aatcttctca 157500
   ctgtgtccat ccctgagcac ctgagtgtat ggcagtgccc ggacttactt gttggctctt 157560
   ggcttgtatt ctgtcatctt ttctctgctc taacgacatt ccagtcttct cagaattttc 157620
   tgtcctataa ctaacttatt ttctcagcat caaaaagtgc cctaggatat atgctgataa 157680
   ggtcctgaaa gaagaaaatt tgccttgcaa tataaacaac cccccatttt cactttctta 157740
   attgttttta ataaatagca tggctttaag taatttctga aactatcttt tatatacaca 157800
   gtagcagtag tttttaattt tctatttttg tcccagttgg agacatccct gccgtctggt 157860
   tttgattctt ctaaaatcat tgctgggata ttgctatata gctttagctg ttccgggctt 157920
   cactatgcaa actagactgg cctcaaactt acagagatct gcctgcctct gcctcccccc 157980
   accaccaccc ccagtactgg agttagaggc atgtgccacc atgctgagct cctaatattt 158040
   ttgaagagca aaaggttgaa atagacattt tccaaagaat gcgtggtcaa aagcacacaa 158100
   aaaaggtgtc cagaatctct aacagtcagg cagtgcttca ttaaaacaat gattatatac 158160
   cacattctgc ctactagaat agtttgatca acaagacaga cagccaggga tgtggctcag 158220
   tggtagatct ctagcatgtg ctaaggtctg ggttccatcc ccagcacttt aaaaaaaaaa 158280
   aaaaagaaag aaaagaaaag aaaagaaaga aagaaaaaag atggttgcaa aaaagaaaaa 158340
   aagaaagaaa gaaagaaaag acatatatat agcacccaga caccgctgga aggggggcaa 158400
   cattgttctt tagaggtaaa actatagagt tcccattcca gtagtttgac tgagatatat 158460
   gcccaagaga taaatactta caccacacaa aaaccagaag cattcgtcat tggtgatagt 158520
   cctaaagtag aaacaaccca aaggttcatc aattgatgaa tggataagtg aaattatttt 158580
   ctgtgcacaa tggaacatta ctcagtgtcc aaaaggaatg aaggatttac gcaatatgat 158640
   gaatctgaaa acagcaagct aaagagtaga ccaggatggg aacaccggat gtccgtcttc 158700
   tactctgttt tgctttcttt ttcacatggt cactgaattg tccaggatac tttgggcact 158760
   ctggtttaag aagacagggt ctccctcccc atagcctggt ttctctctat tattctgtcc 158820
   acagaattag cagagtacag agaaaagccg tgagtgccat ccacagcctt ctgtgttctc 158880
   acgacctgga tccacggtgt cgcaaaccgg aagtgaaagt caaaatcgcc gccctttacc 158940
   tgccgttggt cggcatcatt ctggacgctc tgccacagct ctatgacttt acaggtaagg 159000
   atctgtctcc tatcttagaa cagcgaggcc cctgccagac gcctctcaca caggaagcaa 159060
   gcaagggctc tcaccagctg gtcagtgcct cacagcagtg gtcactaaac cttttttttt 159120
   ttaaccagac aggtttttaa agatacacaa ttatacacac acacacactc acacacacac 159180
   acacacacac acacacacat atatcagaaa ctatttgttc ttcattcatt gatcggctag 159240
   tttatgattg tcatccactg caccgaaagg atgaactata acagagctaa ttactctcct 159300
   ttgggatcgc tgttctgttg ccttcaaata aacctgttca ttctctttca tgaggcttat 159360
   gagggagtag ttaaaatcaa acaacttttg ctcttatacc aaccaactga cacacaatcc 159420
   aaggagtctt tcagtattag gaaaattacg cagcagcaga aaacgctttg cttttggcaa 159480
   taagaggtca attgtacaat tagaagaaaa aaaacacaaa tttaaaaaaa aaaagtcccc 159540
   acgtcttgga tttatgttcc tattatagtg tggaccagag agtataggtg ggagtccagc 159600
   atcctggtca cttccaagca gtccttgctc ggtgagattg tagaggttac catgttggag 159660
   gttgctgatg aatgagctag agtctggaga gtgcctgcct gagtctagcc atccttacaa 159720
   ggcggtcacc agtgagtctc agagaaccat ctgaaccatg aacctgtgag tcttagagaa 159780
   ccatgccact tcctcctagt acagtggcat gcttgactaa acaatttgta aaatcatgac 159840
   aatagcaact atttagctcg cataataatg agactagagt tcaggagata tcttagtaaa 159900
   gagctggcca gccataggaa tctgcattcg attctcagag ctcacattaa aaacctccga 159960
   cttggtgcta tgcacttgtg attccagcac tgggaaggtg agacaggaag atccctggac 160020
   acaatccaat cagccagcca gaaaaaacaa aggtggcgat gcccgaggaa caaaacccaa 160080
   ggtcctctcc ttccctccga atgcatacac atagtctcac atccttcata tacaaacaga 160140
   tacacgaaag agaccaaccg cctagaacgt ctaattcatg cacatcgtag agtgagaagc 160200
   aagcactcct atggacgtag ttatttctct tctttattaa aaatgacaat gacaaaaacc 160260
   cctcctgtaa atcagcagga gaaagctcag cagttaagag cgcttgcttc tcttgcagag 160320
   gacccagact ctgttcccgg cacccacaag gtggcctaca gctgtctgta actccagttc 160380
   cacaagatct ggcaccctct catgtcctct acagtgaaca tgcacacatg cagggcaaac 160440
   gctcatacac acggagtaag aataaacatc taaaaaagac agctcagggt ctggtcagat 160500
   ggcgtaactt aagcgtactt aatgctctcc cagaggatct gggtttggtt ccccgcacct 160560
   cacacctgcc tgtaactcca gttccagaag atctgatgcc ctctcctgac ctcaccaaat 160620
   gcacaaggta cacacacata catacatgca gatatatgca ggcagtaaca cctacacata 160680
   aaatttaaaa aaacttattt ttctttttaa agatacatct catggggctg gagagattgg 160740
   agagatggtt ccatgttgaa gagaactggc tgctcttcca gaggacccag gatcccatgc 160800
   acccatgtag cagctcatag ctgcctgtaa ctctagttcc atgggatcca gtgccctatt 160860
   ctagcctctt agggcatcaa gaatgaaagt ggtatacaga catacatgtt gcaaaacatg 160920
   gaaataaaaa taaaaaaaat taaaaagata aataaaaaag ataaaaataa aaaaataaaa 160980
   aaggtgttaa agatatatct aaaattcaaa cttcagctcc atagattacc ataagtgacc 161040
   tttgacaagt ttggcttttc agtaaaagta gatatgcata tggtgataat aaattagcat 161100
   ttgcttagac ttttgaaatt tagtaattta gtacaggatt tactatagtg acatattatt 161160
   tctacagggt acttattctg tagttatact tcattgaatc attatttaaa acagagtatc 161220
   ataacctagc aaaagtatta caaaacttta aaaactttta ttttttttac catctgaaaa 161280
   attagctcat agcctaagcg tatgctcaac attttgtttt ttaagcctgt ctgctttttc 161340
   tatatatgaa taattcatat agatatatat ctggttttga attacgtgct gacttcagtg 161400
   agtaagatat gtctttcgct tgtcggggtg aagacgggaa aatgtggtct ttggcttgac 161460
   atcacacatg ggaatgttta atggcagatg ctcgcagtgg aaggagtcgt gccagtggct 161520
   cgtatgaaga acaagatgtg gccaacggaa tcaaccagaa tgtggccctg gccatagcgg 161580
   ggaatcactt taatttgaag accagtggag caatgctgtc ttccttggta tgtgtgtact 161640
   gtacatttct tgagtgtatt tcttgttgat gtcacctcct cctaaacttc tgatagaacc 161700
   cagcctagaa cagacatgct ttctgcctca atactgcaat acaggaatcg gtgaagctct 161760
   ccgatcagag agagctatgg gaacagcaag agatgcacgg ggcagggtta gccagtccca 161820
   gtgtgaagct gaatctgggg agtctgaaat cattgcagaa aagatcaaac caaggtgtcc 161880
   atccttgcat tggacgtaga tggggcttcc tccatgtgtt tattagtatt actgttcccc 161940
   tccgataaat gactaaggct ccaaaagcct ccagaacaaa aggcagcctt cctaggtccc 162000
   cagggagtct aaggagattt gtcccccaac agaggggatc tttgtggaat gtacatacct 162060
   ctgctgctgc tgaggtggga atcgcctgtt tgccaatgca ctttgtcaga gcagcagttc 162120
   agcttctagc tcagtgttgt tattgttttg ttcttaattg gcttgacggg ctctgtttca 162180
   tgatcctaga gatgagccta aagtgacccg gggtttgcaa ttgccccgca gtcaattttg 162240
   ttttgcattt caacgtaatg tctgaatctg cagcaaactc gcttggtttt cttctgagaa 162300
   ggcagggtct tgacacaatt tctttactag ctgacttttt atgtcttcct tctagcactt 162360
   agccatctct gcccctttca cccttgccca ggagggttgg ttggagagca gggaaacagg 162420
   acacctgaga ctcggcaaca ctaatcttac tcactaagca gtgccttaga tcacattcta 162480
   cctctgaatc atccacctta ccgaagttac ctgctcaggg cagtgtgtgt cttttatatt 162540
   aattgcctgt aaggagtaga gaaggtgaag ttcacctatt aaaacgtgtc cctgtgaagt 162600
   tctagcggaa gactgtccct ttttagggtc atacttacaa tagcttaaat gaaagcaagt 162660
   ttgccctctg tcacttccat ccaagactaa gattttcttc acacagttca caaattcctc 162720
   actgcaatgt tactgccaca ggccaccgga tattaggtgc accacatgaa gcaaggggtc 162780
   cgctgcacat tgttatctgt cttggtgtta tgagttttag caagtgtgct tagtagcact 162840
   ctgtagtgaa cattcaccaa ggctattaca gcacatggcc tgaggaccca cccaccctca 162900
   agtgaatctt agtgtaggtc ccattgcaga tggctttgcc aagacttgac tcatgtgcta 162960
   accagttcat gatcttgatg tgaaactgaa ctatgaactg ttggattgtt ttatggtatc 163020
   tggtcaggct agtttctctt ccggtcctcc actggccatc ttgaagaaga agtccttgga 163080
   gatcttagca gcaagaaagc ccctttgcat cactggtatt gaagagcaac atcaagatcc 163140
   agagcagggc accagactgt gctcagttca gcggtccata gcttaatcta aactctgccc 163200
   cagcgtgcgt ctcctgagca gccctgcacc atgcacacat tctctaacct cgggaagaca 163260
   gggctactgg aagcagatac ccagccatgt gctggaagta ggcacagtgt tctgaggggt 163320
   tctcatctct tacactttta agttttcctg tggcctgact tcttccctcc cttgccctcc 163380
   cccctccggc cccccatttc gcccccttcc agccctataa gcagtacaac atgctgaatg 163440
   cagacaccac ccgccacctc atgatttgct tcctgtggat catgaaaaat gctgatcaga 163500
   gcctcatcag gaagtggatc gctgacctgc cttccatgca gctcaatagg attctggacc 163560
   tgctgttcat ctgtgtctcc tgctttgaat acaaggcaag tggcaggggt gggggagtgt 163620
   cttagctgtt ccaatcctgt cttcttactc tcttcccttt gggcaacagc agcatcgtct 163680
   gtccttccta cactgtatta caacccacag cctgtttgca tctgcagatt gcaaacagga 163740
   aagaatatct gcctattgca gtgggcctgt tcctgccaat aaagctttat acacaggagc 163800
   ccccagaaaa gtggaatcta tgtctaaact ctccaccatg accctgttct cttttgacaa 163860
   atctctccct ctgcagagat cccactaaac caaatgtgtt tctacatctg ttcttccagg 163920
   gaaagcagag ttctgacaaa gtcagtaacc aggtcctgca gaagtcaaga gacgtcaagg 163980
   ccaagctgga agaggccctg ctccgtgggg aaggagcccg tggggagatg atgcgccgtc 164040
   gcattccagg tgtgcggtag tcctcccatc tgctacacag ctcttcttct tggacgtcac 164100
   tccccgagac agcaagttct gggaccctgt gcttgtcttg tagaatgttt gttccccact 164160
   tctgagggaa agagtcaccc tgaaagtatc cccaaatgcc atgttttaat taagatttaa 164220
   ccgcaaagaa aggccacatc taagaaagtg gttcatgcgc tgtcttccct gtttcaagtc 164280
   cctctagggg acgtgatcaa caaactggag ggctctgcag tccacagtca tatccctgag 164340
   tcattcaaga tgctactgca tagagcagtc tccccaaaaa gcgtgacggt gtaccattgt 164400
   cctagggttc ccatgaccca aacacctgca ctcatcactt taatatgata aatatgatta 164460
   ctacccccag tgaaacagtg acaggagggc atggactctg gagggctttc acctgacacc 164520
   ctgacctacc ttctagcaag tcagggaggc tcacaggaac ccctgctcag gagaactgtg 164580
   gaaggcagac catcttccag agctgcctcc agttcaccca gggcaggtca ccacaggcgt 164640
   agacacggcg gctctgtctc ctcagagcag tacagactga tgaacagggc gtctgtgctg 164700
   atgcttctca gctaagctga gcactgaact tttggttatc ttagagggtt tcatgctaat 164760
   caaattcctg tcgtgtgttt cttaactccc agggactgac cggtttccag gcataaatga 164820
   aaatctgaga tggaggaaag agcagacaca gtggcggcag gctaatgaga agctggacaa 164880
   gtaagttgcc ctgccacagg ctctgcaccg tgttcactaa cagtcttagc tcttgttccc 164940
   aggctacttg tgttactggg gcaactgcat ccttccttct gagggtagta agaaaaggaa 165000
   acatactgtc tgcttaagta cagctccagc tgcccggaat gcccagagac ataagaagct 165060
   gtgacttcag ctcttaacaa tgtatccaga gtttaaagac acctggtttc cacagcccca 165120
   gttgatgaaa gcaattggtg aggagcagag actagaaaaa ggaggacctg gtctcagcct 165180
   cccaactccg catctgtatt tccataatga aaatggatgc aaagatggat attcagagat 165240
   agtgataata gctttacatt ttattgttca aaaaaaatca aaatatattt gattttggaa 165300
   tatatttgat ttgatttgga agtacataac aaagtaccag gttgttacag tgttagggga 165360
   acattaagtg atcaggacca gtgcttggtt ttagagtact cttcaatctc taattacata 165420
   cagtagctgc atttgtaaaa ggccacaaag aaatttacca agacattaat aatgatcttc 165480
   gggttttaaa aatgtaagtg attgatattt tctttttcat agttcttggg tttcctacat 165540
   tggatttgca tttacctatt ataataaaaa atgcaagaaa aatgaaaact aatcttgctt 165600
   agattataac atataccgtg tgtgtgtgtg tgtgtgtgtg tgtgtgtgtg tgtgtgttat 165660
   cttaatttgc tttcagttgc tctaataaaa acaccatgat caaaagcagc ttgaggagga 165720
   cagggcttct tttatcctat gacttaaaat ccatcacaga aggaagtcag agcaggaacc 165780
   tggaggcaga aactaaagca gagggcatga agaaacactg cttatcggtt tgctcttcat 165840
   gacttgctag cctttttcct tatataactc aggactgctt gcccaggggt agcagcccta 165900
   acagcagccc taacatctac cgtcaatcaa gaacatgcct ccatagactt gcctgtaggc 165960
   aagatgatag aggcagtccc tcaattttga gattccctcc tcccagatat gtctaggttt 166020
   gtgtcaagtt gacagaaacc agccagcaga tgtgtatagg tatatggtat atcaaagggg 166080
   gaaaggaggg ggaaagggag ggagggaggg agggagggag ggaggaaaga gagagagaga 166140
   aagagtacca tatataacaa tttagacata tgagcactaa agtgaaatgt gtggggatag 166200
   ataatgtctg tcttctttgc ctctaagcca taggtgccct tatgaatccc agattcactg 166260
   agcaaaacac tgaatggttc aagaacccac atacacaaca aaacatagat agtacagctt 166320
   cactgtgtct gaagtgctca ccatgaggag gggtgagaag atagcagtgt tgtagtaaat 166380
   aaatgttaat gtcttcacca ttaccgtagg ataacacatc tttgttaacc tgtaggactc 166440
   ttagggaggg gctcaaagtt tctctaaaat atcacctgtt ttcctagaaa ccgcttaata 166500
   attattttga aaagcatgcc aaataatccg gtagcccata gcttcttaac aatgaaggat 166560
   gctccttgtg ccagtcggtg tatctgatac caatgacacg gtgatggctt tgccactaag 166620
   caagaatgtg ctcctccgtg gcttggaaag gagacgtcca tagctggcca gtccagagat 166680
   acatggagaa tcacattggt cacttgggtg tcacacggga gccccagttg ttagccttat 166740
   taataatgcc cctttgctta gaagactggc tcgcactgat cttcctagct ggtgtttaag 166800
   aactttgact ctgtcagtac atttttgcac ccggtttcag ggaggaaata tggatttgaa 166860
   gtgacacagc taggcacttg gccctgcttt ctctttttct ctctccttgt ccgattgctc 166920
   tcattcggga ggggagatag ctggtgaaga caagggtgta acaccaaagc tgtgtgcagg 166980
   aggcccagca ctgcttagat gctaaccagg tcatctgctg cacaagatgg agacagtgtg 167040
   gcaaggtgtg tttgactggg ctctgtgaat tgccctggga agccgttccc atttgctgac 167100
   atcagcagtc ttcgctggaa ccgtgcaggt cctgccagtt ctcagcagct ccggctcggg 167160
   tgggctgctc attatcagag tggctgatga ccccctcagt cctgcagccg ggtggcaatt 167220
   aaaaccgtgc aagttagagc tctttggaaa tgttataatt ttgagatgac agcttctgtc 167280
   atttcctctt atgtttattt taattttaat tagtttgact cctgtttctt ttagagcaat 167340
   agtttttagc ctggactaca tattcaaatc tcccgtggga acttccaaag tctgtgtctg 167400
   tgttgctcta cctcctaccc ccacccaccc agggcctctc tatgtagaca aggctgcgta 167460
   cgaactcaca aggtccacct gtctctgcct cctgagtcct gagattgaag gtgtacccac 167520
   cacacccagc gtcaaagtgc ccaaaggtac cctctgccag tttgttcaga acttctaggt 167580
   tagtcgaact ttaaggtgat tcaactgtgc aggcaagtct gaaccagact ggtagacctc 167640
   ccaggagtga tgacaatatt gcatgacaat atgaattact taatgatact gagctatata 167700
   attaaagtga ctaaggtggt aagttctatg ttacatgcat tttatcacaa ttccaaaggc 167760
   aggttaggac tatctgtact gtcgattact ggagctatta accacataca gttctgaagc 167820
   ctggagcctg gagtgtggct gtttcgtatt gagatataga ataaataaat aaataaataa 167880
   ataaataaat aaataaataa aatgtgtgtg caagattgca catttcagca ttgatttttt 167940
   atatttgtta cttattaaca gtatttttag tgtattgggc taaataaaat atggtaaaaa 168000
   gttaatactg gctgggagta ctggcccatg cccttcattc tggtacccaa gaggcagagg 168060
   taggtagatc tctgtgagtt tgagactagc ctggtctaca tggtgagcct ggacttccaa 168120
   gacagtaaaa cctttctaag aaaaagaaaa gggaaaataa tgcccttgat ttcattttat 168180
   cttttgtgtg tgtgtgtgtg tgtttgtgtg tgtgtgtgtg tgtgtgtgtg tgtgtgtgca 168240
   tgcagctatc ataatagata aaacaatgca catggcctct agtacatttc tactgtacag 168300
   tgttggtcaa agtgcggttg gttggttatt ttttttggtt agggaattgc tgttgttgtt 168360
   ttgagacagg attccttagg tagtcttggc tggcttcaga actcactatg taaaccaggc 168420
   tggcctcaaa ctcatagaga tccacctgcc catgcctgtc atataccacc atgtctggca 168480
   tctggccctc ctgctataaa tttaaacata ccagtaaatt aattttaaaa acttgtatat 168540
   tttctcatat agagaaatga ggggaagggt tgtcaaacaa ttaggactga agaatctatc 168600
   ctgaatgtga aatcagttaa aaataccttt ctgagcaacc agtgttgtat atacaactgt 168660
   tcctggataa ggaaaaggaa aagagctatg tggataatcc tggtctaaga catttgaact 168720
   gatagcacaa taccccagca tctgtggtaa catcttggcc actgaaacat attgggaaag 168780
   tcaggttttt cttatccttt aaaactaaat ttggactgta aaaataagtt atagtaatac 168840
   cctttcatct acaggcaatg cactccagat acccaaagct gtgaatggtt ccaatgcctt 168900
   gtctacattt ctatgaaatg tgtcaatcaa tctggtaact aagacagcaa ctcagtaaat 168960
   atcatgtaga tggcattaac agcatggata catacactgg acaaagtgat aatccatcac 169020
   ctgacaggag ccaacgcagt gtagggacca cgccttgctt attctggaag acgctcacta 169080
   actatgggta actgaaccga tgaaggcaaa gctgtgggag aggagagccc ggtctccctt 169140
   tctagcttca cttgttttat gagggtggat gggctagtac acagagtgca ccagctcttt 169200
   aaaaattaaa tctcttagta acttctgttt cctgccgggt agcatccagt ccagtaacct 169260
   ccagcagaat gtttggtgcc ccaccccatg ggatgtgaac tttgcttccc actggagaag 169320
   gaagaaggca ttctcgctct tagtgaagcc accttccctc cttctcagga cgactttgag 169380
   tagtaaagag ctgattctcc cattccacag ttctagctct gtttgccagc gttcaccgag 169440
   atcaagtcgc atcctgtctc tctaccctgc tgcggctctc ccccgcatcc cacaaccacc 169500
   cattcagatc cagagagtgc tcacgcgacg ccccaaggct ttaagcagct atctgatggg 169560
   ctcacctaat tgttagtagt aaccaataat taacttctcg acccattttg tcatttttat 169620
   aattgccatc attattaata accctggggc tatggatggc ctggggactt cactagacca 169680
   ccttcccaac cagacacgaa tctcaggaaa caccttcagc cctgagattg gctatgttga 169740
   ggtggccact ttgtgtggat ttgatggttg acggttgtaa agaatccttc aggacccggt 169800
   gcatgggttg tcatctctgg tacctggccg agccacacaa tgctttaatg ggtgatgtgc 169860
   tttttgtttc ttagaacaaa ggcagagtta gatcaagaag ccttgatcag tggcaacctg 169920
   gctacagaag ctaatttgat catcctggat atgcaggaga acatcatcca ggtgaggatg 169980
   actcactccc catcgggccc gctcgggggt gccattcttg gaaatagaaa gggggttcgt 170040
   catccaaaat tgaaaaagac acaggcgtga ttctagaatg tataaaacta aagtgggaac 170100
   gtttggaatt tgcagcagtt gcctcccttg cagattccag aggtgccagg catcctctgg 170160
   cttttctgac tgctactccc ttggaaagag ttagatgttt gaggagctga ccctcaaagt 170220
   tactctggcc aaggacctgc tacattgata aaggaggcag agccttttag ccaagcacct 170280
   cgagaacatg ggtagctaga ttgctcctgg taggtcggtt ccagctggat aatacaccgg 170340
   cgaggctagg agttgctgat accatggggg aaattggggt cccctcttgg ggtttttagt 170400
   ctccttaata aaagcattta agggtggact caaggagaaa ctgaagggca attttattag 170460
   catcttaaag acaaatcccc aaagctgaca agctgtaagt cacagcggct gcttggagga 170520
   gaagacgaag gaaagccatt ccgtttaagc tggcatggag gttggacaca taacagataa 170580
   gtagtccaca tggcagggaa gggggcccag aacactggca aaccccagag tattggggaa 170640
   agtatgatga gaaaagagag aacgataaaa aggagaagtt agacttttct gaatagtttt 170700
   taaaagcaaa cagatgtaca gtttgtagca ttgactcagt aagccatggt gctaggaggt 170760
   ggggattctt gggagaaaaa gtacactatc ccattaaagg gttaattatt ctacccatct 170820
   cctaagcgtg gcttaaggtg aacctggtac cctgtgggtg gtcccctggc caagtgattt 170880
   gctatcacag ctagaatgtt aggcctaata caggccagag attgtattct cttagccaga 170940
   aggtttcttt tattgggtct ttatttctac tctaactttt atgattgcac tgggtaaaaa 171000
   aacagtgcta agtgtgacaa aatggtgtct gttttggact cggattgctg aaggacaatc 171060
   gtgtgtccca tgaatttgga atccctactg tagggcagta caagtcagaa ctctgttgca 171120
   atggtttcgc atccttaggc ttaaccaaca accactgatg gaaagtactt cagaataaaa 171180
   atgcatctgc actgaacatg tgcaaacttt ttagtttcct atcattgttc catgaacaat 171240
   acagagtatc aatagctgac acgatgcttc aatgttgtgt taggtactga aagtaagcta 171300
   gggataattt aaaacacatg agagaattgt gggttatata tgcaaatact acccccttta 171360
   atatgaggga cttgagcatc caatgctgac tcaaggatct tttgaaagtg tcttaacttc 171420
   ggtaccctaa atatttagca tcacatgaat aatatctttg tacaagctgt ttctgagagc 171480
   aatatacctg ataccaagcc tcaaaatgat ttgtagtttc ctaagaaagc taaactatag 171540
   aaaatactgt ttattattat tctcattagt ccttctgtgg ccaatttgga agttataact 171600
   gccaccaaaa agcctcttgt acttgaagct cgaagttgtt atcgaaacac tgacatgaaa 171660
   tcaggcctac aaagaaaaga acaatccaaa agccatgacc caggctggag ggagatgaac 171720
   cttagaactt ggcttaatat cctaaattgg gcacaagtct cgagtcagtt agttttgaaa 171780
   agagggtcat ggggaaagag agcttacgct ctcatggtgg ttaatgaacg atgtagatgg 171840
   cagctcacag agcccttgcc accttctgct gagaaggcgc atcagtcctc taagataatc 171900
   agcagccgcc tctcctgcct ccagccttac tcagtgatga tgttcttcca ccccacccca 171960
   cccacccccc aggcaagctc cgccctggac tgtaaagaca gcctgctggg aggtgtcctc 172020
   cgggtcctgg tgaattctct gagctgtgac cagagcacca cctacctgac tcactgtttc 172080
   gcaaccctcc gagccctcat cgccaaggta aacatgggat gcttgttttc ttactcttaa 172140
   ttaagagtgc ggttcttgtc tggcttcaca tcgctctgta agcagatcag acgtcatgca 172200
   gggcatggag taaaagcttt tttccctctg aaagcagaca cactgatttt cagagactca 172260
   ctgtggggaa gccaaaccat ggcttcacac ttgcagtgtg ggcccaacta tggaaatttc 172320
   aaatgaccac tcactaaaag tcaatgacac gagagtgaaa gtcaacgagc aactacagtt 172380
   cctctccctt tcgttaatac cttatacagt gagattcaat actaggcctg gctcccatta 172440
   attaatgtga cagaatgacc ataataagat gctggcagat gcccgagaac tgtggctagt 172500
   agtctgttga gattattttc ctcctttgtg ggaaatttta gtagatgggt gccttctaca 172560
   tgggatcttg taaatttaca aagtggaatt ctgttataat aattttctcc ttcttagtca 172620
   ccaataattt ctaaggaacc cataagacac aagtaagtcc tttatagctg tgacacactc 172680
   agcagtgact tcaacctggc taacaatgaa gttaaggaca tagaccttac ctcagccgtg 172740
   tgactcacag gtggacgtca ccgcttacct ccatgtctga cacttgcggc tcattctgtt 172800
   tatggagcac ggcgttaacc cttcaggctc ttggcttgtc cagacagacc ttttgcatgt 172860
   cccaacatcc agccgaagag ctgtgaaagt gctcagggat gcctcatggc cacctgtgtc 172920
   tcttctagtt tggagacctg ctgttcgagg aggagatgga gcagtgtgct gacctgtgtc 172980
   agcgggtgct acatcactgc agcagcagca tggacgtcac acggagccaa gcctgcgcca 173040
   ccctctacct cctcatgcgg ttcagcttcg gagccaccag tgtaagcaca gacgggctgg 173100
   gcggcccaga accaagagag ggcaggaatg aaaggcagag gtagaaggga gctttgcagt 173160
   gtggctttta tgtatgccct gtgcggaaga ccccatgagc accagcattt gagaggaagg 173220
   gctggagggt gtggtgctgc ccagcagtca aagttcctgt gtgtgcgcag gttcaggatt 173280
   cacaaaagag tgtcctgtcc taaagtttga gtccgtacca gttctggatg gtaccaggac 173340
   atttgtggat gagaatggta aatgattgtt gtcatctggg cctgatgaca aacatctcac 173400
   tatgtagccc tggctgtctt ggaactcact atatagacca ggctggcctg aaacgcacag 173460
   atatctgcct gcctctgcct ttgcctcctc tgcatttaaa ggcgtgcacc accatgcctg 173520
   gcctggacct gaatagacat tttgtgagtt cagtttcata gcaaacctgt gccttagttt 173580
   gtgcatattt caggatccat gcttgggact ttatttgcca caatgtcaca ttagtgggac 173640
   atcttttaaa ataatgtttt atagcccaac agggtggtac acacctgtaa tcttagcact 173700
   caggaggcag aggcaggtgg atcactgagt ttgaagctag cctggtctac agagtaagtt 173760
   ccaggacagc cagggttaca tagagaaaca ctgtgtatcg aaaaccaaag atgggggttg 173820
   ggaagagaga gactatgttt taccaaatgt attgtaaatt ctgtaaaaga agatctttaa 173880
   cctcagaaaa gaatttacag atgaaacggg gcagataaat gggctaagag aagaaaggga 173940
   gactccgtag ggttttggtt tgctttgtct tatttttgtc tattttgtgt taattctggg 174000
   aactgaactg aagaccccac aatgctaaat gtttactcct gagctatacc ccaggccctg 174060
   gccctcatga gatgttaggg agaactattt aaaagcttat agggcatgct catgggccct 174120
   taagttgtgg ttttccacag tcctgggaga aaagaagaca tggaaagcta ctgcttactt 174180
   caaatccaaa aaatctcaaa agttctgcct ttatgaatct ctgtgagttc aaggccagcc 174240
   tggtatacag agtgagttcc aggacagtca gagttacacg gtgaaacaca aaacaaacaa 174300
   gcaaaaatgc tgcctcagaa ggttccaaag agactcttgg cacaaagacc aactttatat 174360
   cctgtgacca actttagaac aaatctatca gccctgcagt gtggcggcag cccaaacgca 174420
   aagaaaattt aaagggagcc tgacagactt atgggagcat acggtccatc caacccattt 174480
   caacagcagt gcgtatcttg tgcaaagtgt agctcacagt ttattgagag aaaaggaaat 174540
   gcttaagatg ggaatgaaag aaacaccagg agctgcttgt gtctggcccg cagcctcccg 174600
   ctaggtacct gctgactctc gtttgctcca tctcttctga tgcagaactt tgcaagggta 174660
   aagatgcaag tgaccatggc actggcatcc ctggtaggca aagcaccaga cttcaacgaa 174720
   gagcacctga gaaggtcctt aaggacaatt ttggcctatt cagaagagga cacggccatg 174780
   cagacaactc cttttcccat gcaggtagac ccgtgaacat agctttgtct tatgtatgag 174840
   tttgacatta accatttata agcatataca tttatgattt tgcatataaa attttgcata 174900
   taaaatttat acgtgtgatt tgcaggtatt tttaacttat cctatagttg aatgttctca 174960
   gtgcttgaga aatgcaatac acacaccact gtcctcaact tcaagatggc aaaagtctat 175020
   taagagagca tagtaccaaa acaagaagac tcaaaataag aaaagtgata cctgagtgtg 175080
   tggtaacaaa tactgtacaa gatttttaaa agggagtaca ggggaacagg ggctagtgta 175140
   attcagtggt ggagcctaaa atacacaaaa ctctgggaat gatctccacc tcaacataaa 175200
   ggtagataga tagatagaca gacagacaga cagacagaca gaaacacaca gagatgggta 175260
   gacagatagt gggggtgatt aatgaagaac tagtgctctg gaatgtggga gtagagagga 175320
   gtgaaattcc tagagttgaa ttcaggggag agggagaggg cgtaaggagc atgagtggta 175380
   atgattcgtg tgtaaggcag acgagaagat cctaattaag aagagcttac aaacaagatc 175440
   cgtcctaaag aaaccgggag gacagttacc cttctgtctc gctgccatgc ccatgtggca 175500
   gatgcccatg tgacaagtgc agcctgtctg cctttagctc caggccctag cctattctgt 175560
   agtaggctct cacaccctga tagtccacag aagtttatac ctttgcagtt tacaaatatt 175620
   caaagcccat tcagactgta aagtcaagtg ttggagaggg ggatagtata agggagcttc 175680
   atggattcca gggccctggg tcacctgtgg accttggatt gcacagatga aaatggcccc 175740
   aggagattct tggattttct ctgccttgga tccctgcctc tctctgagca aagccttgcc 175800
   ttgcacttca ctgtctgctc ttttcctgca ggtggaggaa cttctctgca atctgaacag 175860
   cattctgtac gacacagtga agatgaggga attccaggaa gaccctgaga tgcttatgga 175920
   cctcatgtac aggtaaatgc tcccgggaag acagagagca ttggggttag gcgtggtgat 175980
   gcaaaccttc agtcccagct tcagtcccag cactcaagag gaagaggcag ctgagttcaa 176040
   ggccagcctg gtccacagag tgagttccag gatagccagg gctacacaga gaaaccctgt 176100
   cttgaaaaag aaaagaaagg aaaggaaagg aaaggaaagg aaaagaaagg aaaggaaagg 176160
   aaaggaaagg aaaggaaagg aaaggaaagg aaaggaaagg aaaggaaagg aaaggaaagg 176220
   aaaggaaaga gaaaagacat ttgtgatatt tgtgagtctg gatatttgtc taggtgacac 176280
   tgcatctttt ttcttttttt gttaattttt tactttattt atatgaatgt tttgtcggca 176340
   tatatatctg tgtaccatgt tccccggagg ccagaagagg gcatcaaatc ccctggggct 176400
   agagttgtag atggttagga gccagcatgg gggtgctgga gccaacccag gtcctccagg 176460
   agaacagtca gtgatcctga ccactgagcc atctctcaag ccccagttgg agccttctct 176520
   gatgtcatta tcataactaa gataggtagg acagtttggg gtaacatcca aaaatatatg 176580
   agcactaatg gaaaacgagt tggttcattc ctaaatttcc aaaaagatta ccttaactaa 176640
   gtgcaatgct tattttaaat taagagagag agaaaaaaaa aacattggct gttatatgaa 176700
   ttatttccca catgctgtgc tgaagttgta ttcataaaca gaaacagctt tactttagaa 176760
   gtcagtgtac tttaaaccct ttctctttag gttcagtagt ttggggaaaa attacttaca 176820
   cagttaaaaa tactcagatc tgtaagagaa acaatacatt ataactttga gataaaagga 176880
   aggaaattac ttgagatcca tgaaaatgga cgtcatttta tcagactttt ttcctcataa 176940
   gacttgtgct agccgggcag tggtggcaca cacctttaaa tcccagcact tgggaggtag 177000
   aggcaggtgg atttctgagt tcaaggccag cctggtctac agagtgagtt ccaggacagc 177060
   cagggctacg caaagaaacc ctgtctcgaa aaagcatcgg cacccagcac accattgtca 177120
   tctagacaat gttacctttt ctctgttttg aaattgtaaa caaaaatatt aagggtgata 177180
   ataaagctta caaaagagag aaccctgaag caccaccacc accaccacca ccaccccccg 177240
   ccccttaaga gcagtgacgt tgaaaggtta tccatgaaac tttcctaatc tgtcttcaga 177300
   attgccaaga gctaccaggc atcgcctgac ctgcggctga cttggctcca gaacatggca 177360
   gagaaacaca ctaagaagaa gtgcttcaca gaggccgcca tgtgcctggt gcatgcagcc 177420
   gccctggtgg ccgagtacct gagcatgctg gaggaccaca gctacctgcc ggtgggcagc 177480
   gtcagctttc aggtaagaca ggcagtgcat ccaaccagga gctgggttcc ttccacctga 177540
   gggaaggcgt cctcagccac cacaggtgat cctgcatggt acagaggaaa gaccaaggat 177600
   gtttccaccg ttaaaatatc ggttcccggt tttctgtgtt gctaccgttg aaaacccctg 177660
   tgtcaaagcg atatcactgc cttgtctaat ggcctcacca tcttttaaaa catattcatt 177720
   acagtaatgg tacttagatt agaggtaaca gtagctttgg gagatattct cagacatttt 177780
   aggcatttgc ccatccccac aagcctgtga agcaggcaga taaggaaggc agtgttactt 177840
   ctgtttcact gatgtaagaa acaagacggc attttttttt taaaagatca tttaaactct 177900
   ttctggaaca tttgtatttg gcttatctac tggcaaggaa gggacaccag tataatgatt 177960
   ccctcacggg ccagtccctc gattctgttc cctggctttg tgggacccaa ctcttgtcca 178020
   aagctaagaa agaatccaac ttacctgaaa ttggaaagaa atggctccat ggatgctttc 178080
   tgtgaacaac ctgaaattta cccacagcca tcaagggcaa attgtgccag acctaaaaac 178140
   tcttaacagg gctgcccagc atccccttgg actattgtta ctgtaatgcg ctacttagag 178200
   ggcagaaaag tctcaaacaa gtgttatgag tagtttttct taaagagatc taagctacag 178260
   agatgatcat ccccgctgct tacaggacag ccatggaccg ttcccagcca gagctgggaa 178320
   gctggagaca agcgtttata ttgctcttgt ttggatttcc atggatgaat gtaaaaggct 178380
   ttgttctcat tattgttctt caacacagtt ttgtgatgct ttctgtaaag gagaaagaaa 178440
   agacgattct aagtagcttt cccaggctta gagtaaaaga caccgaaact attgctggtg 178500
   cagcccagag cttctggttt ctcagggtct gttcatctgc gagctgggcc ttatctccag 178560
   tcattgctgc cgcctctctt acagtaacaa aaatacttac tcttcgattt cattctcaac 178620
   agataaattt tagtctcaat tcagaaatgt cttttataaa gcccagttca attctcgttc 178680
   agacgcatag aaggattcca tctggttttg agtcaagtga ataagaaact gtaaagcaat 178740
   gtttgttttg tccacttgcc tgtcacggaa ctgctgtagc gctgccctcc agctcctccg 178800
   tgacacactt gtccctttac atcgcactgt cacccttttt tctcagctaa tctactgagc 178860
   tattccttgg ccatctctca agccttttcc taaatgctct actttatggt ttactccttg 178920
   atgcttaaac attatccaaa tttcattttt actattatat ttaataagtc tcgctttatc 178980
   cttattttac ttagaatttg gttgactttc ttattttgat aggtgccaag gtgagataaa 179040
   attccagaac acctgaggaa gtctatgtta tccttacgtg gaatatacag atatatcttc 179100
   ttatatattt ttatctattc cttcttcttt tttttgtctc ttttggctgt ttgttttgtt 179160
   ttatagtgct gttgttcttt gaaacagggg cttgtgatat agcccaggct agcccggggc 179220
   ttccctaggt tgagcagcat agccttaaac tcatagagat ctgcctgccc tgtgccattc 179280
   aagttttcag atcaaaggcc accatgcctg gctatccatt ccttctttca cattcattga 179340
   agatttatcc tgtccacatg aactttatat tctagcaact aatgataaat agaaaggata 179400
   caagatgctg tgaacattat cgtgtgcccc agactagagt caggggaagg atggagattg 179460
   tgagaaggaa ggtggaagga actgtgatgt gtaagggctt cttaatgtgc agaacatgac 179520
   gagccagatg tccttgaata cagaacagac ttcaagccat gctgatgcct atgggagaaa 179580
   tactatatgt ctatgggaga aatactatat tctcccaaga atcctacaaa gtcagtttgg 179640
   atatgggtgc tggaaaggga gtgataccaa gcccacatta acctaaggag atgtggaatc 179700
   cattagcctg tatggggaaa ctagccctca gtcatacttg ccagtctata cctagagacc 179760
   caagctcatc caacctaggc agtgtctcca ccttctgggg tgtgggcatc actcttcaca 179820
   ggcagaattt ccacactaag gatgctcgat taagattcaa gactaggaga gaagacggaa 179880
   atcctccttt ctagagttgc aaagaaagtc tcagggcatc ttattggttc tgagcaggtc 179940
   ttatgtttat ccctgaatca gtcataatgg tcaaagtagt aagatggtct gattatctat 180000
   cccatagtca caggtcacac tgagcattac cagtgttttt cttaaagaaa gttaagatac 180060
   tttctgtcac agaagaataa aggaattcag acagtattag caacaggtat tctctgctga 180120
   cagctctctc tctctctctc tctctctctc tctctctctc tctccttcat ttcttcttcc 180180
   ctccctcacc ctctctcttt ctttgacagg atctcactgg tcttgaactc agagagattg 180240
   cctctgcctc tgcctctgcc tctgcctctg cctctgcctc tgcctctgcc tctgcctctg 180300
   cctctgcctc tgcctctgcc tctgcctctg cctctgcctc tgcctctgcc tctctgcctc 180360
   tgcctctctg cctctgcctc tctgcctctg cctctctgcc tctgcctctc tgcctctgcc 180420
   tctctgcctc tgcctctgcc tctgcctctg cctctgcctc tgcctctgcc tcaagtgctg 180480
   ggattttagg cttgtggccc catgcctgcc tctattgctt cttaaacatc ctgctttagt 180540
   ttttaacctt gctgtaataa gattgagcct ttgggaaaga cttgcaaagc agagataact 180600
   aacttggaag aataacagga gaaatgactt agaaggcagt gaggtccaag ccactgagat 180660
   catcggaatt ctatccctgg agtgcaggca atgctgatct cacaaacaca gatatggttg 180720
   ttacttttac accctcactc tgaagccagg aatgtccgcc cagcatcttt tccccccgtg 180780
   acttacagct actgagctgc ccggctcata cctattcctc tatgagtaga aaaatctcat 180840
   acagtgccag tcgtgctggt gtcctaagcg attagatccc ttcagctgaa ctcttgcccc 180900
   ctctgccatc agttcatcat gagaaatcac gtttggaaga aagattggtc acaagggact 180960
   ttgaaaaaaa ttctgtgttg ataaaactgt attccaatgt aactgtgaca atctatgacg 181020
   actgcctcct tatggattcc taagggtgat tcctgtgacc ttgttcgcca agtcagaaag 181080
   tggacagcct ttatatatca cagagagcat cagagcaatt ttcaatgact ggctggagta 181140
   taccccaacc tcaggggaca gagtgttaac cattgttgag cacagcatta ggaaggcgtg 181200
   tcctgtgtgc tctttgcagt tactacacgc tggaggtttc ccatttcttc tctataccat 181260
   actcacttct tccatagctg taagccaaga atggctcgtg gcctatcaga ggtattgatg 181320
   tccctgccag gatcagagct cagatgaggc aagcggggta tgtgcagcac acagcttaaa 181380
   gagaaactca tgtcagaggc aagcaggtcc caagtcagcc atcccaggac cctgaacatg 181440
   agtgctgcct gaaggcttgt gccctgcgtt tcccatccga gccaaccctg cccgaaatgc 181500
   ttcatcccct gttaagatgc tctcatgcct gtagtttata gaaaggaaaa ccacagaaac 181560
   aaaaggggag ggcagcttct gaattatatc acagcaccca taaagacatt ctcgtccatt 181620
   actttcctat gtaatattac acttatgtag ctcaaggtca agactgcagg agcaacatgt 181680
   ttcagcaaaa caggaatcga ttttctgctc actggccttg cccttaaaat ccaaaacatt 181740
   ccctctttgt ccttagggtc atcgcactaa cagtaaggtt gtttcagcta aactgttact 181800
   acattaagga aagtgtcctt tctagaccgt tgccttatgg ttaaaaattg tagagtttgg 181860
   tgacaagttt caaaatgaat ggccaccaga agctcacagg ttggaaccat agactcgtag 181920
   caatcatggt ctggtgaagg ttgaagagct tagcttcttt atctgtctgg gttttgtggt 181980
   cccttgcttg tcaggtccgc tgttccccgg gctcacagtg ggttctctta cagaatattt 182040
   cttccaatgt gcttgaggag tctgcagtct ctgatgacac cttgtcacct gatgaggacg 182100
   gcgtatgctc tggtcggtac ttcactgaga gtggcctggt gggcctcctg gagcaggctg 182160
   cggagctctt cagcacggtc agtgtctaca gggctcattc acacccacag gagcatgcag 182220
   gacagggctg ggcctggctg gatgcttccg gattggtcca gtggagacgt ccatgtccac 182280
   aattgcacca gttctccaag ggaggagaga aagcctttca ccgtgggcat actcgtattt 182340
   ccacagtgtg ctcattggcc aggagcagtt tcactgtgtt ctttctagtt aataggggta 182400
   gaaactaagc cacggaaagt gaaacagggt gcccaggtta cacaatcaga gatgaggcat 182460
   attctcaatc cctcattttc ccactcacag gaaaaagtag aggaagatcc tctgaggatc 182520
   tatgatgcct tccacatctg acacccaact ggtcttcaag acccataccc agggcagctc 182580
   agagagatgg aactgtggaa gccatagaac tatcccttag cccagatcca gagccacccc 182640
   ttagcccgat ctgggtctcc cagtgaaaga aaagacacag aactacagtc caggtcaagg 182700
   tggcaaggca ctcgtagctt ttcgttttaa ccattgccct ctgcccccat agtggaaccc 182760
   acgtaagtgg agcactgtat ttacctgacc aatctgtttt cctccctaca ttatttctca 182820
   ttctcccctc ttcacctcca gactccctcc ctagactgtc tacgtttggg cgtttgtgtt 182880
   cactaccttt attctcctat ctttgttttc ctagaaaact tttccagaaa ggaactttta 182940
   atttaacttt tagtcacaaa actagctttc ttattacatt ttcccacata catatcatta 183000
   tacttttctc ctatccactc acacacacac acacacacat acccttattt tcctgaaatt 183060
   ctagaaagtc aactggtaag aaccttatta gctccctgag gccctgcatc tggcgagagg 183120
   agaacctaaa aaaaacccaa aagccacaag cacacctctc cacacccctc actgcctggg 183180
   ccatctcctc ccttgttttc agtttgtgaa aagcctcctt agatggcaat gctatgttcg 183240
   ttccttactt acttgacgac ataaagatag caacgtgcct ggtttgacct gaaagctatg 183300
   gctctccaaa gatgcccagc tgggcctgga ttctgtaaag cactgcgatg tccccctgtg 183360
   tggtatttgc ataattaact ctggtccctt gtcttaaacg tccaatgcat taactaatcc 183420
   cgtctttccc agggaggctt gtacgagacg gttaatgaag tctacaagct ggtcatccct 183480
   atcctggagg cacacagaga tttccggaag ctgacctcca ctcacgacaa gctgcagaag 183540
   gccttcgata acatcatcaa caaggtagct ggggatgagc ctggcccata agttctggta 183600
   gcaacgctgc ggcaggcgac cctgccttaa cgctgcgcca ccatctccct ggcgctcatc 183660
   attgctgtaa ctctccatca gacaaggaag ggccggcagt acagtgtggc agattagatt 183720
   gctttatagc taggaaatgt gaggatttta aaatttgagt taaatgaagg ccccagaagt 183780
   ctctcagtgg atttcttttg tcatctaggg tttattatgt acatattaaa tctgcttaac 183840
   agaggggatc ttcgtaatgc cagctgtgag aataaattgc cgcccaataa ccagtccttt 183900
   tagcccagtg ggtcaccagt aagcacagct aaaggaggct tctctttgct cccgggacag 183960
   gaccataaga ggatgtttgg gacctacttc cgagttggtt tctacggatc ccgatttggg 184020
   gatttggatg agcaggagtt cgtgtacaag gaacccgcaa tcacgaagct cccggagatc 184080
   tcacatagac tagaggtgag aaagtgagac tgtgtgcgac cttggacatt cccccccccc 184140
   caagtagggc aatgtgtcat ttttaaaata tgttcattgc tggcctctaa atttcccacc 184200
   ttgcatttgt agtatgggaa agtaggcaag attgggaata ccatactatt gggacatttt 184260
   tctcttgaat cctgtgtgcg ttttacagag tttaaagttt aaatcttgaa tcttgttttt 184320
   ttttgttttt tgtttttttt caagactata aaattcagtc agcatccacc cttccttata 184380
   accccttcca tacatggcca atatgtaccc acagacattg ggcatattgc tttttgactt 184440
   tgaaggtcgt catcttgatt tgggaggcat ctaagaaaac tacagactca ctccactgag 184500
   atacagagca actggaaagt aactgtggcc agctcccagc tgtagcctgg cagctacgct 184560
   gtgaaataga aaacagtagt taagttctga aaagccagag caaaaagaaa tagcatagag 184620
   aaaagccgag acaacagaag cagaagttga gaggaacaaa cataagagcc ctctctgtct 184680
   gagagaccag ggtcgtgtga agagctgact cctccctact taattaacgc tgccacgcca 184740
   cgctttgact gtgtcccctc ttgtgactgg aaagcttaaa aacttgtgct gcgcaattgt 184800
   ttcttgtact gttcagtcct gccaaataag gcaagaagac tcaactctga gaagctgtgg 184860
   cctagtcagg ttgggtggct tgcccaaagt cattcacttc acaacaagga aaagtagttg 184920
   ttggcccagg ttcttaactc cctcatgctg tgtcctctgc ttaagagtag aagtatattt 184980
   cttgtctcct gtactctggc ttgtcgcgtt atgggaagtc tggcccctcc aaattttcag 185040
   ggtacaaaat gcaaactgta aatcatgccc tacccatgga ggtgcctcca gaaatctgat 185100
   gacgttggct gaatttgaga gcacgcatct gtactgtagg atgccgaggt atttcagtgt 185160
   gctgaacttc cttagacttt catttctggt ttaggaagtg cttagctttg cttcttagcc 185220
   cagagggacc atcaataatc aacaaatcct gccttgtgct tgttccaaag gagtgcaagc 185280
   tttcaaactg aactttatat actctgggcc tctcttccct agggatttta tggccagtgt 185340
   ttcggtgcag agtttgtgga agtgataaaa gactctactc cagtggacaa aaccaagttg 185400
   gatcctaaca aggtatggaa aaaatatata ctaaaagtgg ttatcacact ctaccctaac 185460
   agtagaaaat ggcagaccca gaggtctgga ctcccccagt ccttcaaatg tgaccatgtt 185520
   ttagtttgtt ttttaggggg gaggggtgca cacacatgta tgaatggagt gtatgcatgt 185580
   gtgtgtgcag gctcactctt ccatacatgc ccacatgtgc aagctggagt ttagcaccaa 185640
   gtggttttct ctatcatgaa ccacctcatc ttttctaccc gggtttccct gtttggccag 185700
   taaacagcag atctaccgtc cctgccacct ccttccccag cactggggta cacatatgca 185760
   gtggctttta tctgggtact ggggagctga gcaagggttc tcattttacc cacccctccc 185820
   atttaggttt ttttttgaga aggttcttgc tgttagttca gcctggctac acaccagctc 185880
   tgtgtttata taagtcatgt ctcgtcatcc ccctacagcc ctgaggattc ggtttggttg 185940
   tttgtgtttt acaagataag aaagttgagg ctcagaaagg ttggaccact ttccaagatg 186000
   acgctaatgg cggagcggct accccaacat gggatctgat tccacccacg gtcttcttca 186060
   ttactatggc tcattagtgc atttctgtaa aatgtttaaa ctttctactt aaagcatgtt 186120
   tttaaattcc aacttctgag ccaggagaga tcgttcagca gttaagagca cttactgctc 186180
   ttccaaagga cccaagtttg gttccctgca cccacatcag tctgtaattc tggctgcatg 186240
   acatcaactc cctctctggc ctccagaaat gcatgcacac ttgtggcaaa tacaaatagt 186300
   gtatgaatgc gaatgtgtgg gcctgtgcgt gtgtgcatgt gtgtgtgtgt ctaaaaaaat 186360
   gcagcagctt ctggctggcc atacttagtg gccctgttct ttaagctgca ggcattcctt 186420
   ctagccatat gcaggattgc tatggtatta gagaaagggg ggggggcctg gggctttgtt 186480
   gttctctgtt ccataccaag ggtccatccc aggctgagat ggtatgctgt gcgattctga 186540
   tgctgacagc tcacatcata gcatatgatt tcacaagttt aagactgccc ttgtttcaaa 186600
   catgagcctc agctggggcc cctacacctc tcacaccacc catgcttcta gccggctaca 186660
   aatgtaggag ttcttaccac acccttgggt tagataattc tgcaggataa gtgcacggac 186720
   tgaaaacagt accacagttc cgactgcagt tgtacagtaa tggatcccag agtgcaaggt 186780
   caggaggaag gccgatctct gtgccctctc catgtgggtc cctgaattct gtcttccagg 186840
   attgcagaaa gatgatcctg atagggaggg attggccaca tacaagtttt tctacatttg 186900
   tccggacact cacttgagag acattcccca gctgaggccg gagagcagtc atgattagca 186960
   ttggacagaa aagtttggag tccttggaag ataaggctgg ctgtgtgccc agtggtcaga 187020
   acagctcaag aagccttccc agcatcaggg cctttcccct cttttagaat tcccaggcta 187080
   ggctgtgagt ttggaatgag aatcaaccca gaatcacacc cagatttcat tatcctaaag 187140
   cgtgatttac actgtgaggt cagtgaaccg cccttcctcc tcctgggtgg gtgtcccacc 187200
   ctgaaccatt gctcatttcc attcttttcc ttgtgttttt aaagaactct tcattgttaa 187260
   caagcacccc tccctccccc atctcgtgtg tattcgtgtg taatttgctg atttgtcgtt 187320
   tcatgtgttt tgatatatac acataaccag tacttgacag tcaaggtata aaacaaccct 187380
   gttcctctga aaggtttctc tagtctcttt tgccaccact ccctcatctc attcagccct 187440
   tagtggtcac caagctgagc tgtagtcccc gatctgtgta gacttgcctt tctggaggtg 187500
   tgtatgtggc acatgtagcc ctttgaagct ggcttcttct actcagccca ccccatccat 187560
   gactcaccga gttgaaatcg ctccttcctt tccatgctaa gtagtattca ttgtgttgat 187620
   gtgccacact ttgcttagtg tctccactgg agggacactt ggacattttc ttgtcattta 187680
   ttatgaacaa ggttactctt tctcagtggt tttcaaccat cctaatgctg tgacccttga 187740
   atacagttca tgttgtgatg accccaacca tggaactact tcacagctgt aatcttgctt 187800
   ctcttatgaa tcgtgatgtg aaagggactc tccacacccc gagtggggtc atgaccctca 187860
   ggttgagaat ggtggctctc aagcatggcc tcgtgtggct gattattacc ctccgatcct 187920
   cttggtgtta tgtgactctc ctgaggagtc tgcttctgtt cacctcatat cgtgagggca 187980
   gcaacagatc agagaaaggg ttctcttcaa gtctcactcg gtgaatcgat gagtttattg 188040
   gtgttattga cagagcatgg gtactcaaaa tcccctgcac caccatgaaa ccctctatgt 188100
   gatttatggg cagctactcc accaaagaaa ctcctcccct cagcaagtgg agaaaagtgg 188160
   ggaccggcag aggaaggatg ggtgtcgctg cagcagccta cccgcttcct tcatgcgggt 188220
   gatcacatct gctctccttc aaagcagcaa taagcacacc aagctaagga acgccccaca 188280
   caagtactgc gtacgcgtgt ggcagtgggg ctcgtgtggg ggcggcattt ggctgcatga 188340
   ggagccttgg cgtacagagc agccatgcgc cctgcattcc tggtgctcat ttgaacattc 188400
   cagccgctcc gtcctagcaa acacttgcta ttatacatgt cctcagtcta agcagttgca 188460
   cgacctagag ggcgggccct cattagggca actgagtaaa ggaaggtctg cgtccttggg 188520
   acagcagttg gatgacttcg ctgtgctaga ggggccgcag ctggacccgg ctcttctctt 188580
   aagcctgttc ccttccacag cctcggggct tttctttgcc tgggttggag aggaaacagg 188640
   gcagattctg aactccctcc cgcctcgctt gcggtgttag gcattggtgg ttttccagac 188700
   tggaagggag tgtcgggttc tgtcccagcg tgcctttcca aagcgccctg catctctttg 188760
   gctgactggc tcacctcttc ccgtctcctc acctcttcct tcccccaggc ctacattcag 188820
   atcacttttg tggagcctta ctttgatgaa tatgagatga aagaccgggt gacctacttc 188880
   gagaagaatt tcaacctccg gaggttcatg tacaccaccc ccttcaccct ggaggggaga 188940
   ccccggggcg agcttcatga gcaacaccgc agaaacaccg tgctcaccac catgcacgcc 189000
   ttcccctaca tcaagaccag gatccgagtc agccagaaag aggaggtaat gcgccccgcc 189060
   cgtgggaaat ggccactttc gggaagaggg agcctggatg atccccaaga cccacacgcc 189120
   agctctacct catgagactg aagagaaccc tgaaaaaaag aaaagcaaag aatggagggg 189180
   aaaagaaggg ctggacacag tggcgtgtgc ctttagtccc agcactgagg attcaggggc 189240
   aggtggatgg ctgcgaattc aaggccagcc ttatctacat aacaagttcc aagccaacca 189300
   tggctacaca gtaagacaca acaaacacgc ggcagccctg ggagtccatg ctcatgatcc 189360
   cagtactcca gaggctgagg caggagcatc ttgagtttga gagcatcctg ggtggcatag 189420
   caagtctaag gcaacccaaa ctatttaggg agatacagct cagcagtagg gctgttgtct 189480
   ggcctgtaca aagatatcct taaaatattg tctcgaggct attctctcac tctcatgggg 189540
   ccaagggatc tcacgcagtg agttacacct ttcaaaacag tgttaaatat ttagttcctt 189600
   aaagttattt atatagcata gtttacatat ttaatcactg tgtttccatt ttaattataa 189660
   gcttatataa gaggtctgta tagtattccc ttcatgttat taggttttat atccatagaa 189720
   tcaaccaacc ttgaattggc atattgaagg gaaattttac ataaatacag aatttgtatg 189780
   aatttttccc cttaaaaaat aaaaacttca cacacatgta agctctcctg aacagataaa 189840
   gaggaatctg ttaccctgca tggtggcaca caccctaatc ccagcatttg aaaggcagag 189900
   gcaggtagtt ctctgagttc aagccaccct agtctacaga gttccaggac atccaagggt 189960
   tacatagaga aaccctgtct caaacaaaca aacaaacaaa caaacaaaca agaatatagt 190020
   aaagccgttg tctgtgcagt ctttgcagcc cattgggtgt gaaggatttg ctgttacact 190080
   ctagagtgtt tcctaaatgc ttggtcgaaa caactgaggg ggcagagtca ttgtagctca 190140
   gttttcaagg tctcatccat tgtggtgaag aaggcatagc agagctgttc agtgcacacc 190200
   atagtaacca ggaagcagac aataaggctt acaggagggg accaaggaaa agtacagccc 190260
   caagggctta tgctcccatg gacctgtacc actgaactag gcccatcttc tatacttcat 190320
   gagagatcca tccactggtt atgttaaagg tttctgttgg aaaccacacc ctagtgcatg 190380
   aatctgtggg tatacttcat aatcaaacat ctacgtatcc tcagatttca gtacagctgg 190440
   agagaaaagt tcccagaacc atctttccag ggagtatgaa tgcctgtatt taaattttat 190500
   attttatgca gtgtttttct cgagtattag gtactagatt cagagcctcg tgcttactgc 190560
   aatacttcac ctatggcctg taggtgtttc agacactgtt gtattgcaga gagggaatca 190620
   aacaggcagc cccctgacct ggtactccgt gtgggaaggg cacgattgca gccagcacat 190680
   cagcctgctc ctggccccag gttcttccag tgccggtgca ggaggcacat ggctctaacc 190740
   tcttaataaa aatgttggca agaaagacct tatgtacttt ggaagacttt aacccacttt 190800
   aatagagtct taccaagaag atctctcaaa cctcagaatt atgcatagca ccaaccttcc 190860
   attttctcct taaaatccct acgtgttaac tcatatacca cctctttatc ctggactgca 190920
   gccttctctg attctggctt gactcatccc aacctggcac tttctttctt gtatagacca 190980
   cacccagttc tcggtctgct ctctgtgatg cagaatgagg ccttaaaaaa tccttaggaa 191040
   ctgatgtgtg agcggatggc ttctgcacct tgatgagcct agccccaaac ttctttttac 191100
   agttgtcttg tgcataccta atatgacagg aatttttata gcaactcact ttcatgacat 191160
   ctcgcccagg catccagctt gatcttcata gcaacagctc tctttccata cccttacttc 191220
   atcagtttat ccgttgttca ttaagccaac aactagaaca ggcccaatca agtcagtgaa 191280
   catgtgaagg acttgcaacc atccagtcca tggggcagga gctgcatggg ctgagctggc 191340
   tggagagcag ccatctagac cgctgctgag tgatgtagct tcctcagaga ggaggagtca 191400
   cagagcctgc cctggagact gtccatagaa aggtgttggg aaggcatcta gtatgctgct 191460
   tactgtttca agacttttaa acactaagac attctaggca caatacagag atacattgtt 191520
   tatgaaagtg tccttataac tatatgcaat gcccccagag aactactcca ggtttcccat 191580
   tgctccagct atgtaacaaa gtggcccaga ctggatggca taaataacag aaatgtattg 191640
   tcttggcagt tctcgaggct gggaagctca agttcaggga attggcaggt tggttccttg 191700
   tgagggctga gggagaatct gtcccaggcc tgcctcaagc cttgtcagag gacatcttct 191760
   ccttgtgtct gctcatctca tctgtgctat atacaagctg tgccctcatt tcctcatctc 191820
   ttaaagatac caattatgtg aattaggtct gccctaataa acccagttta acttgactac 191880
   cttctgagct cctggaggtt aggcctttaa atgtgaatgg gggggggggg gttgatccat 191940
   cacattcagg aaggacccaa ccccaaacca gaacttagca ccctcttctc aaggtgtcac 192000
   catcacagtg agctggaaaa ggcacaaaag atgtgaaagc ttacgtgatg atgggaaaga 192060
   aaatcatggc ggccatactt tccccttgta atctgagtaa cttgttcaat actaggaaaa 192120
   acaaaacaaa acaaccaacc tagccatgta gaaagaagtt gggctttaga tatagaaata 192180
   attatgacgg ctgagcatgg tggcacacac ctttaatccc agcactcagg aggcagaggc 192240
   aggcagattt ctgagttcaa ggccagcctg ttttacaaag tgagttccag gacagccaag 192300
   gctacacaga gaaaccctgt ctcgaaaaac aaaaataata ataataataa ttatgaccaa 192360
   atttgtacct tagtacttaa tacattttgt gttagtctta taaattcccc ttttacgata 192420
   aagtgaaaag ggcaggcagt tgtttaaagg agaaactcat acacagatgc ggaacatgta 192480
   ggtactcaag aaacatgaag gccatctctg tgtgacctgt tcagattatt tcaaagtacc 192540
   ctgaaagtca ttccttagct gctgcgtcaa atccaggtag cctccctgtc agagctccag 192600
   gtttgccagg gactgtaagg gaggccatgg cctcactctg cggttgtctt ctgttcagtt 192660
   cgttttgact ccgattgagg ttgccattga agatatgaag aagaagaccc tgcagttagc 192720
   cgtggccact caccaggagc cccctgatgc aaagatgctg caaatggtac tgcagggctc 192780
   tgtaggagcc actgtaaatc aggtaagctg ggctcactaa tggcagcttg gttagggctt 192840
   gtttgtttac gtggtctcta ctcttaggag gtgatgagga atcagtctac atgcaatctt 192900
   cctttttaaa aaaatcccag aaaggagaag actgtattta aggagtacca ttgtctcaaa 192960
   gtctccattt ggaaccagat ctggcaatat gacccaggac tagagtcttg ttgcagagag 193020
   ctgacttgtg tccaggatag gtagctatat gcagagcttt ggacaaattc taagaatgtg 193080
   tatgtagaga taagaaaagg caggcctagg ccatctatct cctcggttag gtaccaggta 193140
   ttcccaacat agcaagtacc ttgccaacag gattgatctg tgactggctt ctcaggccac 193200
   tttgacaaga ttagtgtttg cattcctgtc ataaccatca cacacaagtt cagcatagaa 193260
   ctaagactgc ctctgatgga gagacatata agcctagtta gtgctttctg tctagtgggc 193320
   cccttgatgt tggctcttca caccaccaca cccaaaaacc cataaccatt gtccttccca 193380
   gccaacgcct tctctatctg aggatcccct gcagaaattc taagtctgtt caacttaggg 193440
   ggaatcaaag gtagcaaaca ggaggcctgt gaattctctg cttcaaatca tggtttacat 193500
   aaaatgattc atccactggg gccaccagat aagcagatag actcggacag atagaggtag 193560
   ccttcgcttt agaggagccg ctcacagtag cagcctttgc tgccgtcaca ttcattacct 193620
   tgtggtatga agggaaagct gtagggttag cattggattg agaagatgcc cttaggccag 193680
   tccgtagtct cctaaaactc ttggtcctcc tcgggaatcc gtttcctgag aaattcaaga 193740
   cctccctagg ctttttgttt tagtgaaaca agctacaaaa ttaaatgttc agaagtaagg 193800
   agccctcccc acattctctc tctcccaagt catttcattt tcattccaac tccatctcct 193860
   gtttcccctc cctccatcag ggaccactgg aggtggccca agtgttcttg gctgaaattc 193920
   cagctgaccc aaagctctac cgacatcaca acaagctgag gttgtgcttc aaggagttca 193980
   taatgcggta agaagggagg tggtggacac tggagcgtga ctgcatgggg gcaccgctca 194040
   gtactggagt gtttgcccaa cacgcaagag gccctaggtg ccatccccac cactacaaaa 194100
   acaaatagaa tcatatccca ggtaccaccc agagctactg gctcattgac tctggtttaa 194160
   gaaccctgct agatgcttat gatgcacaca cacacactga accttaaggt ccagggcagt 194220
   agaccaatgg tgcttgtact accactgaga aactaacact tcttgtgtag gctgcttcat 194280
   ggctgccatg gcactgggcc tcacagtcgt gtcgtaagac cgatatcgtc atcttcgcct 194340
   tacacacgag ccatgggagg tttggggatg ggagatgact ttactgacgg ccacgcagcc 194400
   agttggcagc cgggctgagt tgcagcaggt cttctggttc ttggccatcc ctgcttctgt 194460
   ctgggctggt agtcactgac acacttaagt caaagctgtt tgtatttata ctatgtaggt 194520
   ttgcaaatta tccagtgatt tcctttagaa gcctaaaact aggaaaggtt ctaaaactgg 194580
   gctttggcaa agatgacaca tacctgggca aggccagcct catctgtcct actgacttct 194640
   tctgtgcgac tggtcttaag ggcagctgct tcagcctaga cagaaaatgg ctctgtagtc 194700
   agagccttgt gattcccgag acacagatag ttacctcagg tctccagtgg ggcctgaaag 194760
   aaaaacgtga tgagatgggg tatgacgcca cttcacttat aagagaagaa aatgtcagaa 194820
   gtgtgtgtac caattgcctt tacttccaga accttggtgc agtgaccgag gacaggaata 194880
   gtcttgcagg gtgggcaggg agaaaggaaa ctaaaagtta gtttgcccaa tttagggtca 194940
   gcccactaga gggctgctgt cgggtgaaac tagagataga gtctcatcac aagctttccg 195000
   gagaaatgcc caactttgct gtgtgagaac acttctccct gcagtgaagt gcaggtattt 195060
   ggaaatgggt atatacaata ggtggcatct agtaatgttg gtatacttgg tttccttttt 195120
   ttatagcaat tctgctataa gaggcatcat tttctttact acatagtact ttattggctt 195180
   ttgttctctc tctctctctc tctctctctc tctctctctc tctctctctc tctctctctc 195240
   tcctttctga aacagtgtcc cactatgtac ctctgaccag cctgggaatt aggtctgggc 195300
   attcctgtga ccatggtgac aggatgaagt gtcacattac tgagtagcaa cagagtgcag 195360
   catgtactga gaactcttaa gaacattcca gatacaggaa tccacacatc agaaaggcca 195420
   ggtccattgt gagctaggat aggctttatg gaaaactatc acctactatt tgtctcgaat 195480
   attcaggtgg aagaagaaag atgatggtga gagggagcca tgactgtcag gaagatggcg 195540
   tggatgtaag actcactcag gaatgccagc tctgctgtct catggaagtc catgccagct 195600
   ggagagcagg ccaggctggg aaatatcaga gccacattag ctctgggtag aatatcacag 195660
   aataggaagt ttgctgaggg aaggggcact tttagggttc acaccaactc cctctgctgg 195720
   atcactcaat cccaaccaag actccacgta gatgaagtgc cctagcccat ccctattggg 195780
   tgtgttctag gtctctggaa actacagtcc gaggacaagg gcaggtggtt atctcgcttc 195840
   ttaagaggat cctttaacag agttactgtc ttagggtttc tattcctgca caaacatcat 195900
   gaccaagaag caagttgggg aggaaagggt ttattcagct tacacttcca cattgctgtt 195960
   catcatcaga ggaagtcagg actggaactc aagcaggaca ggaacttgga ggcaggagct 196020
   gatgcagagg ccatggaggg atgctgctta ctggcttgct tctcctggct tgctcagctt 196080
   gctttcttac acaacccaga actaccagcc cagggatggc actgcccaca gtgggctctc 196140
   ccacccttga tcactaattg agaaaatgcc tcacagctgg atctcatgga ggcatttcct 196200
   taagggaggc tcctttctct gtgatagctc cagcttgtgt caacttgaca cacaaaacca 196260
   gcgggtacag atattcaaag tcaaagtcct taagaaatgc tattctcagc aagttgaggc 196320
   aggagactta ctgtgagttt ggggtcagct ctgatgggca tagtgtgctc cagaccagcc 196380
   tgggaaacat agcaaaactc tctagcagca atgctctggg taaaccagat ggcacttatc 196440
   aaaccaaatg cagactgatg gttcagtaag catggagtag ggcccaagac tctggctttt 196500
   gttagtttgt ttgggttggg ttggggttgt ttgtttgttt gtttgtttgg gggttttgtt 196560
   ttgtgtttaa ggcagggttt cactttgtag tcccaggttg gccttctccc ttgaatcccc 196620
   ctgtctcagc atcctaagtg ctagaatgac aagtgtcggc taccatgcct ggatccagat 196680
   tgtgcagtac tgtgcgatgc gtccttgtgc tggggccgtg ggagcggcgg cctttgggca 196740
   gaagctgctg cttccgggcc tcctgtttat acttctcccc cagctccccc aaccttaact 196800
   cttcaagtca cccctttggt cttcctccct tcaaaaagac aagtatgttc acacagccat 196860
   tctgaaccaa ccctcggtcc atgccagctt gccacaatcc agcataccct ctgtaccacc 196920
   ggtgtggaga ggtgacatcc taaggcagga aggccataga taaaacaatg cttccagcca 196980
   ctaccacatc cacgacgaca cgaccacagt taacctcccc tctcttcatc ccagacaggc 197040
   caaaaatggc agttcatcaa actccttttt tttttttttt tggtttttcg agacagggtt 197100
   tctctgtgta gccctggctg tcctggaact cactctttag accaggctgg tctcgaactc 197160
   agaaatctgc cttcctctgc ctcccgagtg ctggaattaa aggcgtgcgc caccacgcat 197220
   ggccgaacta cgtttaaaag gagtgtaagg atttttaagt tcccacaaag cagtggctga 197280
   agcccccctc tcccacctgc ttgctgctcc caagtccgat gaggaagctg ccactgtttg 197340
   agaccataga caacagagtg gcccactgct gagactcctt ttcactgggc tctgtctcct 197400
   cagcttccca gcagactggt agatatccca gggctcaaag gagacccagc cagccttccc 197460
   gcaaggacag ctctgttgac acagccctgg ccaagtctgt agtggcttct cctgcaccag 197520
   gacagcggct ttggactcag ctgtagcctg gcatgcagca gtcagcactc ctagactctt 197580
   agcactcttc tgatggctgc caagagagga caaggccttc tctgcctttt cctaagagga 197640
   aatgcctgaa aggttcataa ccgcagatgg acaagtgtct cctccctcct ctcccagact 197700
   cactagaccg tcagaggcac tgcagtcggt tctgtgtttt ccctcactta atctgattgc 197760
   tctcactcct gcagagactt ggggcttggg aaatagctca gtggataaga gcatgcattg 197820
   tgcaagtctg aggacttgag ttcaaatccc tgctacctac atataaacct taatgtcgct 197880
   gtgcatgcac cggtgacccc cagcagaggg gtggatgggg caatggctgg ggtttgctgg 197940
   tcaccagcct agttccaggg tcagttaaga gaccctgcct caagaaaaat cctgatgtct 198000
   tcctctggct tctacacatg tacctgcgtc cacatgtgca tacacaccac acatacaaat 198060
   acagttaaca aattataagt attaagtgat ggggggcggt gctgaaaaaa tggttctgca 198120
   gttaaatgtg tgtactgttt tttttcaagg aacctgagtt tggctcccag cacccatact 198180
   aggtagctca caactgcctg ccactctaag gacacacttt cttagggggc acagccaccc 198240
   acacagggag caattcctgc ctacgatgag cttatgagag aaacggcaaa ccaaacttac 198300
   agattaaaaa aaaaatgagg aaagtgctga gttagaaaga gagagaagcc ataagccaga 198360
   tgtgacatgt gcctttaatc ctagcactgg ggaggcaggg caaagcctgc tccagagaga 198420
   tccaacccct ctggcctctt caggcacttg cagtcatgtg tatacataag caagcacaca 198480
   cgtgtacaca cacacacaca cacacaatta acaatagatg attttaaaag gttaagtaaa 198540
   ataaaataaa aataattttt agaaggattc agtgtcttaa attttatttt ttgtatttga 198600
   attagagaca gccatattac aggcactttt acaagactgt gtggctactg tcccagcagt 198660
   gtatatgctg gctcagaggc agacagtaat gtgtgacgtt ggtggttttt aagagggttt 198720
   gggtcagtgt gcatggtgtc tgtggaaaca gatctggatg ctttccgggg agacagtgtt 198780
   aaatgacaga ggcttctagg aatcaggtta acaagggatg acccctgtga ccttgggtct 198840
   cctgtaacac actaaggata gctaagttaa gggcgctaag aagaaatttg agccaggtgt 198900
   agctgcttat gcacacacac acacacacac acacacacac tttctcatgg gcatgagcac 198960
   agctctttgg ccttcctcat ctcacctgta gtgactaaca agaaaactat aaccttggtg 199020
   tagtgatcgt ctctgccctt cctctgcctc accccacccc actcccttgg acacactctc 199080
   tgtccccgtg ccctgtgcct ggaggaacag ttatggaggg aggatttctg gagctgaggc 199140
   tgactcagaa cagctgcagt tatttggtcc cgaaagtcag agaaccacgg gggttgagga 199200
   tttgtctgtg ttaacactag agttccgcca cagtggggca gtcagccctg ttttcagttc 199260
   tggacagtta tccttaccat tcttattgtt tccccagtgg tgtctacctt aacctgtctg 199320
   ttgatttctc ttcgtctctc ctctgttgct gtttgatgag ttgttctgat acaggggttc 199380
   tctggccacc cttactcgtt ttgttttgtt ttgttttttt tcttactttg ttctgtaatc 199440
   tctaattcct tttctcttgg ggggttgggg agcaggggtc tccctgtgta gacctgcctg 199500
   cctctccctc cccagtgcta ggattaaaga cacatgccac acccagctta cagcttctct 199560
   ctctctctct ctctctctct ctctctctct ctctctctct cacccagcac tttcctcatt 199620
   ttttaaatct gtaagtttgg gcttgtcatt tctctcacaa actcacctta ggcaggaatc 199680
   accctcctgt gggtggccat gcaccctaac aaagtgaggg ggggactttc tataggttcc 199740
   tgatgcctta ggacttctta gtttaatgcc ttgtgtggta cgagcttcag attgcaatcc 199800
   tgcaaaataa ctttttcatt aaaggccttg tgctcatagc aagtttgtat tccacctctt 199860
   ggggcagcat ttttctaagc ccatttgccc aagtaagtta gctttcttca aggctctaag 199920
   ctgaatgccc gggctttaga gagaacccca cagttctgac tctgacgcag ggcctaacag 199980
   tctgtgtctt gaaagctcta tgggggatct gataatccat cagggttgaa aacactctta 200040
   gtggggctgt agtgatggct cagtgggtaa gtacacttgc tgcacagaca taaggacctg 200100
   agttcagatc ccagcactcc tgtaacaagc aggatatggt cccacacctt cccataatcc 200160
   cagcgaggag gaaaggggag actgaaagat tggtatctct aaggtttgct ggactgccac 200220
   ggcaaaaatc ataagcccca ggcttagcaa gagaccttgc tcaaagagat aaggcagatg 200280
   taatagaggg gcctacccca tgtcctcctt taatctgtga gtactattac aggcacatgc 200340
   atatatgttc acacacacac atttatatat atactagagt ttgtggttct aaactttaca 200400
   tcgtggtgaa cagaaaaacc tgctaggagt acaggtttat ttggctcacg gtttctggga 200460
   tttcagtcta tgaccaagtg gatccagcct gcaagaaggc agagcagtat ggttggaagc 200520
   ttgctttcca ggaagctgtt catctcacag tggccagatg atgatgaggc tgcagacaag 200580
   atgtactcat ctcaaggact ccttccccgt tttgtcccaa gtgcctatca gactaggacc 200640
   ctatcaaagg actagtctgt gaatgagttc cagagcctca tgacgcaatc acatccccag 200700
   tcaatcccca tccagctgac agccggtgct tcgtcagcac tgtgccttag ggagacactt 200760
   agacaagaag cagacttact gtgactgttt tccagtcact aaagcacaaa cactgaggac 200820
   ctgcaaccgc ctctctctcc ctcagatgcg gagaggccgt ggagaagaac aggcgactca 200880
   tcaccgcaga gcagcgggag taccagcagg agctgaagaa gaactacaac aagctgagag 200940
   acagcctcag gcccatgatt gagcggaaaa tcccagagct ctacaagccc atattcagag 201000
   ttgacagtca gaagaggtaa ggaggggcca gtgtagcctc ttcctgggca tcagagatca 201060
   tgggggcacc acaaatccag cttgctcggc tgctcatgca caaagcagac agcccagggt 201120
   ggactggggc tggagtccag cctccagcca gtctgtggtc tgcattcagt ccaaaccttt 201180
   ccctgaactc accaagcccc ttcttgttgg ccttgtgcca gcgaattccc aagtccatcc 201240
   agtgtagctg gtgtgtgtgt agatgtgtag ggggctggag gggcaggggt gaacaacagg 201300
   aatgggtctc aggtcttctt ttatttacta agttccccag gaccaatagc tttctggttc 201360
   ataattttct cccaaccgtc tctttaaaac agcagtctct ttaaaaggct aaccaatcct 201420
   tctgttctct ctctctctct ctctctcctt aatttcaggg actctttcca cagatctagt 201480
   ttcaggaaat gtgaaaccca gttgtcacag ggcagctgaa aaaagccacc tttgccggtg 201540
   acgactgggg ccccactacc tggaaggact cgctggtacg tccacagcag ccacccaaga 201600
   ctgactccac agtccctgcc cagccagtgc tctggaaact tggggggtct atgaccacag 201660
   aatgataccc aagtgtattg tggactatct tgaccatgtc aggggttggg gtggggtggg 201720
   taaatcaggg gataattgta tttattaaag tatttttcac agtgctgtag aaggaaggcc 201780
   taagctgttt ctgctgagtg gcttggctgg tcacaggcct cccagaaagt gacttcttct 201840
   gacctatgtt tgaattccta agataaactc aaattgtgga cagaagagtg gaacactgtc 201900
   cagcaaccta ttccgagtga atttcactca tccctttggt gagaagaaaa gataaaactc 201960
   cagaagagtg aaccattttc caatatatct gtgagaaaaa gtcttacagg aaatagatgg 202020
   agcgctccac acctctcctg agctgccgga ctatgtgccg tctagttatt gctgcattgt 202080
   tgcttctgtt aagctgagtg agtccacctc tacgcagggg acagttgtac gagacagaca 202140
   gtccaggtcg cccggccaca cagcactgac agcacatgcc ccagagcggg agcagttctc 202200
   ctaggacatc ttgccggttc cctgtacact tgctctcact gcctcctttt tctccgggat 202260
   aaggtcattt ttctacaaga cattcatttc agaacattgt gtcctctgtg tgctacaggg 202320
   atatatgctt tggattgttt ttacagtgaa cttggaagag ggaaagaaac ctatggtttg 202380
   agaattaaat tatatatttt catacaactc atgaccttac ctaatgctaa agacagaata 202440
   agaatgataa gcaagtgggg tgacaatgca cgcctttagt cccagcactc agaagacaga 202500
   gacaggtgga tctctgagtt taaggccagc ctggtctaca gagcgagttc caggacagcc 202560
   agattacaca atgagaccat gtctcataaa taaataaata aataaataaa caaacaaata 202620
   aataaataaa taaataaatt gcaaaatgct tccctgtgcc actgctgttt gtgactttga 202680
   caactcaaag tgctgtgaag cttcagagac cctggtagga ctggccttag tcaagcagtg 202740
   ccctccagag tggccctagt gcaatgtcct aatccttctg gttggaacag agttctcagt 202800
   tgtcagtact gaaatgttca cacataccat tgggcatgag tgaccttatt ctgaaggaag 202860
   atgttggaaa aaataaatgg cttgctggct gcacttattt aaac 202904